(19) Europäisches Patentamt European Patent Office Office européen des brevets

(11) **EP 4 550 337 A1**

(12) **EUROPEAN PATENT APPLICATION**

(43) Date of publication:
**07.05.2025 Bulletin 2025/19**

(21) Application number: **24209902.6**

(22) Date of filing: **30.10.2024**

(51) International Patent Classification (IPC):
**G16B 40/20** (2019.01)

(52) Cooperative Patent Classification (CPC):
**G16B 40/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 US 202363594835 P**

(71) Applicant: **Tempus AI, Inc.**
**Chicago, IL 60654 (US)**

(72) Inventors:
• **BEAUCHAMP, Kyle**
 **Chicago, IL 60654 (US)**

• **ERBE, Rossin**
 **Chicago, IL 60654 (US)**
• **JIN, Ailin**
 **Chicago, IL 60654 (US)**
• **STEIN, Michelle**
 **Chicago, IL 60654 (US)**
• **ZANDER, Alia**
 **Chicago, IL 60654 (US)**

(74) Representative: **Barker Brettell LLP**
**100 Hagley Road**
**Edgbaston**
**Birmingham B16 8QQ (GB)**

(54) **METHODS, SYSTEMS, AND COMPOSITIONS FOR PREDICTING RESPONSE TO IMMUNE ONCOLOGY THERAPIES**

(57) Disclosed herein are systems, methods, and compositions for identifying subjects likely to respond to an immune oncology therapy. The disclosed methods may include applying one or more model components to a machine learning algorithm. The one or more model components are derived from RNA and/or DNA sequencing from a subject and may include a checkpoint related gene signature, an immune exhaustion signature, a immune oncology signature, a tumor mutational burden, or a granulocytic myeloid derived suppressor cell signature.

FIG. 1A

**Description**

CROSS-REFERENCE TO RELATED APPLICATIONS

The present application claims priority to U.S. Provisional Patent Application No. 63/594,835 that was filed October 31, 2023, the entire contents of which are hereby incorporated by reference.

BACKGROUND

Checkpoint inhibitor use has now become standard of care in several indications, e.g., non-small cell lung cancer. Currently, there are only two biomarkers being used in the clinic to prescribe immuno-oncology (IO) therapies (including checkpoint inhibitors): PD-L1 protein level (often measured by expensive, time-consuming immunohistochemical staining methods) and tumor mutational burden (TMB). However, each of these biomarkers has disadvantages. For example, PD-L1 level is not always predictive of patient response to IO, and TMB is only currently approved for prescribing IO therapy to patients on the last line of therapy. Thus, there is an unmet need for diagnostics, biomarkers, and/or tools that complement these methods and aid in clinical decision making, for example, to inform physician management of IO therapy courses. In particular, there is an unmet need for methods to detect subjects with any type of cancer that are likely to respond to an IO therapy.

SUMMARY

Disclosed herein are systems, methods, and compositions for selecting subjects likely to respond to an immune oncology therapy.

In an aspect of the current disclosure, methods of selecting a subject for treatment with an immune oncology (IO) therapy, wherein the subject is in need of treatment for a cancer are provided. In some embodiments, the methods comprise: at a computer system having one or more processors, and memory storing one or more programs for execution by the one or more processors: applying, by the one or more processors, one or more model components derived from sequencing data from a sample of the cancer to one or more machine learning algorithms (MLAs), wherein the sequencing data comprises RNA sequencing data and DNA sequencing data, wherein the one or more model components comprise a tumor mutational burden (TMB), a checkpoint related gene signature, an immune exhaustion signature, a granulocytic myeloid derived suppressor cell (gMDSC) signature, and an immune oncology signature; displaying a report, the report comprising an indication that the subject is selected for an immune oncology therapy. In some embodiments, the subject has a cancer that is PD-L1 low, PD-L1 intermediate, or has a low tumor mutational burden. In some embodiments, the one or more machine learning algorithms (MLAs) are trained on training data from a cohort of subjects diagnosed with cancer. In some embodiments, the one or more MLAs comprise a variational autoencoder, an accelerated failure time model, a parametric survival model, a Cox proportional hazards model, a random forest model, a gradient-boosted survival model, a linear model, a recurrent neural network, a transformer neural network, or a convolutional neural network. In some embodiments, the checkpoint related gene signature comprises expression values for one or more genes selected from CD274, SPP1, CXCL9, CD74, CD276, IDO1, PDCD1LG2, and TNFRSF5. In some embodiments, the checkpoint related gene signature comprises expression values for CD274, SPP1, CXCL9, CD74, CD276, IDO1, PDCD1LG2, and TNFRSF5. In some embodiments, the immune exhaustion signature comprises expression values for the following genes TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, and SLC38A5. In some embodiments, the immune exhaustion signature comprises expression values for one or more genes selected from TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, SLC38A5, TIFA, DOK2, PPP1R2, DMAC1, DNAJB1, TAGAP, GZMA, CD27, GADD45A, HSPH1, STMN1, GZMH, CLIC3, GLIPR1, CHORDC1, CD3E, CD69, BAG3, ATF3, MICB, TRBC2, EZR, ARHGDIB, CASC8, ITM2A, DDX24, CD52, RAC2, TERF2IP, ELF1, FAM96B, GGH, NKG7, LY6E, CITED2, ZFAND2A, SAMSN1, CST7, CDKN3, TCEAL3, BBC3, IL32, MBD4, DNAJA4, TMEM141, UBB, HCST, IGLV1-40, HOPX, RHOH, USB1, H2AFZ, CSRP1, IKZF1, RGS2, IGLC2, CCND2, SELPLG, FUNDC2, IGFBP7, IGKV3-15, SERPINE2, TRDMT1, RGS1, HMOX1, HSP90AB1, HSPA1A, LIME1, TUBB, MRPL10, IFI44L, COTL1, LBH, ZEB2, HMGB2, LDHA, LGALS3, CYLD, PXMP2, CD74, PPIH, CD8A, RFX2, KLRD1, KLF6, LINC02446, HTRA1, TUBA4A, HSPB1, DNAJA1, CD3D, DUSP2, ELL2, TPM1, CKS1B, LGALS1, BEX3, GLRX, CCL4, GBPS, PTPRC, CLK1, IRF4, PIM2, SAT1, CXCR3, ZFP36, CD24, PELI1, CKS2, GYPC, FOXN2, IGLV1-51, IFT46, IGLV1-41, PLA2G16, COMMD8, IPCEF1, SMPDL3B, EVL, EVI2B, RAB11FIP1, DUSP5, HAVCR2, UBC, CRIP1, SRPRB, SERPINA1, PCSK7, BCL2L11, HSPA6, CWC25, CORO1A, TPST2, MBNL2, CKB, TUBA1B, GABARAPL1, PXDC1, SEL1L, PPP1R8, FKBP4, GABARAPL2, JCHAIN, STK17B, ZWINT, CHMP1B, ID2, HERPUD1, ROCK1, SKAP1, S100A4, CXCL10, CASP3, APOC1, ARID5B, SMAP2, CSRNP1, ADIRF, HLA-DPA1, PPP1R15A, DMKN, SCAF4, MYL9, LYAR, ZBTB25, GADD45B, GCHFR, LINC01588, RAB20, LSP1, FCGR2B, HIST2H2AA4, NCF4, LCK, IGHV3-33, LAPTM5, TUBB4B, TPM2, RBM38, RBP4, CCNA2, SERTAD1, ITM2C, PLPP5, DNAJB9, SYNGR2, TUBB2A, ERLEC1, TMED9, IFI6, HSP90AA1, PTPN1, TTL, DKK1, TM2D3, DCAF11, RIC1, SERPING1, DERL3, KDELR3, GEM, KLF9, TYROBP, CERCAM, CCDC84, ODC1, CYP2C9, CFLAR, HLA-DMB, DUSP1, JSRP1, TRIB1, JUN, NFATC2, EMP3, SNRNP70, TMED5, ST8SIA4, IGLV3-1, ZNF394, TNFSF9, CTSW, CUL1, BACH1, RABL3, KPNA2, EPS8L3, IER5, HSPA1B, CADM1, MCL1, RNF19A, ITGA4, CD38, WIPI1, CENPK, HCLS1, SPICE1, HIST1H2BC, MPRIP, FOSB, SERPINB8, FAM126A, CEP55, ATXN1, VCL, SOCS1, PCNX1, SQOR, JUNB, C10orf90, LCP1, STRADB, CREB3L2, GNG7, CCNH, SNX2, IGSF1, CCNL1, FKBP11, DBF4, ICAM1, MAD2L1, TMEM176B, PAIP2B, CD79A, SRXN1, NOB1, IER2, HLA-DRA, ZFP36L1, MZB1, MAGEA4, JUND, CD8B, AARS, TXNDC15, AC016831.7, GNA15, ATM, TSC22D1, GZMK, RAC3, ZNF263, TNFAIP3, H1FX, FGG, FHL2, MBNL1, TMEM205, IGLV6-57, CD96, TUBA1C, UCHL1, PRDM1, SRPK2, NUP37, TMEM87A, THEMIS2, HSPA5, PCMT1, TUBA1A, IGHG1, ANKRD37, MEF2C, XRN1, POU2AF1, BCL6, INAFM1, ADH4, TGFB1I1, PBK, DCN, FCRL5, DNAJB4, HLA-DQA1, TBC1D23, TMEM39A, GCC2, TMEM192, IGHA1, PTHLH, MFAP5, GEMIN6, BIRC3, IGHV4-4, SLC6A6, CYP2R1, HLA-DRB1, PPP1R15B, HMCES, MYC, WISP2, CHN1, ILK, PXN-AS1, LINC01970, CRIP2, PCOLCE2, MTMR6, EDIL3, AGR2, MEF2B, PFKM, KIAA1671, GLIPR2, SSTR2, SERPINB9, HIST1H1E, PTTG1, WSB1, ERN1, Z93241.1, IGLV1-44, SDS, TLE1, NUPR1, IGLV1-47, ICAM2, NXF1, RSPO3, TCF4, AC243960.1, RARRES2, RMDN3, RBFOX2, SEC11C, OLMALINC, FADS2, ITPRIP, FOS, SFTPD, HAUS3, RNF43, HIST1H4C, TIGAR, BIK, ITGA1, TARSL2, AFP, SNORC, MKLN1, BTG2, KRT18, NOC2L, ZFP36L2, NFKBIA, RHOB, HMGA1, BRD3, IGHJ6, U62317.5, SLC2A3, AC034231.1, CLEC11A, EPCAM, SKI, PNOC, MIR155HG, C12orf75, SAMHD1, IGKV3D-15, ACTN1, GSTZ1, TUBB3, CAV1, OAT, COBLL1, SSR4, ACTA2, HBA1, FAM83D, PLA2G2A, RAB14, AC106791.1, RAB23, AC244090.1, KMT5A, SERPINB1, P3H2, XRCC1, AC106782.1, MAL2, EGR1, F8, PLIN2, SOWAHC, IGFBP6, NFKBIZ, XBP1, SLC25A51, IGHM, KCTD5, USP38, FCER1G, PHLDA1, BYSL, HLA-DRB5, RAPH1, DUSP23, FUOM, ISYNA1, TNK2, STAP2, SLC25A4, GALNT2, SGO2, FHL3, ALB, CYP20A1, TM4SF1, ADA, RRP9, DNAH14, BOLA2, BHLHE41, CCL20, AC005537.1, UBALD2, VGLL4, NUDT1, USP10, ADSSL1, PRSS23, FMC1, ARHGAP45, HSPA14-1, CREB5, RBM33, TMX4, ROCK2, ARSK, PALLD, FNDC3B, FOXA3, BATF, PTP4A3, CDC45, IGHV1-2, IMMP2L, STARD10, HIST2H2BF, MTG2, FBXO8, USP32, ADIPOR2, RRM2, DHODH, DDIT4, NFAT5, PPARG, YTHDF3-AS1, GNG4, CSPP1, UBE2S, ZNF473, TIMP1, CPQ, AOC2, H1F0, JRK, EXOSC9, AC012236.1, AC009403.1, C12orf65, AURKA, MYH9, IGKV4-1, IGHMBP2, JADE1, HIST1H3C, TTC39A, SGMS1, LBP, FRYL, DNAJB2, GNG11, HAGHL, ANXA6, MARS, ADD1, KDM4B, TMEM91, AC008915.2, CXCL14, DUSP14, GJB2, PGM1, ETS2, GNPDA1, COL18A1, KLF10, MT1A, TPX2, S100A2, MAP3K5, HIST1H2AE, SLC20A2, ITGB7, SCEL, RSRP1, AKR1B1, GINS1, ZNF296, ALKBH4, UBE2C, ANKRD36C, SULT2B1, SMC5, TSPYL2, TNS4, TIMP3, ID4, SDC1, COX18, CDC42EP2, SQLE, ZNRF1, AKR1B10, NDC80, GFPT2, MAP1B, HIST1H2AG, IDO1, RNF185, UHRF1BP1, ADORA2B, CALD1, PHLDA2, ADH6, TFAP2A, DLG1, MELK, CBWD3, RAB4B, KANSL1L, RCE1, HIST1H2AC, CDK1, TCIM, C17orf67, BRD4, LY6E-DT, SLC1A6, ARL13B, IRF1, DDX3X, RAB2B, MYBBP1A, ARFGAP1, BOP1, IGKV3D-7, KMT2E-AS1, DTNBP1, LAMC2, ATG4C, MYBL2, LRP10, PALMD, ZBTB4, SYTL2, SERPINH1, CD248, CNEP1R1, FURIN, IGLL5, MEST, MDK, NUP205, NRDE2, ECT2, TENT5A, TNKS1BP1, NFXL1, SLC35E3, ECE1, RASD1, SLC52A2, DCBLD2, CP, POLE, COL27A1, SBNO1, SLC7A6, HYKK, SLPI, CFHR1, SPDEF, DACT2, TUBGCP5, AREG, HIST1H2AJ, KIF2A, AL135925.1, NOTCH3, SLC11A1, HEXIM2, IGFBP1, TVP23A, NUDT14, SAMD11, MIR200CHG, PCLAF, SLC43A3, FAM30A, PHRF1, ADM, SIK2, NUSAP1, CFH, KRTCAP3, SPAG4, TPPP3, TSPAN4, AAK1, CST1, CLU, IFRD1, ASPHD2, CNN3, COL4A1, FGA, ANO6, SBSN, FGB, ATP9B, NLGN4Y, HP, EPS8, RNF111, LINC01285, MAOA, IGHV4-31, TNFRSF10D, GSR, IGHGP, TACSTD2, MT1F, RHCG, MUT, PI3, MT1M, LAMB3, MTRNR2L12, SLC35A2, DDX10, RARRES1, MTSS1, CLK2, RPN2, MED29, CYP1B1, TTTY14, DMXL1, AL139246.5, TAF1, DAAM1, MYO1E, MAFB, CDKN1A, F8A3, FABP5, CFB, HSP90B1, SGK3, HMG20B, CDCA5, CLDN4, SYNM, PAWR, TWNK, AC116049.2, RND3, ATP11A, PID1, MALAT1, TMEM168, TFF1, TFRC, RNASET2, SPINK13, PABPC1L, P4HA2, PRSS8, SPINT1, MSC, FMNL1, SLC8B1, UNC13D, SPINT2, DCP1A, NPTN, IGKV3D-11, G6PD, KRT6A, LYPD1, TESC, COL4A2, ELF3, BCAM, AC093323.1, IGHV1-69, LINC00511, PORCN, TPRG1-AS1, EFNB2, PARD6G-AS1, CD9, RGS16, IL6R, FZD3, GLYR1, B3GALT6, LRCH3, MAFK, LINC00491, MT1X, MUC6, PIK3R3, GBP4, PERP, LXN, ZBTB7A, WARS, AC020911.2, MAPK3, ALS2CL, MRE11, TSPAN17, IGHV4-34, IL33, ADAM9, ANGPTL4, TBC1D31, C1R, CTSC, SLC35A4, FST, SGO1, ANKRD36, IGHG3, SLC15A3, HES1, POLR1E, SLC7A5, CAPN12, IGFBP3, FBXO38, FLNA, CSKMT, OAS1, ULK1, PBX1, EXOC4, REEP6, HILPDA, ASF1B, FKBP1B, IL6, CALU, AKR1C1, KLF2, GRTP1, C1S, SMOX, CPLX2, LMNA, BSG, IGHG4, SVIL, HIST1H1B, GCH1, NEAT1, FN1, ESRP1, RFWD3, ADGRE2, SPINK6, HPD, CAVIN1, MT1E, CLDN10, C15orf48, CA9, NR4A1, PPP1R3B, SLC30A1, SLC7A11, VIRMA, NAA25, CCNB1, CFD, AP1G1, H6PD, PSCA, KCNK6, AL161431.1, DVL1, HIST1H2AM, RAB31, CDCA3, SPATA20, PRMT7, PTGR1, SERINC2, IGHG2, GFPT1, TTC22, BTBD1, HIST1H4H, CENPB, ZNF598, GPATCH2L, SPTLC3, CXCL2, CYP24A1, EZH2, GPX2, LMNB2, PTGES, MGLL, NR2F2, KRT19, DNTTIP1, MUC5AC, SDCBP2, IL1R2, AHNAK2, MUC16, AC023090.1, CPE, VNN1, BAMBI, NPW, TK1, IGKV3D-20, ANKRD11, CDC20, CDH1, STK11, IGKC, SLC45A4, TBC1D8, CSTA, AC233755.1, MIGA1, HIST1H2AL, AKAP12, MAP4K4, HOOK2, GGA3, COL7A1, NOS1, ARHGAP26, AKR1C2, TGM2, CENPF, IGHV3-48, CDCA8, TSC2, STC2, PKN3, PVR, CES1, GPRC5A, SEZ6L2, CEP170B, KIF14, IER3, ALDH3B1, TOP2A, SPP1, TXNRD1, LENG8, TRIM15, ALDH3A1, RIMKLB, HECTD4, SMOC1, NEB, RMRP, IGFBP4, MT1G, SCRIB, ERO1A, SOX4, LMO7, RNPEPL1, PLK2, COL6A2, FLRT3, IGHV4-28, SCD, KRT7, PIEZO1, CXCL1, DAPK1, ID1, C3, CXCL3, IGKV3-20, GUCA2B, ITGA3, SFN, IGLV3-21, PLEC, POLR2A, AGRN, MUC1, SERPINB3, S100A8, LAMA5, COL6A1, ITGB4, S100P, SLURP2, MSLN, KRT17, and MUC5B. In some embodiments, the gMDSC signature comprises expression values for one or more genes selected from SERPINB 1, SOD2, S100A8, CTSC, CCL18, CXCL2, PLAUR, NCF2, FPR1, and IL8. In some embodiments, the gMDSC signature comprises expression values for one or more genes selected from SERPINB1, SOD2, S100A8, CTSC, CCL18, CXCL2, PLAUR, NCF2, FPR1, IL8, S100A9, TNFAIP3, CXCL1, BCL2A1, EMR2, LILRB3, SLC11A1, IL6, TREM1, CCL20, LYN, CXCL3, IL1B, IL1R2, AQP9, IL2RA, GPR97, OSM, CXCR1, FPR2, C19orf59, CXCR2, CXCL6, CXCL5, EMR3, MEFV, S100A12, CD300E, FCGR3B, PPBP, LILRA5, LILRA3, and CASP5. In some embodiments, the immune oncology (IO) signature comprises expression values for one or more genes selected from GBP5, IL10RA, NLRC5, CXCL9, RAC2, GBP4, GLUL, IRF1, CD53, CIITA, S100B, GBP2, ITK, SLAMF7, IKZF3, DOCK2, SELL, ARHGAP9, CYTIP, IL2RB, NCKAP1L, APOD, CD96, IL7R, and ZAP70. In some embodiments, the immune oncology (IO) signature comprises expression values for one or more genes selected from ISG20, PCDHGA2, TGFB1I1, ATP8B1, IL7R, IRF8, ETV1, MYLK, GRHL2, THBS4, CYP3A5, FBLIM1, S100B, BICD1, SLAMF7, RAB27A, GATM, ICA1, ITPR1, SLC7A2, ZAP70, LOXL4, CILP, ARHGAP30, ITGB2, KLF5, PRKCA, PCDH7, DPYSL3, RGS2, SPP1, COLGALT2, MPZL2, TNFAIP8, PLAT, ALDH1A3, POF1B, PPP1R9A, SEMA3A, CIITA, DLC1, ARHGAP9, FRAS1, AKAP6, ATP1A2, TTN, LTBP1, NCKAP1L, MAP3K6, MYO1B, MRVI1, FSCN1, GPC1, GBP5, BAMBI, IL2RB, MYO1G, RANBP17, APOD, RASGRP1, CYTIP, ITGA7, CYTH4, PTPRF, KIAA1755, IRF1, GPR37, RAC2, NLRC5, EGFR, ITK, IL10RA, IGFBP2, CD96, RASD1, CD36, TMEM163, IGLL5, IKZF3, PRLR, CDC42BPG, DOCK2, PAM, VEGFA, CD84, SORL1, GBP2, SYTL4, APBB1IP, SIGLEC10, GBP4, COMP, DOCK8, CXCL9, NRP1, EPHB4, CD53, GLUL, DNM1, DSP, SIX4, SELL, DSC3, TNFAIP2, and JAG2. In some embodiments, the TMB is derived from the DNA sequencing data. In some embodiments, the expression values of the checkpoint related gene signature, the immune exhaustion signature, and the granulocytic myeloid derived suppressor cell (gMDSC) signature are derived from the RNA seq data. In some embodiments, the IO therapy is an immune checkpoint inhibitor therapy (ICI). In some embodiments, the ICI comprises pembrolizumab or nivolumab. In some embodiments, the report further comprises an immune profile score (IPS). In some embodiments, the IPS is displayed as an integer from 1-100. In some embodiments, the IPS is further divided into categories or is a categorical result. In some embodiments, the categories are IPS-Low, indeterminate, and IPS-High.

In an aspect of the current disclosure, systems for selecting a subject for treatment with an immune oncology (IO) therapy, wherein the subject is in need of treatment for a cancer are provided. In some embodiments, the systems comprise: a computer system having one or more processors, and memory storing one or more programs for execution by the one or more processors the one or more processors configured to: apply, by the one or more processors, one or more model components derived from sequencing data from a sample of the cancer to one or more machine learning algorithms (MLAs), wherein the sequencing data comprises RNA sequencing data and DNA sequencing data, wherein the one or more model components comprise a tumor mutational burden (TMB), a checkpoint related gene signature, an immune exhaustion signature, a granulocytic myeloid derived suppressor cell (gMDSC) signature, and an immune oncology signature; display a report, the report comprising an indication that the subject is selected for an immune oncology therapy. In some embodiments, the subject has a cancer that is PD-L1 low, PD-L1 intermediate, or has a low tumor mutational burden. In some embodiments, the one or more machine learning algorithms (MLAs) are trained on training data from a cohort of subjects diagnosed with cancer. In some embodiments, the one or more MLAs comprise a variational autoencoder, an accelerated failure time model, a parametric survival model, a Cox proportional hazards model, a random forest model, a gradient-boosted survival model, a linear model, a recurrent neural network, a transformer neural network, or a convolutional neural network. In some embodiments, the checkpoint related gene signature comprises expression values for one or more genes selected from CD274, SPP1, CXCL9, CD74, CD276, IDO1, PDCD1LG2, and TNFRSF5. In some embodiments, the checkpoint related gene signature comprises expression values for CD274, SPP1, CXCL9, CD74, CD276, IDO1, PDCD1LG2, and TNFRSF5. In some embodiments, the immune exhaustion signature comprises expression values for the following genes TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, and SLC38A5. In some embodiments, the immune exhaustion signature comprises expression values for one or more genes selected from TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, SLC38A5, TIFA, DOK2, PPP1R2, DMAC1, DNAJB1, TAGAP, GZMA, CD27, GADD45A, HSPH1, STMN1, GZMH, CLIC3, GLIPR1, CHORDC1, CD3E, CD69, BAG3, ATF3, MICB, TRBC2, EZR, ARHGDIB, CASC8, ITM2A, DDX24, CD52, RAC2, TERF2IP, ELF1, FAM96B, GGH, NKG7, LY6E, CITED2, ZFAND2A, SAMSN1, CST7, CDKN3, TCEAL3, BBC3, IL32, MBD4, DNAJA4, TMEM141, UBB, HCST, IGLV1-40, HOPX, RHOH, USB1, H2AFZ, CSRP1, IKZF1, RGS2, IGLC2, CCND2, SELPLG, FUNDC2, IGFBP7, IGKV3-15, SERPINE2, TRDMT1, RGS1, HMOX1, HSP90AB1, HSPA1A, LIME1, TUBB, MRPL10, IFI44L, COTL1, LBH, ZEB2, HMGB2, LDHA, LGALS3, CYLD, PXMP2, CD74, PPIH, CD8A, RFX2, KLRD1, KLF6, LINC02446, HTRA1, TUBA4A, HSPB1, DNAJA1, CD3D, DUSP2, ELL2, TPM1, CKS1B, LGALS1, BEX3, GLRX, CCL4, GBP5, PTPRC, CLK1, IRF4, PIM2, SAT1, CXCR3, ZFP36, CD24, PELI1, CKS2, GYPC, FOXN2, IGLV1-51, IFT46, IGLV1-41, PLA2G16, COMMD8, IPCEF1, SMPDL3B, EVL, EVI2B, RAB11-FIP1, DUSP5, HAVCR2, UBC, CRIP1, SRPRB, SERPINA1, PCSK7, BCL2L11, HSPA6, CWC25, CORO1A, TPST2, MBNL2, CKB, TUBA1B, GABARAPL1, PXDC1, SEL1L, PPP1R8, FKBP4, GABARAPL2, JCHAIN, STK17B, ZWINT, CHMP1B, ID2, HERPUD1, ROCK1, SKAP1, S100A4, CXCL10, CASP3, APOC1, ARID5B, SMAP2, CSRNP1, ADIRF, HLA-DPA1, PPP1R15A, DMKN, SCAF4, MYL9, LYAR, ZBTB25, GADD45B, GCHFR, LINC01588, RAB20, LSP1, FCGR2B, HIST2H2AA4, NCF4, LCK, IGHV3-33, LAPTM5, TUBB4B, TPM2, RBM38, RBP4, CCNA2, SERTAD1, ITM2C, PLPP5, DNAJB9, SYNGR2, TUBB2A, ERLEC1, TMED9, IFI6, HSP90AA1, PTPN1, TTL, DKK1, TM2D3, DCAF11, RIC1, SERPING1, DERL3, KDELR3, GEM, KLF9, TYROBP, CERCAM, CCDC84, ODC1, CYP2C9, CFLAR, HLA-DMB, DUSP1, JSRP1, TRIB1, JUN, NFATC2, EMP3, SNRNP70, TMED5, ST8SIA4, IGLV3-1, ZNF394, TNFSF9, CTSW, CUL1, BACH1, RABL3, KPNA2, EPS8L3, IER5, HSPA1B, CADM1, MCL1, RNF19A, ITGA4, CD38, WIPI1, CENPK, HCLS1, SPICE1, HIST1H2BC, MPRIP, FOSB, SERPINB8, FAM126A, CEP55, ATXN1, VCL, SOCS1, PCNX1, SQOR, JUNB, C10orf90, LCP1, STRADB, CREB3L2, GNG7, CCNH, SNX2, IGSF1, CCNL1, FKBP11, DBF4, ICAM1, MAD2L1, TMEM176B, PAIP2B, CD79A, SRXN1, NOB1, IER2, HLA-DRA, ZFP36L1, MZB1, MAGEA4, JUND, CD8B, AARS, TXNDC15, AC016831.7, GNA15, ATM, TSC22D1, GZMK, RAC3, ZNF263, TNFAIP3, H1FX, FGG, FHL2, MBNL1, TMEM205, IGLV6-57, CD96, TUBA1C, UCHL1, PRDM1, SRPK2, NUP37, TMEM87A, THEMIS2, HSPA5, PCMT1, TUBA1A, IGHG1, ANKRD37, MEF2C, XRN1, POU2AF1, BCL6, INAFM1, ADH4, TGFB1I1, PBK, DCN, FCRL5, DNAJB4, HLA-DQA1, TBC1D23, TMEM39A, GCC2, TMEM192, IGHA1, PTHLH, MFAP5, GEMIN6, BIRC3, IGHV4-4, SLC6A6, CYP2R1, HLA-DRB1, PPP1R15B, HMCES, MYC, WISP2, CHN1, ILK, PXN-AS1, LINC01970, CRIP2, PCOLCE2, MTMR6, EDIL3, AGR2, MEF2B, PFKM, KIAA1671, GLIPR2, SSTR2, SERPINB9, HIST1H1E, PTTG1, WSB1, ERN1, Z93241.1, IGLV1-44, SDS, TLE1, NUPR1, IGLV1-47, ICAM2, NXF1, RSPO3, TCF4, AC243960.1, RARRES2, RMDN3, RBFOX2, SEC11C, OLMALINC, FADS2, ITPRIP, FOS, SFTPD, HAUS3, RNF43, HIST1H4C, TIGAR, BIK, ITGA1, TARSL2, AFP, SNORC, MKLN1, BTG2, KRT18, NOC2L, ZFP36L2, NFKBIA, RHOB, HMGA1, BRD3, IGHJ6, U62317.5, SLC2A3, AC034231.1, CLEC11A, EPCAM, SKI, PNOC, MIR155HG, C12orf75, SAMHD1, IGKV3D-15, ACTN1, GSTZ1, TUBB3, CAV1, OAT, COBLL1, SSR4, ACTA2, HBA1, FAM83D, PLA2G2A, RAB14, AC106791.1, RAB23, AC244090.1, KMT5A, SERPINB1, P3H2, XRCC1, AC106782.1, MAL2, EGR1, F8, PLIN2, SOWAHC, IGFBP6, NFKBIZ, XBP1, SLC25A51, IGHM, KCTD5, USP38, FCER1G, PHLDA1, BYSL, HLA-DRB5, RAPH1, DUSP23, FUOM, ISYNA1, TNK2, STAP2, SLC25A4, GALNT2, SGO2, FHL3, ALB, CYP20A1, TM4SF1, ADA, RRP9, DNAH14, BOLA2, BHLHE41, CCL20, AC005537.1, UBALD2, VGLL4, NUDT1, USP10, ADSSL1, PRSS23, FMC1, ARHGAP45, HSPA14-1, CREB5, RBM33, TMX4, ROCK2, ARSK, PALLD, FNDC3B, FOXA3, BATF, PTP4A3, CDC45, IGHV1-2, IMMP2L, STARD10, HIST2H2BF, MTG2, FBXO8, USP32, ADIPOR2, RRM2, DHODH, DDIT4, NFAT5, PPARG, YTHDF3-AS1, GNG4, CSPP1, UBE2S, ZNF473, TIMP1, CPQ, AOC2, H1F0, JRK, EXOSC9, AC012236.1, AC009403.1, C12orf65, AURKA, MYH9, IGKV4-1, IGHMBP2, JADE1, HIST1H3C, TTC39A, SGMS1, LBP, FRYL, DNAJB2, GNG11, HAGHL, ANXA6, MARS, ADD1, KDM4B, TMEM91, AC008915.2, CXCL14, DUSP14, GJB2, PGM1, ETS2, GNPDA1, COL18A1, KLF10, MT1A, TPX2, S100A2, MAP3K5, HIST1H2AE, SLC20A2, ITGB7, SCEL, RSRP1, AKR1B1, GINS1, ZNF296, ALKBH4, UBE2C, ANKRD36C, SULT2B1, SMC5, TSPYL2, TNS4, TIMP3, ID4, SDC1, COX18, CDC42EP2, SQLE, ZNRF1, AKR1B10, NDC80, GFPT2, MAP1B, HIST1H2AG, IDO1, RNF185, UHRF1BP1, ADORA2B, CALD1, PHLDA2, ADH6, TFAP2A, DLG1, MELK, CBWD3, RAB4B, KANSL1L, RCE1, HIST1H2AC, CDK1, TCIM, C17orf67, BRD4, LY6E-DT, SLC1A6, ARL13B, IRF1, DDX3X, RAB2B, MYBBP1A, ARFGAP1, BOP1, IGKV3D-7, KMT2E-AS1, DTNBP1, LAMC2, ATG4C, MYBL2, LRP10, PALMD, ZBTB4, SYTL2, SERPINH1, CD248, CNEP1R1, FURIN, IGLL5, MEST, MDK, NUP205, NRDE2, ECT2, TENT5A, TNKS1BP1, NFXL1, SLC35E3, ECE1, RASD1, SLC52A2, DCBLD2, CP, POLE, COL27A1, SBNO1, SLC7A6, HYKK, SLPI, CFHR1, SPDEF, DACT2, TUBGCP5, AREG, HIST1H2AJ, KIF2A, AL135925.1, NOTCH3, SLC11A1, HEXIM2, IGFBP1, TVP23A, NUDT14, SAMD11, MIR200CHG, PCLAF, SLC43A3, FAM30A, PHRF1, ADM, SIK2, NUSAP1, CFH, KRTCAP3, SPAG4, TPPP3, TSPAN4, AAK1, CST1, CLU, IFRD1, ASPHD2, CNN3, COL4A1, FGA, ANO6, SBSN, FGB, ATP9B, NLGN4Y, HP, EPS8, RNF111, LINC01285, MAOA, IGHV4-31, TNFRSF10D, GSR, IGHGP, TACSTD2, MT1F, RHCG, MUT, PI3, MT1M, LAMB3, MTRNR2L12, SLC35A2, DDX10, RARRES1, MTSS1, CLK2, RPN2, MED29, CYP1B1, TTTY14, DMXL1, AL139246.5, TAF1, DAAM1, MYO1E, MAFB, CDKN1A, F8A3, FABP5, CFB, HSP90B1, SGK3, HMG20B, CDCA5, CLDN4, SYNM, PAWR, TWNK, AC116049.2, RND3, ATP11A, PID1, MALAT1, TMEM168, TFF1, TFRC, RNASET2, SPINK13, PABPC1L, P4HA2, PRSS8, SPINT1, MSC, FMNL1, SLC8B1, UNC13D, SPINT2, DCP1A, NPTN, IGKV3D-11, G6PD, KRT6A, LYPD1, TESC, COL4A2, ELF3, BCAM, AC093323.1, IGHV1-69, LINC00511, PORCN, TPRG1-AS1, EFNB2, PARD6G-AS1, CD9, RGS16, IL6R, FZD3, GLYR1, B3GALT6, LRCH3, MAFK, LINC00491, MT1X, MUC6, PIK3R3, GBP4, PERP, LXN, ZBTB7A, WARS, AC020911.2, MAPK3, ALS2CL, MRE11, TSPAN17, IGHV4-34, IL33, ADAM9, ANGPTL4, TBC1D31, C1R, CTSC, SLC35A4, FST, SGO1, ANKRD36, IGHG3, SLC15A3, HES1, POLR1E, SLC7A5, CAPN12, IGFBP3, FBXO38, FLNA, CSKMT, OAS1, ULK1, PBX1, EXOC4, REEP6, HILPDA, ASF1B, FKBP1B, IL6, CALU, AKR1C1, KLF2, GRTP1, C1S, SMOX, CPLX2, LMNA, BSG, IGHG4, SVIL, HIST1H1B, GCH1, NEAT1, FN1, ESRP1, RFWD3, ADGRE2, SPINK6, HPD, CAVIN1, MT1E, CLDN10, C15orf48, CA9, NR4A1, PPP1R3B, SLC30A1, SLC7A11, VIRMA, NAA25, CCNB1, CFD, AP1G1, H6PD, PSCA, KCNK6, AL161431.1, DVL1, HIST1H2AM, RAB31, CDCA3, SPATA20, PRMT7, PTGR1, SERINC2, IGHG2, GFPT1, TTC22, BTBD1, HIST1H4H, CENPB, ZNF598, GPATCH2L, SPTLC3, CXCL2, CYP24A1, EZH2, GPX2, LMNB2, PTGES, MGLL, NR2F2, KRT19, DNTTIP1, MUC5AC, SDCBP2, IL1R2, AHNAK2, MUC16, AC023090.1, CPE, VNN1, BAMBI, NPW, TK1, IGKV3D-20, ANKRD11, CDC20, CDH1, STK11, IGKC, SLC45A4, TBC1D8, CSTA, AC233755.1, MIGA1, HIST1H2AL, AKAP12, MAP4K4, HOOK2, GGA3, COL7A1, NOS1, ARHGAP26, AKR1C2, TGM2, CENPF, IGHV3-48, CDCA8, TSC2, STC2, PKN3, PVR, CES1, GPRC5A, SEZ6L2, CEP170B, KIF14, IER3, ALDH3B1, TOP2A, SPP1, TXNRD1, LENG8, TRIM15, ALDH3A1, RIMKLB, HECTD4, SMOC1, NEB, RMRP, IGFBP4, MT1G, SCRIB, ERO1A, SOX4, LMO7, RNPEPL1, PLK2, COL6A2, FLRT3, IGHV4-28, SCD, KRT7, PIEZO1, CXCL1, DAPK1, ID1, C3, CXCL3, IGKV3-20, GUCA2B, ITGA3, SFN, IGLV3-21, PLEC, POLR2A, AGRN, MUC1, SERPINB3, S100A8, LAMA5, COL6A1, ITGB4, S100P, SLURP2, MSLN, KRT17, and MUC5B. In some embodiments, the gMDSC signature comprises expression values for one or more genes selected from SERPINB 1, SOD2, S100A8, CTSC, CCL18, CXCL2, PLAUR, NCF2, FPR1, and IL8. In some embodiments, the gMDSC signature comprises expression values for one or more genes selected from SERPINB1, SOD2, S100A8, CTSC, CCL18, CXCL2, PLAUR, NCF2, FPR1, IL8, S100A9, TNFAIP3, CXCL1, BCL2A1, EMR2, LILRB3, SLC11A1, IL6, TREM1, CCL20, LYN, CXCL3, IL1B, IL1R2, AQP9, IL2RA, GPR97, OSM, CXCR1, FPR2, C19orf59, CXCR2, CXCL6, CXCL5, EMR3, MEFV, S100A12, CD300E, FCGR3B, PPBP, LILRA5, LILRA3, and CASP5. In some embodiments, the immune oncology (IO) signature comprises expression values for one or more genes selected from GBP5, IL10RA, NLRC5, CXCL9, RAC2, GBP4, GLUL, IRF1, CD53, CIITA, S100B, GBP2, ITK, SLAMF7, IKZF3, DOCK2, SELL, ARHGAP9, CYTIP, IL2RB, NCKAP1L, APOD, CD96, IL7R, and ZAP70. In some embodiments, the immune oncology (IO) signature comprises expression values for one or more genes selected from ISG20, PCDHGA2, TGFB1I1, ATP8B1, IL7R, IRF8, ETV1, MYLK, GRHL2, THBS4, CYP3A5, FBLIM1, S100B, BICD1, SLAMF7, RAB27A, GATM, ICA1, ITPR1, SLC7A2, ZAP70, LOXL4, CILP, ARHGAP30, ITGB2, KLF5, PRKCA, PCDH7, DPYSL3, RGS2, SPP1, COLGALT2, MPZL2, TNFAIP8, PLAT, ALDH1A3, POF1B, PPP1R9A, SEMA3A, CIITA, DLC1, ARHGAP9, FRAS1, AKAP6, ATP1A2, TTN, LTBP1, NCKAP1L, MAP3K6, MYO1B, MRVI1, FSCN1, GPC1, GBP5, BAMBI, IL2RB, MYO1G, RANBP17, APOD, RASGRP1, CYTIP, ITGA7, CYTH4, PTPRF, KIAA1755, IRF1, GPR37, RAC2, NLRC5, EGFR, ITK, IL10RA, IGFBP2, CD96, RASD1, CD36, TMEM163, IGLL5, IKZF3, PRLR, CDC42BPG, DOCK2, PAM, VEGFA, CD84, SORL1, GBP2, SYTL4, APBB1IP, SIGLEC10, GBP4, COMP, DOCK8, CXCL9, NRP1, EPHB4, CD53, GLUL, DNM1, DSP, SIX4, SELL, DSC3, TNFAIP2, and JAG2. In some embodiments, the TMB is derived from the DNA sequencing data. In some embodiments, the expression values of the checkpoint related gene signature, the immune exhaustion signature, and the granulocytic myeloid derived suppressor cell (gMDSC) signature are derived from the RNA seq data. In some embodiments, the IO therapy is an immune checkpoint inhibitor therapy (ICI). In some embodiments, the ICI comprises pembrolizumab or nivolumab. In some embodiments, the report further comprises an immune profile score (IPS). In some embodiments, the IPS is displayed as an integer from 1-100. In some embodiments, the IPS is further divided into categories or is a categorical result. In some embodiments, the categories are IPS-Low, indeterminate, and IPS-High.

In an aspect of the current disclosure, non-transitory computer readable media for selecting a subject for treatment with an immune oncology (IO) therapy, wherein the subject is in need of treatment for a cancer are provided. In some embodiments, the non-transitory computer readable media have stored thereon program code instructions that, when executed by a processor, cause the processor to apply, by the one or more processors, one or more model components derived from sequencing data from a sample of the cancer to one or more machine learning algorithms (MLAs), wherein the sequencing data comprises RNA sequencing data and DNA sequencing data, wherein the one or more model components comprise a tumor mutational burden (TMB), a checkpoint related gene signature, an immune exhaustion signature, a granulocytic myeloid derived suppressor cell (gMDSC) signature, and an immune oncology signature; display a report, the report comprising an indication that the subject is selected for an immune oncology therapy. In some embodiments, the subject has a cancer that is PD-L1 low, PD-L1 intermediate, or has a low tumor mutational burden. In some embodiments, the one or more machine learning algorithms (MLAs) are trained on training data from a cohort of subjects diagnosed with cancer. In some embodiments, the one or more MLAs comprise a variational autoencoder, an accelerated failure time model, a parametric survival model, a Cox proportional hazards model, a random forest model, a gradient-boosted survival model, a linear model, a recurrent neural network, a transformer neural network, or a convolutional neural network. In some embodiments, the checkpoint related gene signature comprises expression values for one or more genes selected from CD274, SPP1, CXCL9, CD74, CD276, IDO1, PDCD1LG2, and TNFRSF5. In some embodiments, the checkpoint related gene signature comprises expression values for CD274, SPP1, CXCL9, CD74, CD276, IDO1, PDCD1LG2, and TNFRSF5. In some embodiments, the immune exhaustion signature comprises expression values for the following genes TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, and SLC38A5. In some embodiments, the immune exhaustion signature comprises expression values for one or more genes selected from TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, SLC38A5, TIFA, DOK2, PPP1R2, DMAC1, DNAJB1, TAGAP, GZMA, CD27, GADD45A, HSPH1, STMN1, GZMH, CLIC3, GLIPR1, CHORDC1, CD3E, CD69, BAG3, ATF3, MICB, TRBC2, EZR, ARHGDIB, CASC8, ITM2A, DDX24, CD52, RAC2, TERF2IP, ELF1, FAM96B, GGH, NKG7, LY6E, CITED2, ZFAND2A, SAMSN1, CST7, CDKN3, TCEAL3, BBC3, IL32, MBD4, DNAJA4, TMEM141, UBB, HCST, IGLV1-40, HOPX, RHOH, USB1, H2AFZ, CSRP1, IKZF1, RGS2, IGLC2, CCND2, SELPLG, FUNDC2, IGFBP7, IGKV3-15, SERPINE2, TRDMT1, RGS1, HMOX1, HSP90AB1, HSPA1A, LIME1, TUBB, MRPL10, IFI44L, COTL1, LBH, ZEB2, HMGB2, LDHA, LGALS3, CYLD, PXMP2, CD74, PPIH, CD8A, RFX2, KLRD1, KLF6, LINC02446, HTRA1, TUBA4A, HSPB1, DNAJA1, CD3D, DUSP2, ELL2, TPM1, CKS1B, LGALS1, BEX3, GLRX, CCL4, GBP5, PTPRC, CLK1, IRF4, PIM2, SAT1, CXCR3, ZFP36, CD24, PELI1, CKS2, GYPC, FOXN2, IGLV1-51, IFT46, IGLV1-41, PLA2G16, COMMD8, IPCEF1, SMPDL3B, EVL, EVI2B, RAB11-FIP1, DUSP5, HAVCR2, UBC, CRIP1, SRPRB, SERPINA1, PCSK7, BCL2L11, HSPA6, CWC25, CORO1A, TPST2, MBNL2, CKB, TUBA1B, GABARAPL1, PXDC1, SEL1L, PPP1R8, FKBP4, GABARAPL2, JCHAIN, STK17B, ZWINT, CHMP1B, ID2, HERPUD1, ROCK1, SKAP1, S100A4, CXCL10, CASP3, APOC1, ARID5B, SMAP2, CSRNP1, ADIRF, HLA-DPA1, PPP1R15A, DMKN, SCAF4, MYL9, LYAR, ZBTB25, GADD45B, GCHFR, LINC01588, RAB20, LSP1, FCGR2B, HIST2H2AA4, NCF4, LCK, IGHV3-33, LAPTM5, TUBB4B, TPM2, RBM38, RBP4, CCNA2, SERTAD1, ITM2C, PLPP5, DNAJB9, SYNGR2, TUBB2A, ERLEC1, TMED9, IFI6, HSP90AA1, PTPN1, TTL, DKK1, TM2D3, DCAF11, RIC1, SERPING1, DERL3, KDELR3, GEM, KLF9, TYROBP, CERCAM, CCDC84, ODC1, CYP2C9, CFLAR, HLA-DMB, DUSP1, JSRP1, TRIB1, JUN, NFATC2, EMP3, SNRNP70, TMED5, ST8SIA4, IGLV3-1, ZNF394, TNFSF9, CTSW, CUL1, BACH1, RABL3, KPNA2, EPS8L3, IER5, HSPA1B, CADM1, MCL1, RNF19A, ITGA4, CD38, WIPI1, CENPK, HCLS1, SPICE1, HIST1H2BC, MPRIP, FOSB, SERPINB8, FAM126A, CEP55, ATXN1, VCL, SOCS1, PCNX1, SQOR, JUNB, C10orf90, LCP1, STRADB, CREB3L2, GNG7, CCNH, SNX2, IGSF1, CCNL1, FKBP11, DBF4, ICAM1, MAD2L1, TMEM176B, PAIP2B, CD79A, SRXN1, NOB1, IER2, HLA-DRA, ZFP36L1, MZB1, MAGEA4, JUND, CD8B, AARS, TXNDC15, AC016831.7, GNA15, ATM, TSC22D1, GZMK, RAC3, ZNF263, TNFAIP3, H1FX, FGG, FHL2, MBNL1, TMEM205, IGLV6-57, CD96, TUBA1C, UCHL1, PRDM1, SRPK2, NUP37, TMEM87A, THEMIS2, HSPA5, PCMT1, TUBA1A, IGHG1, ANKRD37, MEF2C, XRN1, POU2AF1, BCL6, INAFM1, ADH4, TGFB1I1, PBK, DCN, FCRL5, DNAJB4, HLA-DQA1, TBC1D23, TMEM39A, GCC2, TMEM192, IGHA1, PTHLH, MFAP5, GEMIN6, BIRC3, IGHV4-4, SLC6A6, CYP2R1, HLA-DRB1, PPP1R15B, HMCES, MYC, WISP2, CHN1, ILK, PXN-AS1, LINC01970, CRIP2, PCOLCE2, MTMR6, EDIL3, AGR2, MEF2B, PFKM, KIAA1671, GLIPR2, SSTR2, SERPINB9, HIST1H1E, PTTG1, WSB1, ERN1, Z93241.1, IGLV1-44, SDS, TLE1, NUPR1, IGLV1-47, ICAM2, NXF1, RSPO3, TCF4, AC243960.1, RARRES2, RMDN3, RBFOX2, SEC11C, OLMALINC, FADS2, ITPRIP, FOS, SFTPD, HAUS3, RNF43, HIST1H4C, TIGAR, BIK, ITGA1, TARSL2, AFP, SNORC, MKLN1, BTG2, KRT18, NOC2L, ZFP36L2, NFKBIA, RHOB, HMGA1, BRD3, IGHJ6, U62317.5, SLC2A3, AC034231.1, CLEC11A, EPCAM, SKI, PNOC, MIR155HG, C12orf75, SAMHD1, IGKV3D-15, ACTN1, GSTZ1, TUBB3, CAV1, OAT, COBLL1, SSR4, ACTA2, HBA1, FAM83D, PLA2G2A, RAB14, AC106791.1, RAB23, AC244090.1, KMT5A, SERPINB1, P3H2, XRCC1, AC106782.1, MAL2, EGR1, F8, PLIN2, SOWAHC, IGFBP6, NFKBIZ, XBP1, SLC25A51, IGHM, KCTD5, USP38, FCER1G, PHLDA1, BYSL, HLA-DRB5, RAPH1, DUSP23, FUOM, ISYNA1, TNK2, STAP2, SLC25A4, GALNT2, SGO2, FHL3, ALB, CYP20A1, TM4SF1, ADA, RRP9, DNAH14, BOLA2, BHLHE41, CCL20, AC005537.1, UBALD2, VGLL4, NUDT1, USP10, ADSSL1, PRSS23, FMC1, ARHGAP45, HSPA14-1, CREB5, RBM33, TMX4, ROCK2, ARSK, PALLD, FNDC3B, FOXA3, BATF, PTP4A3, CDC45, IGHV1-2, IMMP2L, STARD10, HIST2H2BF, MTG2, FBXO8, USP32, ADIPOR2, RRM2, DHODH, DDIT4, NFAT5, PPARG, YTHDF3-AS1, GNG4, CSPP1, UBE2S, ZNF473, TIMP1, CPQ, AOC2, H1F0, JRK, EXOSC9, AC012236.1, AC009403.1, C12orf65, AURKA, MYH9, IGKV4-1, IGHMBP2, JADE1, HIST1H3C, TTC39A, SGMS1, LBP, FRYL, DNAJB2, GNG11, HAGHL, ANXA6, MARS, ADD1, KDM4B, TMEM91, AC008915.2, CXCL14, DUSP14, GJB2, PGM1, ETS2, GNPDA1, COL18A1, KLF10, MT1A, TPX2, S100A2, MAP3K5, HIST1H2AE, SLC20A2, ITGB7, SCEL, RSRP1, AKR1B1, GINS1, ZNF296, ALKBH4, UBE2C, ANKRD36C, SULT2B1, SMC5, TSPYL2, TNS4, TIMP3, ID4, SDC1, COX18, CDC42EP2, SQLE, ZNRF1, AKR1B10, NDC80, GFPT2, MAP1B, HIST1H2AG, IDO1, RNF185, UHRF1BP1, ADORA2B, CALD1, PHLDA2, ADH6, TFAP2A, DLG1, MELK, CBWD3, RAB4B, KANSL1L, RCE1, HIST1H2AC, CDK1, TCIM, C17orf67, BRD4, LY6E-DT, SLC1A6, ARL13B, IRF1, DDX3X, RAB2B, MYBBP1A, ARFGAP1, BOP1, IGKV3D-7, KMT2E-AS1, DTNBP1, LAMC2, ATG4C, MYBL2, LRP10, PALMD, ZBTB4, SYTL2, SERPINH1, CD248, CNEP1R1, FURIN, IGLL5, MEST, MDK, NUP205, NRDE2, ECT2, TENT5A, TNKS1BP1, NFXL1, SLC35E3, ECE1, RASD1, SLC52A2, DCBLD2, CP, POLE, COL27A1, SBNO1, SLC7A6, HYKK, SLPI, CFHR1, SPDEF, DACT2, TUBGCP5, AREG, HIST1H2AJ, KIF2A, AL135925.1, NOTCH3, SLC11A1, HEXIM2, IGFBP1, TVP23A, NUDT14, SAMD11, MIR200CHG, PCLAF, SLC43A3, FAM30A, PHRF1, ADM, SIK2, NUSAP1, CFH, KRTCAP3, SPAG4, TPPP3, TSPAN4, AAK1, CST1, CLU, IFRD1, ASPHD2, CNN3, COL4A1, FGA, ANO6, SBSN, FGB, ATP9B, NLGN4Y, HP, EPS8, RNF111, LINC01285, MAOA, IGHV4-31, TNFRSF10D, GSR, IGHGP, TACSTD2, MT1F, RHCG, MUT, PI3, MT1M, LAMB3, MTRNR2L12, SLC35A2, DDX10, RARRES1, MTSS1, CLK2, RPN2, MED29, CYP1B1, TTTY14, DMXL1, AL139246.5, TAF1, DAAM1, MYO1E, MAFB, CDKN1A, F8A3, FABP5, CFB, HSP90B1, SGK3, HMG20B, CDCA5, CLDN4, SYNM, PAWR, TWNK, AC116049.2, RND3, ATP11A, PID1, MALAT1, TMEM168, TFF1, TFRC, RNASET2, SPINK13, PABPC1L, P4HA2, PRSS8, SPINT1, MSC, FMNL1, SLC8B1, UNC13D, SPINT2, DCP1A, NPTN, IGKV3D-11, G6PD, KRT6A, LYPD1, TESC, COL4A2, ELF3, BCAM, AC093323.1, IGHV1-69, LINC00511, PORCN, TPRG1-AS1, EFNB2, PARD6G-AS1, CD9, RGS16, IL6R, FZD3, GLYR1, B3GALT6, LRCH3, MAFK, LINC00491, MT1X, MUC6, PIK3R3, GBP4, PERP, LXN, ZBTB7A, WARS, AC020911.2, MAPK3, ALS2CL, MRE11, TSPAN17, IGHV4-34, IL33, ADAM9, ANGPTL4, TBC1D31, C1R, CTSC, SLC35A4, FST, SGO1, ANKRD36, IGHG3, SLC15A3, HES1, POLR1E, SLC7A5, CAPN12, IGFBP3, FBXO38, FLNA, CSKMT, OAS1, ULK1, PBX1, EXOC4, REEP6, HILPDA, ASF1B, FKBP1B, IL6, CALU, AKR1C1, KLF2, GRTP1, C1S, SMOX, CPLX2, LMNA, BSG, IGHG4, SVIL, HIST1H1B, GCH1, NEAT1, FN1, ESRP1, RFWD3, ADGRE2, SPINK6, HPD, CAVIN1, MT1E, CLDN10, C15orf48, CA9, NR4A1, PPP1R3B, SLC30A1, SLC7A11, VIRMA, NAA25, CCNB1, CFD, AP1G1, H6PD, PSCA, KCNK6, AL161431.1, DVL1, HIST1H2AM, RAB31, CDCA3, SPATA20, PRMT7, PTGR1, SERINC2, IGHG2, GFPT1, TTC22, BTBD1, HIST1H4H, CENPB, ZNF598, GPATCH2L, SPTLC3, CXCL2, CYP24A1, EZH2, GPX2, LMNB2, PTGES, MGLL, NR2F2, KRT19, DNTTIP1, MUC5AC, SDCBP2, IL1R2, AHNAK2, MUC16, AC023090.1, CPE, VNN1, BAMBI, NPW, TK1, IGKV3D-20, ANKRD11, CDC20, CDH1, STK11, IGKC, SLC45A4, TBC1D8, CSTA, AC233755.1, MIGA1, HIST1H2AL, AKAP12, MAP4K4, HOOK2, GGA3, COL7A1, NOS1, ARHGAP26, AKR1C2, TGM2, CENPF, IGHV3-48, CDCA8, TSC2, STC2, PKN3, PVR, CES1, GPRC5A, SEZ6L2, CEP170B, KIF14, IER3, ALDH3B1, TOP2A, SPP1, TXNRD1, LENG8, TRIM15, ALDH3A1, RIMKLB, HECTD4, SMOC1, NEB, RMRP, IGFBP4, MT1G, SCRIB, ERO1A, SOX4, LMO7, RNPEPL1, PLK2, COL6A2, FLRT3, IGHV4-28, SCD, KRT7, PIEZO1, CXCL1, DAPK1, ID1, C3, CXCL3, IGKV3-20, GUCA2B, ITGA3, SFN, IGLV3-21, PLEC, POLR2A, AGRN, MUC1, SERPINB3, S100A8, LAMA5, COL6A1, ITGB4, S100P, SLURP2, MSLN, KRT17, and MUC5B. In some embodiments, the gMDSC signature comprises expression values for one or more genes selected from SERPINB 1, SOD2, S100A8, CTSC, CCL18, CXCL2, PLAUR, NCF2, FPR1, and IL8. In some embodiments, the gMDSC signature comprises expression values for one or more genes selected from SERPINB1, SOD2, S100A8, CTSC, CCL18, CXCL2, PLAUR, NCF2, FPR1, IL8, S100A9, TNFAIP3, CXCL1, BCL2A1, EMR2, LILRB3, SLC11A1, IL6, TREM1, CCL20, LYN, CXCL3, IL1B, IL1R2, AQP9, IL2RA, GPR97, OSM, CXCR1, FPR2, C19orf59, CXCR2, CXCL6, CXCL5, EMR3, MEFV, S100A12, CD300E, FCGR3B, PPBP, LILRA5, LILRA3, and CASP5. In some embodiments, the immune oncology (IO) signature comprises expression values for one or more genes selected from GBP5, IL10RA, NLRC5, CXCL9, RAC2, GBP4, GLUL, IRF1, CD53, CIITA, S100B, GBP2, ITK, SLAMF7, IKZF3, DOCK2, SELL, ARHGAP9, CYTIP, IL2RB, NCKAP1L, APOD, CD96, IL7R, and ZAP70. In some embodiments, the immune oncology (IO) signature comprises expression values for one or more genes selected from ISG20, PCDHGA2, TGFB1I1, ATP8B1, IL7R, IRF8, ETV1, MYLK, GRHL2, THBS4, CYP3A5, FBLIM1, S100B, BICD1, SLAMF7, RAB27A, GATM, ICA1, ITPR1, SLC7A2, ZAP70, LOXL4, CILP, ARHGAP30, ITGB2, KLF5, PRKCA, PCDH7, DPYSL3, RGS2, SPP1, COLGALT2, MPZL2, TNFAIP8, PLAT, ALDH1A3, POF1B, PPP1R9A, SEMA3A, CIITA, DLC1, ARHGAP9, FRAS1, AKAP6, ATP1A2, TTN, LTBP1, NCKAP1L, MAP3K6, MYO1B, MRVI1, FSCN1, GPC1, GBP5, BAMBI, IL2RB, MYO1G, RANBP17, APOD, RASGRP1, CYTIP, ITGA7, CYTH4, PTPRF, KIAA1755, IRF1, GPR37, RAC2, NLRC5, EGFR, ITK, IL10RA, IGFBP2, CD96, RASD1, CD36, TMEM163, IGLL5, IKZF3, PRLR, CDC42BPG, DOCK2, PAM, VEGFA, CD84, SORL1, GBP2, SYTL4, APBB1IP, SIGLEC10, GBP4, COMP, DOCK8, CXCL9, NRP1, EPHB4, CD53, GLUL, DNM1, DSP, SIX4, SELL, DSC3, TNFAIP2, and JAG2. In some embodiments, the TMB is derived from the DNA sequencing data. In some embodiments, the expression values of the checkpoint related gene signature, the immune exhaustion signature, and the granulocytic myeloid derived suppressor cell (gMDSC) signature are derived from the RNA seq data. In some embodiments, the IO therapy is an immune checkpoint inhibitor therapy (ICI). In some embodiments, the ICI comprises pembrolizumab or nivolumab. In some embodiments, the report further comprises an immune profile score (IPS). In some embodiments, the IPS is displayed as an integer from 1-100. In some embodiments, the IPS is further divided into categories or is a categorical result. In some embodiments, the categories are IPS-Low, indeterminate, and IPS-High.

In an aspect of the current disclosure, methods of determining an immune profile score (IPS) for a subject diagnosed with a cancer are provided. In some embodiments, the methods comprise: at a computer system having one or more processors, and memory storing one or more programs for execution by the one or more processors: (A) receiving sequencing data from a sample of the cancer from the subject, wherein the sequencing data comprises RNA sequencing data, wherein the RNA sequencing data comprises a plurality of data elements comprising expression values for a plurality of genes; and applying one or more model components one to one or more models to determine the IPS for the subject. In some embodiments, the one or more model components are selected from the group consisting of: tumor mutational burden (TMB), microsatellite instability (MSI), human leukocyte antigen (HLA) typing, HLA loss of heterozygosity, T cell repertoire, B cell repertoire, a level of immune infiltration into the subjects cancer, one or more clinical laboratory results, expression of one or more checkpoint genes, optionally wherein the one or more checkpoint genes are selected from CD274, CTLA4, TIM3, TIGIT, PDCD1, TNFSF4, LAG3, IDO1, and TNFRSF9, the presence of specific pathogenic mutations and alterations in genes related to immunotherapy response and cancer prognosis, optionally, STK11, KEAP1, ARID1A, and LKB1, RNA signatures of specific cell types and/or cell states, optionally, cytotoxic T-cells, biological processes, optionally, tertiary lymphoid structure formation or mechanisms of T-cell formation, responsiveness to immunotherapy, an immune exhaustion signature (IES), or any of the components listed in Table 3.

In some embodiments, the methods comprise: at a computer system having one or more processors, and memory storing one or more programs for execution by the one or more processors: (A) receiving sequencing data from a sample of the cancer from the subject, wherein the sequencing data comprises RNA sequencing data, wherein the RNA sequencing data comprises a plurality of data elements comprising expression values for a plurality of genes; (B) applying, to the plurality of data elements for the subject's cancer, a model that is trained to provide an immune exhaustion signature (IES) for the subject's cancer, wherein the IPS is characterized by positive weights on genes associated with immunosuppression and cancer proliferation and negative weights on cytotoxic genes, wherein the model is trained on a cohort data set comprising RNA sequencing data from a sample of a cancer from a plurality of subjects and clinical data from the plurality of subjects, wherein the clinical data comprises a survival metric; and (C) applying the IPS and, optionally, one or more additional model components to one or more models to determine the IPS for the subject, wherein the IPS and the optional one or more model components are used by the model to determine the IPS for the subject.

In some embodiments, the methods comprise: at a computer system having one or more processors, and memory storing one or more programs for execution by the one or more processors: (A) receiving sequencing data from a sample of the cancer from the subject, wherein the sequencing data comprises RNA sequencing data, wherein the RNA sequencing data comprises a plurality of data elements comprising expression values for a plurality of genes; (B) applying, to the plurality of data elements for the subject's cancer, a model that is trained to provide an immune exhaustion signature (IES) for the subject's cancer, wherein the IPS is characterized by positive weights on genes associated with immunosuppression and cancer proliferation and negative weights on cytotoxic genes, wherein the IPS is calculated using a plurality of biomarkers, wherein each of the plurality of biomarkers are ranked by their weight, wherein the weight of each of the biomarkers determines the biomarker's contribution to the IPS, wherein one or more of the biomarkers are selected from a gene and an associated gene weight listed in Table 1; (C) applying the IPS and, optionally, one or more additional model components to the one or more models to determine the IPS, wherein the IPS and the optional one or more model components are used by the model to determine the IPS for the subject. In some embodiments, the method further comprises: generating a clinical report comprising the immune profile score. In some embodiments, the method further comprises administering a therapeutically effective amount of an immune oncology therapy to the subject. In some embodiments, the method further comprises administering a therapeutically effective amount of an additional therapy to the subject selected from the group consisting of: a chemotherapy, a hormone therapy, a targeted therapy, and a radiation therapy. In some embodiments, the sequencing data comprises DNA sequencing data and RNA sequencing data. In some embodiments, the one or more additional model components are selected from one or more of tumor mutational burden (TMB), microsatellite instability (MSI), human leukocyte antigen (HLA) typing, HLA loss of heterozygosity, T cell repertoire, B cell repertoire, a level of immune infiltration into the subjects cancer, one or more clinical laboratory results, expression of one or more checkpoint genes, optionally wherein the one or more checkpoint genes are selected from CD274, CTLA4, TIM3, TIGIT, PDCD1, TNFSF4, LAG3, IDO1, and TNFRSF9, the presence of specific pathogenic mutations and alterations in genes related to immunotherapy response and cancer prognosis, optionally, STK11, KEAP1, ARID1A, and LKB1, RNA signatures of specific cell types and/or cell states, optionally, cytotoxic T-cells, biological processes, optionally, tertiary lymphoid structure formation or mechanisms of T-cell formation, responsiveness to immunotherapy, or any of the components listed in Table 3. In some embodiments, the model that is trained to provide an immune exhaustion signature (IES) for the subject's cancer comprises a machine learning algorithm selected from the group consisting of: a variational autoencoder, a Cox proportional hazards model, a random forest model, a gradient-boosted survival model, and a convolutional neural network. In some embodiments, the model that is trained to provide an immune exhaustion signature (IES) for the subject's cancer comprises a variational autoencoder. In some embodiments, the clinical report indicates a particular IO therapy for use in treatment of the subject. In some embodiments, the IPS is a numerical value from 1 to 100. In some embodiments, the IPS further comprises 2 or more categories, wherein the categories are based on the likelihood of the subject to respond to an IO therapy. In some embodiments, the sequencing data comprises a targeted panel for sequencing normal-matched tumor tissue, wherein the panel detects single nucleotide variants, insertions and/or deletions, and copy number variants in 598-648 genes and chromosomal rearrangements in 22 genes. In some embodiments, the sequencing data comprises full exome or full transcriptome sequencing. In some embodiments, the IPS indicates that the subject's cancer is likely to progress on an IO therapy, the clinical report indicates one or more additional therapies for use in treating the subject for the cancer. In some embodiments, the methods further comprise administering a therapeutically effective amount of the one or more additional therapies indicated in the clinical report. In some embodiments, the one or more additional therapies are selected from: a chemotherapy, a hormone therapy, a targeted therapy, and a radiation therapy. In some embodiments, the one or more additional therapies comprises a chemotherapy.

In an aspect of the current disclosure, systems for determining an immune profile score (IPS) for a subject diagnosed with cancer are provided. In some embodiments, the systems comprise a computer including a processor, the processor configured to: perform a method comprising: at a computer system having one or more processors, and memory storing one or more programs for execution by the one or more processors: (A) receiving sequencing data from a sample of the cancer from the subject, wherein the sequencing data comprises RNA sequencing data, wherein the RNA sequencing data comprises a plurality of data elements comprising expression values for a plurality of genes; and applying one or more model components one to one or more models to determine the IPS for the subject. In some embodiments, the method further comprises: generating a clinical report comprising the immune profile score. In some embodiments, the method further comprises administering a therapeutically effective amount of an immune oncology therapy to the subject. In some embodiments, the method further comprises administering a therapeutically effective amount of an additional therapy to the subject selected from the group consisting of: a chemotherapy, a hormone therapy, a targeted therapy, and a radiation therapy. In some embodiments, the sequencing data comprises DNA sequencing data and RNA sequencing data. In some embodiments, the one or more additional model components are selected from one or more of tumor mutational burden (TMB), microsatellite instability (MSI), human leukocyte antigen (HLA) typing, HLA loss of heterozygosity, T cell repertoire, B cell repertoire, a level of immune infiltration into the subjects cancer, one or more clinical laboratory results, expression of one or more checkpoint genes, optionally wherein the one or more checkpoint genes are selected from CD274, CTLA4, TIM3, TIGIT, PDCD1, TNFSF4, LAG3, IDO1, and TNFRSF9, the presence of specific pathogenic mutations and alterations in genes related to immunotherapy response and cancer prognosis, optionally, STK11, KEAP1, ARID1A, and LKB1, RNA signatures of specific cell types and/or cell states, optionally, cytotoxic T-cells, biological processes, optionally, tertiary lymphoid structure formation or mechanisms of T-cell formation, responsiveness to immunotherapy, or any of the components listed in Table 3. In some embodiments, the model that is trained to provide an immune exhaustion signature (IES) for the subject's cancer comprises a machine learning algorithm selected from the group consisting of: a variational autoencoder, a Cox proportional hazards model, a random forest model, a gradient-boosted survival model, and a convolutional neural network. In some embodiments, the model that is trained to provide an immune exhaustion signature (IES) for the subject's cancer comprises a variational autoencoder. In some embodiments, the clinical report indicates a particular IO therapy for use in treatment of the subject. In some embodiments, the IPS is a numerical value from 1 to 100. In some embodiments, the IPS further comprises 2 or more categories, wherein the categories are based on the likelihood of the subject to respond to an IO therapy. In some embodiments, the sequencing data comprises a targeted panel for sequencing normal-matched tumor tissue, wherein the panel detects single nucleotide variants, insertions and/or deletions, and copy number variants in 598-648 genes and chromosomal rearrangements in 22 genes. In some embodiments, the sequencing data comprises full exome or full transcriptome sequencing. In some embodiments, the IPS indicates that the subject's cancer is likely to progress on an IO therapy, the clinical report indicates one or more additional therapies for use in treating the subject for the cancer. In some embodiments, the methods further comprise administering a therapeutically effective amount of the one or more additional therapies indicated in the clinical report. In some embodiments, the one or more additional therapies are selected from: a chemotherapy, a hormone therapy, a targeted therapy, and a radiation therapy. In some embodiments, the one or more additional therapies comprises a chemotherapy.

A non-transitory computer readable medium having stored thereon program code instructions that, when executed by a processor, cause the processor to perform a method comprising: at a computer system having one or more processors, and memory storing one or more programs for execution by the one or more processors: (A) receiving sequencing data from a sample of the cancer from the subject, wherein the sequencing data comprises RNA sequencing data, wherein the RNA sequencing data comprises a plurality of data elements comprising expression values for a plurality of genes; and applying one or more model components one to one or more models to determine the IPS for the subject. In some embodiments, the method further comprises: generating a clinical report comprising the immune profile score. In some embodiments, the method further comprises administering a therapeutically effective amount of an immune oncology therapy to the subject. In some embodiments, the method further comprises administering a therapeutically effective amount of an additional therapy to the subject selected from the group consisting of a chemotherapy, a hormone therapy, a targeted therapy, and a radiation therapy. In some embodiments, the sequencing data comprises DNA sequencing data and RNA sequencing data. In some embodiments, the one or more additional model components are selected from one or more of tumor mutational burden (TMB), microsatellite instability (MSI), human leukocyte antigen (HLA) typing, HLA loss of heterozygosity, T cell repertoire, B cell repertoire, a level of immune infiltration into the subjects cancer, one or more clinical laboratory results, expression of one or more checkpoint genes, optionally wherein the one or more checkpoint genes are selected from CD274, CTLA4, TIM3, TIGIT, PDCD1, TNFSF4, LAG3, IDO1, and TNFRSF9, the presence of specific pathogenic mutations and alterations in genes related to immunotherapy response and cancer prognosis, optionally, STK11, KEAP1, ARID1A, and LKB1, RNA signatures of specific cell types and/or cell states, optionally, cytotoxic T-cells, biological processes, optionally, tertiary lymphoid structure formation or mechanisms of T-cell formation, responsiveness to immunotherapy, or any of the components listed in Table 3. In some embodiments, the model that is trained to provide an immune exhaustion signature (IES) for the subject's cancer comprises a machine learning algorithm selected from the group consisting of: a variational autoencoder, a Cox proportional hazards model, a random forest model, a gradient-boosted survival model, and a convolutional neural network. In some embodiments, the model that is trained to provide an immune exhaustion signature (IES) for the subject's cancer comprises a variational autoencoder. In some embodiments, the clinical report indicates a particular IO therapy for use in treatment of the subject. In some embodiments, the IPS is a numerical value from 1 to 100. In some embodiments, the IPS further comprises 2 or more categories, wherein the categories are based on the likelihood of the subject to respond to an IO therapy. In some embodiments, the sequencing data comprises a targeted panel for sequencing normal-matched tumor tissue, wherein the panel detects single nucleotide variants, insertions and/or deletions, and copy number variants in 598-648 genes and chromosomal rearrangements in 22 genes. In some embodiments, the sequencing data comprises full exome or full transcriptome sequencing. In some embodiments, the IPS indicates that the subject's cancer is likely to progress on an IO therapy, the clinical report indicates one or more additional therapies for use in treating the subject for the cancer. In some embodiments, the methods further comprise administering a therapeutically effective amount of the one or more additional therapies indicated in the clinical report. In some embodiments, the one or more additional therapies are selected from: a chemotherapy, a hormone therapy, a targeted therapy, and a radiation therapy. In some embodiments, the one or more additional therapies comprises a chemotherapy.

BRIEF DESCRIPTION OF THE FIGURES

**FIGs. 1A and 1B** show that overall survival is predicted by the disclosed methods in a pan-cancer cohort of subjects.

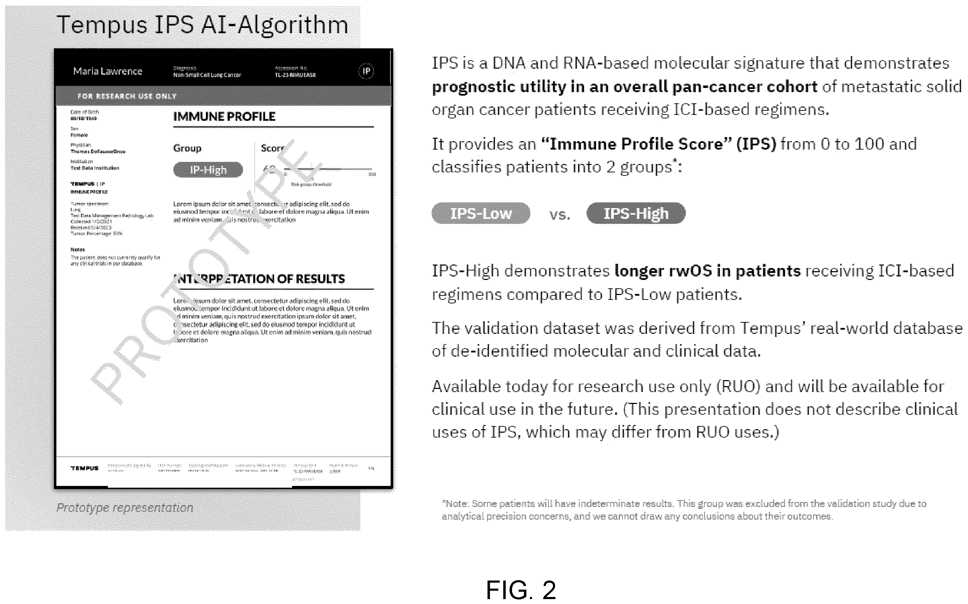

**FIG. 2** shows an exemplary readout of one embodiment of the disclosed methods.

**FIG. 3** shows that for patients receiving ICI + additional treatment in 1L, IPSHigh patients have longer OS regardless of PD-L1 IHC status. PD-L1 subgroups | ICI+additional treatment, LOT1.

**FIG. 4** shows that overall survival is predicted by the disclosed methods in subjects with non-small cell lung cancer (NSCLC), regardless of PD-L1 status.

**FIG. 5** shows For patients receiving ICI monotherapy in 1L, MSS patients have longer OS if they are IPS-High MSI-H patients have similar OS regardless of IPS status (sample size N<50).

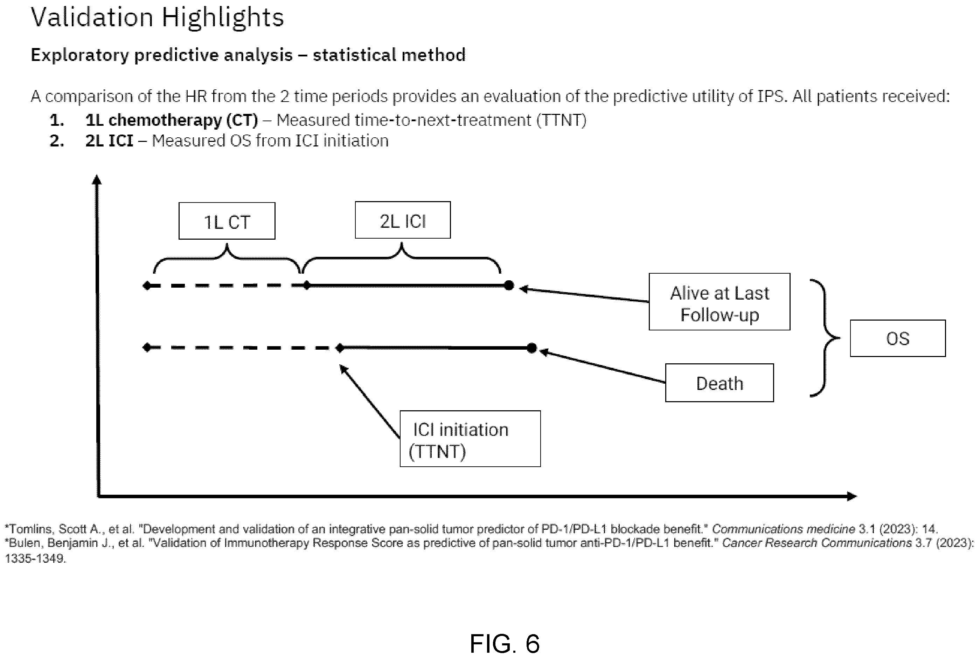

**FIG. 6** shows a comparison of the HR from the 2 time periods provides an evaluation of the predictive utility of IPS. All patients received: 1. 1L chemotherapy (CT) - Measured time-to-next-treatment (TTNT) 2. 2L ICI - Measured OS from ICI initiation.

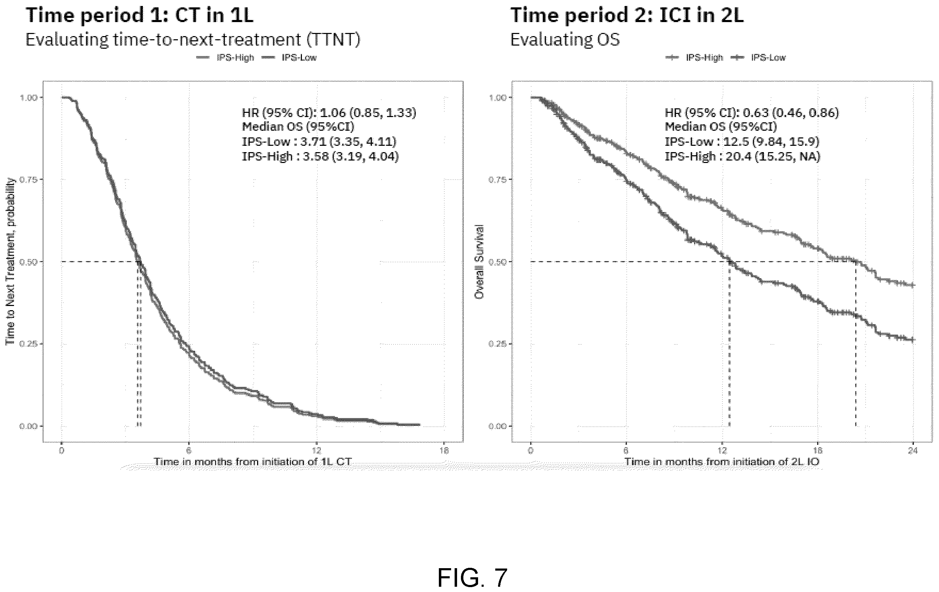

**FIG. 7** shows patients treated with chemotherapy (CT) in 1L, then ICI in 2L. Pan-cancer metastatic solid tumors with ICI approvals - IPS stratifies outcomes following ICI but not CT, interaction test p-value < 0.01.

**FIGs. 8A and 8B** show the inclusion criteria (8A) and exclusion criteria (8B) defining the IPS validation cohort.

**FIG. 9** shows that the IPS has significant prognostic utility beyond tumor mutational burden (TMB), though TMB is still significant in a multivariable model with the IPS, with some attenuation in the hazard ratio.

**FIG. 10** shows that IPS has significant prognostic utility beyond standard PD-L1 histological assessment and PD-L1 expression is not significant in a multivariable model with IPS.

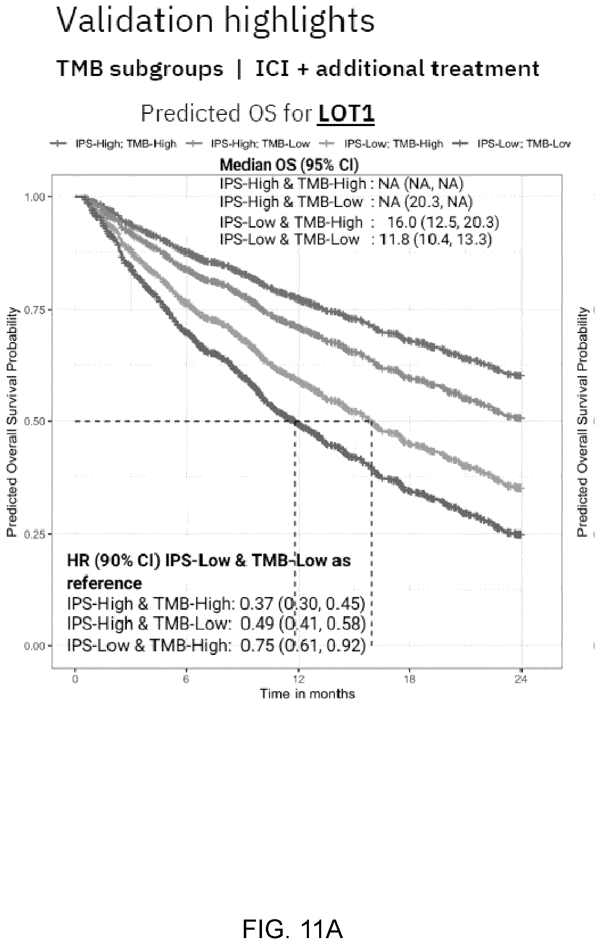

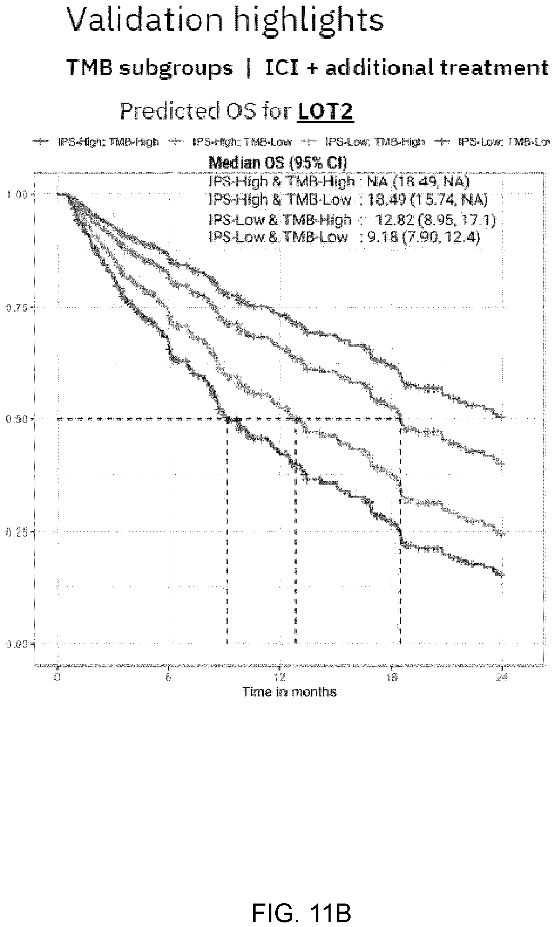

**FIGs. 11A and 11B** show the predicted overall survival for all combinations of TMB and IPS values (i.e., TMB-High+IPS-High, TMB-High+IPS-Low, TMB-Low+IPS-High, TMB-Low+IPS-Low). The same groupings are shown for both line of therapy 1 (Fig. 11A) and line of therapy 2( Fig. 11B).

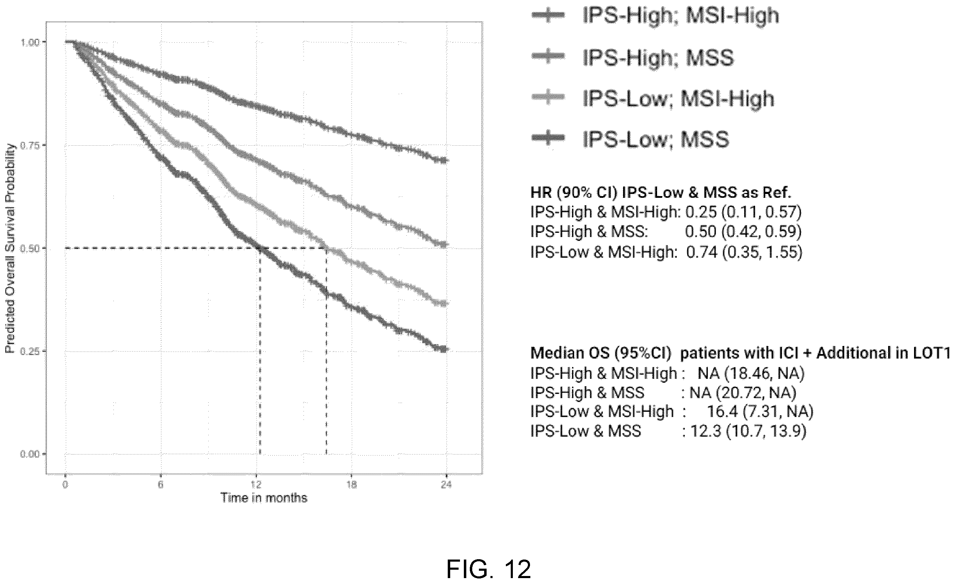

**FIG. 12** shows the predicted overall survival for all combinations of MSI and IPS values (i.e., MSI-High+IPS-High, MSI-High+IPS-Low, MSS+IPS-High, MSS+IPS-Low).

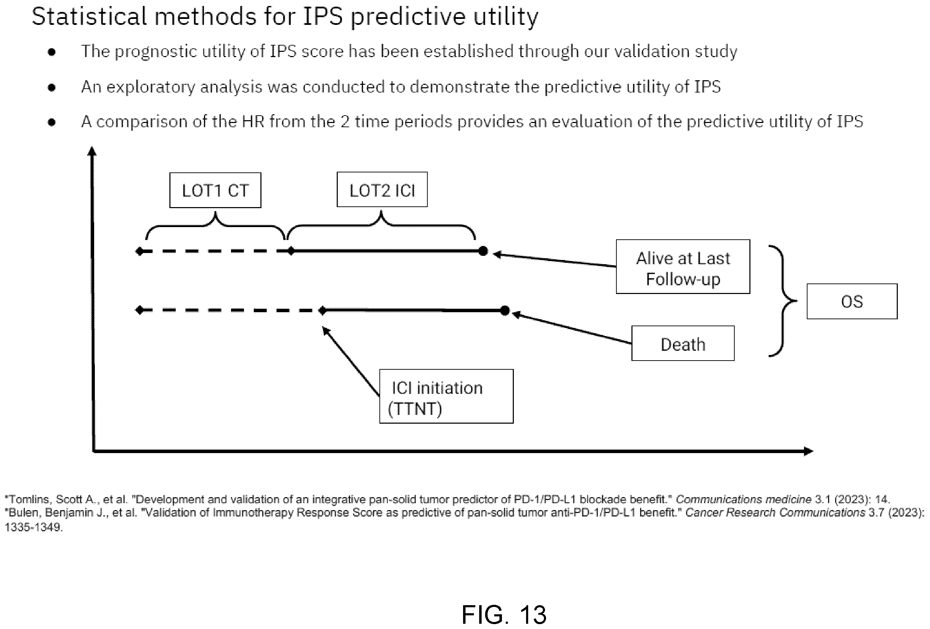

**FIG. 13** shows the statistical method used in assessing predictive utility of IPS (i.e., the analysis underpinning Fig. 7.

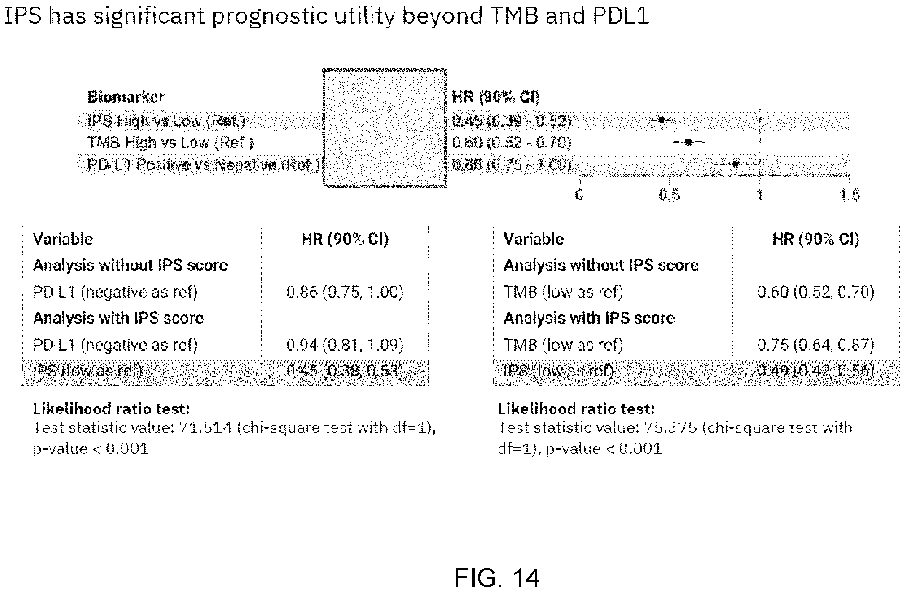

**FIG. 14** shows CoxPH and likelihood ratio results indicating the improved prognostication of IPS versus TMB/PD-L1 alone.

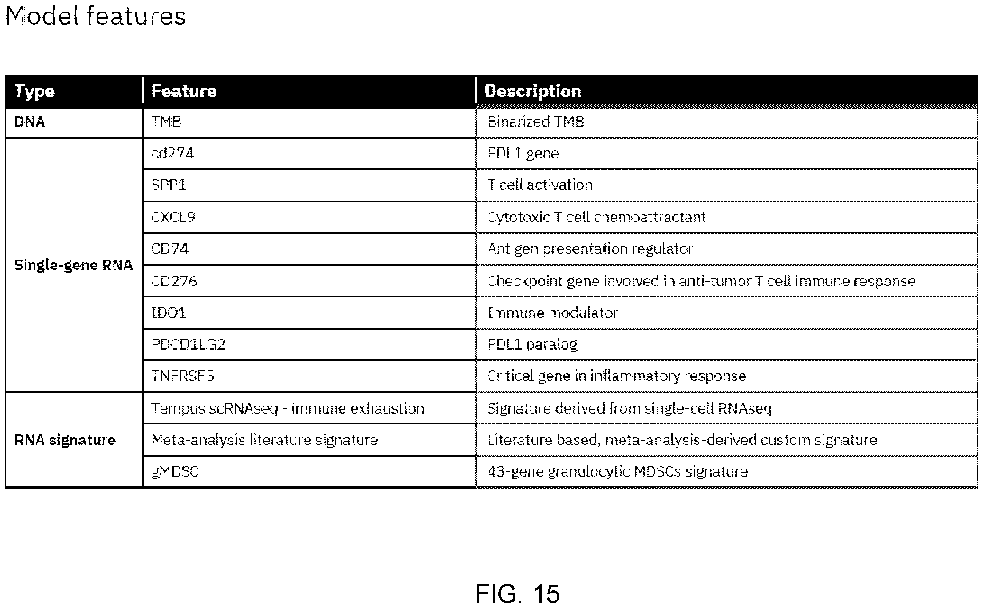

**FIG. 15** shows a brief description of each feature used in the model.

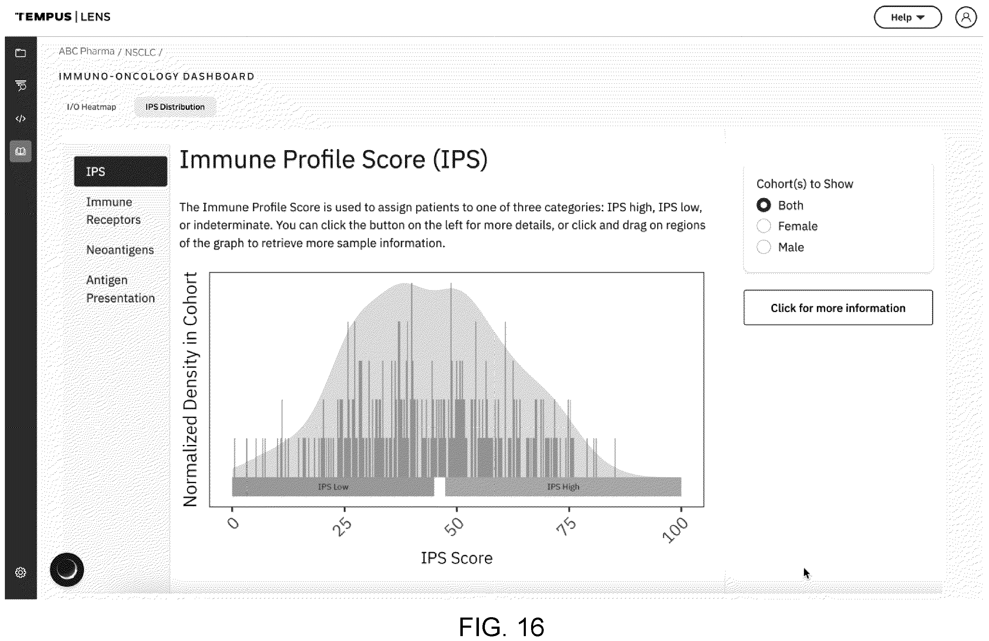

**FIG. 16** shows an exemplary graphical user interface (GUI) of the disclosed methods showing a continuous IPS and density of scores in a cohort labeled on the X axis with the categories IPS low and IPS high.

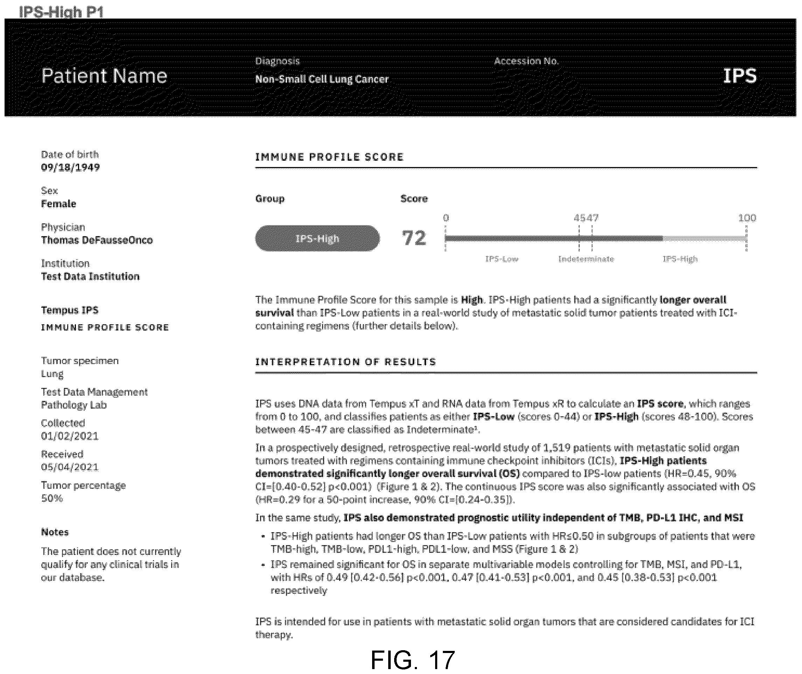

**FIG. 17** shows an example patient report for an IPS-high sample.

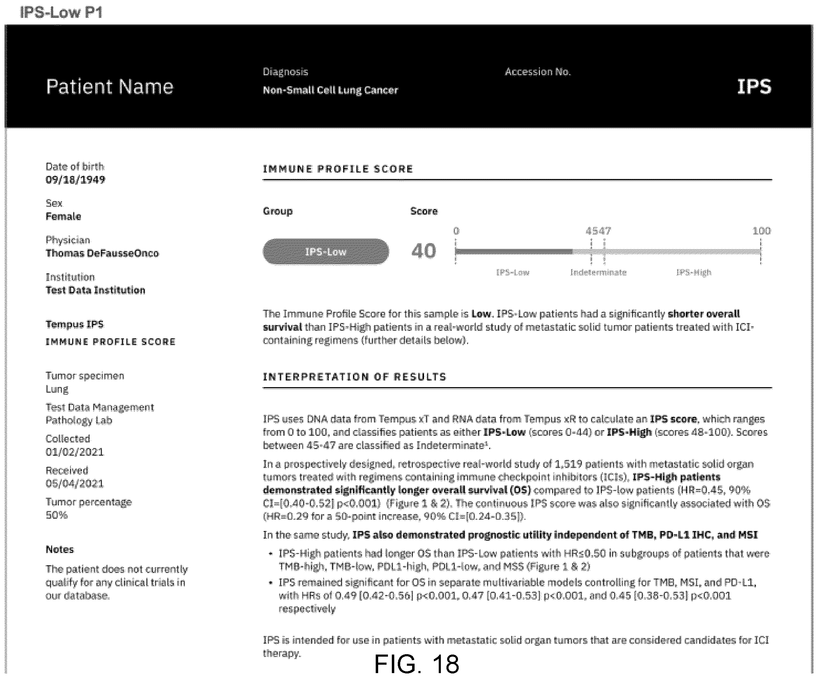

**FIG. 18** shows an example patient report for an IPS-low sample.

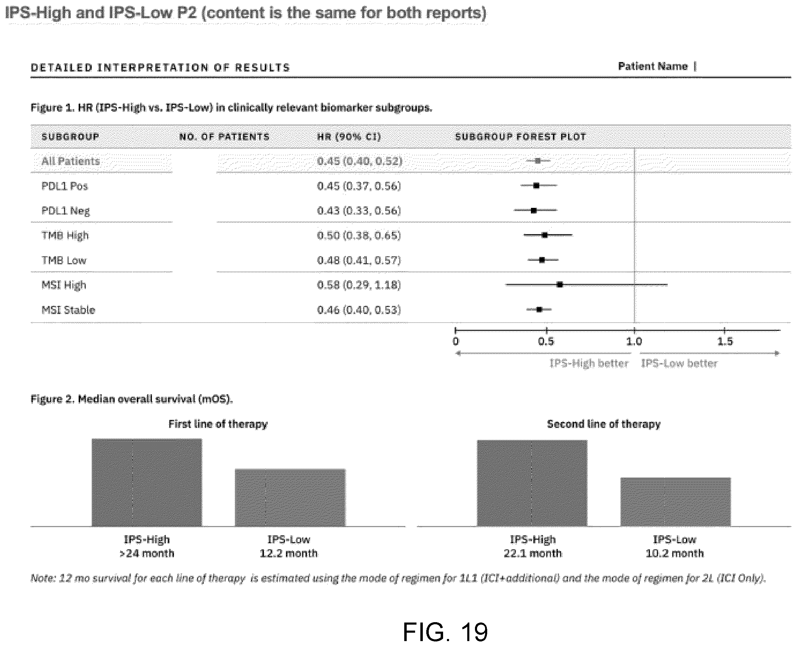

**FIG. 19** shows an example patient rep ort with interpretation page.

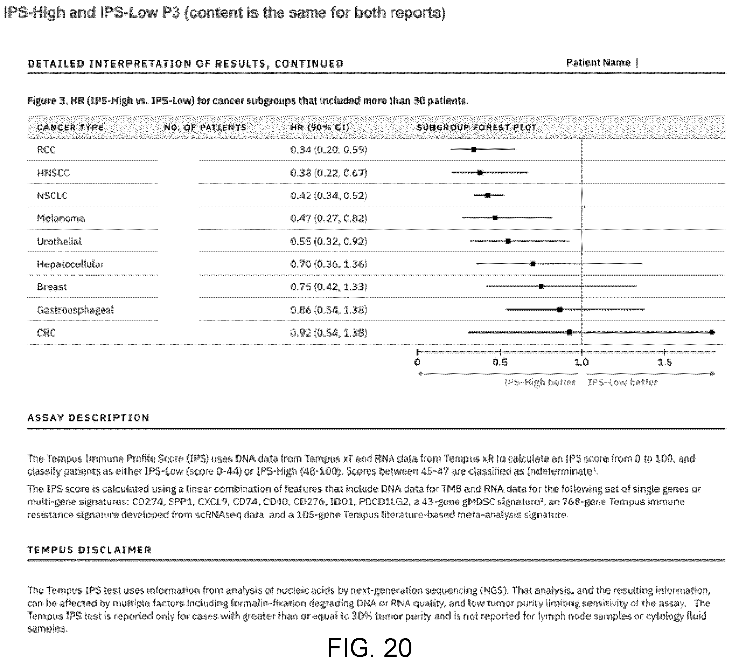

**FIG. 20** shows an example patient report with interpretation page and assay description.

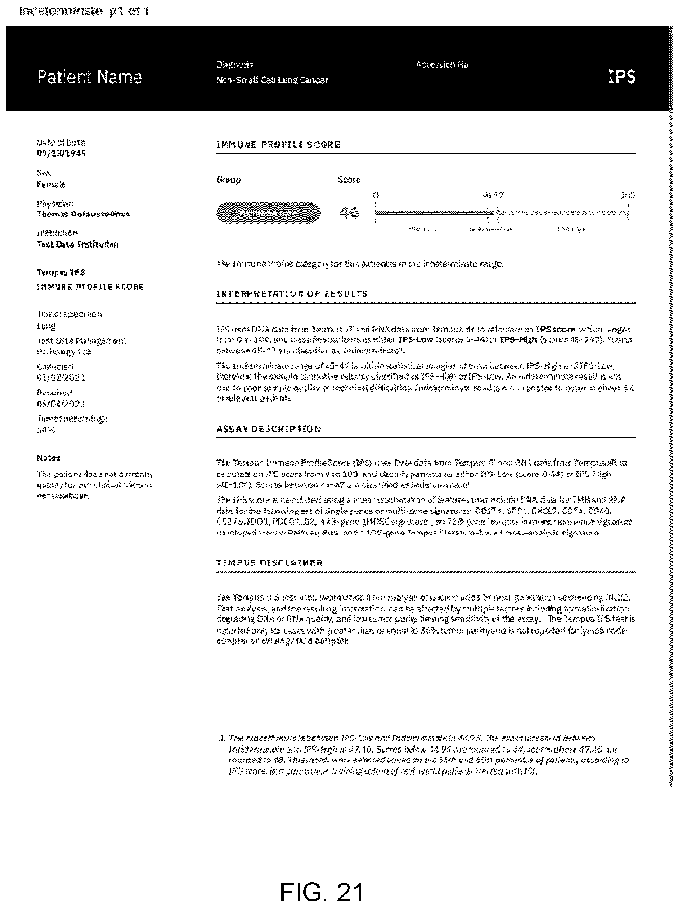

**FIG. 21** shows an example patient report for an IPS-indeterminate sample.

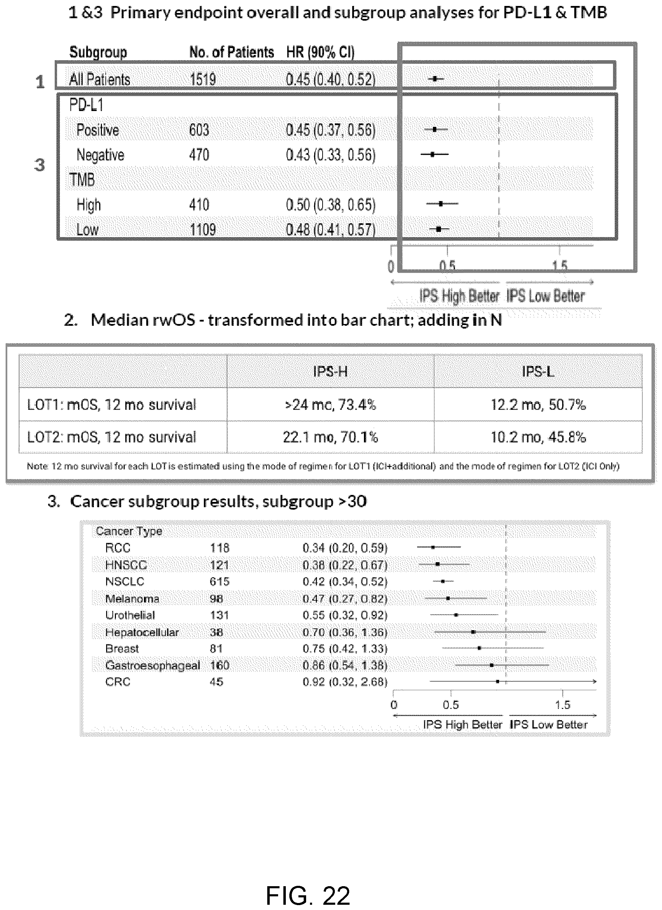

**FIG. 22** shows forest plots indicating significant results for primary endpoints in the IPS clinical validation study.

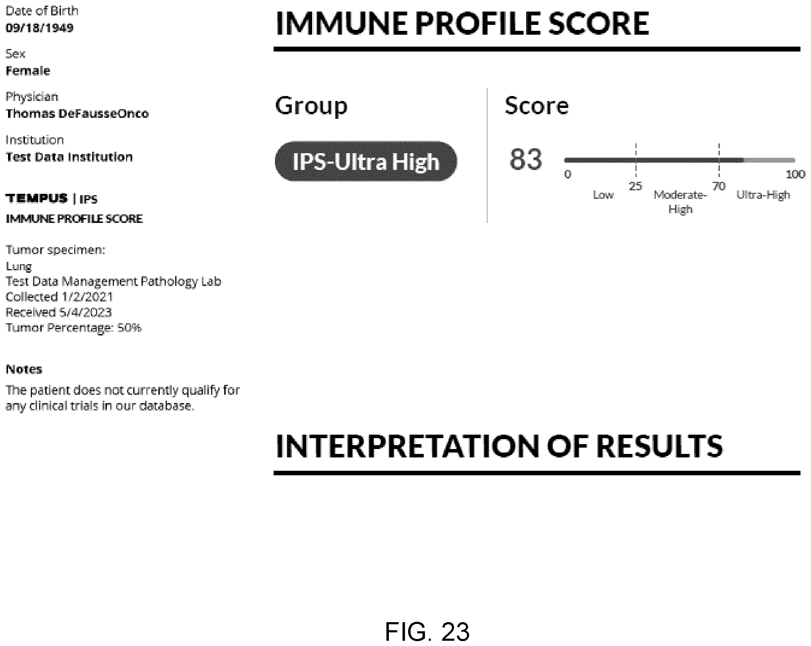

**FIG. 23** shows an example patient report with an ultra-high IPS risk categorization.

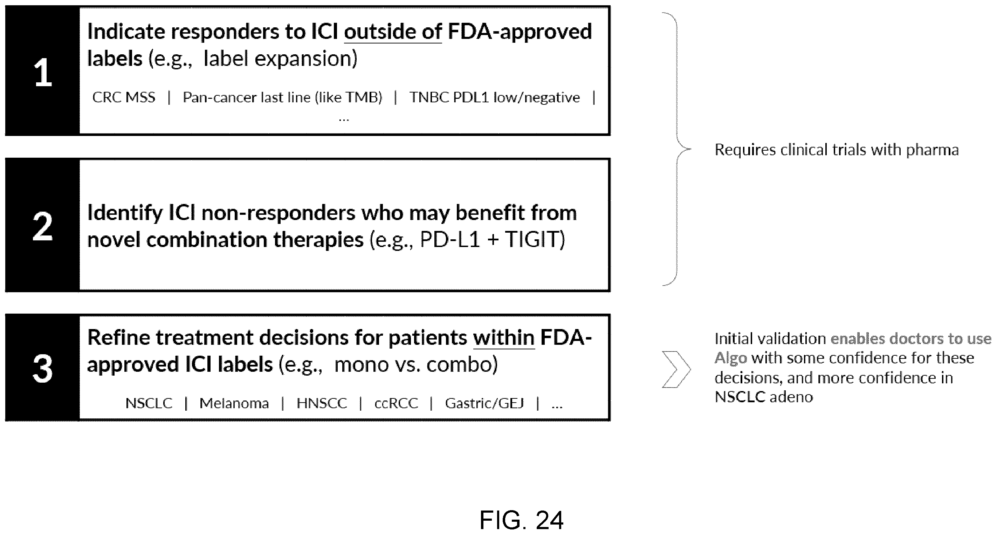

**FIG. 24** shows an example clinical/pharma strategy associated with IPS.

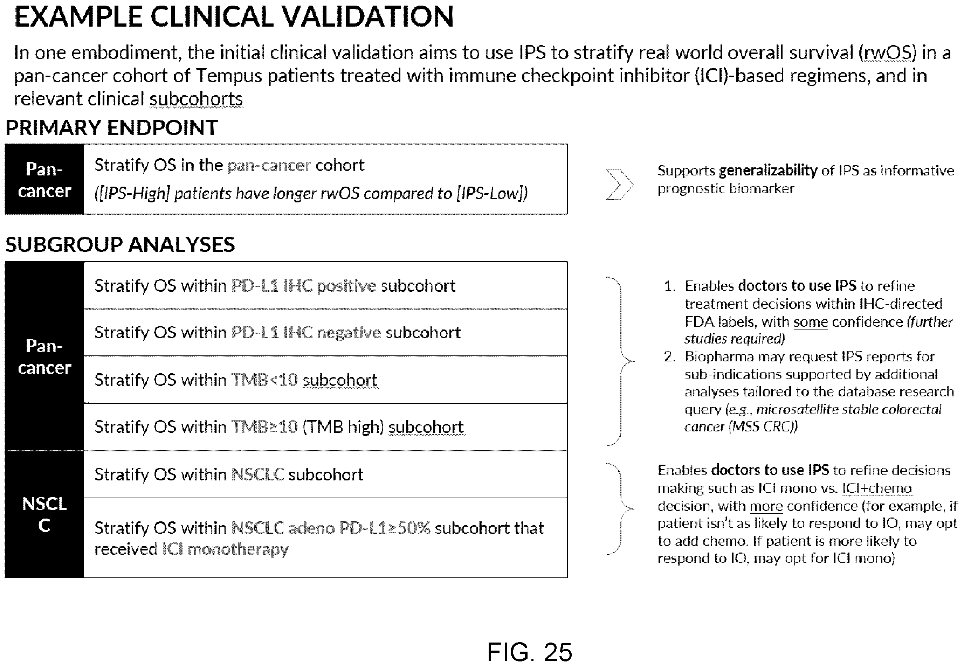

**FIG. 25** shows an example clinical validation strategy associated with IPS

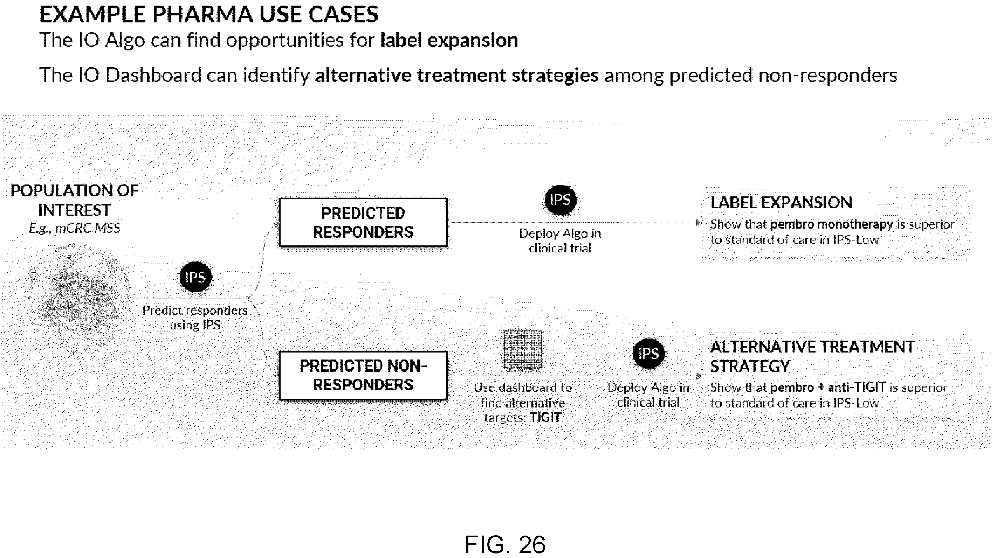

**FIG. 26** shows an example pharma strategy associated with IPS.

**FIG. 27** shows inclusion/exclusion criteria for the study cohort for Example 4.

**FIG. 28** shows that various machine learning (ML) techniques were implemented to reduce the feature space. In one embodiment, the IPS model includes 11 RNA-based features and TMB.

**FIGs. 29A, 29B, and 29C** show that the hazard ratio (HR) for the cohort in Example 4 was 0.45 (0.40, 0.52), p < 0.01. Predicted OS from a CoxPH model for a) 1L monotherapy and b) 2L monotherapy patients. Predicted survival for 1L and 2L combination therapy patients are similar to above, c) The median OS and 95% confidence interval for IPS-H and IPS-L groups for each line of therapy/treatment group combination.

**FIG. 30** shows a forest plot showing IPS-H vs. IPS-L hazard ratios and confidence intervals across demographics and clinically relevant subgroups. Subgroups may have <1519 patients due to availability of data.

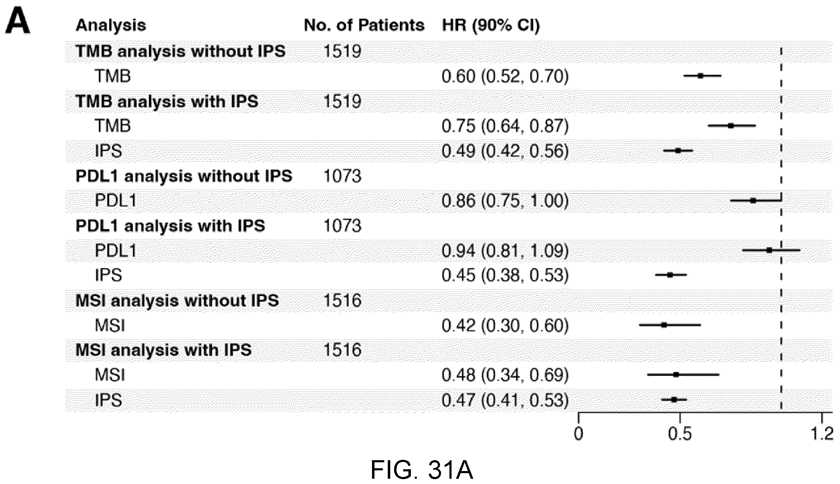

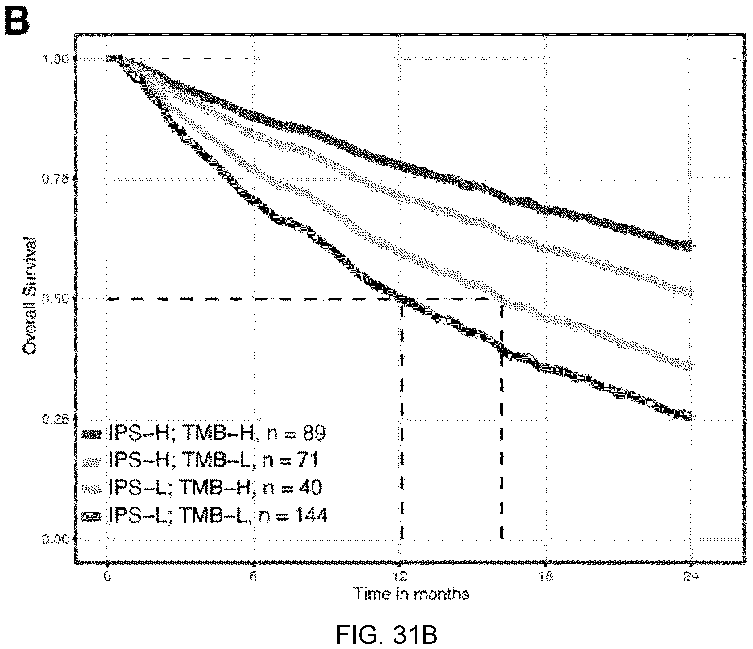

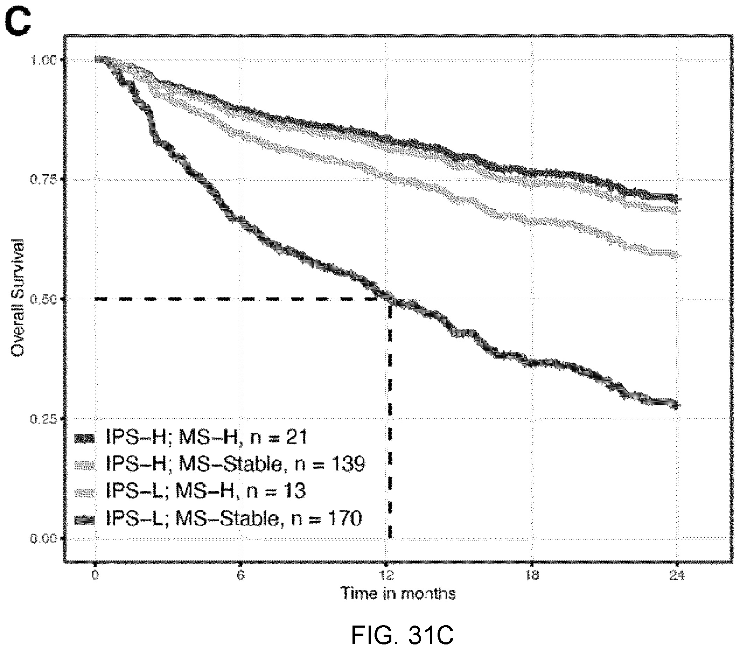

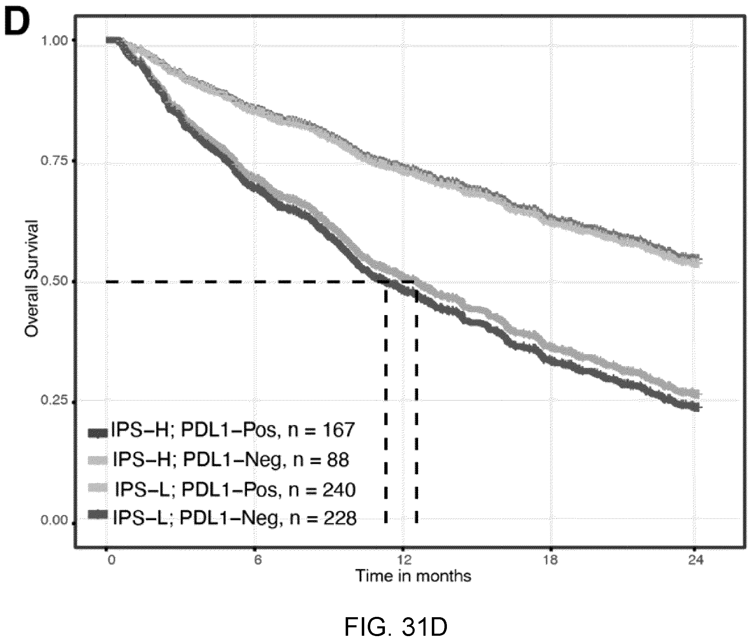

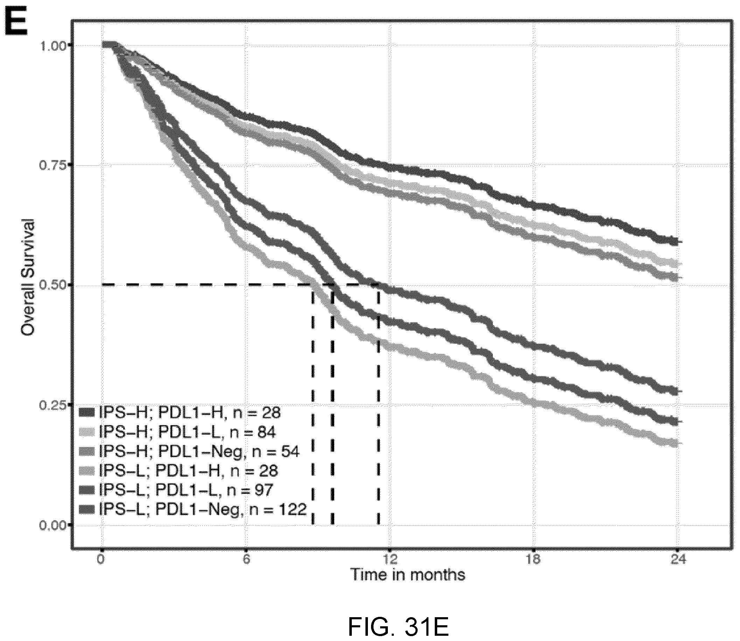

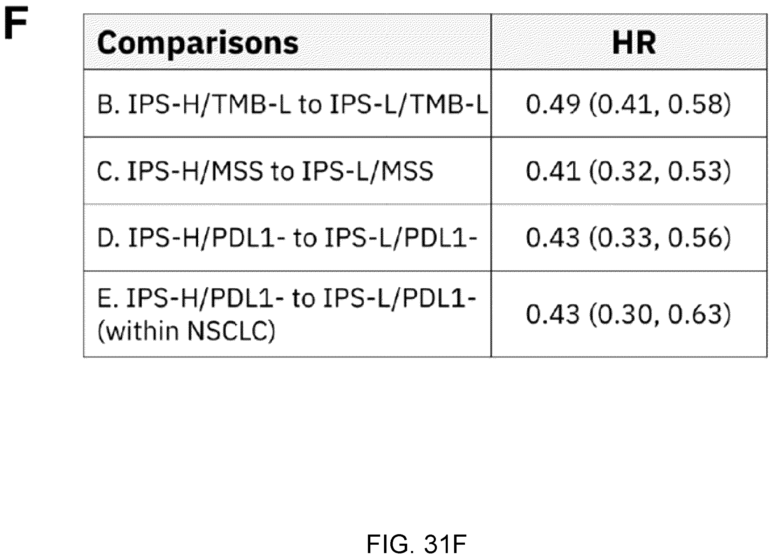

**FIGs. 31A, 31B, 31C, 31D, 31E, and 31F** show A. Forest plot showing univariate (UV) HRs for TMB, PD-L1, MSI and multivariate (MV) HRs that include IPS. A likelihood ratio test between the UV and MV models was significant (p<0.01) for all three biomarkers, indicating that IPS has significant prognostic utility beyond TMB, MSI, and PD-L1. Plots b-e show predicted OS from a model stratified by line of therapy and fit on IPS, treatment group, and the MV model with the listed biomarker: B. TMB pan-cancer, C. MSI pan-cancer, D. PD-L1 pan-cancer and E. PD-L1 in NSCLC patients. The predicted OS curves represent patients treated with monotherapy in 1L for TMB and MSI (B-C), and combination therapy in 1L for PD-L1 and NSCLC (D-E). F. HR and 90% CI for the most relevant curves shown in the predicted OS plots in (B-E).

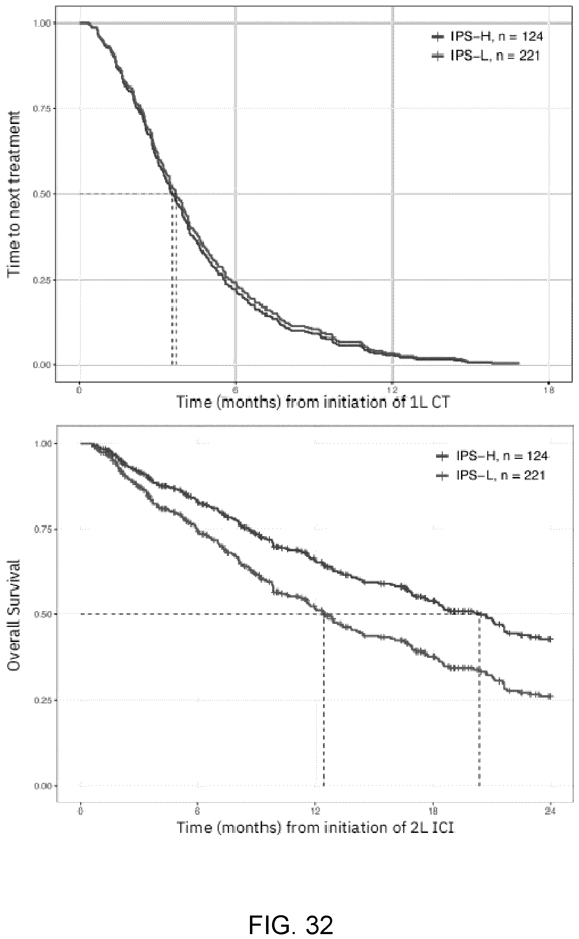

**FIG. 32** shows an exploratory analysis of the predictive utility of the IPS was performed by combining the training and validation cohorts of patients who received chemotherapy (CT) as first line treatment and ICI as second line treatment. Patients served as their own control in this analysis, and outcomes were evaluated for two lines of therapy: time to next treatment (TTNT) on CT and OS on ICI. A conditional model for recurrent events was fit. Top: Predicted TTNT for 1L CT with no significant effect for IPS (HR = 1.06 (0.85, 1.33)). Bottom: Predicted OS for 2L ICI shows that IPS does have a significant effect (HR = 0.63 (0.46, 0.86)). Interaction test p < 0.01, indicating that the HR in 2L ICI is significantly different from HR in 1L CT.

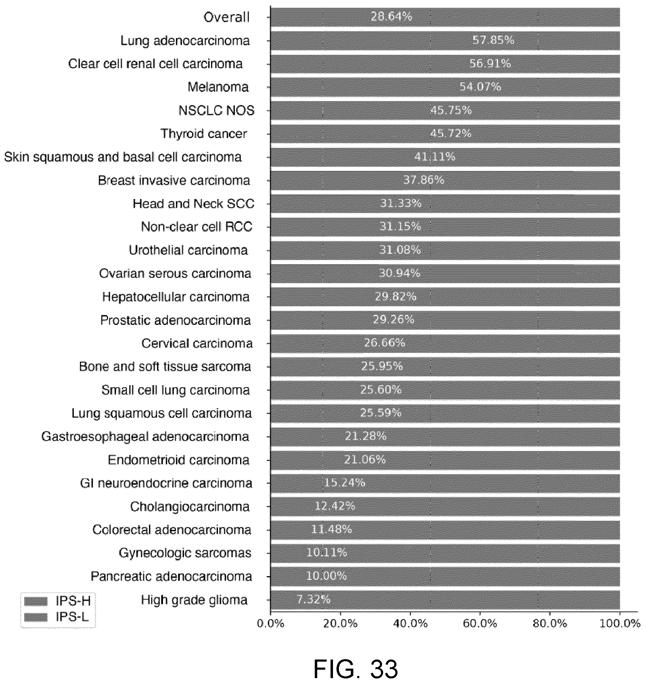

**FIG. 33** shows Prevalence plot showing the percentage of IPS high patients in a large, representative cohort of patients from a multimodal database.

**FIG. 34** shows an example 100 of a system (e.g., a data processing system) for characterizing a protein in accordance with some embodiments of the disclosed subject matter is shown.

DETAILED DESCRIPTION

**Challenges with Current Immunotherapy Biomarkers**

While immunotherapies have dramatically improved outcomes for many cancer patients, there is a massive opportunity to expand the benefits of immunotherapies to patients who are not identified by existing biomarkers as candidates for an immunotherapy. For example, many patients who could benefit from immune checkpoint inhibitors (ICIs) are not being identified by existing biomarkers like PD-L1 *(see* Rizvi H, Sanchez-Vega F, La K, et al. Molecular Determinants of Response to Anti-Programmed Cell Death (PD)-1 and Anti-Programmed Death-Ligand 1 (PD-L1) Blockade in Patients With Non-Small-Cell Lung Cancer Profiled With Targeted Next-Generation Sequencing. J Clin Oncol. 2018;36(7):633-641) and TMB *(see* McGrail DJ, Pilié PG, Rashid NU, et al. High tumor mutation burden fails to predict immune checkpoint blockade response across all cancer types. Ann Oncol. 2021;32(5):661-672). Additionally, some patients identified as PD-L1 and TMB high do not respond to ICIs *(see* Camila Bragança Xavier et al., Association between tumor mutational burden (TMB) and mutational profile and its effect on overall survival: A post hoc analysis of patients with TMB-high and TMB-low metastatic cancer treated with immune checkpoint inhibitors (ICI). JCO 40, 2632-2632(2022). DOI:10.1200/JCO.2022.40.16_suppl.2632).

Furthermore, IO therapies, e.g., ICIs, are costly and have significant risks of side effects. Therefore, developing improved biomarkers for ICI response and/or methods of detecting subjects that are good candidates for ICI therapies have the potential to notably improve trial success rates and patient outcomes, ensuring more accurate identification of patients who could benefit from ICI therapies.

**Advantages of the Disclosed Technologies**

The systems, methods, and compositions described herein relate to an immune profile score that has prognostic utility in a pan-cancer cohort of subjects. Therefore, the disclosed methods and systems may be useful for treatment of any cancer and can be used to direct patient therapy, and in particular, immune checkpoint inhibitor (ICI) therapy.

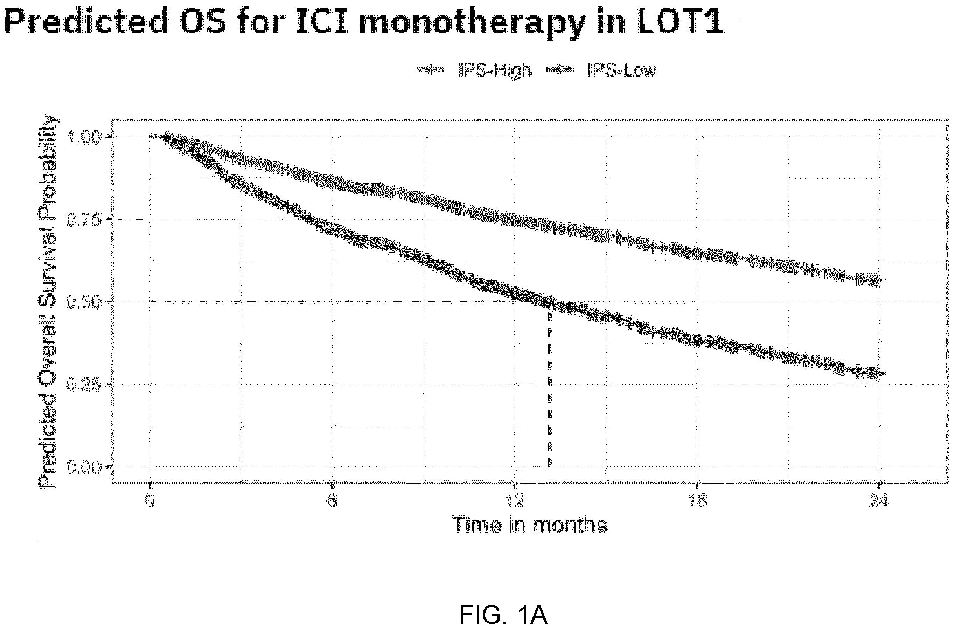

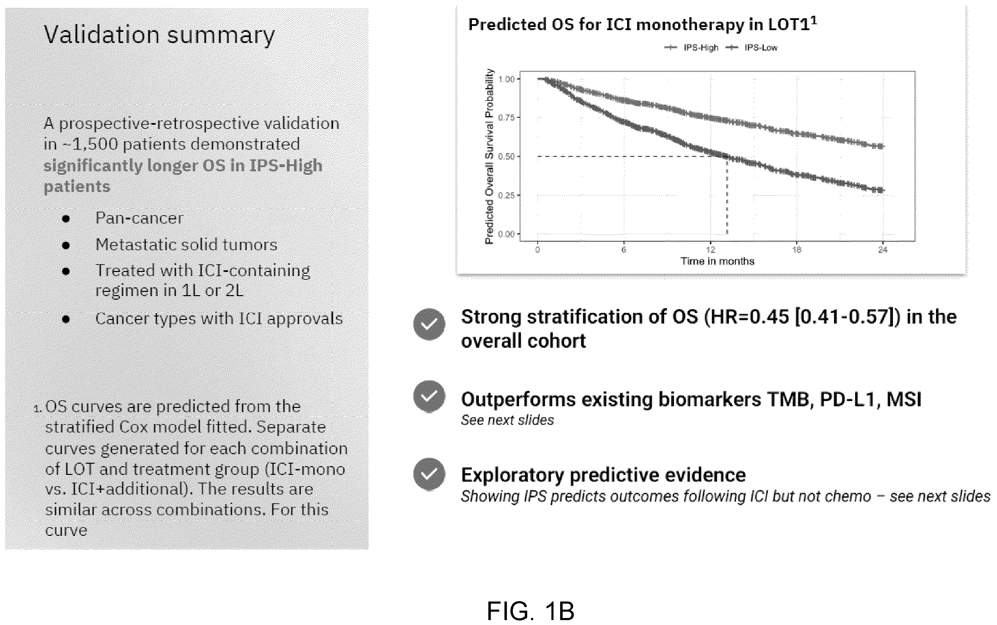

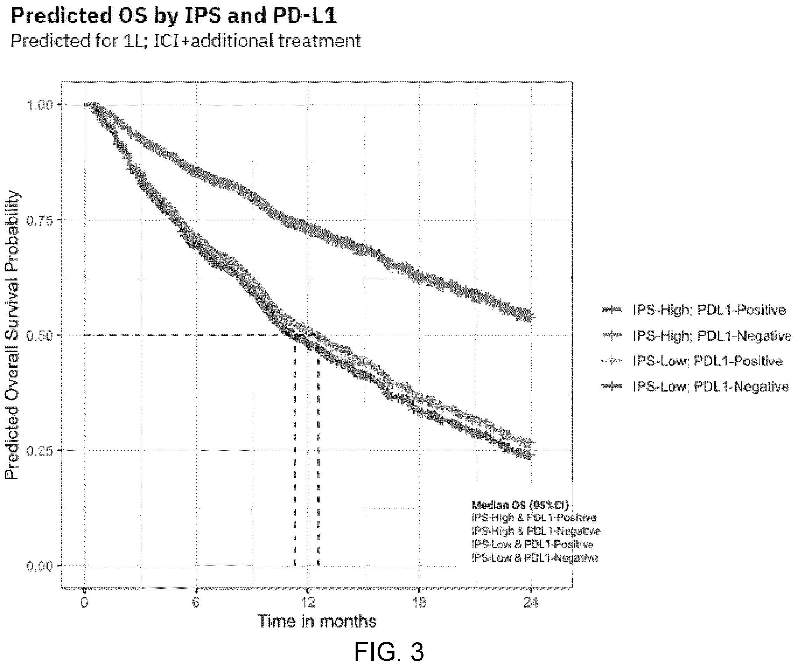

The inventors discovered that the disclosed methods are predictive of overall survival (OS) subsequent to ICI therapy in a pan-cancer cohort of subjects *(see,* e.g., FIG. 1). The pan-cancer cohort of subjects comprised subjects suffering from melanoma, non small cell lung cancer, breast carcinoma renal clear cell carcinoma, cervical carcinoma endometrial serous carcinoma, cholangiocarcinoma lung squamous cell carcinoma, lung adenocarcinoma gastroesophageal adenocarcinoma, urothelial carcinoma urothelial neuroendocrine carcinoma, endometrioid carcinoma head and neck squamous cell carcinoma, hepatocellular carcinoma skin squamous and basal cell carcinoma, colorectal adenocarcinoma gastroesophageal squamous cell carcinoma, and small cell lung carcinoma (NSCLC). As discussed above, a standard biomarker for ICI therapy success is tumor PD-L1 expression. Surprisingly, the disclosed methods are predictive of OS *regardless* of PD-L1 status (FIG. 3). Moreover, the disclosed methods are able to identify a clinically meaningful subset of subjects who are characterized as "PD-L1 low" but are, nonetheless, good candidates for ICI therapy. Thus, the disclosed methods and systems fill a much-needed gap in current diagnostic technology.

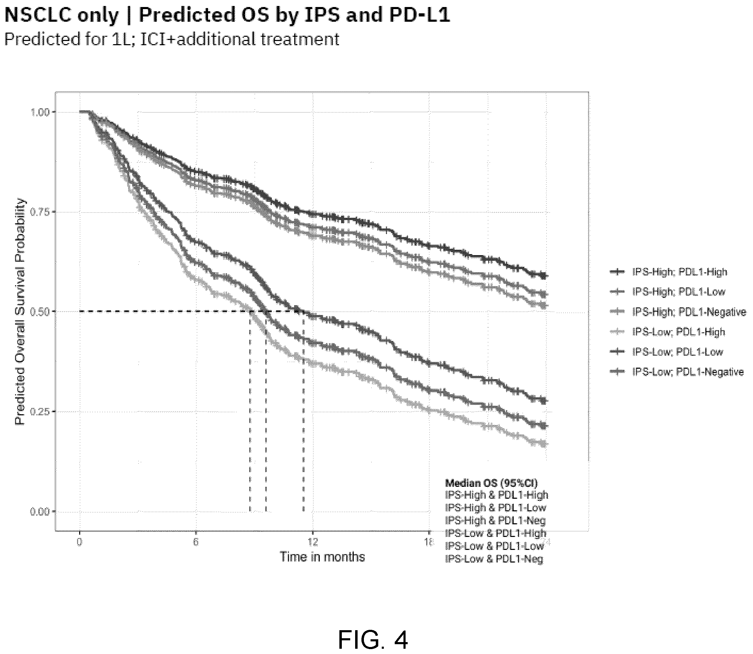

Further, in the context of non-small cell lung cancer (NSCLC), PD-L1 status, either high, low, or negative, is subordinate in its predictive ability compared to the IPS generated by the disclosed methods (for NSCLC patients receiving ICI+ additional treatment in 1L, IPS High patients have longer OS regardless of PD-L1 IHC status (FIG. 4).

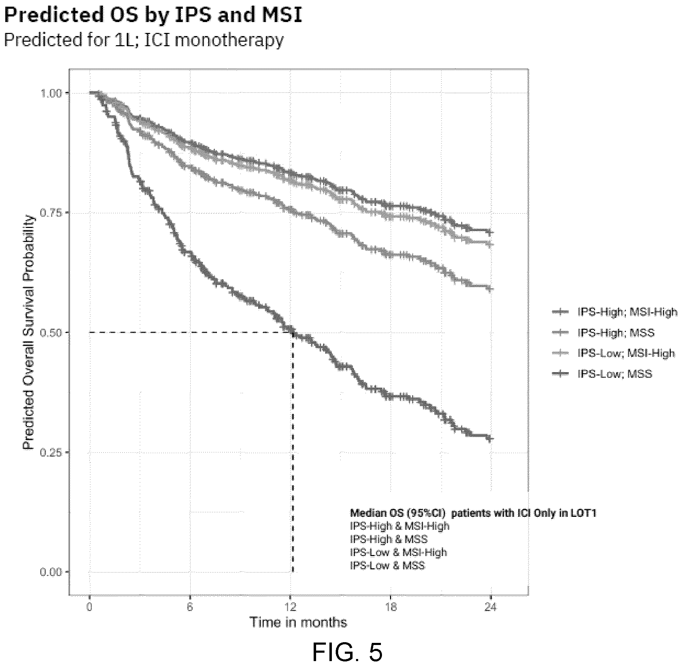

Microsatellite stable (MSS) subjects may be considered to have a poorer prognosis than comparable subjects with microsatellite instability (MSI) when treated with an ICI. Despite this potential confounding factor, the disclosed methods are able to identify a subset of microsatellite stable (MSS) subjects as having a significantly higher likelihood of objective survival following IO therapy, e.g., ICI therapy, if they are IPS-high according to the disclosed methods *(see,* e.g., FIG. 5).

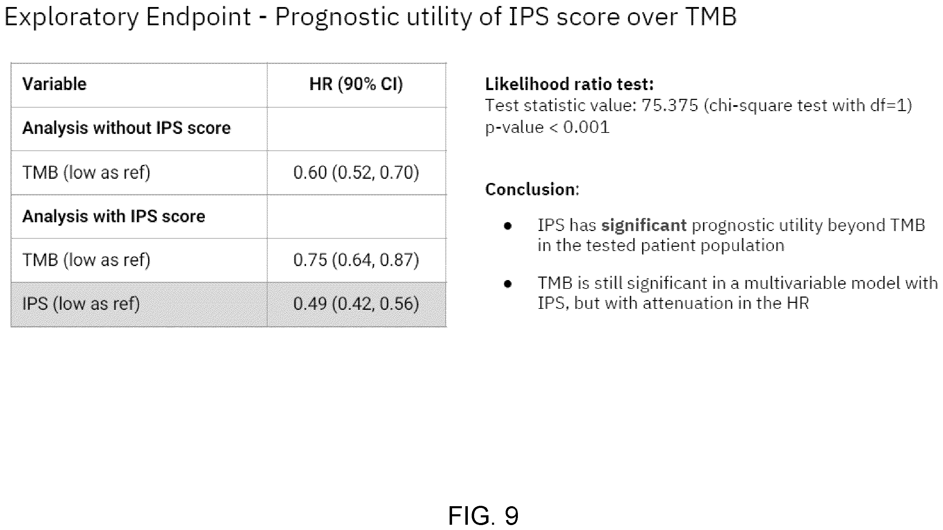

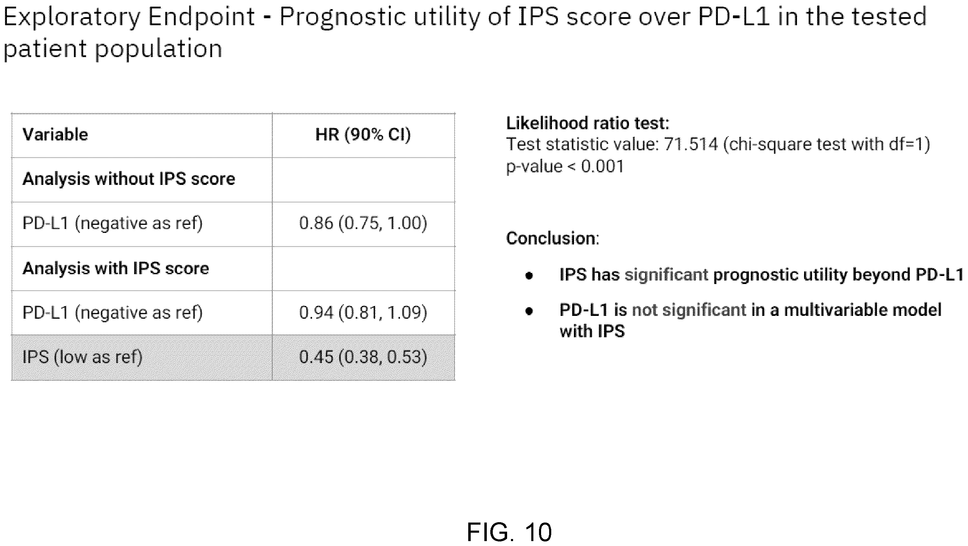

Tumor mutational burden (TMB) is approved as a last-line diagnostic for subjects suffering from any cancer. Referring now to FIG. 9, the inventors demonstrated that the disclosed methods have significant prognostic value over TMB alone as a biomarker. Similarly, FIG. 10 shows that IPS has significant prognostic utility beyond standard PD-L1 histological assessment and PD-L1 expression is not significant in a multivariable model with IPS. However, the disclosed methods may further include PD-L1 status as a model parameter and PD-L1 histological assessment may be used in combination with the IPS, in some embodiments.

Thus, the disclosed methods are significantly more effective at identifying subjects likely to have an increased overall survival subsequent to ICI therapy than existing biomarker technologies and the disclosed methods are able to identify clinically relevant subsets of subjects that would not be considered good candidates to receive an immunotherapy, using current diagnostic technologies. Accordingly, the disclosed methods provide a significant contribution to multiple technical fields including to the fields of oncology and diagnostics.

Further, the disclosed methods may be used to select patients for a clinical trial (for example, certain IPS results as part of inclusion/exclusion criteria, may only want patients who are likely to respond to IO, or patients who are not likely to respond to IO, plan clinical trials (getting estimates of patient population sizes and IPS characteristics), or interpret results (for patients who did not respond to the trial, analysis may be performed to determine if that group have an IPS score that was very different than the responders' scores.

## Methods

In an aspect of the current disclosure, methods are provided. In some embodiments, the methods comprise: at a computer system having one or more processors, and memory storing one or more programs for execution by the one or more processors: applying, by the one or more processors, one or more model components derived from sequencing data from a sample to one or more machine learning algorithms (MLAs).

As used herein, "one or more model components" comprises one or more of an immune exhaustion signature (IES), an immune oncology signature (IOS), a gMDSC signature, a tumor mutational burden (TMB), and a checkpoint related gene signature. The one or more model components may further comprise one or more of the components described in Table 3.

The sequencing data may comprise RNA sequencing data or RNA and/or DNA sequencing data.
The machine learning algorithms include, but are not limited to a variational autoencoder, a Cox proportional hazards model, a random forest model, a gradient-boosted survival model, a recurrent neural network, a transformer neural network, accelerated failure time model, a parametric survival model, or a convolutional neural network.

The methods may be performed using sequencing data obtained from a sample from a subject. Alternatively, sequencing may be performed to obtain the sequencing data.

As used herein, a "subject" may be suffering from any type of cancer, e.g., urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, esophageal cancers, hepatocellular cancers, cholangiocellular cancers, head and neck squamous cell cancer soft tissue sarcoma, and small cell lung cancer. The disclosed methods may be predictive of a subject's response to immune oncology therapies regardless of their particular type of tumor.

The one or more "model components," which may also be referred to as "features" or "model features" may further comprise one or more features from the group consisting of: tumor mutational burden (TMB), microsatellite instability (MSI), human leukocyte antigen (HLA) typing, HLA loss of heterozygosity, T cell repertoire, B cell repertoire, a level of immune infiltration into the subjects cancer, one or more clinical laboratory results, expression of one or more checkpoint genes, optionally wherein the one or more checkpoint genes are selected from CD274, CTLA4, TIM3, TIGIT, PDCD1, TNFSF4, LAG3, IDO1, and TNFRSF9, the presence of specific pathogenic mutations and alterations in genes related to immunotherapy response and cancer prognosis, optionally, STK11, KEAP1, ARID1A, RNA signatures of specific cell types and/or cell states, optionally, cytotoxic T-cells, biological processes, optionally, tertiary lymphoid structure formation or mechanisms of T-cell formation, responsiveness to immunotherapy, and an immune exhaustion signature (IES) or from any of the components listed in Table 3, also referred to as "signatures" or "biomarkers." The model that is trained to provide an immune exhaustion signature (IES) for the subject's cancer or the one or more models to determine the IPS the comprise a machine learning algorithm may be selected from the group consisting of: a variational autoencoder, a Cox proportional hazards model, a random forest model, a gradient-boosted survival model, a recurrent neural network, a transformer neural network, accelerated failure time model, a parametric survival model, or a convolutional neural network.

As used herein, a "survival metric" refers to a metric associated with survival of the subject, e.g., overall survival (OS), progression-free survival (PFS). In some embodiments, the survival metric is measured after the subject has been treated with an IO therapy, e.g., an ICI therapy.

In some embodiments, the methods comprise at a computer system having one or more processors, and memory storing one or more programs for execution by the one or more processors: (A) receiving sequencing data from a sample of the cancer from the subject, wherein the sequencing data comprises RNA sequencing data, wherein the RNA sequencing data comprises a plurality of data elements comprising expression values for a plurality of genes; (B) applying, to the plurality of data elements for the subject's cancer, a model that is trained to provide an immune exhaustion score (IES) for the subject's cancer, wherein the IES is characterized by positive weights on genes associated with immunosuppression and cancer proliferation and negative weights on cytotoxic genes, wherein the IES is calculated using a plurality of biomarkers, wherein each of the plurality of biomarkers are ranked by their weight, wherein the weight of each of the biomarkers determines the biomarker's contribution to the IES, wherein one or more of the biomarkers are selected from a gene and an associated gene weight listed in Table 1; (C) applying the IES and, optionally, one or more additional model components to the one or more models to determine the IES, wherein the IES and the optional one or more model components are used by the model to determine the IPS for the subject.

In some embodiments, the tumor sample comprises formalin-fixed, paraffin-embedded (FFPE) tumor specimens, tissue sections, surgical biopsy, skin biopsy, punch biopsy, prostate biopsy, bone biopsy, bone marrow biopsy, needle biopsy, CT-guided biopsy, ultrasound-guided biopsy, fine needle aspiration, aspiration biopsy, fresh tissue or blood samples. In some embodiments, matched normal samples include matched tumor-free tissue (for example, biopsy) or saliva or blood specimens. In some embodiments, the tumor sample comprises a somatic specimen. In some embodiments, the normal or tumor-free sample comprises a germline specimen. In some embodiments, the sample is not a fine needle aspirate sample.

The methods may be used by clinicians, e.g., to validate specific clinical decisions, e.g., when used in conjunction with established ICI biomarkers and clinicopathologic features for cancers with and without ICI indications. The disclosed methods may be leveraged to identify targetable populations and therapeutic strategies or as an IVD/CDx (in vitro diagnostic/companion diagnostic). The disclosed methods may be implemented in a clinical trial to validate for clinical use. The disclosed methods may be used to design or modify schedules for radiological examination of the subject.

The model components could be, in some embodiments, determined from sources other than sequencing data, e.g., IHC (i.e., protein) data could be used as an input, histology, e.g., hematoxylin and eosin stained sections (H&E) data could be used as an input. H&E stained samples could be used to impute RNA or TMB then use the imputed values of those as input to determine the models, e.g., to extract the elements that make up the models, e.g., expression values.

## Immune exhaustion signature

The inventors discovered an immune exhaustion signature (IES) that is negatively associated with response to immune oncology (IO) therapy, e.g., ICI therapy. The IES may comprise of one or more genes selected from TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, SLC38A5, TIFA, DOK2, PPP1R2, DMAC1, DNAJB1, TAGAP, GZMA, CD27, GADD45A, HSPH1, STMN1, GZMH, CLIC3, GLIPR1, CHORDC1, CD3E, CD69, BAG3, ATF3, MICB, TRBC2, EZR, ARHGDIB, CASC8, ITM2A, DDX24, CD52, RAC2, TERF2IP, ELF1, FAM96B, GGH, NKG7, LY6E, CITED2, ZFAND2A, SAMSN1, CST7, CDKN3, TCEAL3, BBC3, IL32, MBD4, DNAJA4, TMEM141, UBB, HCST, IGLV1-40, HOPX, RHOH, USB1, H2AFZ, CSRP1, IKZF1, RGS2, IGLC2, CCND2, SELPLG, FUNDC2, IGFBP7, IGKV3-15, SERPINE2, TRDMT1, RGS1, HMOX1, HSP90AB1, HSPA1A, LIME1, TUBB, MRPL10, IFI44L, COTL1, LBH, ZEB2, HMGB2, LDHA, LGALS3, CYLD, PXMP2, CD74, PPIH, CD8A, RFX2, KLRD1, KLF6, LINC02446, HTRA1, TUBA4A, HSPB1, DNAJA1, CD3D, DUSP2, ELL2, TPM1, CKS1B, LGALS1, BEX3, GLRX, CCL4, GBP5, PTPRC, CLK1, IRF4, PIM2, SAT1, CXCR3, ZFP36, CD24, PELI1, CKS2, GYPC, FOXN2, IGLV1-51, IFT46, IGLV1-41, PLA2G16, COMMD8, IPCEF1, SMPDL3B, EVL, EVI2B, RAB11FIP1, DUSP5, HAVCR2, UBC, CRIP1, SRPRB, SERPINA1, PCSK7, BCL2L11, HSPA6, CWC25, CORO1A, TPST2, MBNL2, CKB, TUBA1B, GABARAPL1, PXDC1, SEL1L, PPP1R8, FKBP4, GABARAPL2, JCHAIN, STK17B, ZWINT, CHMP1B, ID2, HERPUD1, ROCK1, SKAP1, S100A4, CXCL10, CASP3, APOC1, ARID5B, SMAP2, CSRNP1, ADIRF, HLA-DPA1, PPP1R15A, DMKN, SCAF4, MYL9, LYAR, ZBTB25, GADD45B, GCHFR, LINC01588, RAB20, LSP1, FCGR2B, HIST2H2AA4, NCF4, LCK, IGHV3-33, LAPTM5, TUBB4B, TPM2, RBM38, RBP4, CCNA2, SERTAD1, ITM2C, PLPP5, DNAJB9, SYNGR2, TUBB2A, ERLEC1, TMED9, IFI6, HSP90AA1, PTPN1, TTL, DKK1, TM2D3, DCAF11, RIC1, SERPING1, DERL3, KDELR3, GEM, KLF9, TYROBP, CERCAM, CCDC84, ODC1, CYP2C9, CFLAR, HLA-DMB, DUSP1, JSRP1, TRIB1, JUN, NFATC2, EMP3, SNRNP70, TMED5, ST8SIA4, IGLV3-1, ZNF394, TNFSF9, CTSW, CUL1, BACH1, RABL3, KPNA2, EPS8L3, IER5, HSPA1B, CADM1, MCL1, RNF19A, ITGA4, CD38, WIPI1, CENPK, HCLS1, SPICE1, HIST1H2BC, MPRIP, FOSB, SERPINB8, FAM126A, CEP55, ATXN1, VCL, SOCS1, PCNX1, SQOR, JUNB, C10orf90, LCP1, STRADB, CREB3L2, GNG7, CCNH, SNX2, IGSF1, CCNL1, FKBP11, DBF4, ICAM1, MAD2L1, TMEM176B, PAIP2B, CD79A, SRXN1, NOB1, IER2, HLA-DRA, ZFP36L1, MZB1, MAGEA4, JUND, CD8B, AARS, TXNDC15, AC016831.7, GNA15, ATM, TSC22D1, GZMK, RAC3, ZNF263, TNFAIP3, H1FX, FGG, FHL2, MBNL1, TMEM205, IGLV6-57, CD96, TUBA1C, UCHL1, PRDM1, SRPK2, NUP37, TMEM87A, THEMIS2, HSPA5, PCMT1, TUBA1A, IGHG1, ANKRD37, MEF2C, XRN1, POU2AF1, BCL6, INAFM1, ADH4, TGFB1I1, PBK, DCN, FCRL5, DNAJB4, HLA-DQA1, TBC1D23, TMEM39A, GCC2, TMEM192, IGHA1, PTHLH, MFAP5, GEMIN6, BIRC3, IGHV4-4, SLC6A6, CYP2R1, HLA-DRB1, PPP1R15B, HMCES, MYC, WISP2, CHN1, ILK, PXN-AS1, LINC01970, CRIP2, PCOLCE2, MTMR6, EDIL3, AGR2, MEF2B, PFKM, KIAA1671, GLIPR2, SSTR2, SERPINB9, HIST1H1E, PTTG1, WSB1, ERN1, Z93241.1, IGLV1-44, SDS, TLE1, NUPR1, IGLV1-47, ICAM2, NXF1, RSPO3, TCF4, AC243960.1, RARRES2, RMDN3, RBFOX2, SEC11C, OLMALINC, FADS2, ITPRIP, FOS, SFTPD, HAUS3, RNF43, HIST1H4C, TIGAR, BIK, ITGA1, TARSL2, AFP, SNORC, MKLN1, BTG2, KRT18, NOC2L, ZFP36L2, NFKBIA, RHOB, HMGA1, BRD3, IGHJ6, U62317.5, SLC2A3, AC034231.1, CLEC11A, EPCAM, SKI, PNOC, MIR155HG, C12orf75, SAMHD1, IGKV3D-15, ACTN1, GSTZ1, TUBB3, CAV1, OAT, COBLL1, SSR4, ACTA2, HBA1, FAM83D, PLA2G2A, RAB14, AC106791.1, RAB23, AC244090.1, KMT5A, SERPINB1, P3H2, XRCC1, AC106782.1, MAL2, EGR1, F8, PLIN2, SOWAHC, IGFBP6, NFKBIZ, XBP1, SLC25A51, IGHM, KCTD5, USP38, FCER1G, PHLDA1, BYSL, HLA-DRB5, RAPH1, DUSP23, FUOM, ISYNA1, TNK2, STAP2, SLC25A4, GALNT2, SGO2, FHL3, ALB, CYP20A1, TM4SF1, ADA, RRP9, DNAH14, BOLA2, BHLHE41, CCL20, AC005537.1, UBALD2, VGLL4, NUDT1, USP10, ADSSL1, PRSS23, FMC1, ARHGAP45, HSPA14-1, CREB5, RBM33, TMX4, ROCK2, ARSK, PALLD, FNDC3B, FOXA3, BATF, PTP4A3, CDC45, IGHV1-2, IMMP2L, STARD10, HIST2H2BF, MTG2, FBXO8, USP32, ADIPOR2, RRM2, DHODH, DDIT4, NFAT5, PPARG, YTHDF3-AS1, GNG4, CSPP1, UBE2S, ZNF473, TIMP1, CPQ, AOC2, H1F0, JRK, EXOSC9, AC012236.1, AC009403.1, C12orf65, AURKA, MYH9, IGKV4-1, IGHMBP2, JADE1, HIST1H3C, TTC39A, SGMS1, LBP, FRYL, DNAJB2, GNG11, HAGHL, ANXA6, MARS, ADD1, KDM4B, TMEM91, AC008915.2, CXCL14, DUSP14, GJB2, PGM1, ETS2, GNPDA1, COL18A1, KLF10, MT1A, TPX2, S100A2, MAP3K5, HIST1H2AE, SLC20A2, ITGB7, SCEL, RSRP1, AKR1B1, GINS1, ZNF296, ALKBH4, UBE2C, ANKRD36C, SULT2B1, SMC5, TSPYL2, TNS4, TIMP3, ID4, SDC1, COX18, CDC42EP2, SQLE, ZNRF1, AKR1B10, NDC80, GFPT2, MAP1B, HIST1H2AG, IDO1, RNF185, UHRF1BP1, ADORA2B, CALD1, PHLDA2, ADH6, TFAP2A, DLG1, MELK, CBWD3, RAB4B, KANSL1L, RCE1, HIST1H2AC, CDK1, TCIM, C17orf67, BRD4, LY6E-DT, SLC1A6, ARL13B, IRF1, DDX3X, RAB2B, MYBBP1A, ARFGAP1, BOP1, IGKV3D-7, KMT2E-AS1, DTNBP1, LAMC2, ATG4C, MYBL2, LRP10, PALMD, ZBTB4, SYTL2, SERPINH1, CD248, CNEP1R1, FURIN, IGLL5, MEST, MDK, NUP205, NRDE2, ECT2, TENT5A, TNKS1BP1, NFXL1, SLC35E3, ECE1, RASD1, SLC52A2, DCBLD2, CP, POLE, COL27A1, SBNO1, SLC7A6, HYKK, SLPI, CFHR1, SPDEF, DACT2, TUBGCP5, AREG, HIST1H2AJ, KIF2A, AL135925.1, NOTCH3, SLC11A1, HEXIM2, IGFBP1, TVP23A, NUDT14, SAMD11, MIR200CHG, PCLAF, SLC43A3, FAM30A, PHRF1, ADM, SIK2, NUSAP1, CFH, KRTCAP3, SPAG4, TPPP3, TSPAN4, AAK1, CST1, CLU, IFRD1, ASPHD2, CNN3, COL4A1, FGA, ANO6, SBSN, FGB, ATP9B, NLGN4Y, HP, EPS8, RNF111, LINC01285, MAOA, IGHV4-31, TNFRSF10D, GSR, IGHGP, TACSTD2, MT1F, RHCG, MUT, PI3, MT1M, LAMB3, MTRNR2L12, SLC35A2, DDX10, RARRES1, MTSS1, CLK2, RPN2, MED29, CYP1B1, TTTY14, DMXL1, AL139246.5, TAF1, DAAM1, MYO1E, MAFB, CDKN1A, F8A3, FABP5, CFB, HSP90B1, SGK3, HMG20B, CDCA5, CLDN4, SYNM, PAWR, TWNK, AC116049.2, RND3, ATP11A, PID1, MALAT1, TMEM168, TFF1, TFRC, RNASET2, SPINK13, PABPC1L, P4HA2, PRSS8, SPINT1, MSC, FMNL1, SLC8B1, UNC13D, SPINT2, DCP1A, NPTN, IGKV3D-11, G6PD, KRT6A, LYPD1, TESC, COL4A2, ELF3, BCAM, AC093323.1, IGHV1-69, LINC00511, PORCN, TPRG1-AS1, EFNB2, PARD6G-AS1, CD9, RGS16, IL6R, FZD3, GLYR1, B3GALT6, LRCH3, MAFK, LINC00491, MT1X, MUC6, PIK3R3, GBP4, PERP, LXN, ZBTB7A, WARS, AC020911.2, MAPK3, ALS2CL, MRE11, TSPAN17, IGHV4-34, IL33, ADAM9, ANGPTL4, TBC1D31, C1R, CTSC, SLC35A4, FST, SGO1, ANKRD36, IGHG3, SLC15A3, HES1, POLR1E, SLC7A5, CAPN12, IGFBP3, FBXO38, FLNA, CSKMT, OAS1, ULK1, PBX1, EXOC4, REEP6, HILPDA, ASF1B, FKBP1B, IL6, CALU, AKR1C1, KLF2, GRTP1, C1S, SMOX, CPLX2, LMNA, BSG, IGHG4, SVIL, HIST1H1B, GCH1, NEAT1, FN1, ESRP1, RFWD3, ADGRE2, SPINK6, HPD, CAVIN1, MT1E, CLDN10, C15orf48, CA9, NR4A1, PPP1R3B, SLC30A1, SLC7A11, VIRMA, NAA25, CCNB1, CFD, AP1G1, H6PD, PSCA, KCNK6, AL161431.1, DVL1, HIST1H2AM, RAB31, CDCA3, SPATA20, PRMT7, PTGR1, SERINC2, IGHG2, GFPT1, TTC22, BTBD1, HIST1H4H, CENPB, ZNF598, GPATCH2L, SPTLC3, CXCL2, CYP24A1, EZH2, GPX2, LMNB2, PTGES, MGLL, NR2F2, KRT19, DNTTIP1, MUC5AC, SDCBP2, IL1R2, AHNAK2, MUC16, AC023090.1, CPE, VNN1, BAMBI, NPW, TK1, IGKV3D-20, ANKRD11, CDC20, CDH1, STK11, IGKC, SLC45A4, TBC1D8, CSTA, AC233755.1, MIGA1, HIST1H2AL, AKAP12, MAP4K4, HOOK2, GGA3, COL7A1, NOS1, ARHGAP26, AKR1C2, TGM2, CENPF, IGHV3-48, CDCA8, TSC2, STC2, PKN3, PVR, CES1, GPRC5A, SEZ6L2, CEP170B, KIF14, IER3, ALDH3B1, TOP2A, SPP1, TXNRD1, LENG8, TRIM15, ALDH3A1, RIMKLB, HECTD4, SMOC1, NEB, RMRP, IGFBP4, MT1G, SCRIB, ERO1A, SOX4, LMO7, RNPEPL1, PLK2, COL6A2, FLRT3, IGHV4-28, SCD, KRT7, PIEZO1, CXCL1, DAPK1, ID1, C3, CXCL3, IGKV3-20, GUCA2B, ITGA3, SFN, IGLV3-21, PLEC, POLR2A, AGRN, MUC1, SERPINB3, S100A8, LAMA5, COL6A1, ITGB4, S100P, SLURP2, MSLN, KRT17, and MUC5B.

Table 1 lists 985 genes which may make up the IES in any combination; however, the IES may comprise 1-985, or any number in between 1 and 985 of the genes listed in Table 1 and may further comprise the weights corresponding to the genes listed in Table 1. The IES may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 1, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 1, which are listed by ascending score, the top genes having the most negative value. The IES may comprise expression values for each of the genes listed in Table 1. The IES may consist of expression values for each of the genes listed in Table 1.

Therefore, in some embodiments, the methods comprise at a computer system having one or more processors, and memory storing one or more programs for execution by the one or more processors: applying, by the one or more processors, one or more model components derived from sequencing data from a sample to one or more machine learning algorithms (MLAs), wherein the sequencing data comprises RNA sequencing data and DNA sequencing data, wherein the one or more model components comprise an immune exhaustion signature.

Classification models, such as regularized logistic regression or support vector machines (SVM), can be used to predict progression within a particular time interval after the initiation of an immunotherapy regimen.

Survival models, such as Cox Proportional-Hazards and survival SVMs, can be used to predict the progression free survival, overall survival or time to progression after the initiation of an immunotherapy regimen.

In some embodiments, the systems and methods include an IO Progression Risk predictor that uses outputs generated from two laboratory developed tests (LDTs): a targeted panel DNA sequencing assay (for example, targeting approximately 650 genes) and a whole exome capture RNA sequencing (RNA-seq) assay.

**Table 1. Immune exhaustion signature biomarkers**

| hgnc_symbol (gene) | ensembl_gene_id | weight |
|---|---|---|
| TMSB4X | ENSG00000205542 | -0.8290406 |
| CCL5 | ENSG00000161570 | -0.7483824 |
| TSC22D3 | ENSG00000157514 | -0.6780022 |
| CYTOR | CYTOR | -0.6561841 |
| CXCL13 | ENSG00000156234 | -0.6500419 |
| TXNIP | ENSG00000117289 | -0.6461806 |
| PTPRCAP | ENSG00000213402 | -0.6068093 |
| RGCC | ENSG00000102760 | -0.6012076 |
| IGLC3 | IGLC3 | -0.5772579 |
| CYTIP | ENSG00000115165 | -0.5697271 |
| IGHV1-69D | IGHV1-69D | -0.5619589 |
| CXCR4 | ENSG00000121966 | -0.5381885 |
| HMGN2 | ENSG00000198830 | -0.5348123 |
| HSPD1 | ENSG00000144381 | -0.5272772 |
| NEU1 | ENSG00000204386 | -0.5259638 |
| TPD52 | ENSG00000076554 | -0.5239793 |
| GZMB | ENSG00000100453 | -0.5178311 |
| PIM1 | ENSG00000137193 | -0.5161117 |
| SRGN | ENSG00000122862 | -0.5118545 |
| BST2 | ENSG00000130303 | -0.5105286 |
| PDE4B | ENSG00000184588 | -0.5094234 |
| HSPA8 | ENSG00000109971 | -0.503791 |
| PRF1 | ENSG00000180644 | -0.4960389 |
| CD7 | ENSG00000173762 | -0.4885356 |
| SLC38A5 | ENSG00000017483 | -0.4866104 |
| TIFA | ENSG00000145365 | -0.4858798 |

(continued)

| hgnc_symbol (gene) | ensembl_gene_id | weight |
|---|---|---|
| DOK2 | ENSG00000147443 | -0.4830895 |
| PPP1R2 | ENSG00000184203 | -0.4779025 |
| DMAC1 | DMAC1 | -0.4747319 |
| DNAJB1 | ENSG00000132002 | -0.4739487 |
| TAGAP | ENSG00000164691 | -0.4707387 |
| GZMA | ENSG00000145649 | -0.4562186 |
| CD27 | ENSG00000139193 | -0.4560648 |
| GADD45A | ENSG00000116717 | -0.4557478 |
| HSPH1 | ENSG00000120694 | -0.4556826 |
| STMN1 | ENSG00000117632 | -0.4543601 |
| GZMH | ENSG00000100450 | -0.4493743 |
| CLIC3 | ENSG00000169583 | -0.4460661 |
| GLIPR1 | ENSG00000139278 | -0.4457882 |
| CHORDC1 | ENSG00000110172 | -0.4422807 |
| CD3E | ENSG00000198851 | -0.4404029 |
| CD69 | ENSG00000110848 | -0.4396038 |
| BAG3 | ENSG00000151929 | -0.432024 |
| ATF3 | ENSG00000162772 | -0.430873 |
| MICB | ENSG00000204516 | -0.4305996 |
| TRBC2 | TRBC2 | -0.4292696 |
| EZR | ENSG00000092820 | -0.4255834 |
| ARHGDIB | ENSG00000111348 | -0.4249911 |
| CASC8 | CASC8 | -0.4208658 |
| ITM2A | ENSG00000078596 | -0.4193023 |
| DDX24 | ENSG00000089737 | -0.4189241 |
| CD52 | ENSG00000169442 | -0.4170264 |
| RAC2 | ENSG00000128340 | -0.415641 |
| TERF2IP | ENSG00000166848 | -0.415127 |
| ELF1 | ENSG00000120690 | -0.4107733 |
| FAM96B | ENSG00000166595 | -0.4099359 |
| GGH | ENSG00000137563 | -0.408704 |
| NKG7 | ENSG00000105374 | -0.406886 |
| LY6E | ENSG00000160932 | -0.4065791 |
| CITED2 | ENSG00000164442 | -0.4052196 |
| ZFAND2A | ENSG00000178381 | -0.4034172 |
| SAMSN1 | ENSG00000155307 | -0.4032015 |
| CST7 | ENSG00000077984 | -0.4031606 |
| CDKN3 | ENSG00000100526 | -0.4005753 |
| TCEAL3 | ENSG00000196507 | -0.3974166 |

(continued)

| hgnc_symbol (gene) | ensembl_gene_id | weight |
|---|---|---|
| BBC3 | ENSG00000105327 | -0.3904396 |
| IL32 | ENSG00000008517 | -0.390281 |
| MBD4 | ENSG00000129071 | -0.3897499 |
| DNAJA4 | ENSG00000140403 | -0.3886274 |
| TMEM141 | ENSG00000244187 | -0.3874071 |
| UBB | ENSG00000170315 | -0.3862517 |
| HCST | ENSG00000126264 | -0.3838442 |
| IGLV1-40 | IGLV1-40 | -0.3808413 |
| HOPX | ENSG00000171476 | -0.3801608 |
| RHOH | ENSG00000168421 | -0.3787045 |
| USB1 | ENSG00000103005 | -0.3764477 |
| H2AFZ | ENSG00000164032 | -0.3764411 |
| CSRP1 | ENSG00000159176 | -0.3752883 |
| IKZF1 | ENSG00000185811 | -0.3749183 |
| RGS2 | ENSG00000116741 | -0.3726402 |
| IGLC2 | IGLC2 | -0.3694022 |
| CCND2 | ENSG00000118971 | -0.3692821 |
| SELPLG | ENSG00000110876 | -0.3682601 |
| FUNDC2 | ENSG00000165775 | -0.3675725 |
| IGFBP7 | ENSG00000163453 | -0.3670408 |
| IGKV3-15 | IGKV3-15 | -0.3652932 |
| SERPINE2 | ENSG00000135919 | -0.3637573 |
| TRDMT1 | ENSG00000107614 | -0.3579596 |
| RGS1 | ENSG00000090104 | -0.3558943 |
| HMOX1 | ENSG00000100292 | -0.354847 |
| HSP90AB1 | ENSG00000096384 | -0.3542926 |
| HSPA1A | ENSG00000204389 | -0.3491537 |
| LIME1 | ENSG00000203896 | -0.3490153 |
| TUBB | ENSG00000196230 | -0.348567 |
| MRPL10 | ENSG00000159111 | -0.3475924 |
| IFI44L | ENSG00000137959 | -0.3464634 |
| COTL1 | ENSG00000103187 | -0.3392989 |
| LBH | ENSG00000213626 | -0.3389645 |
| ZEB2 | ENSG00000169554 | -0.3327727 |
| HMGB2 | ENSG00000164104 | -0.3317196 |
| LDHA | ENSG00000134333 | -0.3301483 |
| LGALS3 | ENSG00000131981 | -0.3288205 |
| CYLD | ENSG00000083799 | -0.3274298 |
| PXMP2 | ENSG00000176894 | -0.327215 |

(continued)

| hgnc_symbol (gene) | ensembl_gene_id | weight |
|---|---|---|
| CD74 | ENSG00000019582 | -0.3251709 |
| PPIH | ENSG00000171960 | -0.3246344 |
| CD8A | ENSG00000153563 | -0.3238892 |
| RFX2 | ENSG00000087903 | -0.3237332 |
| KLRD1 | ENSG00000134539 | -0.3233311 |
| KLF6 | ENSG00000067082 | -0.3217616 |
| LINC02446 | LINC02446 | -0.3183214 |
| HTRA1 | ENSG00000166033 | -0.3180989 |
| TUBA4A | ENSG00000127824 | -0.3169468 |
| HSPB1 | ENSG00000106211 | -0.3162788 |
| DNAJA1 | ENSG00000086061 | -0.313121 |
| CD3D | ENSG00000167286 | -0.308495 |
| DUSP2 | ENSG00000158050 | -0.3069069 |
| ELL2 | ENSG00000118985 | -0.3060271 |
| TPM1 | ENSG00000140416 | -0.3058286 |
| CKS1B | ENSG00000173207 | -0.3026863 |
| LGALS1 | ENSG00000100097 | -0.2993232 |
| BEX3 | BEX3 | -0.2925122 |
| GLRX | ENSG00000173221 | -0.2916881 |
| CCL4 | ENSG00000129277 | -0.2912327 |
| GBP5 | ENSG00000154451 | -0.286062 |
| PTPRC | ENSG00000081237 | -0.2834517 |
| CLK1 | ENSG00000013441 | -0.2830338 |
| IRF4 | ENSG00000137265 | -0.2824847 |
| PIM2 | ENSG00000102096 | -0.2800731 |
| SAT1 | ENSG00000130066 | -0.2799943 |
| CXCR3 | ENSG00000186810 | -0.2798519 |
| ZFP36 | ENSG00000128016 | -0.279523 |
| CD24 | CD24 | -0.2789109 |
| PELI1 | ENSG00000197329 | -0.27777 |
| CKS2 | ENSG00000123975 | -0.2775129 |
| GYPC | ENSG00000136732 | -0.2774506 |
| FOXN2 | ENSG00000170802 | -0.2770045 |
| IGLV1-51 | IGLV1-51 | -0.276842 |
| IFT46 | ENSG00000118096 | -0.2744548 |
| IGLV1-41 | IGLV1-41 | -0.2740068 |
| PLA2G16 | ENSG00000176485 | -0.2691798 |
| COMMD8 | ENSG00000169019 | -0.2691174 |
| IPCEF1 | ENSG00000074706 | -0.265221 |

(continued)

| hgnc_symbol (gene) | ensembl_gene_id | weight |
|---|---|---|
| SMPDL3B | ENSG00000130768 | -0.2642688 |
| EVL | ENSG00000196405 | -0.2635641 |
| EVI2B | ENSG00000185862 | -0.262028 |
| RAB11FIP1 | ENSG00000156675 | -0.2619652 |
| DUSP5 | ENSG00000138166 | -0.2608611 |
| HAVCR2 | ENSG00000135077 | -0.2600957 |
| UBC | ENSG00000150991 | -0.2597783 |
| CRIP1 | ENSG00000213145 | -0.2595268 |
| SRPRB | ENSG00000144867 | -0.2583287 |
| SERPINA1 | ENSG00000197249 | -0.2579104 |
| PCSK7 | ENSG00000160613 | -0.2573958 |
| BCL2L11 | ENSG00000153094 | -0.2566683 |
| HSPA6 | ENSG00000173110 | -0.2556002 |
| CWC25 | ENSG00000108296 | -0.2547722 |
| CORO1A | ENSG00000102879 | -0.2542663 |
| TPST2 | ENSG00000128294 | -0.2518063 |
| MBNL2 | ENSG00000139793 | -0.2510812 |
| CKB | ENSG00000166165 | -0.2506911 |
| TUBA1B | ENSG00000123416 | -0.2500131 |
| GABARAPL1 | ENSG00000139112 | -0.2499451 |
| PXDC1 | ENSG00000168994 | -0.2497275 |
| SEL1L | ENSG00000071537 | -0.2467196 |
| PPP1R8 | ENSG00000117751 | -0.2451707 |
| FKBP4 | ENSG00000004478 | -0.2442843 |
| GABARAPL2 | ENSG00000034713 | -0.2430466 |
| JCHAIN | JCHAIN | -0.2429324 |
| STK17B | ENSG00000081320 | -0.2429287 |
| ZWINT | ENSG00000122952 | -0.2427671 |
| CHMP1B | CHMP1B | -0.2423414 |
| ID2 | ENSG00000115738 | -0.2418112 |
| HERPUD1 | ENSG00000051108 | -0.2414653 |
| ROCK1 | ENSG00000067900 | -0.2404299 |
| SKAP1 | ENSG00000141293 | -0.2401503 |
| S100A4 | ENSG00000196154 | -0.2401321 |
| CXCL10 | ENSG00000169245 | -0.2393407 |
| CASP3 | ENSG00000164305 | -0.2378334 |
| APOC1 | ENSG00000130208 | -0.2365321 |
| ARID5B | ENSG00000150347 | -0.2357791 |
| SMAP2 | ENSG00000084070 | -0.2353876 |

(continued)

| hgnc_symbol (gene) | ensembl_gene_id | weight |
|---|---|---|
| CSRNP1 | ENSG00000144655 | -0.2348078 |
| ADIRF | ENSG00000148671 | -0.2340451 |
| HLA-DPA1 | ENSG00000231389 | -0.2337188 |
| PPP1R15A | ENSG00000087074 | -0.2332392 |
| DMKN | ENSG00000161249 | -0.2319452 |
| SCAF4 | ENSG00000156304 | -0.231515 |
| MYL9 | ENSG00000101335 | -0.2307233 |
| LYAR | ENSG00000145220 | -0.2303914 |
| ZBTB25 | ENSG00000089775 | -0.2302441 |
| GADD45B | ENSG00000099860 | -0.2301164 |
| GCHFR | ENSG00000137880 | -0.2297791 |
| LINC01588 | LINC01588 | -0.2272209 |
| RAB20 | ENSG00000139832 | -0.2267677 |
| LSP1 | ENSG00000130592 | -0.2255597 |
| FCGR2B | ENSG00000072694 | -0.2223128 |
| HIST2H2AA4 | ENSG00000203812 | -0.2208335 |
| NCF4 | ENSG00000100365 | -0.2204469 |
| LCK | ENSG00000182866 | -0.2199268 |
| IGHV3-33 | IGHV3-33 | -0.2185143 |
| LAPTM5 | ENSG00000162511 | -0.2182056 |
| TUBB4B | ENSG00000188229 | -0.2176445 |
| TPM2 | ENSG00000198467 | -0.2161625 |
| RBM38 | ENSG00000132819 | -0.2155132 |
| RBP4 | ENSG00000138207 | -0.2151345 |
| CCNA2 | ENSG00000145386 | -0.2145531 |
| SERTAD1 | ENSG00000197019 | -0.2133368 |
| ITM2C | ENSG00000135916 | -0.2127687 |
| PLPP5 | PLPP5 | -0.2110864 |
| DNAJB9 | ENSG00000128590 | -0.2090008 |
| SYNGR2 | ENSG00000108639 | -0.208865 |
| TUBB2A | ENSG00000137267 | -0.2082945 |
| ERLEC1 | ENSG00000068912 | -0.2070148 |
| TMED9 | ENSG00000184840 | -0.2051139 |
| IFI6 | ENSG00000126709 | -0.2046423 |
| HSP90AA1 | ENSG00000080824 | -0.2038517 |
| PTPN1 | ENSG00000196396 | -0.2013339 |
| TTL | ENSG00000114999 | -0.2013168 |
| DKK1 | ENSG00000107984 | -0.1996319 |
| TM2D3 | ENSG00000184277 | -0.1983668 |

(continued)

| hgnc_symbol (gene) | ensembl_gene_id | weight |
|---|---|---|
| DCAF11 | ENSG00000100897 | -0.1981003 |
| RIC1 | RIC1 | -0.1971229 |
| SERPING1 | ENSG00000149131 | -0.1950058 |
| DERL3 | ENSG00000099958 | -0.1947924 |
| KDELR3 | ENSG00000100196 | -0.1944155 |
| GEM | ENSG00000164949 | -0.1931934 |
| KLF9 | ENSG00000119138 | -0.1922351 |
| TYROBP | ENSG00000011600 | -0.1919778 |
| CERCAM | ENSG00000167123 | -0.1911665 |
| CCDC84 | ENSG00000186166 | -0.1909595 |
| ODC1 | ENSG00000115758 | -0.1877338 |
| CYP2C9 | ENSG00000138109 | -0.187254 |
| CFLAR | ENSG00000003402 | -0.1852216 |
| HLA-DMB | ENSG00000242574 | -0.1851799 |
| DUSP1 | ENSG00000120129 | -0.1850796 |
| JSRP1 | ENSG00000167476 | -0.1840335 |
| TRIB1 | ENSG00000173334 | -0.1834214 |
| JUN | ENSG00000177606 | -0.1830259 |
| NFATC2 | ENSG00000101096 | -0.1826242 |
| EMP3 | ENSG00000142227 | -0.1814376 |
| SNRNP70 | ENSG00000104852 | -0.1814164 |
| TMED5 | ENSG00000117500 | -0.1797061 |
| ST8SIA4 | ENSG00000113532 | -0.1774501 |
| IGLV3-1 | IGLV3-1 | -0.1762711 |
| ZNF394 | ENSG00000160908 | -0.1761781 |
| TNFSF9 | ENSG00000125657 | -0.175163 |
| CTSW | ENSG00000172543 | -0.1744902 |
| CUL1 | ENSG00000055130 | -0.1742351 |
| BACH1 | ENSG00000156273 | -0.1742087 |
| RABL3 | ENSG00000144840 | -0.1741873 |
| KPNA2 | ENSG00000182481 | -0.1732765 |
| EPS8L3 | ENSG00000198758 | -0.1732098 |
| IER5 | ENSG00000162783 | -0.1720591 |
| HSPA1B | ENSG00000204388 | -0.1702319 |
| CADM1 | ENSG00000182985 | -0.1698262 |
| MCL1 | ENSG00000143384 | -0.1674024 |
| RNF19A | ENSG00000034677 | -0.1653651 |
| ITGA4 | ENSG00000115232 | -0.1648511 |
| CD38 | ENSG00000004468 | -0.1632596 |

(continued)

| hgnc_symbol (gene) | ensembl_gene_id | weight |
|---|---|---|
| WIPI1 | ENSG00000070540 | -0.162337 |
| CENPK | ENSG00000123219 | -0.1622388 |
| HCLS1 | ENSG00000180353 | -0.1620898 |
| SPICE1 | ENSG00000163611 | -0.1620307 |
| HIST1H2BC | ENSG00000180596 | -0.1609839 |
| MPRIP | ENSG00000133030 | -0.1605812 |
| FOSB | ENSG00000125740 | -0.1597354 |
| SERPINB8 | ENSG00000166401 | -0.156178 |
| FAM126A | ENSG00000122591 | -0.1556618 |
| CEP55 | ENSG00000138180 | -0.1551316 |
| ATXN1 | ENSG00000124788 | -0.1542545 |
| VCL | ENSG00000035403 | -0.1538892 |
| SOCS1 | ENSG00000185338 | -0.1531732 |
| PCNX1 | PCNX1 | -0.1522917 |
| SQOR | SQOR | -0.1520147 |
| JUNB | ENSG00000171223 | -0.1515677 |
| C10orf90 | ENSG00000154493 | -0.1512549 |
| LCP1 | ENSG00000136167 | -0.1511265 |
| STRADB | ENSG00000082146 | -0.1509434 |
| CREB3L2 | ENSG00000182158 | -0.1504384 |
| GNG7 | ENSG00000176533 | -0.1499603 |
| CCNH | ENSG00000134480 | -0.1499527 |
| SNX2 | ENSG00000205302 | -0.149771 |
| IGSF1 | ENSG00000147255 | -0.1478828 |
| CCNL1 | ENSG00000163660 | -0.1463949 |
| FKBP11 | ENSG00000134285 | -0.1451441 |
| DBF4 | ENSG00000006634 | -0.1449207 |
| ICAM1 | ENSG00000090339 | -0.1428717 |
| MAD2L1 | ENSG00000164109 | -0.1427837 |
| TMEM176B | ENSG00000106565 | -0.1422 |
| PAIP2B | ENSG00000124374 | -0.14076 |
| CD79A | ENSG00000105369 | -0.1400287 |
| SRXN1 | ENSG00000271303 | -0.1394223 |
| NOB1 | ENSG00000141101 | -0.1387885 |
| IER2 | ENSG00000160888 | -0.1382321 |
| HLA-DRA | ENSG00000204287 | -0.1375092 |
| ZFP36L1 | ENSG00000185650 | -0.1368896 |
| MZB1 | ENSG00000170476 | -0.1367876 |
| MAGEA4 | ENSG00000147381 | -0.136779 |

(continued)

| hgnc_symbol (gene) | ensembl_gene_id | weight |
|---|---|---|
| JUND | ENSG00000130522 | -0.1361241 |
| CD8B | ENSG00000172116 | -0.1359972 |
| AARS | ENSG00000090861 | -0.1356492 |
| TXNDC15 | ENSG00000113621 | -0.13562 |
| AC016831.7 | AC016831.7 | -0.1352706 |
| GNA15 | ENSG00000060558 | -0.1340825 |
| ATM | ENSG00000149311 | -0.1325106 |
| TSC22D1 | ENSG00000102804 | -0.1305006 |
| GZMK | ENSG00000113088 | -0.1295298 |
| RAC3 | ENSG00000169750 | -0.1284718 |
| ZNF263 | ENSG00000006194 | -0.1284553 |
| TNFAIP3 | ENSG00000118503 | -0.1282892 |
| H1FX | ENSG00000184897 | -0.1277453 |
| FGG | ENSG00000171557 | -0.127668 |
| FHL2 | ENSG00000115641 | -0.1273976 |
| MBNL1 | ENSG00000152601 | -0.1272853 |
| TMEM205 | ENSG00000105518 | -0.1272013 |
| IGLV6-57 | IGLV6-57 | -0.1259549 |
| CD96 | ENSG00000153283 | -0.1251649 |
| TUBA1C | ENSG00000167553 | -0.1249284 |
| UCHL1 | ENSG00000154277 | -0.1240437 |
| PRDM1 | ENSG00000057657 | -0.1238668 |
| SRPK2 | ENSG00000135250 | -0.1237373 |
| NUP37 | ENSG00000075188 | -0.1234859 |
| TMEM87A | ENSG00000103978 | -0.122503 |
| THEMIS2 | ENSG00000130775 | -0.1223713 |
| HSPA5 | ENSG00000044574 | -0.1220345 |
| PCMT1 | ENSG00000120265 | -0.1217614 |
| TUBA1A | ENSG00000167552 | -0.1214192 |
| IGHG1 | IGHG1 | -0.1197335 |
| ANKRD37 | ENSG00000186352 | -0.1196659 |
| MEF2C | ENSG00000081189 | -0.1196321 |
| XRN1 | ENSG00000114127 | -0.1157327 |
| POU2AF1 | ENSG00000110777 | -0.1156388 |
| BCL6 | ENSG00000113916 | -0.1153908 |
| INAFM1 | INAFM1 | -0.1152006 |
| ADH4 | ENSG00000198099 | -0.1135076 |
| TGFB1I1 | ENSG00000140682 | -0.1133195 |
| PBK | ENSG00000168078 | -0.1131905 |

(continued)

| hgnc_symbol (gene) | ensembl_gene_id | weight |
|---|---|---|
| DCN | ENSG00000011465 | -0.112764 |
| FCRL5 | ENSG00000143297 | -0.11156 |
| DNAJB4 | ENSG00000162616 | -0.1087502 |
| HLA-DQA1 | ENSG00000196735 | -0.1086234 |
| TBC1D23 | ENSG00000036054 | -0.1079377 |
| TMEM39A | ENSG00000176142 | -0.1079061 |
| GCC2 | ENSG00000135968 | -0.1075803 |
| TMEM192 | ENSG00000170088 | -0.1061784 |
| IGHA1 | IGHA1 | -0.1056561 |
| PTHLH | ENSG00000087494 | -0.1049335 |
| MFAP5 | ENSG00000197614 | -0.1042597 |
| GEMIN6 | ENSG00000152147 | -0.1041941 |
| BIRC3 | ENSG00000023445 | -0.1032815 |
| IGHV4-4 | IGHV4-4 | -0.1030753 |
| SLC6A6 | ENSG00000131389 | -0.1028621 |
| CYP2R1 | ENSG00000186104 | -0.1024013 |
| HLA-DRB1 | ENSG00000196126 | -0.1022066 |
| PPP1R15B | ENSG00000158615 | -0.1019545 |
| HMCES | ENSG00000183624 | -0.1017539 |
| MYC | ENSG00000136997 | -0.1012028 |
| WISP2 | ENSG00000064205 | -0.1000957 |
| CHN1 | ENSG00000128656 | -0.0992322 |
| ILK | ENSG00000166333 | -0.0973891 |
| PXN-AS1 | PXN-AS1 | -0.0969511 |
| LINC01970 | LINC01970 | -0.0954792 |
| CRIP2 | ENSG00000182809 | -0.094992 |
| PCOLCE2 | ENSG00000163710 | -0.0949521 |
| MTMR6 | ENSG00000139505 | -0.0940945 |
| EDIL3 | ENSG00000164176 | -0.0912204 |
| AGR2 | ENSG00000106541 | -0.0911028 |
| MEF2B | ENSG00000213999 | -0.0908633 |
| PFKM | ENSG00000152556 | -0.0904552 |
| KIAA1671 | ENSG00000197077 | -0.0900812 |
| GLIPR2 | ENSG00000122694 | -0.0900675 |
| SSTR2 | ENSG00000180616 | -0.0900517 |
| SERPINB9 | ENSG00000170542 | -0.0875295 |
| HIST1H1E | ENSG00000168298 | -0.0873188 |
| PTTG1 | ENSG00000164611 | -0.0866534 |
| WSB1 | ENSG00000109046 | -0.0863943 |

(continued)

| hgnc_symbol (gene) | ensembl_gene_id | weight |
|---|---|---|
| ERN1 | ENSG00000178607 | -0.086269 |
| Z93241.1 | Z93241.1 | -0.0862512 |
| IGLV1-44 | IGLV1-44 | -0.0860696 |
| SDS | ENSG00000135094 | -0.0851688 |
| TLE1 | ENSG00000196781 | -0.083979 |
| NUPR1 | ENSG00000176046 | -0.0839728 |
| IGLV1-47 | IGLV1-47 | -0.0823827 |
| ICAM2 | ENSG00000108622 | -0.0823085 |
| NXF1 | ENSG00000162231 | -0.0811781 |
| RSPO3 | ENSG00000146374 | -0.0808593 |
| TCF4 | ENSG00000196628 | -0.0800312 |
| AC243960.1 | AC243960.1 | -0.079305 |
| RARRES2 | ENSG00000106538 | -0.0791681 |
| RMDN3 | ENSG00000137824 | -0.07866 |
| RBFOX2 | ENSG00000100320 | -0.0781518 |
| SEC11C | ENSG00000166562 | -0.0769648 |
| OLMALINC | OLMALINC | -0.0758656 |
| FADS2 | ENSG00000134824 | -0.0735793 |
| ITPRIP | ENSG00000148841 | -0.0728967 |
| FOS | ENSG00000170345 | -0.0723711 |
| SFTPD | ENSG00000133661 | -0.0718835 |
| HAUS3 | ENSG00000214367 | -0.0711247 |
| RNF43 | ENSG00000108375 | -0.0707523 |
| HIST1H4C | ENSG00000197061 | -0.0706203 |
| TIGAR | TIGAR | -0.0704414 |
| BIK | ENSG00000100290 | -0.0699677 |
| ITGA1 | ENSG00000213949 | -0.0694757 |
| TARSL2 | ENSG00000185418 | -0.068867 |
| AFP | ENSG00000081051 | -0.0686708 |
| SNORC | SNORC | -0.0685303 |
| MKLN1 | ENSG00000128585 | -0.0678051 |
| BTG2 | ENSG00000159388 | -0.067453 |
| KRT18 | ENSG00000111057 | -0.0673334 |
| NOC2L | ENSG00000188976 | -0.0672982 |
| ZFP36L2 | ENSG00000152518 | -0.0672711 |
| NFKBIA | ENSG00000100906 | -0.066907 |
| RHOB | ENSG00000143878 | -0.0667935 |
| HMGA1 | ENSG00000137309 | -0.0651953 |
| BRD3 | ENSG00000169925 | -0.0645345 |

(continued)

| hgnc_symbol (gene) | ensembl_gene_id | weight |
|---|---|---|
| IGHJ6 | IGHJ6 | -0.0642042 |
| U62317.5 | U62317.5 | -0.0636437 |
| SLC2A3 | ENSG00000059804 | -0.062742 |
| AC034231.1 | AC034231.1 | -0.0621546 |
| CLEC11A | ENSG00000105472 | -0.0617116 |
| EPCAM | ENSG00000119888 | -0.0614957 |
| SKI | ENSG00000157933 | -0.0613422 |
| PNOC | ENSG00000168081 | -0.0611905 |
| MIR155HG | ENSG00000234883 | -0.061095 |
| C12orf75 | ENSG00000235162 | -0.0610706 |
| SAMHD1 | ENSG00000101347 | -0.0610275 |
| IGKV3D-15 | IGKV3D-15 | -0.0599042 |
| ACTN1 | ENSG00000072110 | -0.0594803 |
| GSTZ1 | ENSG00000100577 | -0.0591872 |
| TUBB3 | ENSG00000258947 | -0.0567281 |
| CAV1 | ENSG00000105974 | -0.0551526 |
| OAT | ENSG00000065154 | -0.0549207 |
| COBLL1 | ENSG00000082438 | -0.0539482 |
| SSR4 | ENSG00000180879 | -0.0528114 |
| ACTA2 | ENSG00000107796 | -0.052349 |
| HBA1 | ENSG00000206172 | -0.052332 |
| FAM83D | ENSG00000101447 | -0.0521586 |
| PLA2G2A | ENSG00000188257 | -0.051089 |
| RAB14 | ENSG00000119396 | -0.0508289 |
| AC106791.1 | AC106791.1 | -0.0497825 |
| RAB23 | ENSG00000112210 | -0.0493934 |
| AC244090.1 | AC244090.1 | -0.0491485 |
| KMT5A | KMT5A | -0.0489599 |
| SERPINB 1 | ENSG00000021355 | -0.0487341 |
| P3H2 | P3H2 | -0.0475112 |
| XRCC1 | ENSG00000073050 | -0.047304 |
| AC106782.1 | AC106782.1 | -0.0471665 |
| MAL2 | ENSG00000147676 | -0.046121 |
| EGR1 | ENSG00000120738 | -0.045691 |
| F8 | ENSG00000185010 | -0.0450744 |
| PLIN2 | ENSG00000147872 | -0.0449649 |
| SOWAHC | ENSG00000198142 | -0.0447953 |
| IGFBP6 | ENSG00000167779 | -0.0430421 |
| NFKBIZ | ENSG00000144802 | -0.0427261 |

(continued)

| hgnc_symbol (gene) | ensembl_gene_id | weight |
|---|---|---|
| XBP1 | ENSG00000100219 | -0.0393507 |
| SLC25A51 | ENSG00000122696 | -0.0383585 |
| IGHM | IGHM | -0.0383192 |
| KCTD5 | ENSG00000167977 | -0.0379597 |
| USP38 | ENSG00000170185 | -0.0378038 |
| FCER1G | ENSG00000158869 | -0.0367767 |
| PHLDA1 | ENSG00000139289 | -0.0366224 |
| BYSL | ENSG00000112578 | -0.0361786 |
| HLA-DRB5 | ENSG00000198502 | -0.035617 |
| RAPH1 | ENSG00000173166 | -0.0354985 |
| DUSP23 | ENSG00000158716 | -0.0348872 |
| FUOM | ENSG00000148803 | -0.034529 |
| ISYNA1 | ENSG00000105655 | -0.0329892 |
| TNK2 | ENSG00000061938 | -0.0322881 |
| STAP2 | ENSG00000178078 | -0.0321043 |
| SLC25A4 | ENSG00000151729 | -0.029497 |
| GALNT2 | ENSG00000143641 | -0.0294967 |
| SGO2 | SGO2 | -0.028765 |
| FHL3 | ENSG00000183386 | -0.0284614 |
| ALB | ENSG00000163631 | -0.0282075 |
| CYP20A1 | ENSG00000119004 | -0.0270327 |
| TM4SF1 | ENSG00000169908 | -0.0268013 |
| ADA | ENSG00000196839 | -0.025933 |
| RRP9 | ENSG00000114767 | -0.0253568 |
| DNAH14 | ENSG00000185842 | -0.0237476 |
| BOLA2 | ENSG00000183336 | -0.0233573 |
| BHLHE41 | ENSG00000123095 | -0.0225121 |
| CCL20 | ENSG00000115009 | -0.0219877 |
| AC005537.1 | AC005537.1 | -0.021938 |
| UBALD2 | ENSG00000185262 | -0.0212678 |
| VGLL4 | ENSG00000144560 | -0.0206353 |
| NUDT1 | ENSG00000106268 | -0.0206234 |
| USP10 | ENSG00000103194 | -0.02015 |
| ADSSL1 | ENSG00000185100 | -0.0200441 |
| PRSS23 | ENSG00000150687 | -0.015428 |
| FMC1 | FMC1 | -0.0141516 |
| ARHGAP45 | ARHGAP45 | -0.0137886 |
| HSPA14-1 | HSPA14-1 | -0.0132293 |
| CREB5 | ENSG00000146592 | -0.0127356 |

(continued)

| hgnc_symbol (gene) | ensembl_gene_id | weight |
|---|---|---|
| RBM33 | ENSG00000184863 | -0.0113459 |
| TMX4 | ENSG00000125827 | -0.009466 |
| ROCK2 | ENSG00000134318 | -0.0091039 |
| ARSK | ENSG00000164291 | -0.0078135 |
| PALLD | ENSG00000129116 | -0.0076409 |
| FNDC3B | ENSG00000075420 | -0.0068282 |
| FOXA3 | ENSG00000170608 | -0.0052306 |
| BATF | ENSG00000156127 | -0.0042389 |
| PTP4A3 | ENSG00000184489 | -0.0038806 |
| CDC45 | ENSG00000093009 | -0.0035675 |
| IGHV1-2 | IGHV1-2 | -0.0027275 |
| IMMP2L | ENSG00000184903 | -0.0026488 |
| STARD10 | ENSG00000214530 | -0.0021082 |
| HIST2H2BF | ENSG00000203814 | -0.0018981 |
| MTG2 | ENSG00000101181 | -0.0018976 |
| FBXO8 | ENSG00000164117 | -0.0010903 |
| USP32 | ENSG00000170832 | -0.000941 |
| ADIPOR2 | ENSG00000006831 | 0.00023297 |
| RRM2 | ENSG00000171848 | 0.00062261 |
| DHODH | ENSG00000102967 | 0.001119 |
| DDIT4 | ENSG00000168209 | 0.00162318 |
| NFAT5 | ENSG00000102908 | 0.00169985 |
| PPARG | ENSG00000132170 | 0.0030336 |
| YTHDF3-AS1 | YTHDF3-AS1 | 0.00351131 |
| GNG4 | ENSG00000168243 | 0.00360821 |
| CSPP1 | ENSG00000104218 | 0.0043984 |
| UBE2S | ENSG00000108106 | 0.00495386 |
| ZNF473 | ENSG00000142528 | 0.00495821 |
| TIMP1 | ENSG00000102265 | 0.00508074 |
| CPQ | ENSG00000104324 | 0.00541804 |
| AOC2 | ENSG00000131480 | 0.00688464 |
| H1F0 | ENSG00000189060 | 0.00762257 |
| JRK | JRK | 0.00820898 |
| EXOSC9 | ENSG00000123737 | 0.00825229 |
| AC012236.1 | AC012236.1 | 0.00849967 |
| AC009403.1 | AC009403.1 | 0.00865458 |
| C12orf65 | ENSG00000130921 | 0.0087269 |
| AURKA | ENSG00000087586 | 0.00895547 |
| MYH9 | ENSG00000100345 | 0.01454932 |

(continued)

| hgnc_symbol (gene) | ensembl_gene_id | weight |
|---|---|---|
| IGKV4-1 | IGKV4-1 | 0.01522631 |
| IGHMBP2 | ENSG00000132740 | 0.015257 |
| JADE1 | ENSG00000077684 | 0.01596038 |
| HIST1H3C | ENSG00000196532 | 0.0167969 |
| TTC39A | ENSG00000085831 | 0.01687531 |
| SGMS1 | ENSG00000198964 | 0.0174353 |
| LBP | ENSG00000129988 | 0.0177654 |
| FRYL | ENSG00000075539 | 0.01801951 |
| DNAJB2 | ENSG00000135924 | 0.01817648 |
| GNG11 | ENSG00000127920 | 0.01937393 |
| HAGHL | ENSG00000103253 | 0.02054714 |
| ANXA6 | ENSG00000197043 | 0.02070843 |
| MARS | ENSG00000166986 | 0.02229895 |
| ADD1 | ENSG00000087274 | 0.02303727 |
| KDM4B | ENSG00000127663 | 0.02307151 |
| TMEM91 | ENSG00000142046 | 0.02406029 |
| AC008915.2 | AC008915.2 | 0.0244961 |
| CXCL14 | ENSG00000145824 | 0.02545583 |
| DUSP14 | ENSG00000161326 | 0.02591071 |
| GJB2 | ENSG00000165474 | 0.0262334 |
| PGM1 | ENSG00000079739 | 0.0269324 |
| ETS2 | ENSG00000157557 | 0.02713344 |
| GNPDA1 | ENSG00000113552 | 0.0278746 |
| COL18A1 | ENSG00000182871 | 0.02822276 |
| KLF10 | ENSG00000155090 | 0.0292351 |
| MT1A | ENSG00000205362 | 0.03073511 |
| TPX2 | ENSG00000088325 | 0.03136912 |
| S100A2 | ENSG00000196754 | 0.03179409 |
| MAP3K5 | ENSG00000197442 | 0.03248026 |
| HIST1H2AE | ENSG00000168274 | 0.0331125 |
| SLC20A2 | ENSG00000168575 | 0.03337477 |
| ITGB7 | ENSG00000139626 | 0.03340733 |
| SCEL | ENSG00000136155 | 0.03353908 |
| RSRP1 | RSRP1 | 0.03359313 |
| AKR1B1 | ENSG00000085662 | 0.03643835 |
| GINS1 | ENSG00000101003 | 0.03706734 |
| ZNF296 | ENSG00000170684 | 0.03785632 |
| ALKBH4 | ENSG00000160993 | 0.03790482 |
| UBE2C | ENSG00000175063 | 0.03957332 |

(continued)

| hgnc_symbol (gene) | ensembl_gene_id | weight |
|---|---|---|
| ANKRD36C | ENSG00000174501 | 0.03977232 |
| SULT2B1 | ENSG00000088002 | 0.04025832 |
| SMC5 | ENSG00000198887 | 0.04084966 |
| TSPYL2 | ENSG00000184205 | 0.04224774 |
| TNS4 | ENSG00000131746 | 0.04248376 |
| TIMP3 | ENSG00000100234 | 0.04467604 |
| ID4 | ENSG00000172201 | 0.04478639 |
| SDC1 | ENSG00000115884 | 0.0465128 |
| COX18 | ENSG00000163626 | 0.04762095 |
| CDC42EP2 | ENSG00000149798 | 0.0479187 |
| SQLE | ENSG00000104549 | 0.04854746 |
| ZNRF1 | ENSG00000186187 | 0.0488868 |
| AKR1B10 | ENSG00000198074 | 0.04935299 |
| NDC80 | ENSG00000080986 | 0.04967183 |
| GFPT2 | ENSG00000131459 | 0.05016553 |
| MAP1B | ENSG00000131711 | 0.05050151 |
| HIST1H2AG | ENSG00000196787 | 0.05193023 |
| IDO1 | ENSG00000131203 | 0.05299858 |
| RNF185 | ENSG00000138942 | 0.05303177 |
| UHRF1BP1 | ENSG00000065060 | 0.05328148 |
| ADORA2B | ENSG00000170425 | 0.0535626 |
| CALD1 | ENSG00000122786 | 0.05363613 |
| PHLDA2 | ENSG00000181649 | 0.05399965 |
| ADH6 | ENSG00000172955 | 0.05460884 |
| TFAP2A | ENSG00000137203 | 0.05522595 |
| DLG1 | ENSG00000075711 | 0.05543325 |
| MELK | ENSG00000165304 | 0.05610831 |
| CBWD3 | ENSG00000196873 | 0.05616215 |
| RAB4B | ENSG00000167578 | 0.05652341 |
| KANSL1L | ENSG00000144445 | 0.05667774 |
| RCE1 | ENSG00000173653 | 0.05731328 |
| HIST1H2AC | ENSG00000180573 | 0.05903386 |
| CDK1 | ENSG00000170312 | 0.05971862 |
| TCIM | TCIM | 0.06100506 |
| C17orf67 | ENSG00000214226 | 0.0610775 |
| BRD4 | ENSG00000141867 | 0.06150705 |
| LY6E-DT | LY6E-DT | 0.0617519 |
| SLC1A6 | ENSG00000105143 | 0.06184738 |
| ARL13B | ENSG00000169379 | 0.06201305 |

(continued)

| hgnc_symbol (gene) | ensembl_gene_id | weight |
|---|---|---|
| IRF1 | ENSG00000125347 | 0.06279108 |
| DDX3X | ENSG00000215301 | 0.06440059 |
| RAB2B | ENSG00000129472 | 0.06440603 |
| MYBBP1A | ENSG00000132382 | 0.0645036 |
| ARFGAP1 | ENSG00000101199 | 0.0669557 |
| BOP1 | ENSG00000170727 | 0.06804563 |
| IGKV3D-7 | IGKV3D-7 | 0.06929084 |
| KMT2E-AS1 | KMT2E-AS1 | 0.07012494 |
| DTNBP1 | ENSG00000047579 | 0.07028198 |
| LAMC2 | ENSG00000058085 | 0.07044349 |
| ATG4C | ENSG00000125703 | 0.07140213 |
| MYBL2 | ENSG00000101057 | 0.07232309 |
| LRP10 | ENSG00000197324 | 0.07428999 |
| PALMD | ENSG00000099260 | 0.07458015 |
| ZBTB4 | ENSG00000174282 | 0.07521748 |
| SYTL2 | ENSG00000137501 | 0.07521786 |
| SERPINH1 | ENSG00000149257 | 0.07534697 |
| CD248 | ENSG00000174807 | 0.07540795 |
| CNEP1R1 | ENSG00000205423 | 0.07642911 |
| FURIN | ENSG00000140564 | 0.07773387 |
| IGLL5 | IGLL5 | 0.07901263 |
| MEST | ENSG00000106484 | 0.08272485 |
| MDK | ENSG00000110492 | 0.08398304 |
| NUP205 | ENSG00000155561 | 0.08600693 |
| NRDE2 | ENSG00000119720 | 0.08663681 |
| ECT2 | ENSG00000114346 | 0.08708529 |
| TENT5A | TENT5A | 0.08718747 |
| TNKS1BP1 | ENSG00000149115 | 0.08775285 |
| NFXL1 | ENSG00000170448 | 0.0878479 |
| SLC35E3 | ENSG00000175782 | 0.08814538 |
| ECE1 | ENSG00000117298 | 0.08817485 |
| RASD1 | ENSG00000108551 | 0.08948933 |
| SLC52A2 | ENSG00000185803 | 0.0906505 |
| DCBLD2 | ENSG00000057019 | 0.09092941 |
| CP | ENSG00000047457 | 0.090947 |
| POLE | ENSG00000177084 | 0.09122142 |
| COL27A1 | ENSG00000196739 | 0.09168626 |
| SBNO1 | ENSG00000139697 | 0.09246919 |
| SLC7A6 | ENSG00000103064 | 0.09376455 |

(continued)

| hgnc_symbol (gene) | ensembl_gene_id | weight |
|---|---|---|
| HYKK | ENSG00000188266 | 0.09461495 |
| SLPI | ENSG00000124107 | 0.096531 |
| CFHR1 | ENSG00000244414 | 0.09682313 |
| SPDEF | ENSG00000124664 | 0.09939881 |
| DACT2 | ENSG00000164488 | 0.10129043 |
| TUBGCP5 | ENSG00000153575 | 0.1022474 |
| AREG | ENSG00000109321 | 0.10349606 |
| HIST1H2AJ | ENSG00000182611 | 0.10391941 |
| KIF2A | ENSG00000068796 | 0.1040777 |
| AL135925.1 | AL135925.1 | 0.10510125 |
| NOTCH3 | ENSG00000074181 | 0.10527227 |
| SLC11A1 | ENSG00000018280 | 0.10548697 |
| HEXIM2 | ENSG00000168517 | 0.10568237 |
| IGFBP1 | ENSG00000146678 | 0.10715199 |
| TVP23A | ENSG00000166676 | 0.10763961 |
| NUDT14 | ENSG00000183828 | 0.10864274 |
| SAMD11 | ENSG00000187634 | 0.10921951 |
| MIR200CHG | MIR200CHG | 0.10931367 |
| PCLAF | PCLAF | 0.10944494 |
| SLC43A3 | ENSG00000134802 | 0.10972944 |
| FAM30A | FAM30A | 0.10996001 |
| PHRF1 | ENSG00000070047 | 0.11063817 |
| ADM | ENSG00000148926 | 0.11264171 |
| SIK2 | ENSG00000170145 | 0.11279737 |
| NUSAP1 | ENSG00000137804 | 0.11295719 |
| CFH | ENSG00000000971 | 0.11500026 |
| KRTCAP3 | ENSG00000157992 | 0.11524822 |
| SPAG4 | ENSG00000061656 | 0.1155683 |
| TPPP3 | ENSG00000159713 | 0.11699977 |
| TSPAN4 | ENSG00000214063 | 0.1176398 |
| AAK1 | ENSG00000115977 | 0.11790302 |
| CST1 | ENSG00000170373 | 0.11816964 |
| CLU | ENSG00000120885 | 0.11852667 |
| IFRD1 | ENSG00000006652 | 0.11953649 |
| ASPHD2 | ENSG00000128203 | 0.12056523 |
| CNN3 | ENSG00000117519 | 0.12182891 |
| COL4A1 | ENSG00000187498 | 0.12192622 |
| FGA | ENSG00000171560 | 0.12269039 |
| ANO6 | ENSG00000177119 | 0.12427127 |

(continued)

| hgnc_symbol (gene) | ensembl_gene_id | weight |
|---|---|---|
| SBSN | ENSG00000189001 | 0.12440735 |
| FGB | ENSG00000171564 | 0.12575339 |
| ATP9B | ENSG00000166377 | 0.12576154 |
| NLGN4Y | ENSG00000165246 | 0.12583522 |
| HP | ENSG00000257017 | 0.12618904 |
| EPS8 | ENSG00000151491 | 0.1264151 |
| RNF111 | ENSG00000157450 | 0.12677036 |
| LINC01285 | LINC01285 | 0.12697746 |
| MAOA | ENSG00000189221 | 0.12701674 |
| IGHV4-31 | IGHV4-31 | 0.12768866 |
| TNFRSF10D | ENSG00000173530 | 0.12900174 |
| GSR | ENSG00000104687 | 0.12977374 |
| IGHGP | IGHGP | 0.12981969 |
| TACSTD2 | ENSG00000184292 | 0.12987906 |
| MT1F | ENSG00000198417 | 0.13014634 |
| RHCG | ENSG00000140519 | 0.13096346 |
| MUT | ENSG00000146085 | 0.13124914 |
| PI3 | ENSG00000124102 | 0.13184208 |
| MT1M | ENSG00000205364 | 0.13290805 |
| LAMB 3 | ENSG00000196878 | 0.13357507 |
| MTRNR2L12 | ENSG00000269028 | 0.1342401 |
| SLC35A2 | ENSG00000102100 | 0.13498922 |
| DDX10 | ENSG00000178105 | 0.1371739 |
| RARRES1 | ENSG00000118849 | 0.13751803 |
| MTSS1 | ENSG00000170873 | 0.13787903 |
| CLK2 | ENSG00000176444 | 0.1379331 |
| RPN2 | ENSG00000118705 | 0.14023371 |
| MED29 | ENSG00000063322 | 0.14141189 |
| CYP1B1 | ENSG00000138061 | 0.14353636 |
| TTTY14 | ENSG00000176728 | 0.14398424 |
| DMXL1 | ENSG00000172869 | 0.144 |
| AL139246.5 | AL139246.5 | 0.14529607 |
| TAF1 | ENSG00000147133 | 0.14557107 |
| DAAM1 | ENSG00000100592 | 0.14616989 |
| MYO1E | ENSG00000157483 | 0.14845465 |
| MAFB | ENSG00000204103 | 0.1486457 |
| CDKN1A | ENSG00000124762 | 0.14898159 |
| F8A3 | ENSG00000185990 | 0.14943731 |
| FABP5 | ENSG00000164687 | 0.14957616 |

(continued)

| hgnc_symbol (gene) | ensembl_gene_id | weight |
|---|---|---|
| CFB | ENSG00000243649 | 0.15005757 |
| HSP90B1 | ENSG00000166598 | 0.1501378 |
| SGK3 | ENSG00000104205 | 0.15084904 |
| HMG20B | ENSG00000064961 | 0.15088087 |
| CDCA5 | ENSG00000146670 | 0.15186115 |
| CLDN4 | ENSG00000189143 | 0.15258871 |
| SYNM | ENSG00000182253 | 0.15287656 |
| PAWR | ENSG00000177425 | 0.15298806 |
| TWNK | TWNK | 0.15414731 |
| AC116049.2 | AC116049.2 | 0.15426011 |
| RND3 | ENSG00000115963 | 0.15436454 |
| ATP11A | ENSG00000068650 | 0.15446725 |
| PID1 | ENSG00000153823 | 0.1545904 |
| MALAT1 | ENSG00000251562 | 0.15489668 |
| TMEM168 | ENSG00000146802 | 0.15731537 |
| TFF1 | ENSG00000160182 | 0.15836668 |
| TFRC | ENSG00000072274 | 0.15930122 |
| RNASET2 | ENSG00000026297 | 0.15940832 |
| SPINK13 | ENSG00000214510 | 0.16061184 |
| PABPC1L | ENSG00000101104 | 0.1626279 |
| P4HA2 | ENSG00000072682 | 0.16369961 |
| PRSS8 | ENSG00000052344 | 0.16441339 |
| SPINT1 | ENSG00000166145 | 0.16447538 |
| MSC | ENSG00000178860 | 0.16484685 |
| FMNL1 | ENSG00000184922 | 0.16662268 |
| SLC8B1 | ENSG00000089060 | 0.1672964 |
| UNC13D | ENSG00000092929 | 0.16775854 |
| SPINT2 | ENSG00000167642 | 0.16797978 |
| DCP1A | ENSG00000162290 | 0.16917971 |
| NPTN | ENSG00000156642 | 0.16941091 |
| IGKV3D-11 | IGKV3D-11 | 0.16972472 |
| G6PD | ENSG00000160211 | 0.17004436 |
| KRT6A | ENSG00000205420 | 0.1701689 |
| LYPD1 | ENSG00000150551 | 0.17033444 |
| TESC | ENSG00000088992 | 0.17201957 |
| COL4A2 | ENSG00000134871 | 0.17230445 |
| ELF3 | ENSG00000163435 | 0.17285524 |
| BCAM | ENSG00000187244 | 0.17286958 |
| AC093323.1 | AC093323.1 | 0.17366225 |

(continued)

| hgnc_symbol (gene) | ensembl_gene_id | weight |
|---|---|---|
| IGHV1-69 | IGHV1-69 | 0.1738128 |
| LINC00511 | LINC00511 | 0.17396097 |
| PORCN | ENSG00000102312 | 0.17418613 |
| TPRG1-AS1 | TPRG1-AS1 | 0.17608684 |
| EFNB2 | ENSG00000125266 | 0.17753741 |
| PARD6G-AS1 | PARD6G-AS1 | 0.17796445 |
| CD9 | ENSG00000010278 | 0.17818554 |
| RGS16 | ENSG00000143333 | 0.17846893 |
| IL6R | ENSG00000160712 | 0.17927676 |
| FZD3 | ENSG00000104290 | 0.18137573 |
| GLYR1 | ENSG00000140632 | 0.18143135 |
| B3GALT6 | ENSG00000176022 | 0.18169077 |
| LRCH3 | ENSG00000186001 | 0.18175747 |
| MAFK | ENSG00000198517 | 0.18250978 |
| LINC00491 | LINC00491 | 0.18303211 |
| MT1X | ENSG00000187193 | 0.1862794 |
| MUC6 | ENSG00000184956 | 0.18707584 |
| PIK3R3 | ENSG00000117461 | 0.18830746 |
| GBP4 | ENSG00000162654 | 0.1885141 |
| PERP | ENSG00000112378 | 0.18881083 |
| LXN | ENSG00000079257 | 0.18946418 |
| ZBTB7A | ENSG00000178951 | 0.19152485 |
| WARS | ENSG00000140105 | 0.19165362 |
| AC020911.2 | AC020911.2 | 0.19170022 |
| MAPK3 | ENSG00000102882 | 0.19214207 |
| ALS2CL | ENSG00000178038 | 0.1927534 |
| MRE11 | MRE11 | 0.19294888 |
| TSPAN17 | ENSG00000048140 | 0.19300817 |
| IGHV4-34 | IGHV4-34 | 0.1949669 |
| IL33 | ENSG00000137033 | 0.1954984 |
| ADAM9 | ENSG00000168615 | 0.19577676 |
| ANGPTL4 | ENSG00000167772 | 0.19629216 |
| TBC1D31 | ENSG00000156787 | 0.19698112 |
| C1R | ENSG00000159403 | 0.19875261 |
| CTSC | ENSG00000109861 | 0.19902864 |
| SLC35A4 | ENSG00000176087 | 0.19967438 |
| FST | ENSG00000134363 | 0.20003097 |
| SGO1 | SGO1 | 0.20042324 |
| ANKRD36 | ENSG00000135976 | 0.20042917 |

(continued)

| hgnc_symbol (gene) | ensembl_gene_id | weight |
|---|---|---|
| IGHG3 | IGHG3 | 0.20214134 |
| SLC15A3 | ENSG00000110446 | 0.20363048 |
| HES1 | ENSG00000114315 | 0.20397656 |
| POLR1E | ENSG00000137054 | 0.20435518 |
| SLC7A5 | ENSG00000103257 | 0.20460984 |
| CAPN12 | ENSG00000182472 | 0.20495103 |
| IGFBP3 | ENSG00000146674 | 0.2066585 |
| FBXO38 | ENSG00000145868 | 0.20672603 |
| FLNA | ENSG00000196924 | 0.20675384 |
| CSKMT | CSKMT | 0.20871642 |
| OAS1 | ENSG00000089127 | 0.20940009 |
| ULK1 | ENSG00000177169 | 0.20950152 |
| PBX1 | ENSG00000185630 | 0.21014394 |
| EXOC4 | ENSG00000131558 | 0.21088976 |
| REEP6 | ENSG00000115255 | 0.21190931 |
| HILPDA | ENSG00000135245 | 0.21375751 |
| ASF1B | ENSG00000105011 | 0.21573824 |
| FKBP1B | ENSG00000119782 | 0.21723895 |
| IL6 | ENSG00000136244 | 0.21756423 |
| CALU | ENSG00000128595 | 0.217784 |
| AKR1C1 | ENSG00000187134 | 0.2184874 |
| KLF2 | ENSG00000127528 | 0.2197309 |
| GRTP1 | ENSG00000139835 | 0.22025041 |
| C1S | ENSG00000182326 | 0.22058915 |
| SMOX | ENSG00000088826 | 0.22372174 |
| CPLX2 | ENSG00000145920 | 0.22384913 |
| LMNA | ENSG00000160789 | 0.22785227 |
| BSG | ENSG00000172270 | 0.22908567 |
| IGHG4 | IGHG4 | 0.22954205 |
| SVIL | ENSG00000197321 | 0.2324116 |
| HIST1H1B | ENSG00000184357 | 0.2326907 |
| GCH1 | ENSG00000131979 | 0.23300366 |
| NEAT1 | ENSG00000245532 | 0.2332629 |
| FN1 | ENSG00000115414 | 0.23388489 |
| ESRP1 | ENSG00000104413 | 0.23603399 |
| RFWD3 | ENSG00000168411 | 0.23635007 |
| ADGRE2 | ADGRE2 | 0.23714031 |
| SPINK6 | ENSG00000178172 | 0.2392691 |
| HPD | ENSG00000158104 | 0.24136677 |

(continued)

| hgnc_symbol (gene) | ensembl_gene_id | weight |
|---|---|---|
| CAVIN1 | CAVIN1 | 0.24193044 |
| MT1E | ENSG00000169715 | 0.24426004 |
| CLDN10 | ENSG00000134873 | 0.24464439 |
| C15orf48 | ENSG00000166920 | 0.2447176 |
| CA9 | ENSG00000107159 | 0.24549681 |
| NR4A1 | ENSG00000123358 | 0.24760291 |
| PPP1R3B | ENSG00000173281 | 0.24885757 |
| SLC30A1 | ENSG00000170385 | 0.24955915 |
| SLC7A11 | ENSG00000151012 | 0.25061235 |
| VIRMA | VIRMA | 0.2509382 |
| NAA25 | ENSG00000111300 | 0.25189295 |
| CCNB1 | ENSG00000134057 | 0.25213915 |
| CFD | ENSG00000197766 | 0.25334427 |
| AP1G1 | ENSG00000166747 | 0.2542073 |
| H6PD | ENSG00000049239 | 0.25436643 |
| PSCA | PSCA | 0.2556265 |
| KCNK6 | ENSG00000099337 | 0.2565629 |
| AL161431.1 | AL161431.1 | 0.25786754 |
| DVL1 | ENSG00000107404 | 0.25854063 |
| HIST1H2AM | ENSG00000233224 | 0.2590186 |
| RAB31 | ENSG00000168461 | 0.25943103 |
| CDCA3 | ENSG00000111665 | 0.25976846 |
| SPATA20 | ENSG00000006282 | 0.26025692 |
| PRMT7 | ENSG00000132600 | 0.26215124 |
| PTGR1 | ENSG00000106853 | 0.26377833 |
| SERINC2 | ENSG00000168528 | 0.2638674 |
| IGHG2 | IGHG2 | 0.26394448 |
| GFPT1 | ENSG00000198380 | 0.26444328 |
| TTC22 | ENSG00000006555 | 0.26678386 |
| BTBD1 | ENSG00000064726 | 0.26690102 |
| HIST1H4H | ENSG00000158406 | 0.27086592 |
| CENPB | ENSG00000125817 | 0.27116215 |
| ZNF598 | ENSG00000167962 | 0.27331212 |
| GPATCH2L | ENSG00000089916 | 0.2821217 |
| SPTLC3 | ENSG00000172296 | 0.2844696 |
| CXCL2 | ENSG00000081041 | 0.2848442 |
| CYP24A1 | ENSG00000019186 | 0.28805703 |
| EZH2 | ENSG00000106462 | 0.29162478 |
| GPX2 | ENSG00000176153 | 0.29347402 |

(continued)

| hgnc_symbol (gene) | ensembl_gene_id | weight |
|---|---|---|
| LMNB2 | ENSG00000176619 | 0.29507056 |
| PTGES | ENSG00000148344 | 0.29507342 |
| MGLL | ENSG00000074416 | 0.29684052 |
| NR2F2 | ENSG00000185551 | 0.29726234 |
| KRT19 | ENSG00000171345 | 0.29860005 |
| DNTTIP1 | ENSG00000101457 | 0.29867128 |
| MUC5AC | ENSG00000215182 | 0.29973653 |
| SDCBP2 | ENSG00000125775 | 0.29999447 |
| IL1R2 | ENSG00000115590 | 0.30178872 |
| AHNAK2 | ENSG00000185567 | 0.3019708 |
| MUC16 | ENSG00000181143 | 0.3021724 |
| AC023090.1 | AC023090.1 | 0.3031951 |
| CPE | ENSG00000109472 | 0.30463472 |
| VNN1 | ENSG00000112299 | 0.30691797 |
| BAMBI | ENSG00000095739 | 0.3087375 |
| NPW | ENSG00000183971 | 0.3118132 |
| TK1 | ENSG00000167900 | 0.31219006 |
| IGKV3D-20 | IGKV3D-20 | 0.31330803 |
| ANKRD11 | ENSG00000167522 | 0.31380752 |
| CDC20 | ENSG00000117399 | 0.31532457 |
| CDH1 | ENSG00000039068 | 0.31652898 |
| STK11 | ENSG00000118046 | 0.3169986 |
| IGKC | IGKC | 0.32296088 |
| SLC45A4 | ENSG00000022567 | 0.32814574 |
| TBC1D8 | ENSG00000204634 | 0.33819315 |
| CSTA | ENSG00000121552 | 0.3392412 |
| AC233755.1 | AC233755.1 | 0.33962315 |
| MIGA1 | MIGA1 | 0.34099814 |
| HIST1H2AL | ENSG00000198374 | 0.34156758 |
| AKAP12 | ENSG00000131016 | 0.34173587 |
| MAP4K4 | ENSG00000071054 | 0.3432171 |
| HOOK2 | ENSG00000095066 | 0.3440207 |
| GGA3 | ENSG00000125447 | 0.34517744 |
| COL7A1 | ENSG00000114270 | 0.3466547 |
| NOS1 | ENSG00000089250 | 0.35232848 |
| ARHGAP26 | ENSG00000145819 | 0.35265827 |
| AKR1C2 | ENSG00000151632 | 0.36026683 |
| TGM2 | ENSG00000198959 | 0.36146182 |
| CENPF | ENSG00000117724 | 0.36182958 |

(continued)

| hgnc_symbol (gene) | ensembl_gene_id | weight |
|---|---|---|
| IGHV3-48 | IGHV3-48 | 0.36285096 |
| CDCA8 | ENSG00000134690 | 0.36302796 |
| TSC2 | ENSG00000103197 | 0.36492628 |
| STC2 | ENSG00000113739 | 0.3696755 |
| PKN3 | ENSG00000160447 | 0.37384662 |
| PVR | ENSG00000073008 | 0.3806 |
| CES1 | ENSG00000198848 | 0.38293198 |
| GPRC5A | ENSG00000013588 | 0.3859542 |
| SEZ6L2 | ENSG00000174938 | 0.38817623 |
| CEP170B | ENSG00000099814 | 0.39238733 |
| KIF14 | ENSG00000118193 | 0.39301777 |
| IER3 | ENSG00000137331 | 0.39397794 |
| ALDH3B1 | ENSG00000006534 | 0.39537683 |
| TOP2A | ENSG00000131747 | 0.39561334 |
| SPP1 | ENSG00000118785 | 0.39639193 |
| TXNRD1 | ENSG00000198431 | 0.39665508 |
| LENG8 | ENSG00000167615 | 0.39838022 |
| TRIM15 | ENSG00000204610 | 0.40109217 |
| ALDH3A1 | ENSG00000108602 | 0.4017077 |
| RIMKLB | ENSG00000166532 | 0.4054596 |
| HECTD4 | ENSG00000173064 | 0.4067108 |
| SMOC1 | ENSG00000198732 | 0.4083209 |
| NEB | ENSG00000183091 | 0.40843356 |
| RMRP | ENSG00000269900 | 0.41210017 |
| IGFBP4 | ENSG00000141753 | 0.41471958 |
| MT1G | ENSG00000125144 | 0.42289618 |
| SCRIB | ENSG00000180900 | 0.4234361 |
| ERO1A | ERO1A | 0.4300462 |
| SOX4 | ENSG00000124766 | 0.43042758 |
| LMO7 | ENSG00000136153 | 0.43147683 |
| RNPEPL1 | ENSG00000142327 | 0.4330034 |
| PLK2 | ENSG00000145632 | 0.4392007 |
| COL6A2 | ENSG00000142173 | 0.4395092 |
| FLRT3 | ENSG00000125848 | 0.44094595 |
| IGHV4-28 | IGHV4-28 | 0.44107214 |
| SCD | ENSG00000099194 | 0.4449068 |
| KRT7 | ENSG00000135480 | 0.4534456 |
| PIEZO1 | ENSG00000103335 | 0.46255627 |
| CXCL1 | ENSG00000163739 | 0.46270853 |

(continued)

| hgnc_symbol (gene) | ensembl_gene_id | weight |
|---|---|---|
| DAPK1 | ENSG00000196730 | 0.47022906 |
| ID1 | ENSG00000125968 | 0.48670167 |
| C3 | ENSG00000125730 | 0.48777086 |
| CXCL3 | ENSG00000163734 | 0.48818576 |
| IGKV3-20 | IGKV3-20 | 0.4918123 |
| GUCA2B | ENSG00000044012 | 0.50782996 |
| ITGA3 | ENSG00000005884 | 0.51895195 |
| SFN | ENSG00000175793 | 0.5279402 |
| IGLV3-21 | IGLV3-21 | 0.5387204 |
| PLEC | ENSG00000178209 | 0.55829024 |
| POLR2A | ENSG00000181222 | 0.596864 |
| AGRN | ENSG00000188157 | 0.60017353 |
| MUC1 | ENSG00000185499 | 0.6115802 |
| SERPINB3 | ENSG00000057149 | 0.6544421 |
| S100A8 | ENSG00000143546 | 0.6660124 |
| LAMA5 | ENSG00000130702 | 0.7110091 |
| COL6A1 | ENSG00000142156 | 0.7224733 |
| ITGB4 | ENSG00000132470 | 0.7254199 |
| S100P | ENSG00000163993 | 0.74276584 |
| SLURP2 | SLURP2 | 0.7436052 |
| MSLN | ENSG00000102854 | 0.74538 |
| KRT17 | ENSG00000128422 | 0.7872183 |
| MUC5B | ENSG00000117983 | 0.8070428 |

Expression levels of the selected genes from Table 1 may be determined by any of a number of methods, and may encompass either or both protein and RNA detection.

The presence or absence of an IPS may be determined in any number of cancer types, and is not limited to NSCLC; the cancer may also be identified as having an altered human leukocyte antigen (HLA) phenotype, *e.g.,* a loss of heterozygosity at the HLA locus. In addition, the subject's cancer treatment regimen, or lack thereof, prior to testing for IPS is not limiting.

**Immune Oncology Signature**

The inventors discovered an immune oncology signature (IOS) that is associated with a subject's likelihood to respond to ICI. The IOS may comprise of one or more genes selected from ISG20, PCDHGA2, TGFB1I1, ATP8B1, IL7R, IRF8, ETV1, MYLK, GRHL2, THBS4, CYP3A5, FBLIM1, S100B, BICD1, SLAMF7, RAB27A, GATM, ICA1, ITPR1, SLC7A2, ZAP70, LOXL4, CILP, ARHGAP30, ITGB2, KLF5, PRKCA, PCDH7, DPYSL3, RGS2, SPP1, COLGALT2, MPZL2, TNFAIP8, PLAT, ALDH1A3, POF1B, PPP1R9A, SEMA3A, CIITA, DLC1, ARHGAP9, FRAS1, AKAP6, ATP1A2, TTN, LTBP1, NCKAP1L, MAP3K6, MYO1B, MRVI1, FSCN1, GPC1, GBP5, BAMBI, IL2RB, MYO1G, RANBP17, APOD, RASGRP1, CYTIP, ITGA7, CYTH4, PTPRF, KIAA1755, IRF1, GPR37, RAC2, NLRC5, EGFR, ITK, IL10RA, IGFBP2, CD96, RASD1, CD36, TMEM163, IGLL5, IKZF3, PRLR, CDC42BPG, DOCK2, PAM, VEGFA, CD84, SORL1, GBP2, SYTL4, APBB1IP, SIGLEC10, GBP4, COMP, DOCK8, CXCL9, NRP1, EPHB4, CD53, GLUL, DNM1, DSP, SIX4, SELL, DSC3, TNFAIP2, and JAG2, shown in Table 2.

Table 2 lists 105 genes whose expression values may be used in the IOS. The IOS may comprise expression values for 1-105, or any number in between 1 and 105 of the genes listed in Table 2 and may further comprise the weights corresponding to the genes listed in Table 2 in any combination. The IOS may comprise expression values for 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 2, e.g., the top 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100 or more of the genes in Table 2. The IOS may comprise expression values for each of the 105 genes listed in Table 2. The IOS may comprise expression values for one or more of the following genes GBP5, IL10RA, NLRC5, CXCL9, RAC2, GBP4, GLUL, IRF1, CD53, CIITA, S100B, GBP2, ITK, SLAMF7, IKZF3, DOCK2, SELL, ARHGAP9, CYTIP, IL2RB, NCKAP1L, APOD, CD96, IL7R, or ZAP70, which, in one embodiment, are ranked as the top 25 genes based on weight.

Therefore, in some embodiments, the methods comprise at a computer system having one or more processors, and memory storing one or more programs for execution by the one or more processors: applying, by the one or more processors, one or more model components derived from sequencing data from a sample to one or more machine learning algorithms (MLAs), wherein the sequencing data comprises RNA sequencing data and DNA sequencing data, wherein the one or more model components comprise an immune oncology signature.

Table 2. Genes and weights in Immune Oncology Signature (IOS).

| ensembl_gene | hgnc_gene_symbol | Weight |
|---|---|---|
| ENSG00000172183 | ISG20 | 0.003804 |
| ENSG00000081853 | PCDHGA2 | -5.84E-05 |
| ENSG00000140682 | TGFB1I1 | -0.00877 |
| ENSG00000081923 | ATP8B1 | -0.00646 |
| ENSG00000168685 | IL7R | 0.006902 |
| ENSG00000140968 | IRF8 | 0.005272 |
| ENSG00000006468 | ETV1 | -0.00621 |
| ENSG00000065534 | MYLK | -0.01362 |
| ENSG00000083307 | GRHL2 | -0.00308 |
| ENSG00000113296 | THBS4 | -0.0144 |
| ENSG00000106258 | CYP3A5 | -0.00244 |
| ENSG00000162458 | FBLIM1 | -0.01636 |
| ENSG00000160307 | S100B | 0.015622 |
| ENSG00000151746 | BICD1 | -0.00564 |
| ENSG00000026751 | SLAMF7 | 0.014074 |
| ENSG00000069974 | RAB27A | 0.003806 |
| ENSG00000171766 | GATM | -0.00248 |
| ENSG00000003147 | ICA1 | -0.0055 |
| ENSG00000150995 | ITPR1 | -0.00174 |
| ENSG00000003989 | SLC7A2 | -0.00448 |
| ENSG00000115085 | ZAP70 | 0.006475 |
| ENSG00000138131 | LOXL4 | -0.00045 |
| ENSG00000138615 | CILP | -0.00083 |
| ENSG00000186517 | ARHGAP30 | 0.000363 |
| ENSG00000160255 | ITGB2 | 0.004448 |
| ENSG00000102554 | KLF5 | -0.00074 |
| ENSG00000154229 | PRKCA | -0.00192 |
| ENSG00000169851 | PCDH7 | -0.00862 |
| ENSG00000113657 | DPYSL3 | -0.0057 |
| ENSG00000116741 | RGS2 | -0.00845 |

(continued)

| ensembl_gene | hgnc_gene_symbol | Weight |
|---|---|---|
| ENSG00000118785 | SPP1 | -0.03861 |
| ENSG00000198756 | COLGALT2 | -0.00076 |
| ENSG00000149573 | MPZL2 | -0.00365 |
| ENSG00000145779 | TNFAIP8 | 0.00054 |
| ENSG00000104368 | PLAT | -0.00167 |
| ENSG00000184254 | ALDH1A3 | -0.0026 |
| ENSG00000124429 | POF1B | -0.00105 |
| ENSG00000158528 | PPP1R9A | -7.20E-06 |
| ENSG00000075213 | SEMA3A | -0.01394 |
| ENSG00000179583 | CIITA | 0.015788 |
| ENSG00000164741 | DLC1 | -0.0127 |
| ENSG00000123329 | ARHGAP9 | 0.008283 |
| ENSG00000138759 | FRAS1 | -0.00651 |
| ENSG00000151320 | AKAP6 | -0.00903 |
| ENSG00000018625 | ATP1A2 | -0.00214 |
| ENSG00000155657 | TTN | 0.002688 |
| ENSG00000049323 | LTBP1 | -0.0005 |
| ENSG00000123338 | NCKAP1L | 0.007521 |
| ENSG00000142733 | MAP3K6 | -0.00211 |
| ENSG00000128641 | MYO1B | -0.00681 |
| NSG00000072952 | MRVI1 | -0.00098 |
| ENSG00000075618 | FSCN1 | -0.02913 |
| ENSG00000063660 | GPC1 | -0.00271 |
| ENSG00000154451 | GBP5 | 0.04021 |
| ENSG00000095739 | BAMBI | 0.000157 |
| ENSG00000100385 | IL2RB | 0.007864 |
| ENSG00000136286 | MYO1G | 0.006105 |
| ENSG00000204764 | RANBP17 | -0.00422 |
| ENSG00000189058 | APOD | 0.007289 |
| ENSG00000172575 | RASGRP1 | 0.003107 |
| ENSG00000115165 | CYTIP | 0.008234 |
| ENSG00000135424 | ITGA7 | -0.00494 |
| ENSG00000100055 | CYTH4 | 0.002811 |
| ENSG00000142949 | PTPRF | -0.00642 |
| ENSG00000149633 | KIAA1755 | -0.00484 |
| ENSG00000125347 | IRF1 | 0.017589 |
| ENSG00000170775 | GPR37 | -0.00261 |
| ENSG00000128340 | RAC2 | 0.022314 |
| ENSG00000140853 | NLRC5 | 0.023683 |

(continued)

| ensembl_gene | hgnc_gene_symbol | Weight |
|---|---|---|
| ENSG00000146648 | EGFR | -0.00094 |
| ENSG00000113263 | ITK | 0.014309 |
| ENSG00000110324 | IL10RA | 0.023836 |
| ENSG00000115457 | IGFBP2 | -0.01201 |
| ENSG00000153283 | CD96 | 0.007115 |
| ENSG00000108551 | RASD1 | -0.00063 |
| ENSG00000135218 | CD36 | -0.00291 |
| ENSG00000152128 | TMEM163 | 0.003094 |
| ENSG00000254709 | IGLL5 | 0.001448 |
| ENSG00000161405 | IKZF3 | 0.011866 |
| ENSG00000113494 | PRLR | -0.00169 |
| ENSG00000171219 | CDC42BPG | -0.00467 |
| ENSG00000134516 | DOCK2 | 0.011565 |
| ENSG00000145730 | PAM | -0.0067 |
| ENSG00000112715 | VEGFA | -0.00048 |
| ENSG00000066294 | CD84 | 0.00346 |
| ENSG00000137642 | SORL1 | -5.42E-05 |
| ENSG00000162645 | GBP2 | 0.015149 |
| ENSG00000102362 | SYTL4 | -0.00108 |
| ENSG00000077420 | APBB1IP | 0.000551 |
| ENSG00000142512 | SIGLEC10 | 0.00142 |
| ENSG00000162654 | GBP4 | 0.020847 |
| ENSG00000105664 | COMP | -0.02475 |
| ENSG00000107099 | DOCK8 | 0.001925 |
| ENSG00000138755 | CXCL9 | 0.02317 |
| ENSG00000099250 | NRP1 | -0.00313 |
| ENSG00000196411 | EPHB4 | -0.00842 |
| ENSG00000143119 | CD53 | 0.015805 |
| ENSG00000135821 | GLUL | 0.020494 |
| ENSG00000106976 | DNM1 | -0.00063 |
| ENSG00000096696 | DSP | -0.0038 |
| ENSG00000100625 | SIX4 | -0.00159 |
| ENSG00000188404 | SELL | 0.00834 |
| ENSG00000134762 | DSC3 | -0.0041 |
| ENSG00000185215 | TNFAIP2 | 0.005226 |
| ENSG00000184916 | JAG2 | -0.00377 |

**Checkpoint Related Gene Signature**

The checkpoint related gene signature may comprise expression levels for one or more of the following genes:

CD274, SPP1, CXCL9, CD74, CD276, IDO1, PDCD1LG2, and TNFRSF5. The checkpoint related gene signature may comprise expression values for 1, 2, 3, 4, 5, 6, 7, or all 8 of the above checkpoint related genes in any combination.

In some embodiments, the methods comprise at a computer system having one or more processors, and memory storing one or more programs for execution by the one or more processors: applying, by the one or more processors, one or more model components derived from sequencing data from a sample to one or more machine learning algorithms (MLAs), wherein the sequencing data comprises RNA sequencing data and DNA sequencing data, wherein the one or more model components comprise a checkpoint related gene signature.

**Granulocytic myeloid derived suppressor cell signature**

As used herein, "granulocytic myeloid derived suppressor cell (gMDSC) signature" refers to a signature comprising expression values for one or more of the following 43 genes: SERPINB1, SOD2, S100A8, CTSC, CCL18, CXCL2, PLAUR, NCF2, FPR1, IL8, S100A9, TNFAIP3, CXCL1, BCL2A1, EMR2, LILRB3, SLC11A1, IL6, TREM1, CCL20, LYN, CXCL3, IL1B, IL1R2, AQP9, IL2RA, GPR97, OSM, CXCR1, FPR2, C19orf59, CXCR2, CXCL6, CXCL5, EMR3, MEFV, S100A12, CD300E, FCGR3B, PPBP, LILRA5, LILRA3, and CASP5.

The gMDSC signature may comprise expression values for 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, or all 43 of the listed genes in any combination.

**Tumor Mutational Burden**

Tumor mutational burden (TMB, also referred to as a TMB score) may be determined by methods known in the art or, for example, as described in U.S. Appl. No. 16/789,288 and published as U.S. Pub. No. 2020/0258601 titled Targeted-Panel Tumor Mutational Burden Calculation Systems and Methods and filed 2/12/2020, herein incorporated by reference in its entirety. In some embodiments, TMB is calculated from mutations identified in a subject's DNA. In some embodiments, TMB is calculated from mutations identified in a subject's RNA. In some embodiments, TMB is calculated from mutations identified in a subject's DNA and RNA.

In some embodiments, a panel of genes is sequenced to determine TMB. In some embodiments, the panel includes 100-1000 genes. In some embodiments, the panel includes about 200, 300, 400, 500, 600, 700, 800, or about 900 genes. In some embodiments, the panel comprises at least about 650 genes. In some embodiments, the panel comprises one or more genes selected from the group consisting of ABCB1, ABCC3, ABL1, ABL2, FAM175A, ACTA2, ACVR1, ACVR1B, AGO1, AJUBA, AKT1, AKT2, AKT3, ALK, AMER1, APC, APLNR, APOB, AR, ARAF, ARHGAP26, ARHGAP35, ARID1A, ARID1B, ARID2, ARID5B, ASNS, ASPSCR1, ASXL1, ATIC, ATM, ATP7B, ATR, ATRX, AURKA, AURKB, AXIN1, AXIN2, AXL, B2M, BAP1, BARD1, BCL10, BCL11B, BCL2, BCL2L1, BCL2L11, BCL6, BCL7A, BCLAF1, BCOR, BCORL1, BCR, BIRC3, BLM, BMPR1A, BRAF, BRCA1, BRCA2, BRD4, BRIP1, BTG1, BTK, BUB1B, C11orf65, C3orf70, C8orf34, CALR, CARD11, CARM1, CASP8, CASR, CBFB, CBL, CBLB, CBLC, CBR3, CCDC6, CCND1, CCND2, CCND3, CCNE1, CD19, CD22, CD274, CD40, CD70, CD79A, CD79B, CDC73, CDH1, CDK12, CDK4, CDK6, CDK8, CDKN1A, CDKN1B, CDKN1C, CDKN2A, CDKN2B, CDKN2C, CEBPA, CEP57, CFTR, CHD2, CHD4, CHD7, CHEK1, CHEK2, CIC, CIITA, CKS1B, CREBBP, CRKL, CRLF2, CSF1R, CSF3R, CTC1, CTCF, CTLA4, CTNNA1, CTNNB1, CTRC, CUL1, CUL3, CUL4A, CUL4B, CUX1, CXCR4, CYLD, CYP1B1, CYP2D6, CYP3A5, CYSLTR2, DAXX, DDB2, DDR2, DDX3X, DICER1, DIRC2, DIS3, DIS3L2, DKC1, DNM2, DNMT3A, DOT1L, DPYD, DYNC2H1, EBF1, ECT2L, EGF, EGFR, EGLN1, EIF1AX, ELF3, TCEB1, C11orf30, ENG, EP300, EPCAM, EPHA2, EPHA7, EPHB1, EPHB2, EPOR, ERBB2, ERBB3, ERBB4, ERCC1, ERCC2, ERCC3, ERCC4, ERCC5, ERCC6, ERG, ERRFI1, ESR1, ETS1, ETS2, ETV1, ETV4, ETV5, ETV6, EWSR1, EZH2, FAM46C, FANCA, FANCB, FANCC, FANCD2, FANCE, FANCF, FANCG, FANCI, FANCL, FANCM, FAS, FAT1, FBXO11, FBXW7, FCGR2A, FCGR3A, FDPS, FGF1, FGF10, FGF14, FGF2, FGF23, FGF3, FGF4, FGF5, FGF6, FGF7, FGF8, FGF9, FGFR1, FGFR2, FGFR3, FGFR4, FH, FHIT, FLCN, FLT1, FLT3, FLT4, FNTB, FOXA1, FOXL2, FOXO1, FOXO3, FOXP1, FOXQ1, FRS2, FUBP1, FUS, G6PD, GABRA6, GALNT12, GATA1, GATA2, GATA3, GATA4, GATA6, GEN1, GLI1, GLI2, GNA11, GNA13, GNAQ, GNAS, GPC3, GPS2, GREM1, GRIN2A, GRM3, GSTP1, H19, H3F3A, HAS3, HAVCR2, HDAC1, HDAC2, HDAC4, HGF, HIF1A, HIST1H1E, HIST1H3B, HIST1H4E, HLA-A, HLA-B, HLA-C, HLA-DMA, HLA-DMB, HLA-DOA, HLA-DOB, HLA-DPA1, HLA-DPB1, HLA-DPB2, HLA-DQA1, HLA-DQA2, HLA-DQB1, HLA-DQB2, HLA-DRA, HLA-DRB1, HLA-DRB5, HLA-DRB6, HLA-E, HLA-F, HLA-G, HNF1A, HNF1B, HOXA11, HOXB13, HRAS, HSD11B2, HSD3B1, HSD3B2, HSP90AA1, HSPH1, IDH1, IDH2, IDO1, IFIT1, IFIT2, IFIT3, IFNAR1, IFNAR2, IFNGR1, IFNGR2, IFNL3, IKBKE, IKZF1, IL10RA, IL15, IL2RA, IL6R, IL7R, ING1, INPP4B, IRF1, IRF2, IRF4, IPS2, ITPKB, JAK1, JAK2, JAK3, JUN, KAT6A, KDM5A, KDM5C, KDM5D, KDM6A, KDR, KEAP1, KEL, KIF1B, KIT, KLF4, KLHL6, KLLN, KMT2A, KMT2B, KMT2C, KMT2D, KRAS, L2HGDH, LAG3, LATS1, LCK, LDLR, LEF1, LMNA, LMO1, LRP1B, LYN, LZTR1, MAD2L2, MAF, MAFB, MAGI2, MALT1, MAP2K1, MAP2K2, MAP2K4, MAP3K1, MAP3K7, MAPK1, MAX, MC1R, MCL1, MDM2, MDM4, MED12, MEF2B, MEN1, MET, MGMT, MIB1, MITF, MKI67, MLH1, MLH3, MLLT3, MN1, MPL, MRE11A, MS4A1, MSH2, MSH3, MSH6, MTAP, MTHFD2, MTHFR, MTOR, MTRR, MUTYH, MYB, MYC, MYCL, MYCN, MYD88, MYH11, NBN, NCOR1, NCOR2, NF1, NF2, NFE2L2, NFKBIA, NHP2, NKX2-1, NOP10, NOTCH1, NOTCH2, NOTCH3, NOTCH4, NPM1, NQO1, NRAS, NRG1, NSD1, WHSC1, NT5C2, NTHL1, NTRK1, NTRK2, NTRK3, NUDT15, NUP98, OLIG2, P2RY8, PAK1, PALB2, PALLD, PAX3, PAX5, PAX7, PAX8, PBRM1, PCBP1, PDCD1, PDCD1LG2, PDGFRA, PDGFRB, PDK1, PHF6, PHGDH, PHLPP1, PHLPP2, PHOX2B, PIAS4, PIK3C2B, PIK3CA, PIK3CB, PIK3CD, PIK3CG, PIK3R1, PIK3R2, PIM1, PLCG1, PLCG2, PML, PMS1, PMS2, POLD1, POLE, POLH, POLQ, POT1, POU2F2, PPARA, PPARD, PPARG, PPM1D, PPP1R15A, PPP2R1A, PPP2R2A, PPP6C, PRCC, PRDM1, PREX2, PRKAR1A, PRKDC, PARK2, PRSS1, PTCH1, PTCH2, PTEN, PTPN11, PTPN13, PTPN22, PTPRD, PTPRT, QKI, RAC1, RAD21, RAD50, RAD51, RAD51B, RAD51C, RAD51D, RAD54L, RAF1, RANBP2, RARA, RASA1, RB1, RBM10, RECQL4, RET, RHEB, RHOA, RICTOR, RINT1, RIT1, RNF139, RNF43, ROS1, RPL5, RPS15, RPS6KB1, RPTOR, RRM1, RSF1, RUNX1, RUNX1T1, RXRA, SCG5, SDHA, SDHAF2, SDHB, SDHC, SDHD, SEC23B, SEMA3C, SETBP1, SETD2, SF3B1, SGK1, SH2B3, SHH, SLC26A3, SLC47A2, SLC9A3R1, SLIT2, SLX4, SMAD2, SMAD3, SMAD4, SMARCA1, SMARCA4, SMARCB1, SMARCE1, SMC1A, SMC3, SMO, SOCS1, SOD2, SOX10, SOX2, SOX9, SPEN, SPINK1, SPOP, SPRED1, SRC, SRSF2, STAG2, STAT3, STAT4, STAT5A, STAT5B, STAT6, STK11, SUFU, SUZ12, SYK, SYNE1, TAF1, TANC1, TAP1, TAP2, TARBP2, TBC1D12, TBL1XR1, TBX3, TCF3, TCF7L2, TCL1A, TERT, TET2, TFE3, TFEB, TFEC, TGFBR1, TGFBR2, TIGIT, TMEM127, TMEM173, TMPRSS2, TNF, TNFAIP3, TNFRSF14, TNFRSF17, TNFRSF9, TOP1, TOP2A, TP53, TP63, TPM1, TPMT, TRAF3, TRAF7, TSC1, TSC2, TSHR, TUSC3, TYMS, U2AF1, UBE2T, UGT1A1, UGT1A9, UMPS, VEGFA, VEGFB, VHL, C10orf54, WEE1, WNK1, WNK2, WRN, WT1, XPA, XPC, XPO1, XRCC1, XRCC2, XRCC3, YEATS4, ZFHX3, ZMYM3, ZNF217, ZNF471, ZNF620, ZNF750, ZNRF3, and ZRSR2. In some embodiments, a panel comprises each of the above-listed genes. In some embodiments, a panel consists of each of the above genes.

In some embodiments, TMB is calculated as the number of non-synonymous somatic mutations identified in the panel divided by the amount of DNA sequenced, using, for example, the variant annotation output from a tumor-normal matched targeted sequencing panel for oncology patient specimens and the bioinformatics variant calling pipeline corresponding to the sequencing panel (see, for example Beaubier *et al.,* (2019) (Equation 1, below). Somatic variants are defined as non-synonymous if the variant results in change to the amino acid sequence of the protein.

$$\text{Equation 1: } TMB = \frac{(number\ of\ non-synonymous\ somatic\ mutations)}{(megabases\ of\ DNA\ sequenced)}$$

Thus, in some embodiments, TMB is calculated as the integer number of non-synonymous somatic mutations divided by the number of megabases of genomic DNA (*e.g.,* using, for example, the variant annotation output from a tumor-normal matched targeted sequencing panel for oncology patient specimens and the bioinformatics variant calling pipeline corresponding to the sequencing panel). In some embodiments, the TMB calculation does not include synonymous mutations. In some embodiments, the TMB calculation does include synonymous mutations.

**Multiple Model Components**

Multiple model components may be used to determine the IPS. For example, the methods may comprise the following exemplary model components: a tumor mutational burden (TMB), checkpoint related gene signature, an immune exhaustion signature, a granulocytic myeloid derived suppressor cell (gMDSC) signature, and an immune oncology signature. Each of the model components may be derived from sequencing data and may be used in the disclosed methods in any combination or sub-combination.

The methods may comprise at a computer system having one or more processors, and memory storing one or more programs for execution by the one or more processors: applying, by the one or more processors, one or more model components derived from sequencing data from a sample to one or more machine learning algorithms (MLAs), wherein the sequencing data comprises RNA sequencing data and DNA sequencing data, wherein the one or more model components comprise a tumor mutational burden (TMB), checkpoint related gene signature, an immune exhaustion signature, a granulocytic myeloid derived suppressor cell (gMDSC) signature, and an immune oncology signature.

In some embodiments, model components may each be applied to different MLAs then integrated using another MLA to generate the IPS. Individual features and/or MLA outputs can also be re-combined with MLAs architectures to produce a meta-model or multi-modal IPS model.

In some embodiments, the models may generate an IPS as a linear combination of the coefficients of each of the model features. The combination of the model features may further be min-max scaled to fall between 0-100. The threshold for IPS-low may be set at all patients below the 55th percentile among the full training cohort, IPS-high may be set at greater than or equal to the 60th percentile, and the patients between the 55th and 60th percentiles may form an indeterminate category.

**Sequencing**

Sequencing of nucleic acids, e.g., next generation sequencing RNA and DNA sequencing may be performed according to known methods. RNA or DNA sequencing may be performed using commercially available reagents and platforms.

The sequencing reactions may be performed using a panel of probes for detecting, e.g., about 100 genes to about 20,000, or any subrange therein, e.g., about 100 genes to about 1000 genes. The panel may detect about 100, about 200, about 300, about 400, about 500, about 600, about 700, about 800, about 900, about 1000 genes, or more.

RNA sequencing may be performed and the read data may be processed to detect genetic fusions, e.g., about 1 to about 100 genetic fusions. The fusions may be pathogenic fusions, including, but not limited to, fusions that result in an activating mutation of an oncogene, a silencing mutation of a tumor suppressor, or a copy number variation of a gene.

The sequencing data may comprise data generated by a targeted panel for sequencing normal-matched tumor tissue, or, in an exemplary embodiment, could be tumor tissue only, wherein the panel detects single nucleotide variants, insertions and/or deletions, and copy number variants in 598-648 genes and chromosomal rearrangements in 22 genes.

The sequencing data may comprise full exome or full transcriptome sequencing data.

In some embodiments, the methods comprise at a computer system having one or more processors, and memory storing one or more programs for execution by the one or more processors: (A) receiving sequencing data from a sample of the cancer from the subject, wherein the sequencing data comprises RNA sequencing data, wherein the RNA sequencing data comprises a plurality of data elements comprising expression values for a plurality of genes; and applying one or more model components to one or more models to determine the IPS for the subject. The sequencing data may comprise both RNA sequencing data and DNA sequencing data. Methods of performing RNA and DNA sequencing and processing data from RNA and DNA sequencing reactions are known in the art.

**Systems and non-transitory computer readable media**

The disclosed systems may comprise a computer comprising one or more processor configured to perform any of the disclosed methods, e.g., methods of determining an IPS for a subject, methods of identifying a subject as a candidate for IO therapy, or methods of treating cancer in a subject in need thereof.

The disclosed non-transitory computer readable medium may comprise instructions that, when executed by a computer comprising one or more processor, cause the processor to perform any of the disclosed methods.

In some embodiments, computer systems are provided, wherein the computer systems comprise one or more processors, and memory storing one or more programs for execution by the one or more processors. In some embodiments, one or more models are also provided in the computer system. In some embodiments, the one or more models are individually or collectively trained to provide output data (for example, a binary output, or a continuous output), wherein the output data is derived from input data to which the one or more models are applied. The output data may be used to determine whether a patient is likely to respond to IO therapy (including checkpoint inhibitor) or likely to experience a progression event within a specified amount of time of starting to receive IO therapy. By way of example, input data may comprise, in electronic form, nucleic acid data, such as sequence reads, and features derived from the nucleic acid data. Input data may also comprise clinical information, genetic information, treatment information, treatment outcome information, tumor-specific information (origin, cancer type, size, description, growth rate, etc.), and the like. Input data may comprise HLA class I gene status, and/or tumor mutation burden information. Additional exemplary features that may be input into the system are described below.

The features can be used alone or combined with clinical and/or genomic (DNA), transcriptomic (RNA), or other molecular features to create a feature set for model training. Examples of features may include TMB (continuous and/or binary), driver vs. passenger status of a variant, HLA LOH, immune repertoire sequencing (for example, TCR and/or BCR sequencing), single-cell data (for example, single-cell DNA and/or RNA sequencing, FACS, single-cell surface protein analysis, single-cell TCR profiling, etc.), Resistance gene mutation status, Pathway mutation status, Co-mutation status, Somatic signatures, CD274 (PDL1) expression, Other checkpoint gene expression, Published IO RNA gene signatures, including CYT index, (Rooney, M. S., Shukla, S. A., Wu, C. J., Getz, G. & Hacohen, N. Molecular and Genetic Properties of Tumors Associated with Local Immune Cytolytic Activity. Cell 160, 48-61 (2015)), GEP score (Ayers, M. et al. IFN-γ-related mRNA profile predicts clinical response to PD-1 blockade. J Clin Invest 127, 2930-2940 (2017).), IMPRES (Auslander, N. et al. Robust prediction of response to immune checkpoint blockade therapy in metastatic melanoma. Nat Med 24, 1545-1549 (2018).), Roh score (Roh, W. et al. Integrated molecular analysis of tumor biopsies on sequential CTLA-4 and PD-1 blockade reveals markers of response and resistance. Sci Transl Med 9, eaah3560 (2017)), NRS score (Huang, A. C. et al. A single dose of neoadjuvant PD-1 blockade predicts clinical outcomes in resectable melanoma. Nat Med 25, 454-461 (2019)). Differentially expressed genes determined by comparing expression levels of progressors and non-progressors at 6 months (or other time periods), Pathway expression, WGCNA gene modules, HLA expression.

In one embodiment, each training RNA data set (for example, each set of RNA data may be associated with a unique RNA sequencing run performed on RNA isolated from a unique specimen and/or cDNA associated with that isolated RNA) used to train the disclosed machine learning algorithms may be associated with a continuous TMB score (for example, number of mutations per sequenced megabase). In another embodiment, each training RNA data set may be associated with a binary TMB score (for example, 1 if TMB is above the TMB threshold and 0 if TMB is below the TMB threshold). In various embodiments, the TMB scores associated with any two training RNA data sets

**Cancers**

The methods, systems, and compositions described herein are not limited to the tumor types exemplified herein (*e.g.*, bladder cancer, non-small cell lung cancer, colorectal cancer, and liver cancer). Any solid tumor may be tested or treated using the disclosed methods.

In some embodiments, the subject is suffering from cancer and has or is suspected of having a loss of heterozygosity in a HLA gene (HLA-LOH). When HLA-LOH occurs in the class I HLA locus in the tumor, CD8+ T cells are no longer able to recognize and kill tumor cells. Studies have shown that this is a common mechanism of immune escape and is associated with worse outcomes for patients treated with immunotherapy, *e.g.*, immune checkpoint blockade (4,5). Surprisingly, however, some patients with HLA-LOH do respond to immunotherapy as measured by progression free survival.

A patient may have an HLA-LOH affecting any HLA class I protein. By way of example only, but not by way of limitation, the patient may have a loss of function mutation in beta-2-microglobulin (B2M), a gene that encodes the beta chain of MHC class I molecules. B2M mutations have been identified in multiple cancer types, including colorectal, uterine, stomach, lung, skin and head and neck cancer. A B2M mutation may suggest that a patient is deficient in HLA-I antigen presentation.

As used herein, "stage 0 cancer" refers to a situation in which there is no cancer, but abnormal cells are present, with the potential to become cancerous.

As used herein, "stage I cancer" refers to a small tumor localized to a single site. Stage 1 cancer is also termed "early stage cancer."

As used herein, the term "stage II cancer" refers to a cancer that is larger (has grown) but has not spread to other tissues or organs.

As used herein, the term "stage III cancer" refers to a cancer that is larger (has grown) and that may have spread to other tissues, organs and/or lymph nodes.

As used herein, the term "stage IV cancer" refers to a cancer that has spread from where it started to other parts of the body, and is also termed "metastatic cancer" or "advanced cancer."

**Engine for Predicting Response to Immunotherapy and/or IO Progression Risk**

In some examples, an engine for predicting a response to immunotherapy may be utilized in accord with patient management. Such an engine may be trained on one or more features or signature disclosed herein. Exemplary non-limiting features are described below. In various embodiments, an engine may be retrained, for example, after training data quality control has been performed, different and/or additional training data have been selected, or training data have been otherwise updated or changed.

**Methods of Treatment**

The present invention further provides methods for treating cancer. The methods may be utilized as assessment of whether the patient will respond favorably or unfavorably to a checkpoint inhibitor therapy or to select subjects that are candidates for IO, e.g., ICI, therapy.

Accordingly, determining the susceptibility of a subject's tumor tissue to a therapeutic agent such as an ICI allows for more effective treatment, resulting in improved treatment outcomes, e.g., overall survival time, tumor regression, complete or partial remission, reduction in the number tumors, reduction in the grade of tumor for subjects suffering from various forms of cancer.

A used herein, a "favorable response" or "favorable outcome" refers to a response to therapy that includes reducing, alleviating, inhibiting or preventing one or more cancer symptoms, reducing, inhibiting or preventing the growth of cancer cells, reducing, inhibiting or preventing metastasis of the cancer cells or invasiveness of the cancer cells or metastasis, or reducing, alleviating, inhibiting or preventing one or more symptoms of the cancer or metastasis thereof, longer progression free survival time, or increasing the survival time of the patient, as compared to an appropriate control. By contrast, an "unfavorable response" or "unfavorable outcome" is any response that does not result in any of the above-mentioned effects.

As used herein, the term or "immuno-oncology treatment" or "IO treatment" is used to refer to a cancer treatment

EP 4 550 337 A1

that stimulates the patient's immune system to destroy cancer cells. An exemplary IO therapy comprises checkpoint inhibitors.

In some embodiments, subjects with cancer and at risk of or diagnosed with HLA-LOH may be candidates for one or more checkpoint inhibitor therapies. As used herein, the term "immune checkpoint inhibitor" or "ICI" refers to molecules that totally or partially reduce, inhibit, interfere with or modulate one or more checkpoint proteins. Checkpoint proteins and their ligands are expressed by certain types of immune cells (*e.g.,* T cells, macrophages) as well as by some cancer cells. Checkpoint proteins serve to keep immune responses in check. However, they also inhibit the activation of T cells, thereby preventing them from responding to or killing cancer cells. Immune checkpoint activation can also limit the duration and intensity of T cell responses. Checkpoint inhibitor therapies commonly work by binding to a checkpoint protein and blocking its ability to interact with T cells. When checkpoint proteins are blocked, their suppressive effect on the immune system is released, allowing T cells to respond to tumor antigens and kill cancer cells.

Common checkpoint inhibitor protein targets include, for example, cytotoxic T-lymphocyte-associated protein 4 (CTLA4; also known as CD152), programmed cell death 1 (PD-1), PD-1 ligand 1 (PD-L1), lymphocyte activation gene-3 (LAG-3), 4-1BB (also known as CD137), B7-H3, OX40, and T-cell immunoglobulin and mucin domain-3 (TIM3). Checkpoint inhibitors are commonly antibodies or derivatives of antibodies. Checkpoint blockade may include immune reactivation. The disclosed methods can potentially be applied to any checkpoint inhibitor regimen that is used to treat solid tumors. Suitable regimens include those that utilize checkpoint inhibitors such as pembrolizumab, nivolumab, ipilimumab, atezolizumab, cemiplimab, durvalumab, and avelumab. A checkpoint inhibitor therapy can be administered with another checkpoint inhibitor therapy or may be administered with another cancer therapy (e.g., radiation, surgery, hormone therapy, a chemotherapy, etc.). Exemplary checkpoint inhibitor combination therapies include but are not limited to the ipilimumab and nivolumab.

In some embodiments, the checkpoint inhibitor is administered as part of a combination therapy. Suitable combination therapies include, for example, pembrolizumab, paclitaxel, and carboplatin; pembrolizumab, nab-paclitaxel, and carboplatin; pembrolizumab, pemetrexed, and carboplatin; atezolizumab, bevacizumab, paclitaxel, and carboplatin; or ipilimumab and nivolumab.

The checkpoint inhibitors used with the present invention should be administered in a therapeutically effective amount. The terms "effective amount" or "therapeutically effective amount" refer to an amount sufficient to effect beneficial or desirable biological or clinical results. That result can be reducing, alleviating, inhibiting or preventing one or more symptoms of a disease or condition, reducing, inhibiting or preventing the growth of cancer cells, reducing, inhibiting or preventing metastasis of the cancer cells or invasiveness of the cancer cells or metastasis, or reducing, alleviating, inhibiting or preventing one or more symptoms of the cancer or metastasis thereof, or any other desired alteration of a biological system. In some embodiments, the effective amount is an amount suitable to provide the desired effect, *e.g.,* anti-tumor response. An anti-tumor response may be demonstrated, for example, by a decrease in tumor size or an increase in immune cell activation (*e.g.,* CD8+ or CD4+ T cell activation).

Methods for determining an effective means of administration and dosage are well known to those of skill in the art and will vary with the formulation used for therapy, the purpose of the therapy, the target cell being treated, and the subject being treated. Single or multiple administrations can be carried out with the dose level and pattern being selected by the treating physician. For example, the checkpoint inhibitor pembrolizumab is typically administered in 200 mg doses every 3 weeks or 400 mg doses every 6 weeks for the treatment of NSCLC. Similarly, when pembrolizumab is administered in combination with paclitaxel and carboplatin it is typically administered in 200 mg doses every 3 weeks or 400 mg doses every 6 weeks.

As described above, therapeutic compositions disclosed herein include checkpoint inhibitors. Such compositions can be formulated and/or administered in dosages and by techniques well known to those skilled in the medical arts taking into consideration such factors as the age, sex, weight, tumor type and stage, condition of the particular patient, and the route of administration.

The compositions may include pharmaceutical solutions comprising carriers, diluents, excipients, preservatives, and surfactants, as known in the art. Further, the compositions may include preservatives (*e.g.*, anti-microbial or anti-bacterial agents such as benzalkonium chloride). The compositions also may include buffering agents (*e.g.*, in order to maintain the pH of the composition between 6.5 and 7.5).

In some embodiments, compositions are formulated for systemic delivery, such as oral or parenteral delivery. In some embodiments, minimally invasive microneedles and/or iontophoresis may be used to administer the composition. In some embodiments, compositions are formulated for site-specific administration, such as by injection into a specific tissue or organ, topical administration (*e.g.*, by patch applied to the target tissue or target organ).

The therapeutic composition may include, in addition to checkpoint inhibitor, one or more additional active agents. By way of example, the one or more active agents may include an additional chemotherapeutic drug, an antibiotic, anti-inflammatory agent, a steroid, or a non-steroidal anti-inflammatory drug.

In some embodiments, in addition to one or more therapeutic formulations, a subject is also administered an additional cancer treatment, such as surgery, radiation, immunotherapy, stem cell therapy, and hormone therapy.

In some embodiments, improvements in the condition of the subject's cancer status and overall health is observed more quickly than if no treatment is provided for the same or similar condition or disease.

In some embodiments, the therapeutic composition comprises a bispecific antibody that targets immune cells, such as cytotoxic CD4+ T cells, to tumors. A bispecific antibody is an artificial protein that can simultaneously bind to two different antigens. For example, the bispecific antibody may have a fIPSt domain that binds to a cytotoxic CD4+ T cell-specific cell surface marker and a second domain that binds to a tumor-specific antigen, thereby bring the T cells into close proximity with the tumor. Exemplary, non-limiting cytotoxic CD4+ T cell markers include CD4, granzymes, and perforin, and exemplary, non-limiting tumor specific antigens include CEA, EpCAM, HER2 and EGFR.

With respect to the IO Progression Risk, in some embodiments, a score reflecting probability of a progression event occurring in 3 months and a score reflecting probability of a progression event occurring in 6 months may be provided. This score can then be converted to categories based on a predefined operating point (for example, a user defined threshold) and results are reported to physicians as either 'increased progression risk' or 'no increased progression risk detected.'

Such information will help the clinician interpret patient symptoms, for example, with cross-sectional imaging for monitoring of IO treated patients. In one possible scenario, the clinician could opt for shorter intervals between imaging studies for 'increased risk' subjects, or interpret radiographic changes on cross-sectional imaging with a higher pre-test probability for disease progression and prepare for testing such as CNS imaging and/or transitioning toward the next line of therapy. Accurately refining pre-test probability may inform clinical judgment and lead to better outcomes by identifying progression events sooner, limiting usage of ineffective and costly IO regimens, and improving patient quality of life by potentially transitioning to the next line of therapy before asymptomatic progression becomes symptomatic progression.

**Reports**

The methods may further comprise generating a clinical report comprising the immune profile score (IPS). The clinical report may be electronic or be produced in a paper form.

The methods may further comprise administering a therapeutically effective amount of an immune oncology therapy to the subject based on the report.

The methods may further comprise administering a therapeutically effective amount of an additional therapy to the subject selected from the group consisting of a chemotherapy, a hormone therapy, a targeted therapy, and a radiation therapy, based on the report.

The clinical report may indicate a particular IO therapy for use in treatment of the subject. In other words, the method may determine that a genus or IO therapies, or a particular IO therapy may be most successful for treatment of the subject, which may be reflected in the report. The IO therapy may be an immune checkpoint inhibitor (ICI).

The IPS may be reported as a numerical value from 1 to 100. In other embodiments, another numerical range may be used. For example, the range may be 0 to 1, 1 to 50, -1 to 1, - 10 to 10, etc.

The IPS may be reported categorically. The reported IPS may comprise 2 or more categories, wherein the categories are based on the likelihood of the subject to respond to an IO therapy, e.g., an ICI therapy. For example, the categories may comprise IPS-High, IPS-Intermediate, and IPS-Low, wherein subjects determined to be IPS-High are more likely to have a longer survival or progression-free survival after treatment with an IO therapy. The categories may be IPS-Low, indeterminate, and IPS-High. The categories may be determined empirically, e.g., the thresholds for each category may be determined for a pan-cancer cohort of subjects or a sub-cohort of subjects, e.g., subjects with a single type of cancer, e.g., NSCLC and may be determined using a separate MLA to optimize the thresholds for the categories. The categories may be as follows: IPS-Low (scores 0-44), IPS-High (scores 48-100) and scores between 45-47 may be classified as Indeterminate.

The IPS may indicate that the subject's cancer is likely to progress on an IO therapy, and, accordingly, the clinical report indicates one or more additional therapies for use in treating the subject for the cancer.

The methods may further comprise administering a therapeutically effective amount of the one or more additional therapies indicated in the clinical report. The one or more additional therapies may be selected from: a chemotherapy, a hormone therapy, a targeted therapy, and a radiation therapy.

**IO Progression Risk**

As described above, the tumor immune microenvironment (TIME) modulates tumor killing by immune cells and has prognostic value in determining the clinical course and survival of an individual patient. The methods and systems disclosed herein may be used to analyze DNA and RNA sequences to measure tumor and immune intrinsic mechanisms of sensitization to IO in the TIME, including the tumor mutational burden of the cancer (TMB) and the cytotoxicity of tumor infiltrating immune cells (e.g., by determining the presence or absence of an IPS.).

In some embodiments, the systems and methods disclosed herein comprise a predictive algorithm that analyzes measurements associated with a patient specimen to generate a score reflecting probability of a progression event.

In some embodiments, the IPS reflects the probability of an event occurring in 3 months; in some embodiments, the IPS score reflects the probability of a progression event occurring in 6 months. In some embodiments, the IPS reflects the probability of a progression event occurring in 3 months and 6 months. In some embodiments, a single model assigns patients into high and low risk populations, or to 2 or more different populations. By way of example, using the Kaplan Meier methods, we can estimate what fraction in each population is likely to progress within 3 months and within 6 months.

For example, a clinician could opt for shorter intervals between imaging studies for a subject with an 'increased risk' result or interpret radiographic changes on cross-sectional imaging with a higher pre-test probability for disease progression and prepare for testing such as CNS imaging and/or transitioning toward the next line of therapy. Accurately refining pre-test probability may inform clinical judgment and lead to better outcomes by identifying progression events sooner, limiting usage of ineffective and costly IO regimens, and improving patient quality of life by potentially transitioning to the next line of therapy before asymptomatic progression becomes symptomatic progression.

In some embodiments, an IPS is used for patients diagnosed with non-small cell lung cancer (NSCLC) with a non-squamous histology subtype that will be prescribed IO therapy regimens. In some embodiments, patients have stage IV disease or an earlier stage disease with a metastasis event and have had no prior treatment with IO therapy regimens.

**Indications**

The disclosed methods can be used to detect subjects that are good candidates for IO therapies or are likely to respond to IO therapies regardless of the type of cancer from which the subject is suffering. However, the disclosed methods may be used for assisting decision making in additional clinical situations including the following non-limiting list:

I. Metastatic non-small cell lung carcinoma (mNSCLC) (adenocarcinoma)

Patient population: mNSCLC, adenocarcinoma, PD-L1≥50%
Line of treatment: 1st line in metastatic setting
Decision: IO monotherapy vs. IO+chemo combotherapy
Context: For PD-L1≥50% patients, both mono- and combotherapy are available. Currently, the decision is made primarily based on signs of aggressive disease (e.g., tumor burden, STK11, KEAP1) and patient tolerance for chemo
Basic inclusion/exclusion (I/E):

mNSCLC
Anti-PD-(L)1 alone or with chemo in metastatic 1st line setting
No driver mutations in EGFR, ALK, ROS1, RET, NTRK1/2/3, or HER2

II. mNSCLC Squamous (same as adeno)

Patient population: mNSCLC, squamous, PD-L1≥50%
Line of treatment: 1st line in metastatic setting
Decision:

PDL1 >50 is still relevant group to assess
IO monotherapy vs. IO+chemo combotherapy
Context: For PD-L1≥50% patients, both mono- and combotherapy are available. Currently, the decision is made primarily based on signs of aggressive disease (e.g., tumor burden, STK11, KEAP1) and patient tolerance for chemo
Basic I/E:

mNSCLC
Anti-PD-(L)1 alone or with chemo in metastatic 1st line setting
No driver mutations in EGFR, ALK, ROS1, RET, NTRK1/2/3, or HER2
Notes: More central disease needs rapid treatment

III. HNSCC

Patient population: Squamous histology of the head and neck, Metastatic / advanced, Received IO in the fIPSt line
Basic I/E: PDL1 >1

Notes

Control cohorts of interest, PDL1 >1 who received non-IO treatments in the fIPStline, We can identify these patients using RNAseq if no PDL1 IHC available, Regimens of interest will include doublet chemo or chemo + TKI, HPV status (can determined using sequencing data), Smoking

Prognostic factors - Patient-specific factors that influence prognosis need to be considered when choosing therapy: Factors associated with longer survival in patients include the following: Ambulatory performance status (Eastern Cooperative Oncology Group [ECOG] 0 or 1 versus 2 (table 4)), Prior response to chemotherapy, Longer time since completion of definitive therapy, HPV associated oropharyngeal cancers,

Factors associated with a poor prognosis include the following: Weight loss, Poor performance status, Prior radiation therapy, Active smoking, Significant comorbidity

## IV. Advanced / metastatic clear cell renal cell carcinoma

Line of treatment: FIPSt or second line

Decision: Whether to give IO/IO (ipi/nivo), IO/TKI, or TKI as fIPSt line or second line of treatment

Basic I/E: Clear cell renal cell carcinoma ccRCC, Exclude any patients who received IO in the adjuvant setting

Notes, Covariates, Adjuvant therapy y/n, Cytoreduction y/n, Prognostic group based on International Metastatic Renal Cell Carcinoma Database Consortium (IMDC) prognostic model, Karnofsky performance status (KPS) <80 percent, Time from diagnosis to treatment <1 year, Hemoglobin concentration less than the lower limit of normal, Serum calcium greater than the upper limit of normal, Neutrophil count greater than the upper limit of normal, Platelet count greater.

## V. Bladder cancer

Patient population: Advanced / metastatic urothelial carcinoma 0

Line of treatment: FIPSt or second line

Decision: Currently IO in the fIPSt line is given to patients who are not eligible for cisplatin based regimens, or it is given in the second line after progression on cisplatin. It may be worth evaluating IO biomarkers in both the fIPSt and second line as different subgroups.

Context:

Basic I/E:

IO therapy given in the fIPSt or second line

Notes

Control group

Cisplatin treated patients in the fIPSt line.

## VI. Melanoma

**Patient population:** Advanced / metastatic cutaneous melanoma

**Line of treatment:** FIPSt line

**Decision:**

IPI/NIVO vs Nivo

Influenced by prognostic factors

**Context:**

**Basic I/E:**

Cutaneous melanoma

Any IO treated patients in the fIPSt line

Exclude patients who received adjuvant IO therapy

Analysis

Primary

BRAFwt

Secondary / optional

BRAFmut

IO treated - informs emerging use case
TKI treated - could be used to demonstrate signatures are predictive vs prognostic

**Notes**

May want to consider dosing change in ipi - 3mg / kg (trials) versus 1mg / kg (in practice) Approvals for Melanoma:

| Therapy | Stage IV | Earlier stages |
|---|---|---|
| pembrolizumab | for the treatment of patients with unresectable or metastatic melanoma. | for the adjuvant treatment of adult and pediatric (12 years and older) patients with Stage IIB, IIC, or III melanoma following complete resection. |
| Nivolumab (+/- Ipilimumab) | adult and pediatric (12 years and older) patients with unresectable or metastatic melanoma, as a single agent or in combination with ipilimumab. adult and pediatric (12 years and older) patients with melanoma with lymph node involvement or metastatic disease who have undergone complete resection, in the adjuvant setting. | |
| Nivolumab (+ Relatlimab) | indicated for the treatment of adult and pediatric patients 12 years of age or older with unresectable or metastatic melanoma. | |
| Atezolizumab | in combination with cobimetinib and vemurafenib for the treatment of adult patients with **BRAF V600 mutation-positive** unresectable or metastatic melanoma. | |

**PAN-CANCER LAST LINE** (clinical trial)
*Similar to the TMB pan-cancer indication*
**Patient population:** Pan-cancer (unresectable or metastatic)
**Line of treatment:** Last line (patients that have progressed following prior treatment and who have no satisfactory alternative treatment options)
**Decision:** Should patient receive ICI monotherapy or not
**Triple negative breast cancer (TNBC)** (clinical trial)
**Patient population:** TNBC, PD-L1=0 and/or PD-L1 1-9%
**Line of treatment:** FIPSt line metastatic , any treatment
**Decision:**

There might be a decision point around whether to give IO or other treatments like Enhertu in patients with PDL1 >10
How many patients above and below CPS 10

**Context:**
**Basic I/E:**

Received IO in first line
BRCA negative
PDL1 >1

**Notes**
Subcohort analysis will likely be PDL1 1-10
**Colorectal cancer (CRC)** (clinical trial)
**Patient population:** CRC MSS
**Line of treatment:** Any
**Decision:** No current approval for IO in MSS

**Context:** NA
**Basic I/E:**

MSS CRC
Gastric / GEJ

**Decision:** 1st line ICI+chemo vs. chemo for PD-L1 low

**Further Applications and advantages of the immune profile score**

While the majority of metastatic NSCLC patients are being treated with checkpoint inhibitor (CPI) agents in the fIPSt line as part of the standard of care, there are few tools for assessing a patients' risk for progression prior to the start of treatment. As currently practiced, there is substantial variation in acceptable surveillance regimens for NSCLC patients during IO treatment, with routine follow-ups consisting of CT scans scheduled every three to six months with the purpose of detecting recurrent tumors. However, such routine scheduled follow-ups can delay diagnosis and treatment if recurrence occurs between planned visits. Furthermore, the standard of care on-treatment radiologic assessments of response can be more challenging to interpret for this patient population due to the risk of pseudo-progression, which is a transient enlargement of the tumor from elevated immune infiltration rather than a true increase in tumor burden. With the IPS test, a physician will have additional information on a patient's risk of progression when deciding the cadence of on-treatment radiologic assessments and when interpreting inconclusive radiology results. The IPS test would support physicians in identifying the optimal scan intervals for their patients.

Metastatic NSCLC patients have a substantial symptom burden and physicians seek to balance using aggressive treatment for reducing tumor burden with management of patient quality of life. The IPS test aids physicians in identifying patients at higher risk for disease progression on CPI. These high-risk patients can then be prioritized for more frequent radiologic scans to facilitate earlier detection of their disease progression, allowing physicians to begin considering alternative therapies or the transition to palliative care sooner. This improved patient management may lead to improved clinical care.

In various embodiments, the systems and methods might inform the choice of immune checkpoint regimen when multiple options exist for specific patient subsets (for example, if PD-L1 IHC > 50%).

The sooner disease progression on CPI can be identified, the earlier physicians can begin considering alternative treatment regimens that may be more effective or the transition to palliative care to optimize patient comfort.

In some embodiments, computing device 104 and/or server 116 can be any suitable computing device or combination of devices, such as a desktop computer, a laptop computer, a smartphone, a tablet computer, a wearable computer, a server computer, a virtual machine being executed by a physical computing device, etc. As described herein, system 100 can present information about the characterized protein to a user (e.g., a researcher and/or a physician).

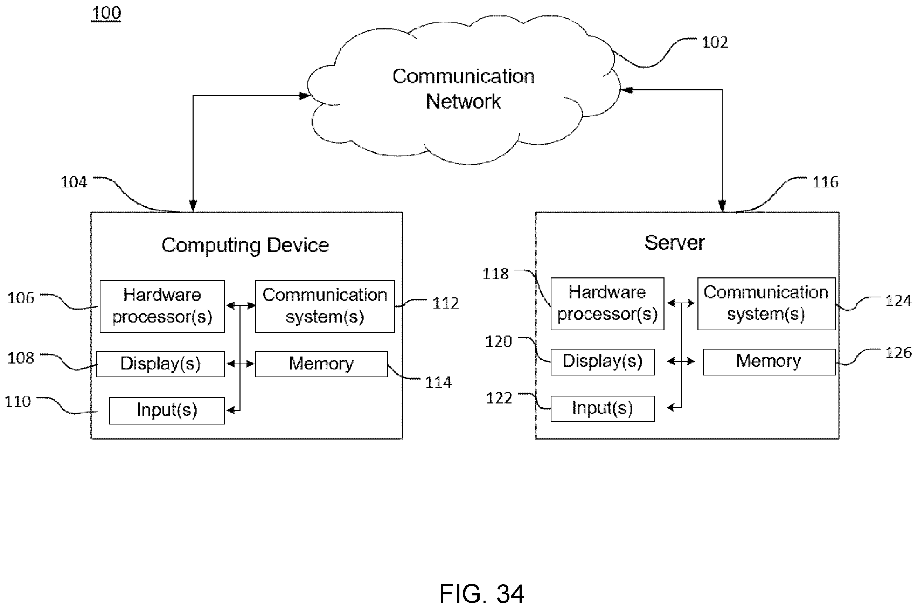

In some embodiments, communication network 102 can be any suitable communication network or combination of communication networks. In some embodiments, communication network 1002 can be any suitable communication network or combination of communication networks. For example, communication network 102 can include a Wi-Fi network (which can include one or more wireless routers, one or more switches, etc.), a peer-to-peer network (e.g., a Bluetooth network), a cellular network (e.g., a 4G network, a 5G network, etc., complying with any suitable standard, such as CDMA, GSM, LTE, LTE Advanced, WiMAX, etc.), a wired network, etc. In some embodiments, communication network 102 can be a local area network, a wide area network, a public network (e.g., the Internet), a private or semi-private network (e.g., a corporate or university intranet), any other suitable type of network, or any suitable combination of networks. Communications links shown in FIG. 34 can each be any suitable communications link or combination of communications links, such as wired links, fiber optic links, Wi-Fi links, Bluetooth links, cellular links, etc.

FIG. 34 additionally shows an example of hardware that can be used to implement computing device 104 and server 116 in accordance with some embodiments of the disclosed subject matter. In some embodiments, computing device 104 can be used to execute one or more set of instructions to identify a behavioral catalog. In other embodiments, computing device 104 can be used to identify therapeutic interventions. In still other embodiments, computing device 104 can be used to identify a configuration of parameter of a gene regulatory network to perform a desired function.

As shown in FIG. 34, computing device 104 can include one or more hardware processor 106, one or more displays 108, one or more inputs 110, one or more communications 112, and/or memory 114. In some embodiments, processor 106 can be any suitable hardware processor or combination of processors, such as central processing unit, a graphics processing unit, etc. In some embodiments, display 108 can include any suitable display devices, such as a computer monitor, a touchscreen, a television, etc. In some embodiments, inputs 110 can include any suitable input device and/or sensors that can be used to receive user input, such as a keyboard, a mouse, a touchscreen, a microphone, etc.

In some embodiments, communication systems 112 can include any suitable hardware, firmware, and/or software for communicating information over communication network 102 and/or any other suitable communication networks. For example, communications systems 112 can include one or more transceivers, one or more communication chips and/or chip sets, etc. In a more particular example, communications systems 112 can include hardware, firmware and/or software that can be used to establish a Wi-Fi connection, a Bluetooth connection, a cellular connection, an Ethernet connection, etc.

In some embodiments, memory 114 can include any suitable storage device or devices that can be used to store instructions, values, etc., that can be used, for example, by processor 1006 to present content using display 1008, to communicate with server 1016 via communications system(s) 1012, etc.

Memory 114 can include any suitable volatile memory, non-volatile memory, storage, or any suitable combination thereof. For example, memory 114 can include RAM, ROM, EEPROM, one or more flash drives, one or more hard disks, one or more solid state drives, one or more optical drives, etc. In some embodiments, memory 114 can have encoded thereon a computer program for controlling operation of computing device 1004. In such embodiments, processor 106 can execute at least a portion of the computer program to present content (e.g., images, user interfaces, graphics, tables, etc.), receive content from server 116, transmit information to server 116, etc.

In some embodiments, server 116 can include a processor 118, a display 120, one or more inputs 122, one or more communications systems 124, and/or memory 126. In some embodiments, processor 118 can be any suitable hardware processor or combination of processors, such as a central processing unit, a graphics processing unit, etc. In some embodiments, display 120 can include any suitable display devices, such as a computer monitor, a touchscreen, a television, etc. In some embodiments, inputs 122 can include any suitable input devices and/or sensors that can be used to receive user input, such as a keyboard, a mouse, a touchscreen, a microphone, etc.

In some embodiments, communications systems 124 can include any suitable hardware, firmware, and/or software for communicating information over communication network 102 and/or any other suitable communication networks. For example, communications systems 124 can include one or more transceivers, one or more communication chips and/or chip sets, etc. In a more particular example, communications systems 124 can include hardware, firmware and/or software that can be used to establish a Wi-Fi connection, a Bluetooth connection, a cellular connection, an Ethernet connection, etc.

In some embodiments, memory 126 can include any suitable storage device or devices that can be used to store instructions, values, etc., that can be used, for example, by processor 118 to present content using display 120, to communicate with one or more computing devices 104, etc. Memory 126 can include any suitable volatile memory, non-volatile memory, storage, or any suitable combination thereof. For example, memory 126 can include RAM, ROM, EEPROM, one or more flash drives, one or more hard disks, one or more solid state drives, one or more optical drives, etc. In some embodiments, memory 126 can have encoded thereon a server program for controlling operation of server 116. In such embodiments, processor 118 can execute at least a portion of the server program to transmit information and/or content (e.g., results of a tissue identification and/or classification, a user interface, etc.) to one or more computing devices 104, receive information and/or content from one or more computing devices 104, receive instructions from one or more devices (e.g., a personal computer, a laptop computer, a tablet computer, a smartphone, etc.), etc.

In some embodiments, any suitable computer readable media can be used for storing instructions for performing the functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

Additionally or alternatively, the method can include assembling training data from sequencing data and/or other biological marker data using a computer system. This step may include assembling the sequencing data and/or other biological marker data into an appropriate data structure on which the machine learning model and/or algorithm can be trained. Assembling the training data may include assembling feature data, sequencing data, and other relevant data. For instance, assembling the training data may include generating labeled data and including the labeled data in the training data. Labeled data may include labeled sequencing data, and/or labeled biological marker data, segmented medical images, or other relevant data that have been labeled as belonging to, or otherwise being associated with, one or more different classifications or categories. For instance, labeled data may include medical images and/or segmented medical images that have been labeled based on the image-localized genetic and/or other biological marker data. The labeled data may include data that are classified on a voxel-by-voxel basis, or a regional or larger volume basis.

Appropriate feature selection can be implemented to reduce the risk of overfitting when the input variables are high-dimensional. As a non-limiting example, a forward stepwise selection can be used, which starts with an empty feature set and adds one feature at each step that maximally improves a pre-defined criterion until no more improvement can be achieved. To avoid overfitting, the accuracy computed on a validation set can be used as an evaluation criterion; when the sample size is limited, cross-validation accuracy can be adopted.

One or more machine learning models and/or algorithms may be trained on the training data. In general, the machine learning model can be trained by optimizing model parameters based on minimizing a loss function. As one non-limiting example, the loss function may be a mean squared error loss function.

The machine learning model may have various architectures. The architecture may include units or nodes which are connected by edges. The output of each node is computed by a function which may be referred to as an activation function. The network architecture may be organized into different layers. The layers may include an input layer, output layer, and intermediate layers which may be referred to as hidden layers. The input layer receives external data (e.g., sequencing data). The output layer produces the ultimate result of the neural network. The network architecture may include two or more hidden layers. Layers may be fully connected or pooled.

Training a machine learning model may include initializing the model, such as by computing, estimating, or otherwise selecting initial model parameters. Training data can then be input to the initialized machine learning model, generating output as genetic and/or other biological marker data and predictive uncertainty data that indicate an uncertainty in those genetic and/or other biological marker predictions. The quality of the output data can then be evaluated, such as by passing the output data to the loss function to compute an error. The current machine learning model can then be updated based on the calculated error (e.g., using backpropagation methods based on the calculated error).

The machine learning model can be updated by updating the model parameters in order to minimize the loss according to the loss function. When the error has been minimized (e.g., by determining whether an error threshold or other stopping criterion has been satisfied), the current model and its associated model parameters represent the trained machine learning model.

The one or more trained neural networks are then stored for later use. Storing the neural network(s) may include storing network parameters (e.g., weights, biases, or both), which have been computed or otherwise estimated by training the neural network(s) on the training data. Storing the trained neural network(s) may also include storing the particular neural network architecture to be implemented. For instance, data pertaining to the layers in the neural network architecture (e.g., number of layers, type of layers, ordering of layers, connections between layers, hyperparameters for layers) may be stored.

In general, the machine learning model is trained, or has been trained, on training data in order to predict subject signatures, e.g., IPS, based on sequencing data and to quantify the uncertainty of those predictions.

**Additional Definitions**

To aid in understanding the invention, several additional terms are defined below.

Unless defined otherwise, all technical and scientific terms used herein have the same meaning as commonly understood by one of skill in the art. Although any methods and materials similar to or equivalent to those described herein can be used in the practice or testing of the claims, the exemplary methods and materials are described herein.

Moreover, reference to an element by the indefinite article "a" or "an" does not exclude the possibility that more than one element is present, unless the context clearly requires that there be one and only one element. The indefinite article "a" or "an" thus usually means "at least one."

The term "about" means within a statistically meaningful range of a value or values such as a stated concentration, length, molecular weight, pH, time frame, temperature, pressure or volume. Such a value or range can be within an order of magnitude, typically within 20%, more typically within 10%, and even more typically within 5% of a given value or range. The allowable variation encompassed by "about" will depend upon the particular system under study.

The terms "comprising," "having," "including," and "containing" are to be construed as open-ended terms (*i.e.,* meaning "including, but not limited to,") unless otherwise noted.

Recitation of ranges of values herein are merely intended to serve as a shorthand method of referring individually to each separate value falling within the range, and includes the endpoint boundaries defining the range, unless otherwise indicated herein, and each separate value is incorporated into the specification as if it were individually recited herein.

As used herein, the term "subject" may be used interchangeably with the term "patient" or "individual" and may include an "animal" and in particular a mammal. Mammalian subjects may include humans and other primates, domestic animals, farm animals, and companion animals such as dogs, cats, guinea pigs, rabbits, rats, mice, horses, cattle, cows, and the like.

In some embodiments, the subject has been diagnosed with cancer. In some embodiments, the subject has an altered human leukocyte antigen (HLA) phenotype in a population of cells of the tumor. As used herein, the term "altered HLA phenotype" refers to a phenotype in which the expression of at least one HLA gene is altered relative to wild-type HLA gene expression. The "HLA complex" is the major histocompatibility complex (MHC) in humans, and it comprises a group of related cell-surface proteins that regulate the immune system.

In some embodiments, the altered phenotype comprises a mutation in at least one HLA class I gene. The HLA complex is located at 6p21.3 on chromosome 6, and downregulation or loss of HLA class I expression in tumor cells is a known mechanism of cancer immune evasion. Loss of heterozygosity (LOH) is the most common mechanism of HLA haplotype absence in a malignant tumor, and the frequency of LOH-6p21 has been reported in many cancer types. Furthermore, LOH has been implicated in carcinogenesis and its presence is a useful prognostic marker in many malignant tumors. Thus, one mechanism of immune escape for tumors is loss of heterozygosity in HLA genes (HLA-LOH), which reduces the total number of neoantigens available for presentation to T cells.

As used herein a "subject sample" or a "biological sample" from the subject refers to a sample taken from the subject, such as, but not limited to a tissue sample (*e.g.,* fat, muscle, skin, neurological, tumor, etc.) or fluid sample (*e.g.,* saliva, blood, serum, plasma, urine, stool, cerebrospinal fluid, etc.), and or cells or sub-cellular structures. In some embodiments, a subject sample comprise a tumor sample, such as a biopsy. Such a sample may be fresh, frozen, or formalin fixed paraffin embedded (FFPE).

As used herein, the term "CD8+ T cells" refers to a subpopulation of HLA class I-restricted T lymphocytes that express the co-receptor protein CD8. CD8+ T cells recognize peptides presented by HLA Class I molecules, found on all nucleated cells. CD8+ T cells include cytotoxic T cells, which are important for killing cancerous, virally infected cells, and cells that are damaged in other ways, and CD8-positive suppressor T cells, which restrain certain types of immune response.

As used herein, the term "CD4+ T cells" refers to a subpopulation of HLA class II-restricted T lymphocytes that express the co-receptor protein CD4. CD4+ T cells are also referred to as "T helper cells" because they "help" the activity of other immune cells by releasing cytokines, small protein mediators that alter the behavior of target cells that express receptors for those cytokines. Studies have shown that a subset of CD4+ T cells with a cytotoxic gene profile can mediate direct killing of tumor cells (1,2,3). Specifically, these CD4+ T cells express proteins, such as perforin (a pore-forming protein) and granzymes (a family of serine proteases), which are commonly associated with CD8+ T cells. T cells use a combination of perforin and granzymes to induce apoptosis in virus-infected or transformed cells.

As noted previously, an immune resistance signature is characterized by the expression level and associated weight of one or more of the genes listed in Table 1 in a tumor sample from the subject.

In some embodiments, the control level or the predetermined threshold value is derived from healthy matched tissue, or matched tissue known to lack an IPS. By "matched tissue" is meant the same tissue type, *e.g.*, lung tissue control if the tumor is lung cancer, liver tissue control if the tumor is liver cancer, etc. By way of example but not by way of limitation, in some embodiments, a control level or threshold level is derived from whole transcriptome expression score data from a tissue matched, non-tumor sample. If the subject's immune resistance signature gene expression level is greater than the control or threshold, the subject's tumor is indicated as having an immune resistance signature.

A variety of techniques may be used to determine whether a tumor sample comprises an immune resistance signature, including single cell RNA sequencing , whole-transcriptome RNA sequencing, and immunohistochemistry (IHC) staining.

**Table 3. Exemplary components for use in determining an IPS.**

| Biomarker | Data Source (RNA or DNA) | Reference |
|---|---|---|
| APOBEC SBS 2, SBS 13 | DNA | As found in, e.g., Alexandrov, Nature 2013 |
| Smoking SBS 4 | DNA | As found in, e.g., Alexandrov, Nature 2013 |
| TLS | RNA | As found in, e.g., Andersson, Nat Comms 2021 |
| IMPRES score | RNA | As found in, e.g., Auslander, Nat Med 2018 |
| IFNgamma TIS | RNA | As found in, e.g., Ayers, JCI 2017 |
| IFN gamma score | RNA | As found in, e.g. Beaubier, Nat Biotech 2019 |
| STK11, KEAP1 mutations | DNA | As found in, e.g. Biton Clin Cancer Res 2018 |
| | | Cancer Disc 2018 |
| | | |
| TLS | RNA | As found in, e.g., Cabrita, Nature 2020 |
| HLA-LOH | DNA | As found in, e.g., Chowell Science 2018 |
| TLS Chemokine | RNA | As found in, e.g., Coppola, Am J Pathol 2021 |
| Angiogenesis | RNA | As found in, e.g., Cristescu, Clin Cancer Res 2022 |
| gMDSC | RNA | As found in, e.g., Cristescu, Clin Cancer Res 2022 |

(continued)

| Biomarker | Data Source (RNA or DNA) | Reference |
|---|---|---|
| mDMSC | RNA | As found in, e.g., Cristescu, Clin Cancer Res 2022 |
| Glycolysis | RNA | As found in, e.g., Cristescu, Clin Cancer Res 2022 |
| Hypoxia | RNA | As found in, e.g., Cristescu, Clin Cancer Res 2022 |

| | | |
|---|---|---|
| Proliferation | RNA | As found in, e.g., Cristescu, Clin Cancer Res 2022 |
| Stroma | RNA | As found in, e.g., Cristescu, Clin Cancer Res 2022 |
| NRS Score | RNA | As found in, e.g., Huang, Nat Med 2019 |
| Immune resistance program | RNA | As found in, e.g., Jerby-Arnon Cell 2018 |
| | | |
| Cytotoxic Score | RNA | As found in, e.g., Lau, Nat Comms 2022 |
| CXCL9 | RNA | As found in, e.g., Litchfield, Cell 2021 |
| HLA Promiscuity Score | DNA | As found in, e.g., Manczinger, Nat Cancer 2021 |
| Immune Score | RNA | As found in, e.g., Roh Sci Trans Med 2017 |
| Cytolytic Index | RNA | As found in, e.g., Rooney, Cell 2015 |
| T cell exhaustion score | RNA | As found in, e.g., Sade-Feldman, Cell 2018 |
| MIRACLE score | RNA | As found in, e.g., Turan, BJC 2020 |
| APM score | RNA | As found in, e.g., Thompson, J Immunother Cancer 2020 |
| | | |
| IPS model | RNA+DNA | As found in, e.g., Tomlins, Commun Med 2023 |
| T cell resilience | RNA | As found in, e.g., Zhang, Nt Med 2022 |

Each of the references listed in Table 3 are incorporated by reference herein in their entireties.

**Exemplary embodiments**

Provided below is a list of exemplary embodiments of the instant disclosure.

1. A method comprising:
at a computer system having one or more processors, and memory storing one or more programs for execution by the one or more processors:
applying, by the one or more processors, one or more model components derived from sequencing data from a sample to one or more machine learning algorithms (MLAs), wherein the sequencing data comprises RNA sequencing data and DNA sequencing data, wherein the one or more model components comprise an immune exhaustion signature.
2. A method comprising:
at a computer system having one or more processors, and memory storing one or more programs for execution by the one or more processors:
applying, by the one or more processors, one or more model components derived from sequencing data from a sample to one or more machine learning algorithms (MLAs), wherein the sequencing data comprises RNA sequencing data and DNA sequencing data, wherein the one or more model components comprise an immune oncology signature.
3. A method comprising:
at a computer system having one or more processors, and memory storing one or more programs for execution by the one or more processors:
applying, by the one or more processors, one or more model components derived from sequencing data from a sample to one or more machine learning algorithms (MLAs), wherein the sequencing data comprises RNA sequencing data and DNA sequencing data, wherein the one or more model components comprise a tumor mutational burden (TMB), checkpoint related gene signature, an immune exhaustion signature, a granulocytic myeloid derived suppressor cell (gMDSC) signature, and an immune oncology signature.

4. A method comprising:

sequencing sample of nucleic acids from a sample of a tumor to generate sequencing data;
applying, by the one or more processors, one or more model components derived from the sequencing data to one or more machine learning algorithms (MLAs), wherein the sequencing data comprises RNA sequencing data and DNA sequencing data, wherein the one or more model components comprise a tumor mutational burden (TMB), checkpoint related gene signature, an immune exhaustion signature, a granulocytic myeloid derived suppressor cell (gMDSC) signature, and an immune oncology signature.

5. A method comprising:

sequencing sample of nucleic acids from a tumor from a subject to generate sequencing data, wherein the sequencing data comprises RNA sequencing data and DNA sequencing data;
deriving one or more model components from the sequencing data, wherein the one or more model components comprise a tumor mutational burden (TMB), checkpoint related gene signature, an immune exhaustion signature, a granulocytic myeloid derived suppressor cell (gMDSC) signature, and an immune oncology signature;
applying, by the one or more processors, the one or more model components derived from the sequencing data to one or more machine learning algorithms (MLAs).

6. The method of any one of preceding embodiments, wherein the one or more MLAs are trained to determine an immune profile score (IPS) for the subject based on the one or more model components.

7. A method of determining an immune profile score (IPS) for a subject, the method comprising:

at a computer system having one or more processors, and memory storing one or more programs for execution by the one or more processors:

applying, by the one or more processors, one or more model components derived from sequencing data from a sample from a subject to one or more machine learning algorithms (MLAs), wherein the sequencing data comprises RNA sequencing data and DNA sequencing data, wherein the one or more model components comprise a tumor mutational burden (TMB), checkpoint related gene signature, an immune exhaustion signature, a granulocytic myeloid derived suppressor cell (gMDSC) signature, and an immune oncology signature, wherein the one or more MLAs are trained to determine an IPS for the subject based on the one or more model components.

8. The method of any one of preceding embodiments, wherein the TMB is derived from the DNA sequencing data.

9. The method of any one of preceding embodiments, wherein the expression values of the panel of genes, the immune exhaustion signature, and the granulocytic myeloid derived suppressor cell (gMDSC) signature are derived from the RNA seq data.

10. The method of any one of the preceding embodiments, wherein the method further comprises displaying the IPS in the form of a report.

11. The method of embodiment 10, wherein the method further comprises comparing the IPS to a pre-determined threshold.

12. The method of embodiment 11, wherein the IPS, as compared to the threshold, indicates that the subject is likely to experience a progression event if treated with an immune oncology therapy.

13. The method of embodiment 12, wherein the method further comprises increasing a frequency of radiological examinations of the subject.

14 The method of embodiment 11, wherein the IPS, as compared to the threshold, indicates that the subject is not likely to experience a progression event if treated with an immune oncology therapy.

15. The method of embodiment 14, wherein the method further comprises reducing a frequency of radiological examinations of the subject.

16. The method of any one of the preceding embodiments, wherein the method further comprises administering a therapeutically effective amount of an immune oncology therapy to the subject.

17. The method of embodiment 16, wherein the immune oncology therapy is a checkpoint inhibitor therapy.

18. The method of embodiment 17, wherein the checkpoint inhibitor therapy is a PD-1/PD-L1 axis inhibitor.

19 The method of embodiment 18, wherein the PD-1/PD-L1 axis inhibitor is an anti-PD-1 monoclonal antibody.

20. The method of any one of preceding embodiments, wherein the IPS is displayed as a number from 1-100.

21. The method of any one of preceding embodiments, wherein the IPS is displayed as an integer from 1-100.

22. The method of any one of preceding embodiments, wherein the IPS is further divided into categories or is further interpreted to yield a categorical result.

23. The method of embodiment 22, wherein the categories are IPS-Low, indeterminate, and IPS-High.

**EP 4 550 337 A1**

24. The method of any one of preceding embodiments, wherein the one or more MLAs comprise a variational autoencoder, a Cox proportional hazards model, a random forest model, a gradient-boosted survival model, or a convolutional neural network.

25. The method of any one of preceding embodiments, wherein the sample is a tumor sample.

26. The method of any one of preceding embodiments, wherein checkpoint related gene signature comprises expression values for a panel of genes comprising CD274, SPP1, CXCL9, CD74, CD276, IDO1, PDCD1LG2, and TNFRSF5.

27. The method of any one of preceding embodiments, wherein the checkpoint related gene signature CD274, SPP1, and CXCL9.

28. The method of any one of preceding embodiments, wherein checkpoint related gene signature comprises CD274, SPP1, CXCL9, and CD74.

29. The method of any one of preceding embodiments, wherein the checkpoint related gene signature comprises CD274, SPP1, CXCL9, CD74, and CD276.

30. The method of any one of preceding embodiments, wherein the checkpoint related gene signature comprises CD274, SPP1, CXCL9, CD74, CD276, and IDO1.

31. The method of any one of preceding embodiments, wherein the checkpoint related gene signature comprises CD274, SPP1, CXCL9, CD74, CD276, IDO1, and PDCD1LG2.

32. The method of any one of preceding embodiments, wherein the checkpoint related gene signature comprises CD274, SPP1, CXCL9, CD74, CD276, IDO1, PDCD1LG2, and TNFRSF5.

33. The method of any one of preceding embodiments, wherein the immune exhaustion signature comprises expression values for one or more genes selected from TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, SLC38A5, TIFA, DOK2, PPP1R2, DMAC1, DNAJB1, TAGAP, GZMA, CD27, GADD45A, HSPH1, STMN1, GZMH, CLIC3, GLIPR1, CHORDC1, CD3E, CD69, BAG3, ATF3, MICB, TRBC2, EZR, ARHGDIB, CASC8, ITM2A, DDX24, CD52, RAC2, TERF2IP, ELF1, FAM96B, GGH, NKG7, LY6E, CITED2, ZFAND2A, SAMSN1, CST7, CDKN3, TCEAL3, BBC3, IL32, MBD4, DNAJA4, TMEM141, UBB, HCST, IGLV1-40, HOPX, RHOH, USB1, H2AFZ, CSRP1, IKZF1, RGS2, IGLC2, CCND2, SELPLG, FUNDC2, IGFBP7, IGKV3-15, SERPINE2, TRDMT1, RGS1, HMOX1, HSP90AB1, HSPA1A, LIME1, TUBB, MRPL10, IFI44L, COTL1, LBH, ZEB2, HMGB2, LDHA, LGALS3, CYLD, PXMP2, CD74, PPIH, CD8A, RFX2, KLRD1, KLF6, LINC02446, HTRA1, TUBA4A, HSPB1, DNAJA1, CD3D, DUSP2, ELL2, TPM1, CKS1B, LGALS1, BEX3, GLRX, CCL4, GBP5, PTPRC, CLK1, IRF4, PIM2, SAT1, CXCR3, ZFP36, CD24, PELI1, CKS2, GYPC, FOXN2, IGLV1-51, IFT46, IGLV1-41, PLA2G16, COMMD8, IPCEF1, SMPDL3B, EVL, EVI2B, RAB11FIP1, DUSP5, HAVCR2, UBC, CRIP1, SRPRB, SERPINA1, PCSK7, BCL2L11, HSPA6, CWC25, CORO1A, TPST2, MBNL2, CKB, TUBA1B, GABARAPL1, PXDC1, SEL1L, PPP1R8, FKBP4, GABARAPL2, JCHAIN, STK17B, ZWINT, CHMP1B, ID2, HERPUD1, ROCK1, SKAP1, S100A4, CXCL10, CASP3, APOC1, ARID5B, SMAP2, CSRNP1, ADIRF, HLA-DPA1, PPP1R15A, DMKN, SCAF4, MYL9, LYAR, ZBTB25, GADD45B, GCHFR, LINC01588, RAB20, LSP1, FCGR2B, HIST2H2AA4, NCF4, LCK, IGHV3-33, LAPTM5, TUBB4B, TPM2, RBM38, RBP4, CCNA2, SERTAD1, ITM2C, PLPP5, DNAJB9, SYNGR2, TUBB2A, ERLEC1, TMED9, IFI6, HSP90AA1, PTPN1, TTL, DKK1, TM2D3, DCAF11, RIC1, SERPING1, DERL3, KDELR3, GEM, KLF9, TYROBP, CERCAM, CCDC84, ODC1, CYP2C9, CFLAR, HLA-DMB, DUSP1, JSRP1, TRIB1, JUN, NFATC2, EMP3, SNRNP70, TMED5, ST8SIA4, IGLV3-1, ZNF394, TNFSF9, CTSW, CUL1, BACH1, RABL3, KPNA2, EPS8L3, IER5, HSPA1B, CADM1, MCL1, RNF19A, ITGA4, CD38, WIPI1, CENPK, HCLS1, SPICE1, HIST1H2BC, MPRIP, FOSB, SERPINB8, FAM126A, CEP55, ATXN1, VCL, SOCS1, PCNX1, SQOR, JUNB, C10orf90, LCP1, STRADB, CREB3L2, GNG7, CCNH, SNX2, IGSF1, CCNL1, FKBP11, DBF4, ICAM1, MAD2L1, TMEM176B, PAIP2B, CD79A, SRXN1, NOB1, IER2, HLA-DRA, ZFP36L1, MZB1, MAGEA4, JUND, CD8B, AARS, TXNDC15, AC016831.7, GNA15, ATM, TSC22D1, GZMK, RAC3, ZNF263, TNFAIP3, H1FX, FGG, FHL2, MBNL1, TMEM205, IGLV6-57, CD96, TUBA1C, UCHL1, PRDM1, SRPK2, NUP37, TMEM87A, THEMIS2, HSPA5, PCMT1, TUBA1A, IGHG1, ANKRD37, MEF2C, XRN1, POU2AF1, BCL6, INAFM1, ADH4, TGFB1I1, PBK, DCN, FCRL5, DNAJB4, HLA-DQA1, TBC1D23, TMEM39A, GCC2, TMEM192, IGHA1, PTHLH, MFAP5, GEMIN6, BIRC3, IGHV4-4, SLC6A6, CYP2R1, HLA-DRB1, PPP1R15B, HMCES, MYC, WISP2, CHN1, ILK, PXN-AS1, LINC01970, CRIP2, PCOLCE2, MTMR6, EDIL3, AGR2, MEF2B, PFKM, KIAA1671, GLIPR2, SSTR2, SERPINB9, HIST1H1E, PTTG1, WSB1, ERN1, Z93241.1, IGLV1-44, SDS, TLE1, NUPR1, IGLV1-47, ICAM2, NXF1, RSPO3, TCF4, AC243960.1, RARRES2, RMDN3, RBFOX2, SEC11C, OLMALINC, FADS2, ITPRIP, FOS, SFTPD, HAUS3, RNF43, HIST1H4C, TIGAR, BIK, ITGA1, TARSL2, AFP, SNORC, MKLN1, BTG2, KRT18, NOC2L, ZFP36L2, NFKBIA, RHOB, HMGA1, BRD3, IGHJ6, U62317.5, SLC2A3, AC034231.1, CLEC11A, EPCAM, SKI, PNOC, MIR155HG, C12orf75, SAMHD1, IGKV3D-15, ACTN1, GSTZ1, TUBB3, CAV1, OAT, COBLL1, SSR4, ACTA2, HBA1, FAM83D, PLA2G2A, RAB14, AC106791.1, RAB23, AC244090.1, KMT5A, SERPINB1, P3H2, XRCC1, AC106782.1, MAL2, EGR1, F8, PLIN2, SOWAHC, IGFBP6, NFKBIZ, XBP1, SLC25A51, IGHM, KCTD5, USP38, FCER1G, PHLDA1, BYSL, HLA-DRB5, RAPH1, DUSP23, FUOM, ISYNA1, TNK2, STAP2, SLC25A4, GALNT2, SGO2, FHL3, ALB, CYP20A1, TM4SF1, ADA, RRP9, DNAH14, BOLA2, BHLHE41, CCL20, AC005537.1, UBALD2, VGLL4, NUDT1, USP10, ADSSL1, PRSS23, FMC1, ARHGAP45, HSPA14-1, CREB5, RBM33, TMX4, ROCK2, ARSK, PALLD, FNDC3B, FOXA3, BATF, PTP4A3, CDC45, IGHV1-2, IMMP2L, STARD10, HIST2H2BF, MTG2, FBXO8, USP32, ADIPOR2, RRM2, DHODH, DDIT4, NFAT5, PPARG, YTHDF3-AS1, GNG4, CSPP1, UBE2S, ZNF473, TIMP1, CPQ, AOC2, H1F0, JRK, EXOSC9, AC012236.1, AC009403.1, C12orf65, AURKA, MYH9, IGKV4-1, IGHMBP2, JADE1, HIST1H3C, TTC39A, SGMS1, LBP, FRYL, DNAJB2, GNG11, HAGHL, ANXA6, MARS, ADD1, KDM4B, TMEM91, AC008915.2, CXCL14, DUSP14, GJB2, PGM1, ETS2, GNPDA1, COL18A1, KLF10, MT1A, TPX2, S100A2, MAP3K5, HIST1H2AE, SLC20A2, ITGB7, SCEL, RSRP1, AKR1B1, GINS1, ZNF296, ALKBH4, UBE2C, ANKRD36C, SULT2B1, SMC5, TSPYL2, TNS4, TIMP3, ID4, SDC1, COX18, CDC42EP2, SQLE, ZNRF1, AKR1B10, NDC80, GFPT2, MAP1B, HIST1H2AG, IDO1, RNF185, UHRF1BP1, ADORA2B, CALD1, PHLDA2, ADH6, TFAP2A, DLG1, MELK, CBWD3, RAB4B, KANSL1L, RCE1, HIST1H2AC, CDK1, TCIM, C17orf67, BRD4, LY6E-DT, SLC1A6, ARL13B, IRF1, DDX3X, RAB2B, MYBBP1A, ARFGAP1, BOP1, IGKV3D-7, KMT2E-AS1, DTNBP1, LAMC2, ATG4C, MYBL2, LRP10, PALMD, ZBTB4, SYTL2, SERPINH1, CD248, CNEP1R1, FURIN, IGLL5, MEST, MDK, NUP205, NRDE2, ECT2, TENT5A, TNKS1BP1, NFXL1, SLC35E3, ECE1, RASD1, SLC52A2, DCBLD2, CP, POLE, COL27A1, SBNO1, SLC7A6, HYKK, SLPI, CFHR1, SPDEF, DACT2, TUBGCP5, AREG, HIST1H2AJ, KIF2A, AL135925.1, NOTCH3, SLC11A1, HEXIM2, IGFBP1, TVP23A, NUDT14, SAMD11, MIR200CHG, PCLAF, SLC43A3, FAM30A, PHRF1, ADM, SIK2, NUSAP1, CFH, KRTCAP3, SPAG4, TPPP3, TSPAN4, AAK1, CST1, CLU, IFRD1, ASPHD2, CNN3, COL4A1, FGA, ANO6, SBSN, FGB, ATP9B, NLGN4Y, HP, EPS8, RNF111, LINC01285, MAOA, IGHV4-31, TNFRSF10D, GSR, IGHGP, TACSTD2, MT1F, RHCG, MUT, PI3, MT1M, LAMB3, MTRNR2L12, SLC35A2, DDX10, RARRES1, MTSS1, CLK2, RPN2, MED29, CYP1B1, TTTY14, DMXL1, AL139246.5, TAF1, DAAM1, MYO1E, MAFB, CDKN1A, F8A3, FABP5, CFB, HSP90B1, SGK3, HMG20B, CDCA5, CLDN4, SYNM, PAWR, TWNK, AC116049.2, RND3, ATP11A, PID1, MALAT1, TMEM168, TFF1, TFRC, RNASET2, SPINK13, PABPC1L, P4HA2, PRSS8, SPINT1, MSC, FMNL1, SLC8B1, UNC13D, SPINT2, DCP1A, NPTN, IGKV3D-11, G6PD, KRT6A, LYPD1, TESC, COL4A2, ELF3, BCAM, AC093323.1, IGHV1-69, LINC00511, PORCN, TPRG1-AS1, EFNB2, PARD6G-AS1, CD9, RGS16, IL6R, FZD3, GLYR1, B3GALT6, LRCH3, MAFK, LINC00491, MT1X, MUC6, PIK3R3, GBP4, PERP, LXN, ZBTB7A, WARS, AC020911.2, MAPK3, ALS2CL, MRE11, TSPAN17, IGHV4-34, IL33, ADAM9, ANGPTL4, TBC1D31, C1R, CTSC, SLC35A4, FST, SGO1, ANKRD36, IGHG3, SLC15A3, HES1, POLR1E, SLC7A5, CAPN12, IGFBP3, FBXO38, FLNA, CSKMT, OAS1, ULK1, PBX1, EXOC4, REEP6, HILPDA, ASF1B, FKBP1B, IL6, CALU, AKR1C1, KLF2, GRTP1, C1S, SMOX, CPLX2, LMNA, BSG, IGHG4, SVIL, HIST1H1B, GCH1, NEAT1, FN1, ESRP1, RFWD3, ADGRE2, SPINK6, HPD, CAVIN1, MT1E, CLDN10, C15orf48, CA9, NR4A1, PPP1R3B, SLC30A1, SLC7A11, VIRMA, NAA25, CCNB1, CFD, AP1G1, H6PD, PSCA, KCNK6, AL161431.1, DVL1, HIST1H2AM, RAB31, CDCA3, SPATA20, PRMT7, PTGR1, SERINC2, IGHG2, GFPT1, TTC22, BTBD1, HIST1H4H, CENPB, ZNF598, GPATCH2L, SPTLC3, CXCL2, CYP24A1, EZH2, GPX2, LMNB2, PTGES, MGLL, NR2F2, KRT19, DNTTIP1, MUC5AC, SDCBP2, IL1R2, AHNAK2, MUC16, AC023090.1, CPE, VNN1, BAMBI, NPW, TK1, IGKV3D-20, ANKRD11, CDC20, CDH1, STK11, IGKC, SLC45A4, TBC1D8, CSTA, AC233755.1, MIGA1, HIST1H2AL, AKAP12, MAP4K4, HOOK2, GGA3, COL7A1, NOS1, ARHGAP26, AKR1C2, TGM2, CENPF, IGHV3-48, CDCA8, TSC2, STC2, PKN3, PVR, CES1, GPRC5A, SEZ6L2, CEP170B, KIF14, IER3, ALDH3B1, TOP2A, SPP1, TXNRD1, LENG8, TRIM15, ALDH3A1, RIMKLB, HECTD4, SMOC1, NEB, RMRP, IGFBP4, MT1G, SCRIB, ERO1A, SOX4, LMO7, RNPEPL1, PLK2, COL6A2, FLRT3, IGHV4-28, SCD, KRT7, PIEZO1, CXCL1, DAPK1, ID1, C3, CXCL3, IGKV3-20, GUCA2B, ITGA3, SFN, IGLV3-21, PLEC, POLR2A, AGRN, MUC1, SERPINB3, S100A8, LAMA5, COL6A1, ITGB4, S100P, SLURP2, MSLN, KRT17, and MUC5B.

34. The method of any one of preceding embodiments, wherein the immune exhaustion signature comprises expression values for one or more of the following genes TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, and SLC38A5.

35. The method of any one of preceding embodiments, wherein the immune exhaustion signature comprises expression values for TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, and BST2.

36. The method of any one of preceding embodiments, wherein the immune exhaustion signature comprises expression values for TMSB4X, CCL5, TSC22D3, CYTOR, CXCL13, TXNIP, PTPRCAP, RGCC, IGLC3, and CYTIP.

37. The method of any one of preceding embodiments, wherein the immune exhaustion signature comprises expression values for the following genes: TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, SLC38A5, TIFA, DOK2, PPP1R2, DMAC1, DNAJB1, TAGAP, GZMA, CD27, GADD45A, HSPH1, STMN1, GZMH, CLIC3, GLIPR1, CHORDC1, CD3E, CD69, BAG3, ATF3, MICB, TRBC2, EZR, ARHGDIB, CASC8, ITM2A, DDX24, CD52, RAC2, TERF2IP, ELF1, FAM96B, GGH, NKG7, LY6E, CITED2, ZFAND2A, SAMSN1, CST7, CDKN3, TCEAL3, BBC3, IL32, MBD4, DNAJA4, TMEM141, UBB, HCST, IGLV1-40, HOPX, RHOH, USB1, H2AFZ, CSRP1, IKZF1, RGS2, IGLC2, CCND2, SELPLG, FUNDC2, IGFBP7, IGKV3-15, SERPINE2, TRDMT1, RGS1, HMOX1, HSP90AB1, HSPA1A, LIME1, TUBB, MRPL10, IFI44L, COTL1, LBH, ZEB2, HMGB2, LDHA, LGALS3, CYLD, PXMP2, CD74, PPIH, CD8A, RFX2, KLRD1, KLF6, LINC02446, HTRA1, TUBA4A, HSPB1, DNAJA1, CD3D, DUSP2, ELL2, TPM1, CKS1B, LGALS1, BEX3, GLRX, CCL4, GBP5, PTPRC, CLK1, IRF4, PIM2, SAT1, CXCR3, ZFP36, CD24, PELI1, CKS2, GYPC, FOXN2, IGLV1-51, IFT46, IGLV1-41, PLA2G16, COMMD8, IPCEF1, SMPDL3B, EVL, EVI2B, RAB11FIP1, DUSP5, HAVCR2, UBC, CRIP1, SRPRB, SERPINA1, PCSK7, BCL2L11, HSPA6, CWC25, CORO1A, TPST2, MBNL2, CKB, TUBA1B, GABARAPL1, PXDC1, SEL1L, PPP1R8, FKBP4, GABARAPL2, JCHAIN, STK17B, ZWINT, CHMP1B, ID2, HERPUD1, ROCK1, SKAP1, S100A4, CXCL10, CASP3, APOC1, ARID5B, SMAP2, CSRNP1, ADIRF, HLA-DPA1, PPP1R15A, DMKN, SCAF4, MYL9, LYAR, ZBTB25, GADD45B, GCHFR, LINC01588, RAB20, LSP1, FCGR2B, HIST2H2AA4, NCF4, LCK, IGHV3-33, LAPTM5, TUBB4B, TPM2, RBM38, RBP4, CCNA2, SERTAD1, ITM2C, PLPP5, DNAJB9, SYNGR2, TUBB2A, ERLEC1, TMED9, IFI6, HSP90AA1, PTPN1, TTL, DKK1, TM2D3, DCAF11, RIC1, SERPING1, DERL3, KDELR3, GEM, KLF9, TYROBP, CERCAM, CCDC84, ODC1, CYP2C9, CFLAR, HLA-DMB, DUSP1, JSRP1, TRIB1, JUN, NFATC2, EMP3, SNRNP70, TMED5, ST8SIA4, IGLV3-1, ZNF394, TNFSF9, CTSW, CUL1, BACH1, RABL3, KPNA2, EPS8L3, IER5, HSPA1B, CADM1, MCL1, RNF19A, ITGA4, CD38, WIPI1, CENPK, HCLS1, SPICE1, HIST1H2BC, MPRIP, FOSB, SERPINB8, FAM126A, CEP55, ATXN1, VCL, SOCS1, PCNX1, SQOR, JUNB, C10orf90, LCP1, STRADB, CREB3L2, GNG7, CCNH, SNX2, IGSF1, CCNL1, FKBP11, DBF4, ICAM1, MAD2L1, TMEM176B, PAIP2B, CD79A, SRXN1, NOB1, IER2, HLA-DRA, ZFP36L1, MZB1, MAGEA4, JUND, CD8B, AARS, TXNDC15, AC016831.7, GNA15, ATM, TSC22D1, GZMK, RAC3, ZNF263, TNFAIP3, H1FX, FGG, FHL2, MBNL1, TMEM205, IGLV6-57, CD96, TUBA1C, UCHL1, PRDM1, SRPK2, NUP37, TMEM87A, THEMIS2, HSPA5, PCMT1, TUBA1A, IGHG1, ANKRD37, MEF2C, XRN1, POU2AF1, BCL6, INAFM1, ADH4, TGFB1I1, PBK, DCN, FCRL5, DNAJB4, HLA-DQA1, TBC1D23, TMEM39A, GCC2, TMEM192, IGHA1, PTHLH, MFAP5, GEMIN6, BIRC3, IGHV4-4, SLC6A6, CYP2R1, HLA-DRB1, PPP1R15B, HMCES, MYC, WISP2, CHN1, ILK, PXN-AS1, LINC01970, CRIP2, PCOLCE2, MTMR6, EDIL3, AGR2, MEF2B, PFKM, KIAA1671, GLIPR2, SSTR2, SERPINB9, HIST1H1E, PTTG1, WSB1, ERN1, Z93241.1, IGLV1-44, SDS, TLE1, NUPR1, IGLV1-47, ICAM2, NXF1, RSPO3, TCF4, AC243960.1, RARRES2, RMDN3, RBFOX2, SEC11C, OLMALINC, FADS2, ITPRIP, FOS, SFTPD, HAUS3, RNF43, HIST1H4C, TIGAR, BIK, ITGA1, TARSL2, AFP, SNORC, MKLN1, BTG2, KRT18, NOC2L, ZFP36L2, NFKBIA, RHOB, HMGA1, BRD3, IGHJ6, U62317.5, SLC2A3, AC034231.1, CLEC11A, EPCAM, SKI, PNOC, MIR155HG, C12orf75, SAMHD1, IGKV3D-15, ACTN1, GSTZ1, TUBB3, CAV1, OAT, COBLL1, SSR4, ACTA2, HBA1, FAM83D, PLA2G2A, RAB14, AC106791.1, RAB23, AC244090.1, KMT5A, SERPINB1, P3H2, XRCC1, AC106782.1, MAL2, EGR1, F8, PLIN2, SOWAHC, IGFBP6, NFKBIZ, XBP1, SLC25A51, IGHM, KCTD5, USP38, FCER1G, PHLDA1, BYSL, HLA-DRB5, RAPH1, DUSP23, FUOM, ISYNA1, TNK2, STAP2, SLC25A4, GALNT2, SGO2, FHL3, ALB, CYP20A1, TM4SF1, ADA, RRP9, DNAH14, BOLA2, BHLHE41, CCL20, AC005537.1, UBALD2, VGLL4, NUDT1, USP10, ADSSL1, PRSS23, FMC1, ARHGAP45, HSPA14-1, CREB5, RBM33, TMX4, ROCK2, ARSK, PALLD, FNDC3B, FOXA3, BATF, PTP4A3, CDC45, IGHV1-2, IMMP2L, STARD10, HIST2H2BF, MTG2, FBXO8, USP32, ADIPOR2, RRM2, DHODH, DDIT4, NFAT5, PPARG, YTHDF3-AS1, GNG4, CSPP1, UBE2S, ZNF473, TIMP1, CPQ, AOC2, H1F0, JRK, EXOSC9, AC012236.1, AC009403.1, C12orf65, AURKA, MYH9, IGKV4-1, IGHMBP2, JADE1, HIST1H3C, TTC39A, SGMS1, LBP, FRYL, DNAJB2, GNG11, HAGHL, ANXA6, MARS, ADD1, KDM4B, TMEM91, AC008915.2, CXCL14, DUSP14, GJB2, PGM1, ETS2, GNPDA1, COL18A1, KLF10, MT1A, TPX2, S100A2, MAP3K5, HIST1H2AE, SLC20A2, ITGB7, SCEL, RSRP1, AKR1B1, GINS1, ZNF296, ALKBH4, UBE2C, ANKRD36C, SULT2B1, SMC5, TSPYL2, TNS4, TIMP3, ID4, SDC1, COX18, CDC42EP2, SQLE, ZNRF1, AKR1B10, NDC80, GFPT2, MAP1B, HIST1H2AG, IDO1, RNF185, UHRF1BP1, ADORA2B, CALD1, PHLDA2, ADH6, TFAP2A, DLG1, MELK, CBWD3, RAB4B, KANSL1L, RCE1, HIST1H2AC, CDK1, TCIM, C17orf67, BRD4, LY6E-DT, SLC1A6, ARL13B, IRF1, DDX3X, RAB2B, MYBBP1A, ARFGAP1, BOP1, IGKV3D-7, KMT2E-AS1, DTNBP1, LAMC2, ATG4C, MYBL2, LRP10, PALMD, ZBTB4, SYTL2, SERPINH1, CD248, CNEP1R1, FURIN, IGLL5, MEST, MDK, NUP205, NRDE2, ECT2, TENT5A, TNKS1BP1, NFXL1, SLC35E3, ECE1, RASD1, SLC52A2, DCBLD2, CP, POLE, COL27A1, SBNO1, SLC7A6, HYKK, SLPI, CFHR1, SPDEF, DACT2, TUBGCP5, AREG, HIST1H2AJ, KIF2A, AL135925.1, NOTCH3, SLC11A1, HEXIM2, IGFBP1, TVP23A, NUDT14, SAMD11, MIR200CHG, PCLAF, SLC43A3, FAM30A, PHRF1, ADM, SIK2, NUSAP1, CFH, KRTCAP3, SPAG4, TPPP3, TSPAN4, AAK1, CST1, CLU, IFRD1, ASPHD2, CNN3, COL4A1, FGA, ANO6, SBSN, FGB, ATP9B, NLGN4Y, HP, EPS8, RNF111, LINC01285, MAOA, IGHV4-31, TNFRSF10D, GSR, IGHGP, TACSTD2, MT1F, RHCG, MUT, PI3, MT1M, LAMB3, MTRNR2L12, SLC35A2, DDX10, RARRES1, MTSS1, CLK2, RPN2, MED29, CYP1B1, TTTY14, DMXL1, AL139246.5, TAF1, DAAM1, MYO1E, MAFB, CDKN1A, F8A3, FABP5, CFB, HSP90B1, SGK3, HMG20B, CDCA5, CLDN4, SYNM, PAWR, TWNK, AC116049.2, RND3, ATP11A, PID1, MALAT1, TMEM168, TFF1, TFRC, RNASET2, SPINK13, PABPC1L, P4HA2, PRSS8, SPINT1, MSC, FMNL1, SLC8B1, UNC13D, SPINT2, DCP1A, NPTN, IGKV3D-11, G6PD, KRT6A, LYPD1, TESC, COL4A2, ELF3, BCAM, AC093323.1, IGHV1-69, LINC00511, PORCN, TPRG1-AS1, EFNB2, PARD6G-AS1, CD9, RGS16, IL6R, FZD3, GLYR1, B3GALT6, LRCH3, MAFK, LINC00491, MT1X, MUC6, PIK3R3, GBP4, PERP, LXN, ZBTB7A, WARS, AC020911.2, MAPK3, ALS2CL, MRE11, TSPAN17, IGHV4-34, IL33, ADAM9, ANGPTL4, TBC1D31, C1R, CTSC, SLC35A4, FST, SGO1, ANKRD36, IGHG3, SLC15A3, HES1, POLR1E, SLC7A5, CAPN12, IGFBP3, FBXO38, FLNA, CSKMT, OAS1, ULK1, PBX1, EXOC4, REEP6, HILPDA, ASF1B, FKBP1B, IL6, CALU, AKR1C1, KLF2, GRTP1, C1S, SMOX, CPLX2, LMNA, BSG, IGHG4, SVIL, HIST1H1B, GCH1, NEAT1, FN1, ESRP1, RFWD3, ADGRE2, SPINK6, HPD, CAVIN1, MT1E, CLDN10, C15orf48, CA9, NR4A1, PPP1R3B, SLC30A1, SLC7A11, VIRMA, NAA25, CCNB1, CFD, AP1G1, H6PD, PSCA, KCNK6, AL161431.1, DVL1, HIST1H2AM, RAB31, CDCA3, SPATA20, PRMT7, PTGR1, SERINC2, IGHG2, GFPT1, TTC22, BTBD1, HIST1H4H, CENPB, ZNF598, GPATCH2L, SPTLC3, CXCL2, CYP24A1, EZH2, GPX2, LMNB2, PTGES, MGLL, NR2F2, KRT19, DNTTIP1, MUC5AC, SDCBP2, IL1R2, AHNAK2, MUC16, AC023090.1, CPE, VNN1, BAMBI, NPW, TK1, IGKV3D-20, ANKRD11, CDC20, CDH1, STK11, IGKC, SLC45A4, TBC1D8, CSTA, AC233755.1, MIGA1, HIST1H2AL, AKAP12, MAP4K4, HOOK2, GGA3, COL7A1, NOS1, ARHGAP26, AKR1C2, TGM2, CENPF, IGHV3-48, CDCA8, TSC2, STC2, PKN3, PVR, CES1, GPRC5A, SEZ6L2, CEP170B, KIF14, IER3, ALDH3B1, TOP2A, SPP1, TXNRD1, LENG8, TRIM15, ALDH3A1, RIMKLB, HECTD4, SMOC1, NEB, RMRP, IGFBP4, MT1G, SCRIB, ERO1A, SOX4, LMO7, RNPEPL1, PLK2, COL6A2, FLRT3, IGHV4-28, SCD, KRT7, PIEZO1, CXCL1, DAPK1, ID1, C3, CXCL3, IGKV3-20, GUCA2B, ITGA3, SFN, IGLV3-21, PLEC, POLR2A, AGRN, MUC1, SERPINB3, S100A8, LAMA5, COL6A1, ITGB4, S100P, SLURP2, MSLN, KRT17, and MUC5B.

38. The method of any one of preceding embodiments, wherein the immune exhaustion signature further comprises a weight corresponding to each of the genes in the signature.

39. The method of any one of preceding embodiments, wherein the gMDSC signature comprises expression values for one or more genes selected from SERPINB1, SOD2, S100A8, CTSC, CCL18, CXCL2, PLAUR, NCF2, FPR1, IL8, S100A9, TNFAIP3, CXCL1, BCL2A1, EMR2, LILRB3, SLC11A1, IL6, TREM1, CCL20, LYN, CXCL3, IL1B, IL1R2, AQP9, IL2RA, GPR97, OSM, CXCR1, FPR2, C19orf59, CXCR2, CXCL6, CXCL5, EMR3, MEFV, S100A12, CD300E, FCGR3B, PPBP, LILRA5, LILRA3, and CASP5.

40. The method of any one of preceding embodiments, wherein the immune oncology (IO) signature comprises expression values for one or more genes selected from ISG20, PCDHGA2, TGFB1I1, ATP8B1, IL7R, IRF8, ETV1, MYLK, GRHL2, THBS4, CYP3A5, FBLIM1, S100B, BICD1, SLAMF7, RAB27A, GATM, ICA1, ITPR1, SLC7A2, ZAP70, LOXL4, CILP, ARHGAP30, ITGB2, KLF5, PRKCA, PCDH7, DPYSL3, RGS2, SPP1, COLGALT2, MPZL2, TNFAIP8, PLAT, ALDH1A3, POF1B, PPP1R9A, SEMA3A, CIITA, DLC1, ARHGAP9, FRAS1, AKAP6, ATP1A2, TTN, LTBP1, NCKAP1L, MAP3K6, MYO1B, MRVI1, FSCN1, GPC1, GBP5, BAMBI, IL2RB, MYO1G, RANBP17, APOD, RASGRP1, CYTIP, ITGA7, CYTH4, PTPRF, KIAA1755, IRF1, GPR37, RAC2, NLRC5, EGFR, ITK, IL10RA, IGFBP2, CD96, RASD1, CD36, TMEM163, IGLL5, IKZF3, PRLR, CDC42BPG, DOCK2, PAM, VEGFA, CD84, SORL1, GBP2, SYTL4, APBB1IP, SIGLEC10, GBP4, COMP, DOCK8, CXCL9, NRP1, EPHB4, CD53, GLUL, DNM1, DSP, SIX4, SELL, DSC3, TNFAIP2, and JAG2.

**Further exemplary embodiments**

41. A method of determining an immune profile score (IPS) for a subject diagnosed with a cancer, the method comprising:
at a computer system having one or more processors, and memory storing one or more programs for execution by the one or more processors:

(A) receiving sequencing data from a sample of the cancer from the subject, wherein the sequencing data comprises RNA sequencing data, wherein the RNA sequencing data comprises a plurality of data elements comprising expression values for a plurality of genes; and applying one or more model components one to one or more models to determine the IPS for the subject.

42. The method of embodiment 1, wherein the one or more model components are selected from the group consisting of: tumor mutational burden (TMB), microsatellite instability (MSI), human leukocyte antigen (HLA) typing, HLA loss of heterozygosity, T cell repertoire, B cell repertoire, a level of immune infiltration into the subjects cancer, one or more clinical laboratory results, expression of one or more checkpoint genes, optionally wherein the one or more checkpoint genes are selected from CD274, CTLA4, TIM3, TIGIT, PDCD1, TNFSF4, LAG3, IDO1, and TNFRSF9, the presence of specific pathogenic mutations and alterations in genes related to immunotherapy response and cancer prognosis, optionally, STK11, KEAP1, ARID1A, and LKB1, RNA signatures of specific cell types and/or cell states, optionally, cytotoxic T-cells, biological processes, optionally, tertiary lymphoid structure formation or mechanisms of T-cell formation, responsiveness to immunotherapy, an immune exhaustion signature (IES), or any of the components listed in Table 2.

43. A method of determining an immune profile score (IPS) for a subject diagnosed with a cancer, the method comprising:

at a computer system having one or more processors, and memory storing one or more programs for execution by the one or more processors:

(A) receiving sequencing data from a sample of the cancer from the subject, wherein the sequencing data comprises RNA sequencing data, wherein the RNA sequencing data comprises a plurality of data elements comprising expression values for a plurality of genes;
(B) applying, to the plurality of data elements for the subject's cancer, a model that is trained to provide an immune exhaustion signature (IES) for the subject's cancer, wherein the IRS is characterized by positive weights on genes associated with immunosuppression and cancer proliferation and negative weights on cytotoxic genes, wherein the model is trained on a cohort data set comprising RNA sequencing data from a sample of a cancer from a plurality of subjects and clinical data from the plurality of subjects, wherein the clinical data comprises a survival metric; and
(C) applying the IRS and, optionally, one or more additional model components to one or more models to determine the IPS for the subject, wherein the IRS and the optional one or more model components are used by the model to determine the IPS for the subject.

44. A method comprising:
at a computer system having one or more processors, and memory storing one or more programs for execution by the one or more processors:

(A) receiving sequencing data from a sample of the cancer from the subject, wherein the sequencing data comprises RNA sequencing data, wherein the RNA sequencing data comprises a plurality of data elements comprising expression values for a plurality of genes;
(B) applying, to the plurality of data elements for the subject's cancer, a model that is trained to provide an immune exhaustion signature (IES) for the subject's cancer, wherein the IRS is characterized by positive weights on genes associated with immunosuppression and cancer proliferation and negative weights on cytotoxic genes, wherein the IRS is calculated using a plurality of biomarkers, wherein each of the plurality of biomarkers are ranked by their weight, wherein the weight of each of the biomarkers determines the biomarker's contribution to the IRS, wherein one or more of the biomarkers are selected from a gene and an associated gene weight listed in Table 1;
(C) applying the IRS and, optionally, one or more additional model components to the one or more models to determine the IPS, wherein the IRS and the optional one or more model components are used by the model to determine the IPS for the subject.

45. The method of any one of embodiments 41-44, wherein the method further comprises:
generating a clinical report comprising the immune profile score.
46. The method of any one of embodiments 41-45, wherein the method further comprises administering a therapeutically effective amount of an immune oncology therapy to the subject.
47. The method of any one of embodiments 41-46, wherein the method further comprises administering a therapeutically effective amount of an additional therapy to the subject selected from the group consisting of: a chemotherapy, a hormone therapy, a targeted therapy, and a radiation therapy.
48. The method of any one of embodiments 41-47, wherein the sequencing data comprises DNA sequencing data and RNA sequencing data.
49. The method of any one of embodiments 43-48, wherein the one or more additional model components are selected from one or more of tumor mutational burden (TMB), microsatellite instability (MSI), human leukocyte antigen (HLA) typing, HLA loss of heterozygosity, T cell repertoire, B cell repertoire, a level of immune infiltration into the subjects cancer, one or more clinical laboratory results, expression of one or more checkpoint genes, optionally wherein the one or more checkpoint genes are selected from CD274, CTLA4, TIM3, TIGIT, PDCD1, TNFSF4, LAG3, IDO1, and TNFRSF9, the presence of specific pathogenic mutations and alterations in genes related to immunotherapy response and cancer prognosis, optionally, STK11, KEAP1, ARID1A, and LKB1, RNA signatures of specific cell types and/or cell states, optionally, cytotoxic T-cells, biological processes, optionally, tertiary lymphoid structure formation or mechanisms of T-cell formation, responsiveness to immunotherapy, or any of the components listed in Table 2.
50. The method of any one of embodiments 43-49, wherein the model that is trained to provide an immune exhaustion signature (IES) for the subject's cancer or the one or more models to determine the IPS the comprise a machine learning algorithm selected from the group consisting of: a variational autoencoder, a Cox proportional hazards model, a random forest model, a gradient-boosted survival model, and a convolutional neural network.
51. The method of any one of embodiments 43-50, wherein the model that is trained to provide an immune exhaustion signature (IES) for the subject's cancer comprises a variational autoencoder.

52. The method of any one of embodiments 45-51, wherein the clinical report indicates a particular IO therapy for use in treatment of the subject.

53. The method of any one of embodiments 41-52, wherein the IPS is a numerical value from 1 to 100.

54. The method of any one of embodiments 41-53, wherein the IPS further comprises 2 or more categories, wherein the categories are based on the likelihood of the subject to respond to an IO therapy.

55. The method of any one of embodiments 41-54, wherein the sequencing data comprises a targeted panel for sequencing normal-matched tumor tissue, wherein the panel detects single nucleotide variants, insertions and/or deletions, and copy number variants in 598-648 genes and chromosomal rearrangements in 22 genes.

56. The method of any one of embodiments 41-55, wherein the sequencing data comprises full exome or full transcriptome sequencing.

57. The method of any one of embodiments 41-56, wherein the IPS indicates that the subject's cancer is likely to progress on an IO therapy, the clinical report indicates one or more additional therapies for use in treating the subject for the cancer.

58. The method of embodiment 57, further comprising administering a therapeutically effective amount of the one or more additional therapies indicated in the clinical report.

59. The method of any one of embodiments 57-58, wherein the one or more additional therapies are selected from: a chemotherapy, a hormone therapy, a targeted therapy, and a radiation therapy.

60. The method of embodiment 59, wherein the one or more additional therapies comprises a chemotherapy.

61. A system for selecting a subject for treatment with an immune oncology (IO) therapy, wherein the subject is in need of treatment for a cancer, comprising a computer including a processor, the processor configured to: perform the method of any one of the preceding embodiments.

62. A non-transitory computer readable medium having stored thereon program code instructions that, when executed by a processor, cause the processor to perform the method of any one of the preceding embodiments.

EXAMPLES

The following Examples are illustrative and are not intended to limit the scope of the claimed subject matter.

**Example 1. Immune profile score can be used to detect subjects likely to respond to immune oncology therapies**

An IO Algo, or IPS algorithm, can be used to determine an immune profile score of a subject. The results of the IO Algo will be determined by a machine learning model, that uses a combination of existing and understood biomarkers that are relevant to ICI response. The biomarkers can include immune inflammatory biomarkers such as NRS score, Cytotoxic score, TLS chemokine, Immune score, TLS scores, IFN$\gamma$, Cytotoxic index, IFN$\gamma$ TIS, APM, T cell resilience, IPS model, MIRACLE score, or IMPRES score. The biomarkers can also include information regarding immune resistance and tumor proliferation, such as an immune resistance score, T-cell exhaustion, angiogenesis, hypoxia, proliferation, and stroma. The biomarkers can further include immune checkpoint genes, such as CD274, CTLA4, TIM3, TIGIT, PDCD1, TNFSF4, LAG3, IDO1, TNFRSF9. The biomarkers can further include tumor-intrinsic features such as TMB, neoantigen burden, PD-L1, IHC TPS, APOBEC SBS, Smoking SBS, tumor purity, KEAP1 mutation, STK11 mutation, and HLA-LOH.

There are several components of the IO Algo model. The model can include features that include multiple different types of biomarkers. For instance, DNA may be one component of the model, and features can include (genomic) TMB, the presence of specific pathogenic mutations and alterations in genes related to immunotherapy response and cancer prognosis, genes like STK11, KEAP1, ARID1A, and LKB1, or other types of DNA alterations such as HLA-LOH. RNA can be a second components of the model and RNA features can include expression levels of single genes that are important in immune cell function, immune checkpoint, or other immune-related functions, like PD-L1, PD-1, CTLA-4, IDO1, IFN-gamma, and TGF-$\beta$, or RNA signatures of specific cell types and/or cell states (like cytotoxic T-cells), biological processes (like Tertiary lymphoid structure formation or mechanisms of T-cell formation), responsiveness to immunotherapy, or others.

A third component of the model can be an immune resistance signature, or an RNA signature identifying tumor immune resistance, which is derived from single-cell sequencing. A variational autoencoder can be used to identify the immune resistance signature, including reducing the dimensionality of the signature. Specifically, a signature can be projected into bulk RNAseq data using gene weights learned in scRNAseq data. The signature can then be characterized by positive weights on genes associated with immunosuppression (S100A8, SERPINB3), cancer proliferation (KRT17) and negative weights on cytotoxic genes (GZMB, PRF1). An example of gene weights associated with an immune resistance signature can be found in Table 1.

The model can be trained using a database of ICI-treated patients. The database of ICI-treated patients with outcomes as well as an immunotherapy platform is used to characterize known biological features relevant to response and resistance to immunotherapy. These features are used to build a pan-cancer Immune Profile Score (IPS) to identify subjects that are likely to respond well to ICI. For instance, a subset of the ICI-treated patients, referred to as a training set, can be used to train a machine learning algorithm to stratify patients.

The biomarkers that make up the model will contribute to the final model output in a way that is determined by machine learning using the Tempus database and possibly public databases. Various learning techniques (Cox PH models, random forest models, gradient-boosted survival models, neural networks, etc.) can be used to train a model that predicts which patients will have longer survival after treatment with ICIs. In general, higher scores on individual biomarkers that predict immunotherapy response will contribute to a higher overall model score. Higher scores on individual biomarkers that predict immunotherapy resistance will contribute to a lower overall model score. For instance, higher TMB may produce a higher model score. Higher cytotoxic T cells may produce a higher model score. In contrast, higher immune resistance may produce a lower model score. A tumor proliferation gene signature may produce a lower model score.

The IO Algo may be a clinical lab test and use DNA and RNA from a patient and a machine learning model to generate its output. Its outputs may include a numeric score, a categorical group, and potentially include model components. The numeric score may be a continuous score, likely from 0 to 100, which represents the model's prediction for likely response to immune checkpoint inhibitors (ICI). In some cases, a higher score corresponds to a longer predicted survival following ICI. The categorical group may be two or more groups corresponding to certain ranges of the numeric score to which the patient can be assigned. For example, the groups may be named "IPS-High," "IPS-Intermediate," or "IPS-Low." The model may also show the patient's score on the sub-components of the model, in a numerical and/or categorical way. For instance, if an RNA-based "cytotoxic T-cell" score is part of the model, the report may show the patient's "cytotoxic T-cell" score.

### Example 2. Detection of immune profile score and administration of therapeutics

In one example, the disclosed methods, systems, and compositions, also referred to as "algorithms," or "algo," or "IO algo," can be used to recommend treatments for a subject suffering from non-small cell lung cancer (NSCLC) with no driver mutation and PD-L1 $\geq$ 50%. Potential treatments for subjects with NSCLC may be administering immune checkpoint inhibitors (ICI) or administering ICI as well as chemotherapy. The IO Algo may be validated for predicting which treatment a subject is likely to benefit most from. For instance, subjects with the classification IPS-Low may be predicted to have the best outcomes from treatment of ICI along with chemotherapy, whereas subjects with the classification IPS-High may be predicted to have the best outcomes from treatment of ICI alone. IPS-Low subjects may survive longer if they receive the recommended treatment of ICI along with chemotherapy, and IPS-High subjects may survive similarly as long on ICI alone and experience lower toxicity than if they had received ICI along with chemotherapy. Signs and symptoms of NSCLC may be reduced by the administration of the recommended treatment. The recommended treatment may be administered daily, every other day, every third day, or on a schedule as determined by the patient's progress, pursuant to a physician's decision. It is anticipated that the subject may experience an increase in the quality of life associated with the reduction in signs or symptoms of NSCLC as compared to an untreated subject, or a subject receiving a treatment that was not predicted to lead to the best outcomes. Methods of measuring reduction in signs and symptoms of NSCLC are known in the art, e.g., reduction in tumor burden as measured by imaging modalities, e.g., magnetic resonance imaging (MRI) or computer aided tomography (CAT) scans.

### Example 3 - Exemplary analysis of real world cancer treatment data

Methodology for exploratory analysis of predictive utility in the study population: LOT2 patients who received CT in LOT1 were evaluated in this analysis. Restricted to patients with sample collection before LOT1 (N=159). Thus, each patient has 2 time periods: receipt of LOT1 CT in the 1st and LOT2 IO in the 2nd. Predictive utility was evaluated by estimating the effect of IPS in each time period. A recurrent event survival model is used to model the ordered events in the 2 time periods. (1) TTNT in time period 1 on LOT1 CT (i.e. time to initiation of IO in 2L) and (2) death in time period 2 on LOT2 IO.

Specifically, a stratified Cox model for the gap time (Prentice, Williams and Peterson or PWP*) was fit to the data. Robust variance is used to account for the correlation between the 2 time periods (both are from the same patient). A comparison of the HR from the 2 time periods provides an evaluation of the predictive utility of IPS.

Subjects in the analysis included those suffering from melanoma, non small cell lung cancer, breast carcinoma renal clear cell carcinoma, cervical carcinoma endometrial serous carcinoma, cholangiocarcinoma lung squamous cell carcinoma, lung adenocarcinoma gastroesophageal adenocarcinoma, urothelial carcinoma urothelial neuroendocrine carcinoma, endometrioid carcinoma head and neck squamous cell carcinoma, hepatocellular carcinoma skin squamous and basal cell carcinoma, colorectal adenocarcinoma gastroesophageal squamous cell carcinoma, and small cell lung carcinoma.

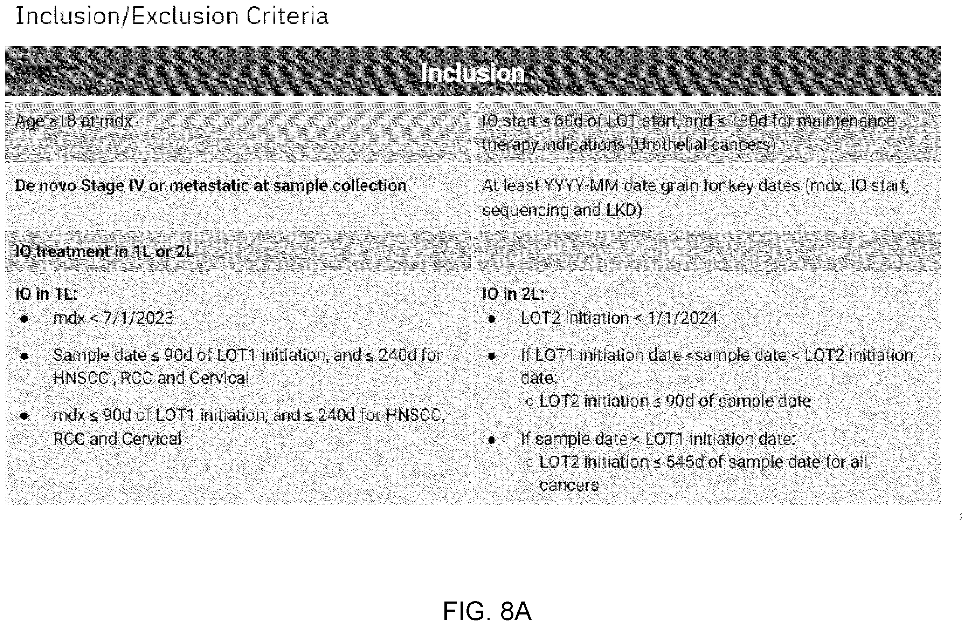

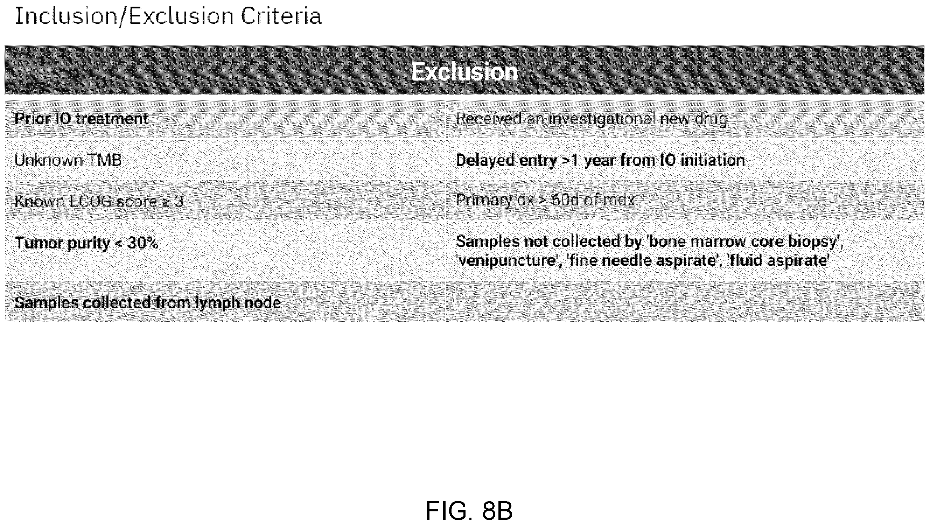

Inclusion and Exclusion criteria are shown in FIG. 8A and 8B.

Definition of PD-L1 positivity

1. Cancers with PDL1 IHC criteria in the FDA indication or practice guidelines:

 a. NSCLC: TPS≥1
 b. GEJ: CPS≥1
 c. Cervix: CPS≥1
 d. TNBC: CPS≥10
 e. Bladder: CPS≥10
 f. HNSCC: CPS≥1

2. Cancers with PDL1 IHC-agnostic FDA indication: PD-L1 IHC TPS or CPS ≥1% a. Including but not limited to Melanoma, RCC, Sarcoma
3. Exclude:

 a. MSI-H cancers
 b. Cancers without FDA indication

**Example 4** - **Development and validation of the Immune Profile Score (IPS) algorithm, a novel multi-omic approach for stratifying outcomes in a real-world cohort of late stage solid cancer patients treated with immune checkpoint inhibitors**

**Methods:** A de-identified pan-cancer cohort from the Tempus multimodal real-world database was used for the development and validation of the Immune Profile Score (IPS) algorithm leveraging Tempus xT (648 gene DNA panel) and xR (RNAseq). The cohort consisted of advanced stage cancer patients treated with any ICI-containing regimen as the first or second line of therapy. The IPS model was developed utilizing a machine learning framework that includes tumor mutational burden (TMB) and 8 RNA-based biomarkers as features.

**Conclusions:** Our results demonstrate that IPS is a generalizable multi-omic biomarker that can be widely utilized clinically as a prognosticator of ICI based regimens.

**What is already known on this topic** - Despite advances in immune checkpoint inhibitor (ICI) biomarker molecular testing, there remains an unmet clinical need for more sensitive and generalizable biomarkers to better predict patient outcomes to ICI. This has been challenging due to the limited availability of multi-omic testing and validation cohorts.

**What this Example adds** - Our results demonstrate that IPS is a generalizable multi-omic biomarker that can be widely utilized clinically as a prognosticator of ICI based regimens. Importantly, IPS-high may identify patients within subgroups (TMB-L, MSS, PD-L1 negative) who benefit from ICI beyond what is predicted by existing biomarkers.

**How this study might affect research, practice, or policy** - IPS results can support patient stratification across pan-solid tumor cohorts to help inform clinicians and researchers which patients are more likely to benefit from ICI based regimens.

Cancer immunotherapies, particularly immune checkpoint inhibitors (ICIs) targeting PD-[L] 1 and CTLA-4, have transformed the oncology treatment landscape. This transformation has been especially notable in cases where conventional systemic therapy options were associated with poor long-term outcomes [1]. Despite substantial improvements, the majority of patients do not benefit from ICIs, emphasizing the need for predictive biomarkers to inform treatment decisions [2].

To date, identifying candidates for immunotherapy relies on myriad PD-L1 immunohistochemistry (IHC) staining criteria across cancer types in addition to pan-cancer biomarkers of microsatellite instability (MSI) status and tumor mutational burden (TMB). Although PD-L1 positivity or high TMB may suggest potential responsiveness to ICIs, there remains a clinical need to improve our ability to determine whether patients will benefit from ICI treatment given the significant number of patients who do not under current guidelines [3].

Translational research efforts have made significant strides in identifying molecular biomarkers beyond PD-L1 IHC, TMB, and MSI, which characterize various aspects of the cancer-immunity cycle that hold promise as predictive immunotherapy biomarkers [4]. Advancements in RNA profiling technologies for both fresh tissue and formalin fixed paraffin embedded tissues have been essential in enabling analysis of routine pathology samples from clinical trials. As evidenced in the comprehensive analysis from Litchfield *et al* of publically available ICI clinical trial data sets, RNA biomarkers hold significant value in complementing DNA biomarkers for characterizing ICI response across solid organ cancers. [5]. However, while large-panel DNA sequencing is commonly performed in advanced-stage cancers to guide treatment decisions, the clinical utility and routine implementation of RNA sequencing are still emerging. As a result, RNA sequencing is less frequently available in academic and reference molecular pathology laboratories [6]. Additionally, the clinical validation of predictive biomarkers is constrained by the limited availability of large-scale multi-omic datasets that include high-quality clinical outcomes data [7]. Driven by these challenges and unmet clinical needs, we developed and validated a multi-omic, pan-solid cancer biomarker using the Tempus testing platform, incorporating both DNA and RNA analysis, to predict outcomes of ICI therapy.

## Methods

### Patient Cohorts

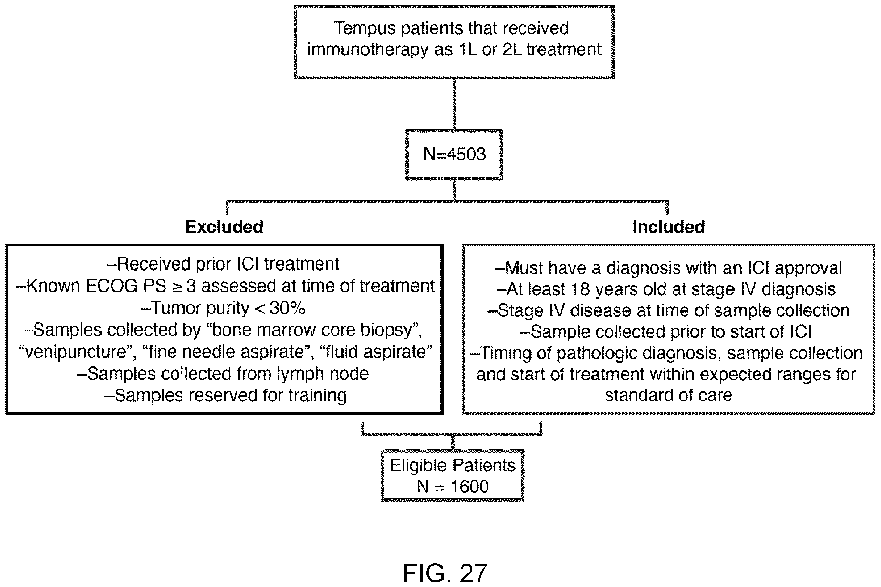

The model development and validation cohorts consist of patients from the de-identified Tempus real-world multimodal database, all of whom underwent clinical next-generation sequencing. **Figure 27** illustrates the CONSORT diagram for the validation cohort. Patients included in the study were diagnosed with stage IV cancer and received an approved ICI in 1L or 2L therapy after January 1, 2018 and before July 1, 2023 (1L) or January 1, 2024 (2L). Patients with an ECOG score $\geq$ 3 were excluded. To be eligible, samples had to be collected prior to any exposure to ICI therapy, with the time between sample collection and treatment within the standard of care range. Exclusion criteria included low tumor purity (<20% for development, <30% for validation) and samples collected from cytology or lymph node biopsies due to ambiguity of anatomic location of lymph node biopsy, high expression of immune genes in the lymph node, and background noise. Eligible patients were then representatively divided into development (n=1707) and validation (n=1600) cohorts. Further characterization of the overall validation cohort is listed in **Table 4**.

### NGS-based DNA and RNA sequencing

The Tempus testing platform includes both a targeted DNA sequencing assay (xT), and an exome capture RNA sequencing assay (xR) [8-10]. The current xT assay targets 648 genes, with a panel size of 1.9MB. Prior versions of xT assay, including a 596-gene version and other DNA sequencing assays, were also utilized in the analysis. TMB was calculated by dividing the number of nonsynonymous mutations by the size of the panel size (PMID: 37129893). The xT assay also includes probes for loci frequently unstable in tumors with mismatch repair deficiencies, allowing for the assessment of microsatellite instability (MSI) and classifies tumors into MSI-H, and MSS categories (PMID: 31040929). The xR assay is based on the IDT xGen Exome Research Panel v2 backbone, comprising >415K individually synthesized probes and spans a 34 Mb target region (19,433 genes) of the human genome. Tempus-specific custom spike-in probes are added to enhance target region detection in key areas like fusion and viral probes. Clinically, the xR assay is used for reporting gene fusions, alternative gene splicing, and gene expression algorithms [9-12].

### PD-L1 Immunohistochemistry

PD-L1 status for each patient was determined by clinical Tempus testing or curated from pathology reports associated with external PD-L1 IHC testing performed at the referring pathology lab. PD-L1 positive and negative classification for each cancer subtype was defined per the FDA guidelines or clinical trials. For cancer types lacking established PD-L1 IHC criteria, a generalized threshold of TPS greater than one was used to define positivity, this criteria was also generalizable across PD-L1 clones used in testing.

### Model development / ML and AI methodologies

DNA and RNA features adapted to the Tempus IO platform were used as the basis for feature selection for the Tempus IPS assay. The features in the IO platform consists of a comprehensive list of DNA and RNA based IO biomarkers that have been established in the literature as associated with tumor immune biology and IO outcomes [13]. In addition to the candidate features selected from the literature, two novel gene signatures were developed by Tempus as part of this study. The first is a signature of tumor-intrinsic immune resistance derived from single-cell RNA-sequencing data, which we term the single-cell immune resistance (scIR) signature [14]. Briefly, this signature was created using a variational autoencoder to extract biological signal from a single-cell RNA-sequencing sample taken from a lung adenocarcinoma patient. The scIR signature was strongly weighted in a small population of tumor cells within a highly immune-activated tumor environment and included known pathways of immune inhibitory signaling on tumor associated macrophages. The second signature was created to capture known literature meta-analysis signals using 105 genes [15].

Using a cohort of 1707 patients treated with ICI, 1094 patients were used to select the features for the model and 613 were held out for model evaluation. This train-evaluation split was performed to create comparable cohorts, stratified on line of therapy and cancer type. To avoid overreliance on this training set, candidate features were further evaluated in publicly available ICI data sets [5-8] using univariate Cox models. Features that did not reach $p < 0.05$ in any of these datasets were excluded from consideration. Using the remaining features, we fit a multivariate Cox proportional hazards model, stratifying by line of therapy (1L or 2L). The model was trained using 10-fold cross-validation, where balanced L1/L2 regularization was applied to remove redundant features, with cross-validation used to determine the regularization weights. The resulting model was then applied to the remaining 613 held-out patients to verify that the model performed consistently outside of the initial training data. After this assessment, the model's final feature coefficients were determined from the full 1707 patient training cohort. The IPS score was calculated as a linear combination of the coefficients and was min-max scaled to fall between 0-100. The threshold for IPS-low was set at all patients below the 55th percentile among the full training cohort, IPS-high at greater than or equal to the 60th percentile, and the patients between the 55th and 60th percentiles form an indeterminate category.

**Statistical analyses**

The analyses conducted in this study were defined prospectively in a statistical analysis plan. The primary objective was to demonstrate in a pan-cancer ICI treated population that IPS-High patients had longer overall survival compared to IPS-Low patients. A stratified Cox proportional hazards model was employed for the primary endpoint of overall survival, with adjustment for treatment regimen type (ICI only vs. ICI+additional), and stratification by line of therapy (first-line vs. second-line). Risk set adjustment was applied in patients where sequencing (and therefore study entry) occurred after the initiation of ICI [16]. The significance of the hazard ratio (HR) was evaluated using a one-sided Wald test at a 5% significance level. Consequently, the one-sided upper 95% confidence interval is provided for all survival analyses. The primary endpoint was also descriptively evaluated across several subgroups. These subgroups included PD-L1 positive and negative patients (based on available IHC data), TMB high and low (<10 mut/Mb and ≥10mut/Mb) and , age categories (<65 and ≥65), sex (male and female), regimens (ICI only vs. ICI+additional), and cancer types (restricted to those with at least 15 patients in both the IPS-High and IPS-Low groups). For each of these subgroups, a stratified Cox PH model (incorporating risk set adjustment) similar to the one described in the primary endpoint analysis was fit.
The prognostic utility of IPS over PD-L1 and TMB was evaluated by a likelihood ratio test that compared the full Cox model including both PD-L1 and IPS to a reduced Cox model that included PD-L1 alone (Methods - Statistical analysis). The prognostic utility of the IPS score in relation to TMB and MSI-H was assessed using a similar approach.
An exploratory analysis of the predictive utility of the IPS score was performed by combining the training and validation cohorts of patients who received chemotherapy (CT) as first line treatment and ICI as second line treatment. Patients served as their own control in this analysis, and outcomes were evaluated for two lines of therapy: time to next treatment (TTNT) on CT and OS on ICI. If IPS was purely prognostic, time to next treatment (as a surrogate for progression) would be anticipated to be longer in IPS-H patients than in IPS-Low patients. The HR for TTNT of IPS-H to IPS-L would then be of a similar magnitude as the HR for OS on second line treatment with ICI. A conditional model for recurrent events was fit to the selected subset of patients. Specifically, a Cox proportional hazards model, stratified by line of therapy, was used to model the two ordered time periods: period 1 in which the patient received CT and period 2 in which the patient received ICI. A Wald test p-value of less than 0.05 for the interaction between IPS and line of treatment would indicate a significant difference in the hazard ratios between the two time periods.

**Statement of Ethics**

This study was conducted in accordance with HIPAA regulations, where applicable, and IRB exempt determinations (Advarra Pro00076072, Pro00072742).

**Data Availability**

Deidentified data used in the research were collected in a real-world health care setting and subject to controlled access for privacy and proprietary reasons. When possible, derived data supporting the findings of this study have been made available within the paper and its Supplementary Figures and Tables.

**Results**

**IPS model development and feature characterization**

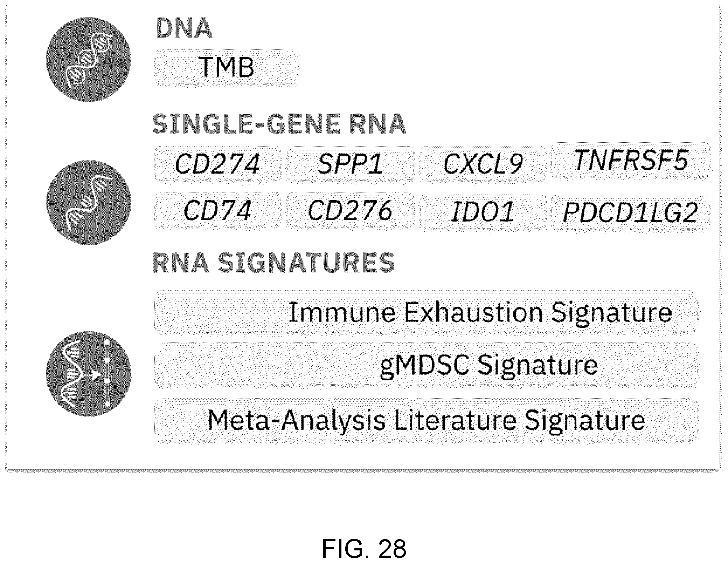

To develop a biomarker that robustly stratifies outcomes in pan-cancer, solid tumor, metastatic ICI-treated patients, we randomly divided the Tempus ICI cohort into a 1,707 development patient cohort and held out 1,600 patients for clinical validation. The development cohort was further subdivided into 1,094 patients for feature selection and model training and 613 were reserved for initial model evaluation. Potential features included in the model were drawn from a comprehensive set of RNA and DNA biomarkers that had been previously implicated in tumor-immune biology or associated with IO-related outcomes. We also considered two novel gene signatures developed as a part of this study that characterize expression patterns of tumor-intrinsic immune resistance (see "Model development", Methods). Candidate model features were initially selected using a combination of biological plausibility, association with rwOS in publicly available ICI studies, and favorable analytical properties. [5-8]. These candidate biomarkers were included in a preliminary multivariate Cox model, stratified by line of therapy. Final feature weights were determined using the combined development and evaluation cohorts (n=1,707) and included the following features: TMB, expression of *CD74, CD274, CD276, CXCL9, IDO1, PDCD1LG2, SPP1, TNFRSF5,* scIR signature, the meta-analysis literature signature, and a gMDSC signature **(Figure 28).** The IPS-low and IPS-high thresholds were set as the 55th and 60th percentile of the full training cohort respectively. Patients that fell between the 55th and 60th percentile thresholds were classified as indeterminate and excluded from further analysis.

**Patient characterization of validation cohort**

The validation cohort was comprised of 1600 patients with stage IV cancer: median (IQR) age of 65.0 (58.0-73.0) years, 40% female (n=645), 1,114 (70%) were treated at community-based hospital or medical practices, and 1,043 (65%) were smokers, 1,016 patients (64%) were White **(Table 4).** The majority of patients in the study were *de novo* stage IV at the time of diagnosis (1,219 [76%]). There were 16 cancer types included in the validation study. The most common cancer was NSCLC (330 patients [49.0%]), followed by GEJ (171 [11%]), urothelial (137 [9%]), RCC (131 [8%]) and HNSCC (125 [8%]). Of note, the following cancer subtype roll-ups were used for NSCLC (lung adenocarcinoma - 371 [23%], lung squamous carcinoma - 155 [9.7%], and NSCLC-NOS - 121 [7.6%]), gastro-esophageal (gastroesophageal adenocarcinoma - 147 [9.2%], gastroesophageal squamous cell carcinoma - 24 [1.5%]). The highest rates of IPS-H were observed in colorectal cancer (27 [59%]), melanoma (56 [55%]), and RCC (69 [53%]) subcohorts **(table 4).** Consistent with current standards of care, 91% of the colorectal cancer patients were MSI-H. The lowest rates of IPS-H were observed in GEJ (26 [15%]), urothelial (36 [26%]) and HNSCC (35 [28%]). PD-L1 IHC results were available on 1,132 patients (PD-L1 positive - [637], PD-L1 negative - [495]), the vast majority of cases were stained with PD-L1 22c3 (1,145). Notably, a higher proportion of IPS-H patients were PD-L1 positive (250 [43%]) versus PD-L1 negative (149 [26%]). TMB data were available on all patients in the study, and a higher proportion (%?) of IPS-L patients are TMB-L versus TMB-H.

Patients were treated with one of ten FDA-approved ICIs. The majority of patients received ICI therapy as part of the first line (1,326 [83%]) versus the second line (274 [17%]). Treatment patterns with ICI were generally consistent with established standards of care. Of the ICI regimen types, ICI+chemotherapy (869 [54%]) was the most common, followed by ICI monotherapy (381 [24%]) and ICI doublet (153 [9.6%]). Notable cancer types and regimens include NSCLC (ICI mono - ( 92 [14%]), ICI doublet ( 30 [4.6%]) ICI+chemo - ( 525 [81%]), ), melanoma (ICI mono - ( 56 [55%]), ICI doublet - ( 40 [39%])), and RCC (ICI doublet - (53 [40%]), ICI+other ( 66 [50%]). Of the patients receiving ICI+other, the "other" consisted mainly of tyrosine kinase inhibitors (78 [4.8%]) of which the majority was used in RCC patients (ICI+TKI - [66]), and ICI with a biologic such as anti-VEGF in hepatocellular carcinoma (Biologic + ICI [26]) and anti-EGFR in GEJ (Biologic + Chemo + ICI - [30]).

The median follow-up time was 21.2 months (IPS-H) or 18.9 months (IPS-L); follow-up time was calculated from reverse Kaplan Meier.

**Clinical validation of IPS as a pan-cancer ICI biomarker**

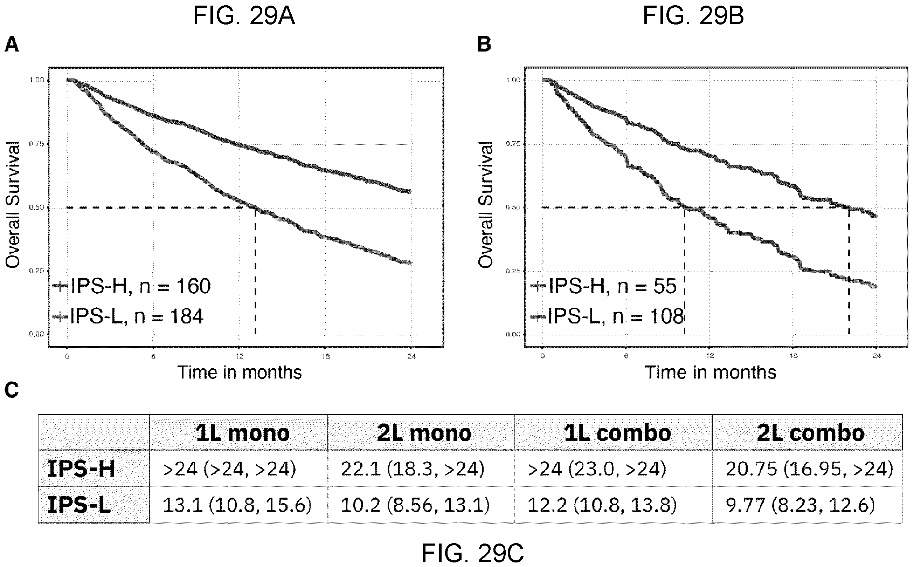

A multivariate CoxPH controlling for regimen (ICI monotherapy or ICI in combination with other therapies), and stratified by line of therapy (1L or 2L), was used to assess the prognostic association of IPS with patient outcomes. OS was demonstrated to be significantly longer in patients with tumors classified as IPS-H vs IPS-L (HR = 0.45 (0.40, 0.52), p-value < 0.01) **(Figure 29.).** Differences in survival between IPS-H and IPS-L were consistent across lines of therapy and regimens. The predicted OS from the CoxPH model is shown in **(Fig 29a,b)** for the setting of ICI only in 1L or 2L. Notably, the predicted OS curves for ICI combination therapy in 1L and 2L demonstrate a similar relationship of IPS result and predicted OS)

**Performance of IPS in clinical and biomarker subgroups**

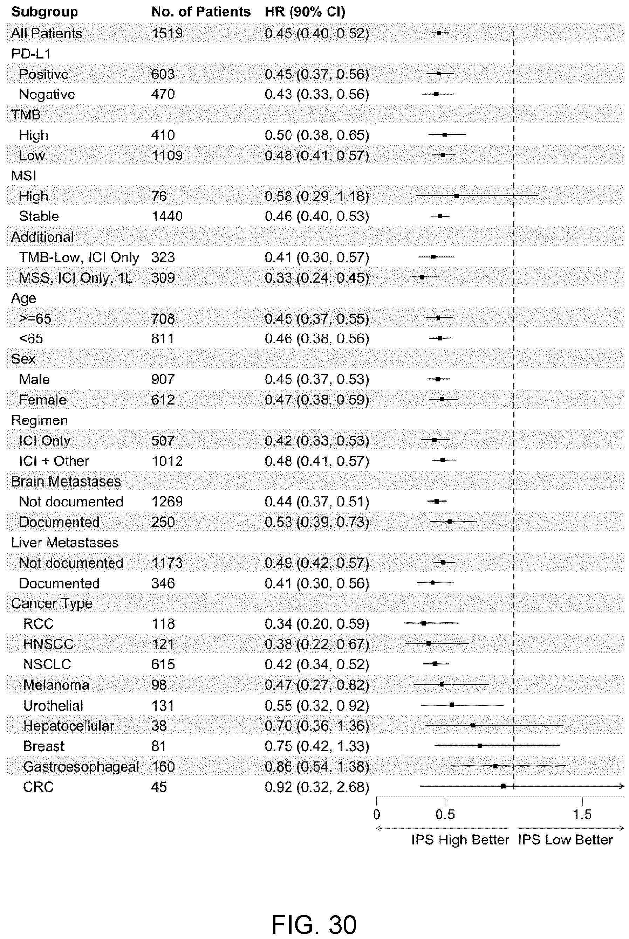

The prognostic association of IPS was also evaluated in clinical and biomarker subgroups. Patients whose tumors were classified as IPS-H had significantly longer OS than IPS-L tumors across all subgroups. Notably, significant associations were maintained across molecular biomarker subgroups of TMB-H, TMB-L, PD-L1+, PD-L1-, and MSI-H as well as clinical subgroups of presence/absence of brain or liver metastasis **(Figure 30).** HR subgroup estimates were similar in direction and magnitude to that of the overall estimate. Among the cancer subgroups evaluated, RCC (0.34 [0.20, 0.59]), HNSCC (0.38 [0.22, 0.67]), NSCLC (0.42 [0.34, 0.52]), and melanoma (0.47 [0.27, 0.82]) had the largest effects while the smallest effects for the IPS score were observed in GEJ, HCC, breast and CRC An exploratory subgroup analysis was performed in RCC and melanoma to evaluate IPS in patients receiving ICI-doublet regimens which are enriched in those disease groups, with melanoma showing (HR=0.56 [0.25-1.23]) and RCC (HR=0.25 [0.10-0.63]).

## IPS has prognostic utility beyond TMB, PD-L1, and MSI

To demonstrate the prognostic association of IPS score beyond the clinically established biomarkers of TMB, PD-L1 IHC, and MSI, we compared the full model including both IPS score and the biomarker of interest to a reduced model of either TMB, PD-L1 IHC, or MSI without IPS (see Methods). We observed a significant association of IPS over TMB, PD-L1 IHC, and MSI (p < 0.001).

The predicted OS curves for these biomarker subgroups, categorized by IPS status, are presented in patients treated with ICI monotherapy in 1L **(Fig. 31b-d)**. For pan-cancer Similar predicted OS curves for treatment conditions and lines of therapy now shown are shown in the supplementary data. Given the size and clinical significance of the NSCLC cohort, these results are also broken out for NSCLC by PD-L1 status. The predicted OS curve is shown for combination therapy in 1L along with similar predicted OS curves for monotherapy and 2L treated patients **(Fig 31e)**. HRs and 90% CI for the most relevant curves shown in the predicted OS plots are listed in **Fig 31f**.

## Exploratory evaluation of predictive utility for IPS

In an exploratory analysis to test the potential predictive utility of IPS, we examined a combined cohort of training and validation patients that had been exposed to non-ICI and ICI therapies in 1L and 2L respectively. While IPS was not associated with TTNT on CT in 1L (HR = 1.06 (0.85, 1.33); **Fig. 32a)**, it was significantly associated with OS in patients receiving 2L ICI (HR = 0.63 [0.46, 0.86]; **Fig. 32b)**. An interaction test between the two lines was significant (p<0.01) indicating that the HR for 2L ICI between IPS-H and IPS-L is significantly different from the HR for 1L CT.

To further evaluate prognostication of IPS in non-ICI treated patients as a means to understand predictive utility, an exploratory analysis was performed in stage IV patients from The Cancer Genome Atlas (TCGA, N=722, patient selection criteria is described in Supplemental Methods). The TCGA enrollment period was prior to the approval and usage of ICI therapies thus ensuring a representative non-ICI comparator cohort that also had DNA and RNA sequencing available to generate a modified version of IPS. There was a significant association of IPS with OS in this cohort (HR=0.75 [0.56-0.99]), however the hazard ratio was attenuated relative to the IPS validation cohort.

## Tumor distribution and IPS prevalence in a expanded pan-cancer cohort

In order to characterize IPS prevalence more generally including in cancer types without approved ICI indications, we examined the distribution of IPS-H and IPS-L in an expanded pan-cancer cohort of patients sequenced at Tempus. In the entire cohort encompassing 25 different cancer types, prevalence of IPS-H was 28.64%. Lung adenocarcinoma, RCC, and melanoma had IPS-H prevalence greater than 50%. On the opposite side of the spectrum, GI neuroendocrine cancer, cholangiocarcinoma, CRC, gynecologic sarcomas, and PDAC all had IPS-H prevalence of less than 20%. Of note, lung squamous cell carcinoma had a prevalence of 25.59% and NSCLC-NOS had a prevalence of 45.75% indicating a likely high proportion of lung adenocarcinomas in the NOS group of patients. To further characterize how IPS may identify ICI responders outside of current cancer type or pan-cancer biomarker ICI approvals, we calculated the proportion of patients who are IPS-H and TMB-L (14.1%) after excluding cancer types with an ICI approval or tumors that were MSI-H. We also generated a more granular cancer subtype type visualization of IPS status in relation to TMB status.

Discussion/Conclusions

Leveraging the Tempus xT / xR assays and the IO platform along with real-world data from ICI treated patients, we developed and validated the multi-omic IPS algorithm in a prospectively designed retrospective study using a real-world cohort of advanced solid organ cancer patients treated with an ICI containing regimen in the first or second line of therapy. Using a prespecified statistical analysis plan, IPS was validated as a generalizable pan-cancer prognostic biomarker demonstrating that IPS-high patients have significantly longer OS then IPS-low patients. Additionally, the validation demonstrated that IPS-high patients had significantly longer OS compared to IPS-low patients across relevant ICI biomarker subgroups, including PD-L1 status, TMB levels, and microsatellite stability. Specifically, in TMB-low patients receiving ICI-only therapy, and microsatellite-stable (MSS) patients treated with ICI in their first line of therapy, IPS-high patients showed substantially longer survival than their IPS-low counterparts. Notably, IPS retained its prognostic significance in multivariable models, even when controlling for TMB, MSI status, and PD-L1 expression. Overall these analyses demonstrate the clinical value of IPS to assess potential benefit to ICI regimens beyond the current standard of care biomarkers. Finally, a post-hoc exploratory analysis into the predictive capabilities of IPS was performed with patients who received chemotherapy in the first line of therapy and ICI in the second line. IPS did not predict time to the next treatment following chemotherapy, however, IPS was a significant predictor of OS when patients were subsequently treated with ICI.

Our study results build upon the growing body of evidence supporting that multi-omic biomarkers developed using machine learning / artificial intelligence methodologies, high-throughput commercial NGS assays, and real-world clinical data can provide insights into tumor/immune biology and clinical outcomes. The current treatment paradigm of approved immunotherapies therapies in addition to the vast number of clinical trials (including ALCHEMIST, OptimICE-PCR, EQUATE, PET-Stop trials) utilizing immunotherapies with a diverse range of mechanisms and targets highlights opportunities and unmet clinical needs for patient selection using multi-omic biomarkers [17].

In the current treatment paradigm of stage IV solid organ cancers, there are opportunities for biomarkers to help inform clinical management for approved ICI regimens in indications with equipoise between regimens or indications that lack biomarkers for patient selection. This opportunity is perhaps most apparent in NSCLC where patients of all PD-L1 levels are approved for ICI+chemo while in tumors with PD-L1 IHC high (TPS > 50) patients can receive ICI+chemo or ICI monotherapy [18]. A significant focus of clinical research has therefore focused on further sub-stratification of PD-L1 IHC. Aguilar *et al.* showed in an RWD retrospective analysis that patients with TPS scores greater than 90 have significantly better outcomes than patients with TPS between 50 and 89, which may be informative for ICI monotherapy patient selection [19]. In our exploratory analysis of NSCLC patients receiving ICI monotherapy and subgrouped by PD-L1 IHC levels, patients with IPS high tumors in all PD-L1 IHC subgroups were observed to have longer OS then patients with IPS low tumors. This finding may represent the importance of CD274 (PD-L1) gene expression as a continuous feature in the IPS model. The analysis is notably limited by small sample sizes but generally highlights the potential of IPS to capture tumor immune biomarker granularity and precision. Currently the INSIGNA study which is a large randomized control trial in NSCLC has aims focused on elucidating the optimal clinical management for these patients [20].

Regarding the potential for IPS to inform new treatment indication strategies, **Figure 32** highlights the cancer specific IPS-high/low prevalence in an expanded cohort that include diseases that currently lack ICI indications. Of note, MSS colorectal cancer, and pancreatic cancer were shown to have among the lowest IPS high rates which tracks with the historically limited response rates seen in ICI monotherapy trials for those cancer types [21-23]. IPS therefore could have value in identifying the rare potential responders in these or similarly challenging diseases for ICI. Additionally, we showed the proportion of patients who are IPS-H / TMB-L / MSS in cancer subtypes that currently lack an ICI approval, indicating the potential pan-cancer role of IPS-H for identifying ICI responders in stage IV patients that currently lack an approved use. Prospective clinical trial designs utilizing IPS stratification or selection may be considered in the future [24]. However, perhaps even more impactful than development of monotherapy ICI studies is the potential application of multi-omic biomarkers such as IPS to inform patient selection for novel ICI combinatorial strategies and the next generation of immunotherapy modalities such as T-cell/NK-cell engagers and RNA cancer vaccines. These novel applications may require modified versions of IPS along with additional biomarkers that characterize the cancer-immunity cycle relevant to a specific combinatorial strategy [4]. Lastly, as ICI based regimens move into neoadjuvant and adjuvant settings, there are significant opportunities for patient selection strategies to reduce ICI exposure in patients unlikely to respond and therefore reducing the number of overall adverse events.

Limitations of this study reflect the real-world, retrospective nature of the validation cohort. While our study inclusion and exclusion criteria attempted to control for confounding variables and non-standard care scenarios, additional biases may be unaccounted for. Regarding our attempts to characterize the predictive nature of IPS, Tempus clinical testing and our subsequent clinical-molecular data set was generated predominantly in the post ICI-era. Therefore, we did not have the ability to perform case-control matching with patients who received non-ICI regimens prior to the approvals. We attempted to address this limitation with an analysis of stage IV patients who did not receive ICI, collected from TCGA. Among these patients, we observed a significant difference in OS between patients classified as IPS-high versus IPS-low. This result suggests that the IPS model has generalized prognostic utility, as would be biologically expected given the known prognostic association of immune infiltration in tumors [25] which the model is intended to capture. However, given the attenuated hazard ratio we observed in these non-ICI treated patients in TCGA versus the ICI treated patients in the study cohort, IPS appears to have additionally predictive utility. Also of note, the proportion of patients in each cancer type and biomarker subgroup is representative of clinical testing at Tempus which expectedly results in disproportionately sized cancer subgroups in the development and validation cohorts representative of cancer prevalence and NGS testing frequency. The variability of cohort size across cancer types therefore limits the ability to comprehensively evaluate the heterogeneity of IPS performance across cancer types. Additionally, the IPS model did not include clinical and lab features that have been demonstrated to add prognostic utility in combination with molecular markers such as TMB as evidenced by Chowell *et al,* which could be considered for future model iterations [PMID: 34725502].

In summary, we demonstrated in a large RWD clinical validation study that IPS is a generalizable multi-omic biomarker that can be widely utilized clinically as a prognosticator of ICI based regimens. Importantly, IPS-high may identify patients within subgroups (TMB-L, MSS, PD-L1 negative) who benefit from ICI beyond what is predicted by existing biomarkers. Future prospective predictive utility studies are planned for evaluating the numerous clinical applications of IPS.

**Supplemental Methods**

*Clinical Data Abstraction*

Clinical data were extracted from the Tempus real-world oncology database. This encompassed longitudinal structured and unstructured data from geographically diverse oncology practices, including integrated delivery networks, academic institutions, and community practices. Structured data from electronic health record systems were integrated with unstructured data collected from patient records via technology-enabled chart abstraction and corresponding molecular data, if applicable. Patients with no recorded date of death across all mortality sources were censored at the date of last recorded interaction with the medical system (i.e., date of last follow-up).

*TMB*

TMB was calculated by dividing the number of nonsynonymous variations by the size of the panel (2.4 Mb for the panel size of xT.v2 and 1.9Mb for the panel coding region of xT.v4). All non-silent somatic coding variations such as missense, indel, and stop-loss variants with coverage greater than $\times$ 100 and an allelic fraction greater than 5% are included in the count of nonsynonymous variations. TMB calculated using the assay is highly correlated with TMB calculated from whole exome TCGA data (R = 0.986, P < 2.2 $\times$ 10-16).16 The xT.v2 TMB score is adjusted for differences in denominators between the versions to be directly comparable to xT.v4. All analyses are completed incorporating both assays, with tumors considered TMB-H if they have an adjusted TMB score of 10 mut/Mb or more.

*TCGA Supplemental Methods*

FASTQ files from RNA sequencing data for the TCGA cohort were downloaded from the Genomic Data Commons (cite) and processed through the Tempus RNA pipeline as described below. The clinical data for the cohort was obtained from cBioportal (cite). Patients included were required to have been Stage 4 at sample collection and have RNA-sequencing, TMB, and OS data available. The period from OS anchor date to the 24 month maximum follow up date was required to be before first FDA approval of ICI. This criteria yielded a cohort of 752 patients. The RNA-sequencing data was reprocessed from raw abundance files using the Tempus RNA-processing pipeline (as described in Methods). Linear batch correction was further applied so that the normalized counts were comparable to the data used in the IPS validation cohort. The IPS model was run on the resulting data set without adjustment. Of the 752 patients, 722 were assigned an IPS-high or IPS-low category, with 30 receiving a score in the indeterminate range.

*Analytical Validation*

The Tempus IPS assay was analytically validated to ensure consistent performance across a variety of experimental conditions associated with the underlying IPS assay inputs (xT - TMB, xR - RNA features) to test the precision and analytical accuracy of computing the IPS score and the IPS result (IPS high or IPS low) under CAP/CLIA standards. Precision was tested through repeatability and reproducibility studies using tumor samples from five cancer types: NSCLC, HNSCC, melanoma, urothelial carcinoma, and RCC. These samples were run in triplicate, incorporating both DNA (xT) and RNA (xR) replicates to generate the IPS score. Repeatability was evaluated within a single assay run, while reproducibility was tested across multiple runs involving different instruments, reagent lots, and operators. 30 tumor samples were used in replicates, with DNA and RNA extracted from the same patients but placed on separate plates for independent processing in each run. The study utilized about 12 different flowcell reagent lots, 20 unique flowcells, and 11 distinct sequencers, 4 different operators, ensuring a comprehensive evaluation of reproducibility across different conditions. The repeatability overall percent agreement (OPA) was calculated to be 97%, while the reproducibility OPA was determined to be 95%. Scatter plots (Fig. S-AV-1 and S-AV-2) demonstrated tight clustering of replicate IPS scores around the expected diagonal, with over 95% of replicate pairs producing highly consistent IPS scores, affirming the assay's robust repeatability and reproducibility across varied experimental conditions.

Analytic sensitivity was assessed by testing RNA inputs of 25 ng, 50 ng, 100 ng, and 300ng to ensure robust performance at varying RNA levels. In addition, retrospective real-world data from clinically sequenced samples using the xT and xR assays (the IPS RWD cohort) were used for validation of reportable range and analytic sensitivity, leveraging the combined DNA and RNA features to ensure the assay's reliable performance across diverse tumor sites, procedures, and tumor purity thresholds. New prospective data generated in the wet lab were used in precision experiments and in laboratory concordance studies, ensuring that the IPS assay produces consistent and reproducible results across all solid tumors.

Lastly, we tested the effect of macrodissection and changes in tumor purity on IPS results given the practice of pathologist discretionary macrodissection in the xT and xR sample workflow. Unstained slides from 29 samples that were previously macrodissected as part of the clinical workflow underwent whole slide scraping, resulting in non-macrodissected samples with lower tumor purity than the original microdissected samples. These samples had tumor purities ranging from 40% to 80%, representing borderline macrodissectable cases. A comparison of pre- and post-macrodissected samples revealed a Pearson correlation coefficient of 0.979 and an overall percent agreement in risk classification of 85.7%, indicating a very strong positive correlation between IPS scores before and after macrodissection. This suggests that the macrodissection process does not significantly impact the assay's results. The robustness of the IPS assay was further confirmed by consistent risk classification across various cancer types, ensuring its reliability for clinical decision-making even in samples with borderline tumor purity.

Table 4 - Cohort summary. Overall category sums down column; IPS-H, IPS-L, and Indeterminate sum across rows

| Characteristics | Overall N=1600 | IPS-High N=576 | IPS-Low N=943 | Indeterminate N=81 |
|---|---|---|---|---|
| Age | | | | |
| Mean (SD) | 64.6 (11.8) | 64.9 (12.1) | 64.5 (11.6) | 64.2 (11.7) |
| Median | 65.0 | 65.0 | 65.0 | 66.0 |
| [IQR] | [58.0, 73.0] | [59.0, 74.0] | [58.0, 73.0] | [56.0, 74.0] |
| Min / Max | 20.0 / 89.0 | 27.0 / 89.0 | 20.0 / 88.0 | 34.0 / 83.0 |
| Sex | | | | |
| Female | 645 (40%) | 252 (44%) | 360 (38%) | 33 (41%) |
| Male | 955 (60%) | 324 (56%) | 583 (62%) | 48 (59%) |
| Line of therapy | | | | |
| 1 | 1,326 (83%) | 482 (84%) | 774 (82%) | 70 (86%) |
| 2 | 274 (17%) | 94 (16%) | 169 (18%) | 11 (14%) |
| Treatment regimen | | | | |
| IO Only | 534 (33%) | 215 (37%) | 292 (31%) | 27 (33%) |
| IO mono | 381 (24%) | 146 (25%) | 219 (23%) | 16 (20%) |
| IO doublet | 153 (9.6%) | 69 (12%) | 73 (7.7%) | 11 (14%) |
| IO + Other | 1,066 (67%) | 361 (63%) | 651 (69%) | 54 (67%) |
| IO+Chemo | 869 (54%) | 283 (49%) | 542 (57%) | 44 (54%) |
| IO+Chemo+Other | 72 (4.5%) | 15 (2.6%) | 55 (5.8%) | 2 (2.5%) |
| IO+Other | 125 (7.8%) | 63 (11%) | 54 (5.7%) | 8 (9.9%) |
| ECOG | | | | |
| 0 | 334 (21%) | 137 (24%) | 186 (20%) | 11 (14%) |
| 1 | 473 (30%) | 168 (29%) | 279 (30%) | 26 (32%) |
| 2 | 140 (9%) | 45 (8%) | 93 (10%) | 2 (2%) |
| Unknown/Missing | 653 (40%) | 226 (39%) | 385 (40%) | 42 (52%) |
| Stage at primary dx | | | | |
| Stage I | 47 (3%) | 22 (4%) | 23 (2%) | 2 (2%) |
| Stage II | 68 (4%) | 25 (4%) | 41 (4%) | 2 (2%) |
| Stage III | 94 (6%) | 33 (6%) | 58 (6%) | 3 (4%) |
| Stage IV | 1,219 (76%) | 430 (75%) | 721 (76%) | 68 (84%) |
| Unknown/Missing | 172 (11%) | 66 (11%) | 100 (11%) | 6 (7%) |
| Cancer type * | | | | |
| Breast | 86 (5%) | 40 (47%) | 41 (48%) | 5 (6%) |
| CRC | 46 (3%) | 27 (59%) | 18 (39%) | 1 (2%) |
| Gastroesophageal | 171 (11%) | 26 (15%) | 134 (78%) | 11(6%) |
| Hepatocellular | 40 (2%) | 16 (40%) | 22 (55%) | 2 (5%) |

(continued)

| Characteristics | Overall N=1600 | IPS-High N=576 | IPS-Low N=943 | Indeterminate N=81 |
|---|---|---|---|---|
| HNSCC | 125 (8%) | 35 (28%) | 86 (69%) | 4 (3%) |
| Melanoma | 102 (6%) | 56 (55%) | 42 (41%) | 4 (4%) |
| NSCLC | 647 (40%) | 248 (38%) | 367 (57%) | 32 (5%) |
| RCC | 131 (8%) | 69 (53%) | 49 (37%) | 13 (10%) |
| Urothelial | 137 (9%) | 36 (26%) | 95 (69%) | 6 (4%) |
| Other | 115 (7%) | 23 (20%) | 89 (77%) | 3 (3%) |
| Brain metastases Documented | 265 (17%) | 107 (19%) | 143 (15%) | 15 (19%) |
| Liver metastases Documented | 362 (23%) | 94 (16%) | 252 (27%) | 16 (20%) |
| PD-L1 by IHC Negative | 495 (31%) | 149 (26%) | 321 (34%) | 25 (31%) |
| Positive | 637 (40%) | 250 (43%) | 353 (37%) | 34 (42%) |
| Unknown/Missing | 468 (29%) | 177 (31%) | 269 (29%) | 22 (27%) |
| TMB High | 430 (27%) | 250 (43%) | 160 (17%) | 20 (25%) |
| Low | 1,170 (73%) | 326 (57%) | 783 (83%) | 61 (75%) |
| MSI High | 80 (5.0%) | 45 (7.8%) | 31 (3.3%) | 4 (4.9%) |
| Stable | 1517 (95%) | 531 (92%) | 909 (96%) | 77 (95%) |
| Undetermined | 3 (0.2%) | 0 (0%) | 3 (0.3%) | 0 (0%) |

References for Example 4

1 Herbst RS, Baas P, Kim D-W, et al. Pembrolizumab versus docetaxel for previously treated, PD-L1-positive, advanced non-small-cell lung cancer (KEYNOTE-010): a randomised controlled trial. Lancet. 2016;387:1540-50.

2 Zhao B, Zhao H, Zhao J. Efficacy of PD-1/PD-L1 blockade monotherapy in clinical trials. Ther Adv Med Oncol. 2020;12:1758835920937612.

3 Kim MS, Prasad V. Pembrolizumab for all. J Cancer Res Clin Oncol. 2023;149:1357-60.

4 Chen DS, Mellman I. Oncology meets immunology: the cancer-immunity cycle. Immunity. 2013;39:1-10.

5 Litchfield K, Reading JL, Puttick C, et al. Meta-analysis of tumor- and T cell-intrinsic mechanisms of sensitization to checkpoint inhibition. Cell. 2021;184:596-614.e14.

6 Lin HM, Wu Y, Yin Y, et al. Real-world ALK testing trends in patients with advanced non-small-cell lung cancer in the United States. Clin Lung Cancer. 2023;24:e39-49.

7 Anti-PD1 response prediction dream challenge. DREAM Challenges. https://dreamchallenges.org/anti-pd1-response-prediction-dream-challenge/ (accessed 12 September 2024)

8 Beaubier N, Bontrager M, Huether R, et al. Integrated genomic profiling expands clinical options for patients with cancer. Nat Biotechnol. 2019;37:1351-60.

9 Beaubier N, Tell R, Lau D, et al. Clinical validation of the tempus xT next-generation targeted oncology sequencing assay. Oncotarget. 2019;10:2384-96.

10 Wenric S, Davison JM, Guittar J, et al. Real-world data validation of the PurIST pancreatic ductal adenocarcinoma gene expression classifier and its prognostic implications. medRxiv. 2023;2023.02.23.23286356.

11 Michuda J, Breschi A, Kapilivsky J, et al. Validation of a Transcriptome-Based Assay for Classifying Cancers of Unknown Primary Origin. Mol Diagn Ther. 2023;27:499-511.

12 Leibowitz BD, Dougherty BV, Bell JSK, et al. Validation of genomic and transcriptomic models of homologous recombination deficiency in a real-world pan-cancer cohort. BMC Cancer. 2022;22:587.

13 Jain P, Stein MM, Fields P, et al. 163 A multi-modal, pan-cancer atlas of tumor-immune states across primary and metastatic disease using a large, real-world database. Regular and Young Investigator Award Abstracts. BMJ Publishing Group Ltd 2023.

14 Erbe R, Stein MM, Rand TA, et al. Abstract 2281: A tumor-intrinsic signature involving immunosuppression via MIF-CD74 signaling is associated with overall survival in ICT-treated lung adenocarcinoma. Cancer Res. 2024;84:2281-2281.

15 Bareche Y, Kelly D, Abbas-Aghababazadeh F, et al. Leveraging big data of immune checkpoint blockade response identifies novel potential targets. Ann Oncol. 2022;33:1304-17.

16 Tsai W-Y, Jewell NP, Wang M-C. A note on the product-limit estimator under right censoring and left truncation. Biometrika. 1987;74:883.

17 Upadhaya S, Hubbard-Lucey VM, Yu JX. Immuno-oncology drug development forges on despite COVID-19. Nat Rev Drug Discov. 2020;19:751-2.

18 Merck & Co., Inc. KEYTRUDA (pembrolizumab) [package insert]. U.S. Food and Drug Administration website .

19 Aguilar EJ, Ricciuti B, Gainor JF, et al. Outcomes to first-line pembrolizumab in patients with non-small-cell lung cancer and very high PD-L1 expression. Ann Oncol. 2019;30:1653-9.

20 Hossein Borghaei, ECOG-ACRIN Cancer Research Group. Testing the Timing of Pembrolizumab Alone or With Chemotherapy as First Line Treatment and Maintenance in Non-small Cell Lung Cancer. ClinicalTrials.gov. https://clinicaltrials.gov/study/NCT03793179 (accessed 24 September 2024)

21 Sahin IH, Ciombor KK, Diaz LA, et al. Immunotherapy for microsatellite stable colorectal cancers: Challenges and novel therapeutic avenues. Am Soc Clin Oncol Educ Book. 2022;42:1-12.

22 Marabelle A, Le DT, Ascierto PA, et al. Efficacy of pembrolizumab in patients with noncolorectal high microsatellite instability/mismatch repair-deficient cancer: Results from the phase II KEYNOTE-158 study. J Clin Oncol. 2020;38:1-10.

23 Reardon DA, Brandes AA, Omuro A, et al. Effect of nivolumab vs bevacizumab in patients with recurrent glioblastoma: The CheckMate 143 phase 3 randomized clinical trial. JAMA Oncol. 2020;6:1003-10.

24 Marabelle A, Fakih M, Lopez J, et al. Association of tumour mutational burden with outcomes in patients with advanced solid tumours treated with pembrolizumab: prospective biomarker analysis of the multicohort, open-label, phase 2 KEYNOTE-158 study. Lancet Oncol. 2020;21:1353-65.

25 Fridman WH, Zitvogel L, Sautès-Fridman C, et al. The immune contexture in cancer prognosis and treatment. Nat Rev Clin Oncol. 2017;14:717-34.

Example 5 - Clinical validation of a novel multi-omic algorithm for stratifying outcomes in a real-world cohort of advanced solid cancer patients treated with immune checkpoint inhibitors

Despite advances in immune checkpoint inhibitor (ICI) biomarker molecular testing, there remains an unmet clinical need for more sensitive and generalizable biomarkers to better predict patient outcomes on ICI. This has been challenging due to the limited availability of multi-omic testing and validation cohorts. An integrated DNA/RNA ICI biomarker can address this critical unmet need.

A de-identified pan-cancer cohort from the Tempus multimodal real-world database was used for the development and validation of the Immune Profile Score (IPS) algorithm leveraging Tempus xT (648 gene DNA panel) and xR (RNAseq). The cohort (n=1707 training [T]; n=1600 validation [V]) consisted of advanced stage cancer patients treated with any ICI containing regimen as the first (1L) or second (2L) line of therapy. The IPS model was developed utilizing a machine learning framework that includes tumor mutational burden (TMB) and 11 RNA-based biomarkers as features. Cox Proportional Hazards (CoxPH) models were fit to demonstrate prognostic utility. Predictive utility of IPS was evaluated in an exploratory analysis using a Cox model for recurrent events.

Our results demonstrate that IPS is a generalizable multi-omic biomarker that can be widely used clinically as a prognosticator of ICI-based regimens. IPS-high may identify patients (e.g. within TMB-L, MSS, PD-L1 low subgroups) who may benefit from ICI beyond what is predicted by standard biomarkers. An exploratory analysis is suggestive of predictive utility. Future prospective predictive utility studies are planned.

It should be understood that the examples given above are illustrative and do not limit the uses of the systems and methods described herein in combination with a digital and laboratory health care platform.

In the foregoing description, it will be readily apparent to one skilled in the art that varying substitutions and modifications may be made to the invention disclosed herein without departing from the scope and spirit of the invention. The invention illustratively described herein suitably may be practiced in the absence of any element or elements, limitation or limitations which is not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention. Thus, it should be understood that although the present invention has been illustrated by specific embodiments and optional features, modification and/or variation of the concepts herein disclosed may be resorted to by those skilled in the art, and that such modifications and variations are considered to be within the scope of this invention.

All methods described herein can be performed in any suitable order unless otherwise indicated herein or otherwise clearly contradicted by context. The use of any and all examples provided herein, is intended merely to better illuminate the invention and does not pose a limitation on the scope of the invention unless otherwise claimed. No language in the specification should be construed as indicating any non-claimed element as essential to the practice of the invention.

Citations to a number of patent and non-patent references are made herein. The cited references are incorporated by reference herein in their entireties. In the event that there is an inconsistency between a definition of a term in the specification as compared to a definition of the term in a cited reference, the term should be interpreted based on the definition in the specification.

It will be understood by one of ordinary skill in the art that reaction components are routinely stored as separate solutions, each containing a subset of the total components, for reasons of convenience, storage stability, or to allow for application- dependent adjustment of the component concentrations, and that reaction components are combined prior to the reaction to create a complete reaction mixture. Furthermore, it will be understood by one of ordinary skill in the art that reaction components are packaged separately for commercialization and that useful commercial kits may contain any subset of the reaction components of the invention.

The methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred aspects of this invention are described herein, including the best mode known to the inventors for carrying out the invention. Variations of those preferred aspects may become apparent to those of ordinary skill in the art upon reading the foregoing description. The inventors expect a person having ordinary skill in the art to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

**Claims**

1. A method of selecting a subject for treatment with an immune oncology (IO) therapy, wherein the subject is in need of treatment for a cancer, the method comprising:
   at a computer system having one or more processors, and memory storing one or more programs for execution by the one or more processors:
   applying, by the one or more processors, one or more model components derived from sequencing data from a sample of the cancer to one or more machine learning algorithms (MLAs), wherein the sequencing data comprises RNA sequencing data and DNA sequencing data, wherein the one or more model components comprise a tumor mutational burden (TMB), a checkpoint related gene signature, an immune exhaustion signature, a granulocytic myeloid derived suppressor cell (gMDSC) signature, and an immune oncology signature;
   displaying a report, the report comprising an indication that the subject is selected for an immune oncology therapy.

2. The method of claim 1, wherein the subject has a cancer that is PD-L1 low, PD-L1 intermediate, or has a low tumor mutational burden.

3. The method of any one of claim 1 or claim 2, wherein:

   (a) the one or more machine learning algorithms (MLAs) are trained on training data from a cohort of subjects diagnosed with cancer; and/or
   (b) the one or more MLAs comprise a variational autoencoder, an accelerated failure time model, a parametric survival model, a Cox proportional hazards model, a random forest model, a gradient-boosted survival model, a linear model, a recurrent neural network, a transformer neural network, or a convolutional neural network.

4. The method of any one of the preceding claims, wherein the checkpoint related gene signature comprises expression values for:

   (a) one or more genes selected from CD274, SPP1, CXCL9, CD74, CD276, IDO1, PDCD1LG2, and TNFRSF5; or

(b) CD274, SPP1, CXCL9, CD74, CD276, IDO1, PDCD1LG2, and TNFRSF5.

5. The method of any one of the preceding claims, wherein the immune exhaustion signature comprises expression values for the following genes TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, and SLC38A5.

6. The method of any one of the preceding claims, wherein the immune exhaustion signature comprises expression values for one or more genes selected from TMSB4X, CCL5, TSC22D3, CYTOR, CXCL13, TXNIP, PTPRCAP, RGCC, IGLC3, CYTIP, IGHV1-69D, CXCR4, HMGN2, HSPD1, NEU1, TPD52, GZMB, PIM1, SRGN, BST2, PDE4B, HSPA8, PRF1, CD7, SLC38A5, TIFA, DOK2, PPP1R2, DMAC1, DNAJB1, TAGAP, GZMA, CD27, GADD45A, HSPH1, STMN1, GZMH, CLIC3, GLIPR1, CHORDC1, CD3E, CD69, BAG3, ATF3, MICB, TRBC2, EZR, ARHGDIB, CASC8, ITM2A, DDX24, CD52, RAC2, TERF2IP, ELF1, FAM96B, GGH, NKG7, LY6E, CITED2, ZFAND2A, SAMSN1, CST7, CDKN3, TCEAL3, BBC3, IL32, MBD4, DNAJA4, TMEM141, UBB, HCST, IGLV1-40, HOPX, RHOH, USB1, H2AFZ, CSRP1, IKZF1, RGS2, IGLC2, CCND2, SELPLG, FUNDC2, IGFBP7, IGKV3-15, SERPINE2, TRDMT1, RGS1, HMOX1, HSP90AB1, HSPA1A, LIME1, TUBB, MRPL10, IFI44L, COTL1, LBH, ZEB2, HMGB2, LDHA, LGALS3, CYLD, PXMP2, CD74, PPIH, CD8A, RFX2, KLRD1, KLF6, LINC02446, HTRA1, TUBA4A, HSPB1, DNAJA1, CD3D, DUSP2, ELL2, TPM1, CKS1B, LGALS1, BEX3, GLRX, CCL4, GBP5, PTPRC, CLK1, IRF4, PIM2, SAT1, CXCR3, ZFP36, CD24, PELI1, CKS2, GYPC, FOXN2, IGLV1-51, IFT46, IGLV1-41, PLA2G16, COMMD8, IPCEF1, SMPDL3B, EVL, EVI2B, RAB11FIP1, DUSP5, HAVCR2, UBC, CRIP1, SRPRB, SERPINA1, PCSK7, BCL2L11, HSPA6, CWC25, CORO1A, TPST2, MBNL2, CKB, TUBA1B, GABARAPL1, PXDC1, SEL1L, PPP1R8, FKBP4, GABARAPL2, JCHAIN, STK17B, ZWINT, CHMP1B, ID2, HERPUD1, ROCK1, SKAP1, S100A4, CXCL10, CASP3, APOC1, ARID5B, SMAP2, CSRNP1, ADIRF, HLA-DPA1, PPP1R15A, DMKN, SCAF4, MYL9, LYAR, ZBTB25, GADD45B, GCHFR, LINC01588, RAB20, LSP1, FCGR2B, HIST2H2AA4, NCF4, LCK, IGHV3-33, LAPTM5, TUBB4B, TPM2, RBM38, RBP4, CCNA2, SERTAD1, ITM2C, PLPP5, DNAJB9, SYNGR2, TUBB2A, ERLEC1, TMED9, IFI6, HSP90AA1, PTPN1, TTL, DKK1, TM2D3, DCAF11, RIC1, SERPING1, DERL3, KDELR3, GEM, KLF9, TYROBP, CERCAM, CCDC84, ODC1, CYP2C9, CFLAR, HLA-DMB, DUSP1, JSRP1, TRIB1, JUN, NFATC2, EMP3, SNRNP70, TMED5, ST8SIA4, IGLV3-1, ZNF394, TNFSF9, CTSW, CUL1, BACH1, RABL3, KPNA2, EPS8L3, IER5, HSPA1B, CADM1, MCL1, RNF19A, ITGA4, CD38, WIPI1, CENPK, HCLS1, SPICE1, HIST1H2BC, MPRIP, FOSB, SERPINB8, FAM126A, CEP55, ATXN1, VCL, SOCS1, PCNX1, SQOR, JUNB, C10orf90, LCP1, STRADB, CREB3L2, GNG7, CCNH, SNX2, IGSF1, CCNL1, FKBP11, DBF4, ICAM1, MAD2L1, TMEM176B, PAIP2B, CD79A, SRXN1, NOB1, IER2, HLA-DRA, ZFP36L1, MZB1, MAGEA4, JUND, CD8B, AARS, TXNDC15, AC016831.7, GNA15, ATM, TSC22D1, GZMK, RAC3, ZNF263, TNFAIP3, H1FX, FGG, FHL2, MBNL1, TMEM205, IGLV6-57, CD96, TUBA1C, UCHL1, PRDM1, SRPK2, NUP37, TMEM87A, THEMIS2, HSPA5, PCMT1, TUBA1A, IGHG1, ANKRD37, MEF2C, XRN1, POU2AF1, BCL6, INAFM1, ADH4, TGFB1I1, PBK, DCN, FCRL5, DNAJB4, HLA-DQA1, TBC1D23, TMEM39A, GCC2, TMEM192, IGHA1, PTHLH, MFAP5, GEMIN6, BIRC3, IGHV4-4, SLC6A6, CYP2R1, HLA-DRB1, PPP1R15B, HMCES, MYC, WISP2, CHN1, ILK, PXN-AS1, LINC01970, CRIP2, PCOLCE2, MTMR6, EDIL3, AGR2, MEF2B, PFKM, KIAA1671, GLIPR2, SSTR2, SERPINB9, HIST1H1E, PTTG1, WSB1, ERN1, Z93241.1, IGLV1-44, SDS, TLE1, NUPR1, IGLV1-47, ICAM2, NXF1, RSPO3, TCF4, AC243960.1, RARRES2, RMDN3, RBFOX2, SEC11C, OLMALINC, FADS2, ITPRIP, FOS, SFTPD, HAUS3, RNF43, HIST1H4C, TIGAR, BIK, ITGA1, TARSL2, AFP, SNORC, MKLN1, BTG2, KRT18, NOC2L, ZFP36L2, NFKBIA, RHOB, HMGA1, BRD3, IGHJ6, U62317.5, SLC2A3, AC034231.1, CLEC11A, EPCAM, SKI, PNOC, MIR155HG, C12orf75, SAMHD1, IGKV3D-15, ACTN1, GSTZ1, TUBB3, CAV1, OAT, COBLL1, SSR4, ACTA2, HBA1, FAM83D, PLA2G2A, RAB14, AC106791.1, RAB23, AC244090.1, KMT5A, SERPINB1, P3H2, XRCC1, AC106782.1, MAL2, EGR1, F8, PLIN2, SOWAHC, IGFBP6, NFKBIZ, XBP1, SLC25A51, IGHM, KCTD5, USP38, FCER1G, PHLDA1, BYSL, HLA-DRB5, RAPH1, DUSP23, FUOM, ISYNA1, TNK2, STAP2, SLC25A4, GALNT2, SGO2, FHL3, ALB, CYP20A1, TM4SF1, ADA, RRP9, DNAH14, BOLA2, BHLHE41, CCL20, AC005537.1, UBALD2, VGLL4, NUDT1, USP10, ADSSL1, PRSS23, FMC1, ARHGAP45, HSPA14-1, CREB5, RBM33, TMX4, ROCK2, ARSK, PALLD, FNDC3B, FOXA3, BATF, PTP4A3, CDC45, IGHV1-2, IMMP2L, STARD10, HIST2H2BF, MTG2, FBXO8, USP32, ADIPOR2, RRM2, DHODH, DDIT4, NFAT5, PPARG, YTHDF3-AS1, GNG4, CSPP1, UBE2S, ZNF473, TIMP1, CPQ, AOC2, H1F0, JRK, EXOSC9, AC012236.1, AC009403.1, C12orf65, AURKA, MYH9, IGKV4-1, IGHMBP2, JADE1, HIST1H3C, TTC39A, SGMS1, LBP, FRYL, DNAJB2, GNG11, HAGHL, ANXA6, MARS, ADD1, KDM4B, TMEM91, AC008915.2, CXCL14, DUSP14, GJB2, PGM1, ETS2, GNPDA1, COL18A1, KLF10, MT1A, TPX2, S100A2, MAP3K5, HIST1H2AE, SLC20A2, ITGB7, SCEL, RSRP1, AKR1B1, GINS1, ZNF296, ALKBH4, UBE2C, ANKRD36C, SULT2B1, SMC5, TSPYL2, TNS4, TIMP3, ID4, SDC1, COX18, CDC42EP2, SQLE, ZNRF1, AKR1B10, NDC80, GFPT2, MAP1B, HIST1H2AG, IDO1, RNF185, UHRF1BP1, ADORA2B, CALD1, PHLDA2, ADH6, TFAP2A, DLG1, MELK, CBWD3, RAB4B, KANSL1L, RCE1, HIST1H2AC, CDK1, TCIM, C17orf67, BRD4, LY6E-DT, SLC1A6, ARL13B, IRF1, DDX3X, RAB2B, MYBBP1A, ARFGAP1, BOP1, IGKV3D-7, KMT2E-AS1, DTNBP1, LAMC2, ATG4C, MYBL2, LRP10, PALMD, ZBTB4, SYTL2, SERPINH1, CD248, CNEP1R1, FURIN, IGLL5, MEST, MDK, NUP205, NRDE2, ECT2, TENT5A, TNKS1BP1, NFXL1, SLC35E3, ECE1, RASD1, SLC52A2, DCBLD2, CP, POLE, COL27A1, SBNO1, SLC7A6, HYKK, SLPI, CFHR1, SPDEF, DACT2, TUBGCP5, AREG, HIST1H2AJ, KIF2A, AL135925.1, NOTCH3, SLC11A1, HEXIM2, IGFBP1, TVP23A, NUDT14, SAMD11, MIR200CHG, PCLAF, SLC43A3, FAM30A, PHRF1, ADM, SIK2, NUSAP1, CFH, KRTCAP3, SPAG4, TPPP3, TSPAN4, AAK1, CST1, CLU, IFRD1, ASPHD2, CNN3, COL4A1, FGA, ANO6, SBSN, FGB, ATP9B, NLGN4Y, HP, EPS8, RNF111, LINC01285, MAOA, IGHV4-31, TNFRSF10D, GSR, IGHGP, TACSTD2, MT1F, RHCG, MUT, PI3, MT1M, LAMB3, MTRNR2L12, SLC35A2, DDX10, RARRES1, MTSS1, CLK2, RPN2, MED29, CYP1B1, TTTY14, DMXL1, AL139246.5, TAF1, DAAM1, MYO1E, MAFB, CDKN1A, F8A3, FABP5, CFB, HSP90B1, SGK3, HMG20B, CDCA5, CLDN4, SYNM, PAWR, TWNK, AC116049.2, RND3, ATP11A, PID1, MALAT1, TMEM168, TFF1, TFRC, RNASET2, SPINK13, PABPC1L, P4HA2, PRSS8, SPINT1, MSC, FMNL1, SLC8B1, UNC13D, SPINT2, DCP1A, NPTN, IGKV3D-11, G6PD, KRT6A, LYPD1, TESC, COL4A2, ELF3, BCAM, AC093323.1, IGHV1-69, LINC00511, PORCN, TPRG1-AS1, EFNB2, PARD6G-AS1, CD9, RGS16, IL6R, FZD3, GLYR1, B3GALT6, LRCH3, MAFK, LINC00491, MT1X, MUC6, PIK3R3, GBP4, PERP, LXN, ZBTB7A, WARS, AC020911.2, MAPK3, ALS2CL, MRE11, TSPAN17, IGHV4-34, IL33, ADAM9, ANGPTL4, TBC1D31, C1R, CTSC, SLC35A4, FST, SGO1, ANKRD36, IGHG3, SLC15A3, HES1, POLR1E, SLC7A5, CAPN12, IGFBP3, FBXO38, FLNA, CSKMT, OAS1, ULK1, PBX1, EXOC4, REEP6, HILPDA, ASF1B, FKBP1B, IL6, CALU, AKR1C1, KLF2, GRTP1, C1S, SMOX, CPLX2, LMNA, BSG, IGHG4, SVIL, HIST1H1B, GCH1, NEAT1, FN1, ESRP1, RFWD3, ADGRE2, SPINK6, HPD, CAVIN1, MT1E, CLDN10, C15orf48, CA9, NR4A1, PPP1R3B, SLC30A1, SLC7A11, VIRMA, NAA25, CCNB1, CFD, AP1G1, H6PD, PSCA, KCNK6, AL161431.1, DVL1, HIST1H2AM, RAB31, CDCA3, SPATA20, PRMT7, PTGR1, SERINC2, IGHG2, GFPT1, TTC22, BTBD1, HIST1H4H, CENPB, ZNF598, GPATCH2L, SPTLC3, CXCL2, CYP24A1, EZH2, GPX2, LMNB2, PTGES, MGLL, NR2F2, KRT19, DNTTIP1, MUC5AC, SDCBP2, IL1R2, AHNAK2, MUC16, AC023090.1, CPE, VNN1, BAMBI, NPW, TK1, IGKV3D-20, ANKRD11, CDC20, CDH1, STK11, IGKC, SLC45A4, TBC1D8, CSTA, AC233755.1, MIGA1, HIST1H2AL, AKAP12, MAP4K4, HOOK2, GGA3, COL7A1, NOS1, ARHGAP26, AKR1C2, TGM2, CENPF, IGHV3-48, CDCA8, TSC2, STC2, PKN3, PVR, CES1, GPRC5A, SEZ6L2, CEP170B, KIF14, IER3, ALDH3B1, TOP2A, SPP1, TXNRD1, LENG8, TRIM15, ALDH3A1, RIMKLB, HECTD4, SMOC1, NEB, RMRP, IGFBP4, MT1G, SCRIB, ERO1A, SOX4, LMO7, RNPEPL1, PLK2, COL6A2, FLRT3, IGHV4-28, SCD, KRT7, PIEZO1, CXCL1, DAPK1, ID1, C3, CXCL3, IGKV3-20, GUCA2B, ITGA3, SFN, IGLV3-21, PLEC, POLR2A, AGRN, MUC1, SERPINB3, S100A8, LAMA5, COL6A1, ITGB4, S100P, SLURP2, MSLN, KRT17, and MUC5B.

7. The method of any one of the preceding claims, wherein the gMDSC signature comprises expression values for one or more genes selected from:

    (a) SERPINB1, SOD2, S100A8, CTSC, CCL18, CXCL2, PLAUR, NCF2, FPR1, and IL8; or
    (b) SERPINB1, SOD2, S100A8, CTSC, CCL18, CXCL2, PLAUR, NCF2, FPR1, IL8, S100A9, TNFAIP3, CXCL1, BCL2A1, EMR2, LILRB3, SLC11A1, IL6, TREM1, CCL20, LYN, CXCL3, IL1B, IL1R2, AQP9, IL2RA, GPR97, OSM, CXCR1, FPR2, C19orf59, CXCR2, CXCL6, CXCL5, EMR3, MEFV, S100A12, CD300E, FCGR3B, PPBP, LILRA5, LILRA3, and CASP5.

8. The method of any one of the preceding claims, wherein the immune oncology (IO) signature comprises expression values for one or more genes selected from GBP5, IL10RA, NLRC5, CXCL9, RAC2, GBP4, GLUL, IRF1, CD53, CIITA, S100B, GBP2, ITK, SLAMF7, IKZF3, DOCK2, SELL, ARHGAP9, CYTIP, IL2RB, NCKAP1L, APOD, CD96, IL7R, and ZAP70.

9. The method of any one of the preceding claims, wherein the immune oncology (IO) signature comprises expression values for one or more genes selected from ISG20, PCDHGA2, TGFB1I1, ATP8B1, IL7R, IRF8, ETV1, MYLK, GRHL2, THBS4, CYP3A5, FBLIM1, S100B, BICD1, SLAMF7, RAB27A, GATM, ICA1, ITPR1, SLC7A2, ZAP70, LOXL4, CILP, ARHGAP30, ITGB2, KLF5, PRKCA, PCDH7, DPYSL3, RGS2, SPP1, COLGALT2, MPZL2, TNFAIP8, PLAT, ALDH1A3, POF1B, PPP1R9A, SEMA3A, CIITA, DLC1, ARHGAP9, FRAS1, AKAP6, ATP1A2, TTN, LTBP1, NCKAP1L, MAP3K6, MYO1B, MRVI1, FSCN1, GPC1, GBP5, BAMBI, IL2RB, MYO1G, RANBP17, APOD, RASGRP1, CYTIP, ITGA7, CYTH4, PTPRF, KIAA1755, IRF1, GPR37, RAC2, NLRC5, EGFR, ITK, IL10RA, IGFBP2, CD96, RASD1, CD36, TMEM163, IGLL5, IKZF3, PRLR, CDC42BPG, DOCK2, PAM, VEGFA, CD84, SORL1, GBP2, SYTL4, APBB1IP, SIGLEC10, GBP4, COMP, DOCK8, CXCL9, NRP1, EPHB4, CD53, GLUL, DNM1, DSP, SIX4, SELL, DSC3, TNFAIP2, and JAG2.

10. The method of any one of the preceding claims, wherein the TMB is derived from the DNA sequencing data.

11. The method of any one of the preceding claims, wherein the expression values of the checkpoint related gene signature, the immune exhaustion signature, and the granulocytic myeloid derived suppressor cell (gMDSC) signature are derived from the RNA seq data.

12. The method of any one of the preceding claims, wherein the IO therapy is an immune checkpoint inhibitor therapy (ICI), wherein optionally the ICI comprises pembrolizumab or nivolumab.

13. The method of any one of the preceding claims, wherein the report further comprises an immune profile score (IPS).

14. The method of claim 13, wherein the IPS is displayed as an integer from 1-100.

15. The method of claim 13, wherein the IPS is further divided into categories or is a categorical result, wherein optionally the categories are IPS-Low, indeterminate, and IPS-High.

# Predicted OS for ICI monotherapy in LOT1

FIG. 1A

EP 4 550 337 A1

EP 4 550 337 A1

## Validation summary

A prospective-retrospective validation in ~1,500 patients demonstrated **significantly longer OS in IPS-High patients**

- Pan-cancer
- Metastatic solid tumors
- Treated with ICI-containing regimen in 1L or 2L
- Cancer types with ICI approvals

1. OS curves are predicted from the stratified Cox model fitted. Separate curves generated for each combination of LOT and treatment group (ICI-mono vs. ICI+additional). The results are similar across combinations. For this curve

**Predicted OS for ICI monotherapy in LOT1[1]**

IPS-High · IPS-Low

✓ **Strong stratification of OS (HR=0.45 [0.41-0.57]) in the overall cohort**

✓ **Outperforms existing biomarkers TMB, PD-L1, MSI**
*See next slides*

✓ **Exploratory predictive evidence**
*Showing IPS predicts outcomes following ICI but not chemo – see next slides*

FIG. 1B

## Tempus IPS AI-Algorithm

Prototype representation

IPS is a DNA and RNA-based molecular signature that demonstrates **prognostic utility in an overall pan-cancer cohort** of metastatic solid organ cancer patients receiving ICI-based regimens.

It provides an **"Immune Profile Score" (IPS)** from 0 to 100 and classifies patients into 2 groups[*]:

IPS-Low    vs.    IPS-High

IPS-High demonstrates **longer rwOS in patients** receiving ICI-based regimens compared to IPS-Low patients.

The validation dataset was derived from Tempus' real-world database of de-identified molecular and clinical data.

Available today for research use only (RUO) and will be available for clinical use in the future. (This presentation does not describe clinical uses of IPS, which may differ from RUO uses.)

*Note: Some patients will have indeterminate results. This group was excluded from the validation study due to analytical precision concerns, and we cannot draw any conclusions about their outcomes.

## FIG. 2

EP 4 550 337 A1

**Predicted OS by IPS and PD-L1**

Predicted for 1L; ICI+additional treatment

FIG. 3

**NSCLC only | Predicted OS by IPS and PD-L1**

Predicted for 1L; ICI+additional treatment

Legend:
- IPS-High; PDL1-High
- IPS-High; PDL1-Low
- IPS-High; PDL1-Negative
- IPS-Low; PDL1-High
- IPS-Low; PDL1-Low
- IPS-Low; PDL1-Negative

**Median OS (95%CI)**
IPS-High & PDL1-High
IPS-High & PDL1-Low
IPS-High & PDL1-Neg
IPS-Low & PDL1-High
IPS-Low & PDL1-Low
IPS-Low & PDL1-Neg

FIG. 4

Predicted OS by IPS and MSI
Predicted for 1L; ICI monotherapy

FIG. 5

# Validation Highlights

## Exploratory predictive analysis – statistical method

A comparison of the HR from the 2 time periods provides an evaluation of the predictive utility of IPS. All patients received:

1. **1L chemotherapy (CT)** – Measured time-to-next-treatment (TTNT)
2. **2L ICI** – Measured OS from ICI initiation

*Tomlins, Scott A., et al. "Development and validation of an integrative pan-solid tumor predictor of PD-1/PD-L1 blockade benefit." *Communications medicine* 3.1 (2023): 14.
*Bulen, Benjamin J., et al. "Validation of Immunotherapy Response Score as predictive of pan-solid tumor anti-PD-1/PD-L1 benefit." *Cancer Research Communications* 3.7 (2023): 1335-1349.

FIG. 6

**Time period 1: CT in 1L**
Evaluating time-to-next-treatment (TTNT)

IPS-High — IPS-Low

HR (95% CI): 1.06 (0.85, 1.33)
Median OS (95%CI)
IPS-Low : 3.71 (3.35, 4.11)
IPS-High : 3.58 (3.19, 4.04)

Time to Next Treatment, probability

Time in months from initiation of 1L CT

**Time period 2: ICI in 2L**
Evaluating OS

IPS-High — IPS-Low

HR (95% CI): 0.63 (0.46, 0.86)
Median OS (95%CI)
IPS-Low : 12.5 (9.84, 15.9)
IPS-High : 20.4 (15.25, NA)

Overall Survival

Time in months from initiation of 2L IO

FIG. 7

# Inclusion/Exclusion Criteria

| Inclusion | |
|---|---|
| Age ≥18 at mdx | IO start ≤ 60d of LOT start, and ≤ 180d for maintenance therapy indications (Urothelial cancers) |
| **De novo Stage IV or metastatic at sample collection** | At least YYYY-MM date grain for key dates (mdx, IO start, sequencing and LKD) |
| **IO treatment in 1L or 2L** | |
| **IO in 1L:**<br>● mdx < 7/1/2023<br><br>● Sample date ≤ 90d of LOT1 initiation, and ≤ 240d for HNSCC , RCC and Cervical<br><br>● mdx ≤ 90d of LOT1 initiation, and ≤ 240d for HNSCC, RCC and Cervical | **IO in 2L:**<br>● LOT2 initiation < 1/1/2024<br><br>● If LOT1 initiation date <sample date < LOT2 initiation date:<br>  ○ LOT2 initiation ≤ 90d of sample date<br><br>● If sample date < LOT1 initiation date:<br>  ○ LOT2 initiation ≤ 545d of sample date for all cancers |

FIG. 8A

EP 4 550 337 A1

## Inclusion/Exclusion Criteria

| Exclusion | |
|---|---|
| **Prior IO treatment** | Received an investigational new drug |
| Unknown TMB | **Delayed entry >1 year from IO initiation** |
| Known ECOG score ≥ 3 | Primary dx > 60d of mdx |
| **Tumor purity < 30%** | **Samples not collected by 'bone marrow core biopsy', 'venipuncture', 'fine needle aspirate', 'fluid aspirate'** |
| **Samples collected from lymph node** | |

FIG. 8B

# Exploratory Endpoint - Prognostic utility of IPS score over TMB

| Variable | HR (90% CI) |
|---|---|
| **Analysis without IPS score** | |
| TMB (low as ref) | 0.60 (0.52, 0.70) |
| **Analysis with IPS score** | |
| TMB (low as ref) | 0.75 (0.64, 0.87) |
| IPS (low as ref) | 0.49 (0.42, 0.56) |

**Likelihood ratio test:**
Test statistic value: 75.375 (chi-square test with df=1)
p-value < 0.001

**Conclusion:**

- IPS has **significant** prognostic utility beyond TMB in the tested patient population

- TMB is still significant in a multivariable model with IPS, but with attenuation in the HR

FIG. 9

# Exploratory Endpoint - Prognostic utility of IPS score over PD-L1 in the tested patient population

| Variable | HR (90% CI) |
|---|---|
| **Analysis without IPS score** | |
| PD-L1 (negative as ref) | 0.86 (0.75, 1.00) |
| **Analysis with IPS score** | |
| PD-L1 (negative as ref) | 0.94 (0.81, 1.09) |
| IPS (low as ref) | 0.45 (0.38, 0.53) |

**Likelihood ratio test:**
Test statistic value: 71.514 (chi-square test with df=1)
p-value < 0.001

**Conclusion:**

- IPS has significant prognostic utility beyond PD-L1

- PD-L1 is not significant in a multivariable model with IPS

FIG. 10

EP 4 550 337 A1

# Validation highlights

## TMB subgroups | ICI + additional treatment

### Predicted OS for LOT1

FIG. 11A

FIG. 11B

FIG. 12

# Statistical methods for IPS predictive utility

- The prognostic utility of IPS score has been established through our validation study

- An exploratory analysis was conducted to demonstrate the predictive utility of IPS

- A comparison of the HR from the 2 time periods provides an evaluation of the predictive utility of IPS

*Tomlins, Scott A., et al. "Development and validation of an integrative pan-solid tumor predictor of PD-1/PD-L1 blockade benefit." *Communications medicine* 3.1 (2023): 14.
*Bulen, Benjamin J., et al. "Validation of Immunotherapy Response Score as predictive of pan-solid tumor anti-PD-1/PD-L1 benefit." *Cancer Research Communications* 3.7 (2023): 1335-1349.

FIG. 13

# IPS has significant prognostic utility beyond TMB and PDL1

| Biomarker | HR (90% CI) | |
|---|---|---|
| IPS High vs Low (Ref.) | 0.45 (0.39 - 0.52) | |
| TMB High vs Low (Ref.) | 0.60 (0.52 - 0.70) | |
| PD-L1 Positive vs Negative (Ref.) | 0.86 (0.75 - 1.00) | |

0          0.5          1          1.5

| Variable | HR (90% CI) |
|---|---|
| **Analysis without IPS score** | |
| PD-L1 (negative as ref) | 0.86 (0.75, 1.00) |
| **Analysis with IPS score** | |
| PD-L1 (negative as ref) | 0.94 (0.81, 1.09) |
| IPS (low as ref) | 0.45 (0.38, 0.53) |

**Likelihood ratio test:**
Test statistic value: 71.514 (chi-square test with df=1), p-value < 0.001

| Variable | HR (90% CI) |
|---|---|
| **Analysis without IPS score** | |
| TMB (low as ref) | 0.60 (0.52, 0.70) |
| **Analysis with IPS score** | |
| TMB (low as ref) | 0.75 (0.64, 0.87) |
| IPS (low as ref) | 0.49 (0.42, 0.56) |

**Likelihood ratio test:**
Test statistic value: 75.375 (chi-square test with df=1), p-value < 0.001

FIG. 14

EP 4 550 337 A1

# Model features

| Type | Feature | Description |
|---|---|---|
| **DNA** | TMB | Binarized TMB |
| **Single-gene RNA** | cd274 | PDL1 gene |
| | SPP1 | T cell activation |
| | CXCL9 | Cytotoxic T cell chemoattractant |
| | CD74 | Antigen presentation regulator |
| | CD276 | Checkpoint gene involved in anti-tumor T cell immune response |
| | IDO1 | Immune modulator |
| | PDCD1LG2 | PDL1 paralog |
| | TNFRSF5 | Critical gene in inflammatory response |
| **RNA signature** | Tempus scRNAseq - immune exhaustion | Signature derived from single-cell RNAseq |
| | Meta-analysis literature signature | Literature based, meta-analysis-derived custom signature |
| | gMDSC | 43-gene granulocytic MDSCs signature |

FIG. 15

FIG. 16

| Patient Name | Diagnosis<br>Non-Small Cell Lung Cancer | Accession No. | IPS |
|---|---|---|---|

Date of birth
**09/18/1949**

Sex
**Female**

Physician
**Thomas DeFausseOnco**

Institution
**Test Data Institution**

**Tempus IPS**
IMMUNE PROFILE SCORE

Tumor specimen
Lung

Test Data Management
Pathology Lab

Collected
01/02/2021

Received
05/04/2021

Tumor percentage
50%

**Notes**

The patient does not currently qualify for any clinical trials in our database.

## IMMUNE PROFILE SCORE

**Group**

IPS-High

**Score**

**72**

0      45 47      100

IPS-Low    Indeterminate    IPS-High

The Immune Profile Score for this sample is **High**. IPS-High patients had a significantly **longer overall survival** than IPS-Low patients in a real-world study of metastatic solid tumor patients treated with ICI-containing regimens (further details below).

## INTERPRETATION OF RESULTS

IPS uses DNA data from Tempus xT and RNA data from Tempus xR to calculate an **IPS score**, which ranges from 0 to 100, and classifies patients as either **IPS-Low** (scores 0-44) or **IPS-High** (scores 48-100). Scores between 45-47 are classified as Indeterminate[1].

In a prospectively designed, retrospective real-world study of 1,519 patients with metastatic solid organ tumors treated with regimens containing immune checkpoint inhibitors (ICIs), **IPS-High patients demonstrated significantly longer overall survival (OS)** compared to IPS-low patients (HR=0.45, 90% CI=[0.40-0.52] p<0.001) (Figure 1 & 2). The continuous IPS score was also significantly associated with OS (HR=0.29 for a 50-point increase, 90% CI=[0.24-0.35]).

In the same study, **IPS also demonstrated prognostic utility independent of TMB, PD-L1 IHC, and MSI**

- IPS-High patients had longer OS than IPS-Low patients with HR≤0.50 in subgroups of patients that were TMB-high, TMB-low, PDL1-high, PDL1-low, and MSS (Figure 1 & 2)
- IPS remained significant for OS in separate multivariable models controlling for TMB, MSI, and PD-L1, with HRs of 0.49 [0.42-0.56] p<0.001, 0.47 [0.41-0.53] p<0.001, and 0.45 [0.38-0.53] p<0.001 respectively

IPS is intended for use in patients with metastatic solid organ tumors that are considered candidates for ICI therapy.

FIG. 17

| | | | |
|---|---|---|---|
| **Patient Name** | Diagnosis<br>**Non-Small Cell Lung Cancer** | Accession No. | **IPS** |

Date of birth
**09/18/1949**

Sex
**Female**

Physician
**Thomas DeFausseOnco**

Institution
**Test Data Institution**

**Tempus IPS**
**IMMUNE PROFILE SCORE**

Tumor specimen
Lung

Test Data Management
Pathology Lab

Collected
01/02/2021

Received
05/04/2021

Tumor percentage
50%

**Notes**

The patient does not currently
qualify for any clinical trials in
our database.

## IMMUNE PROFILE SCORE

| Group | Score |
|---|---|
| IPS-Low | 40 |

0         4547        100

IPS-Low     Indeterminate     IPS-High

The Immune Profile Score for this sample is **Low**. IPS-Low patients had a significantly **shorter overall survival** than IPS-High patients in a real-world study of metastatic solid tumor patients treated with ICI-containing regimens (further details below).

## INTERPRETATION OF RESULTS

IPS uses DNA data from Tempus xT and RNA data from Tempus xR to calculate an **IPS score**, which ranges from 0 to 100, and classifies patients as either **IPS-Low** (scores 0-44) or **IPS-High** (scores 48-100). Scores between 45-47 are classified as Indeterminate[1].

In a prospectively designed, retrospective real-world study of 1,519 patients with metastatic solid organ tumors treated with regimens containing immune checkpoint inhibitors (ICIs), **IPS-High patients demonstrated significantly longer overall survival (OS)** compared to IPS-low patients (HR=0.45, 90% CI=[0.40-0.52] p<0.001) (Figure 1 & 2). The continuous IPS score was also significantly associated with OS (HR=0.29 for a 50-point increase, 90% CI=[0.24-0.35]).

In the same study, **IPS also demonstrated prognostic utility independent of TMB, PD-L1 IHC, and MSI**

- IPS-High patients had longer OS than IPS-Low patients with HR≤0.50 in subgroups of patients that were TMB-high, TMB-low, PDL1-high, PDL1-low, and MSS (Figure 1 & 2)
- IPS remained significant for OS in separate multivariable models controlling for TMB, MSI, and PD-L1, with HRs of 0.49 [0.42-0.56] p<0.001, 0.47 [0.41-0.53] p<0.001, and 0.45 [0.38-0.53] p<0.001 respectively

IPS is intended for use in patients with metastatic solid organ tumors that are considered candidates for ICI therapy.

## FIG. 18

# IPS-High and IPS-Low P2 (content is the same for both reports)

DETAILED INTERPRETATION OF RESULTS                                    Patient Name |

Figure 1. HR (IPS-High vs. IPS-Low) in clinically relevant biomarker subgroups.

| SUBGROUP | NO. OF PATIENTS | HR (90% CI) | SUBGROUP FOREST PLOT |
|----------|-----------------|-------------|----------------------|
| All Patients | | 0.45 (0.40, 0.52) | |
| PDL1 Pos | | 0.45 (0.37, 0.56) | |
| PDL1 Neg | | 0.43 (0.33, 0.56) | |
| TMB High | | 0.50 (0.38, 0.65) | |
| TMB Low | | 0.48 (0.41, 0.57) | |
| MSI High | | 0.58 (0.29, 1.18) | |
| MSI Stable | | 0.46 (0.40, 0.53) | |

IPS-High better    IPS-Low better

Figure 2. Median overall survival (mOS).

| First line of therapy | | Second line of therapy | |
|----------|----------|----------|----------|
| IPS-High >24 month | IPS-Low 12.2 month | IPS-High 22.1 month | IPS-Low 10.2 month |

Note: 12 mo survival for each line of therapy is estimated using the mode of regimen for 1L1 (ICI+additional) and the mode of regimen for 2L (ICI Only).

FIG. 19

IPS-High and IPS-Low P3 (content is the same for both reports)

Figure 3. HR (IPS-High vs. IPS-Low) for cancer subgroups that included more than 30 patients.

| CANCER TYPE | NO. OF PATIENTS | HR (90% CI) | SUBGROUP FOREST PLOT |
|---|---|---|---|
| RCC | | 0.34 (0.20, 0.59) | |
| HNSCC | | 0.38 (0.22, 0.67) | |
| NSCLC | | 0.42 (0.34, 0.52) | |
| Melanoma | | 0.47 (0.27, 0.82) | |
| Urothelial | | 0.55 (0.32, 0.92) | |
| Hepatocellular | | 0.70 (0.36, 1.36) | |
| Breast | | 0.75 (0.42, 1.33) | |
| Gastroesphageal | | 0.86 (0.54, 1.38) | |
| CRC | | 0.92 (0.54, 1.38) | |

IPS-High better     IPS-Low better

## ASSAY DESCRIPTION

The Tempus Immune Profile Score (IPS) uses DNA data from Tempus xT and RNA data from Tempus xR to calculate an IPS score from 0 to 100, and classify patients as either IPS-Low (score 0-44) or IPS-High (48-100). Scores between 45-47 are classified as Indeterminate[1].

The IPS score is calculated using a linear combination of features that include DNA data for TMB and RNA data for the following set of single genes or multi-gene signatures: CD274, SPP1, CXCL9, CD74, CD40, CD276, IDO1, PDCD1LG2, a 43-gene gMDSC signature[2], an 768-gene Tempus immune resistance signature developed from scRNAseq data and a 105-gene Tempus literature-based meta-analysis signature.

## TEMPUS DISCLAIMER

The Tempus IPS test uses information from analysis of nucleic acids by next-generation sequencing (NGS). That analysis, and the resulting information, can be affected by multiple factors including formalin-fixation degrading DNA or RNA quality, and low tumor purity limiting sensitivity of the assay. The Tempus IPS test is reported only for cases with greater than or equal to 30% tumor purity and is not reported for lymph node samples or cytology fluid samples.

<div align="center">

# FIG. 20

</div>

Indeterminate p1 of 1

**Patient Name**

Diagnosis
Non-Small Cell Lung Cancer

Accession No

**IPS**

Date of birth
**09/18/1949**

Sex
**Female**

Physician
**Thomas DeFausseOnco**

Institution
**Test Data Institution**

**Tempus IPS**
**IMMUNE PROFILE SCORE**

Tumor specimen
Lung

Test Data Management
Pathology Lab

Collected
01/02/2021

Received
05/04/2021

Tumor percentage
50%

**Notes**

The patient does not currently
qualify for any clinical trials in
our database.

**IMMUNE PROFILE SCORE**

Group              Score

[Indeterminate]    46

0                          45 47                          100

IPS-Low      Indeterminate      IPS-High

The Immune Profile category for this patient is in the indeterminate range.

**INTERPRETATION OF RESULTS**

IPS uses DNA data from Tempus xT and RNA data from Tempus xR to calculate an **IPS score**, which ranges from 0 to 100, and classifies patients as either **IPS-Low** (scores 0-44) or **IPS-High** (scores 48-100). Scores between 45-47 are classified as Indeterminate[1].

The Indeterminate range of 45-47 is within statistical margins of error between IPS-High and IPS-Low; therefore the sample cannot be reliably classified as IPS-High or IPS-Low. An indeterminate result is not due to poor sample quality or technical difficulties. Indeterminate results are expected to occur in about 5% of relevant patients.

**ASSAY DESCRIPTION**

The Tempus Immune Profile Score (IPS) uses DNA data from Tempus xT and RNA data from Tempus xR to calculate an IPS score from 0 to 100, and classify patients as either IPS-Low (score 0-44) or IPS-High (48-100). Scores between 45-47 are classified as Indeterminate[1].

The IPS score is calculated using a linear combination of features that include DNA data for TMB and RNA data for the following set of single genes or multi-gene signatures: CD274, SPP1, CXCL9, CD74, CD40, CD276, IDO1, PDCD1LG2, a 43-gene gMDSC signature[1], an 768-gene Tempus immune resistance signature developed from scRNAseq data, and a 105-gene Tempus literature-based meta-analysis signature.

**TEMPUS DISCLAIMER**

The Tempus IPS test uses information from analysis of nucleic acids by next-generation sequencing (NGS). That analysis, and the resulting information, can be affected by multiple factors including formalin-fixation degrading DNA or RNA quality, and low tumor purity limiting sensitivity of the assay. The Tempus IPS test is reported only for cases with greater than or equal to 30% tumor purity and is not reported for lymph node samples or cytology fluid samples.

1. *The exact threshold between IPS-Low and Indeterminate is 44.95. The exact threshold between Indeterminate and IPS-High is 47.40. Scores below 44.95 are rounded to 44, scores above 47.40 are rounded to 48. Thresholds were selected based on the 55th and 60th percentile of patients, according to IPS score, in a pan-cancer training cohort of real-world patients treated with ICI.*

**FIG. 21**

## 1 &3  Primary endpoint overall and subgroup analyses for PD-L1 & TMB

| | Subgroup | No. of Patients | HR (90% CI) | |
|---|---|---|---|---|
| 1 | All Patients | 1519 | 0.45 (0.40, 0.52) | |
| | PD-L1 | | | |
| 3 | Positive | 603 | 0.45 (0.37, 0.56) | |
| | Negative | 470 | 0.43 (0.33, 0.56) | |
| | TMB | | | |
| | High | 410 | 0.50 (0.38, 0.65) | |
| | Low | 1109 | 0.48 (0.41, 0.57) | |

IPS High Better    IPS Low Better

## 2.  Median rwOS - transformed into bar chart; adding in N

| | IPS-H | IPS-L |
|---|---|---|
| LOT1: mOS, 12 mo survival | >24 mo, 73.4% | 12.2 mo, 50.7% |
| LOT2: mOS, 12 mo survival | 22.1 mo, 70.1% | 10.2 mo, 45.8% |

Note: 12 mo survival for each LOT is estimated using the mode of regimen for LOT1 (ICI+additional) and the mode of regimen for LOT2 (ICI Only)

## 3.  Cancer subgroup results, subgroup >30

| Cancer Type | | | |
|---|---|---|---|
| RCC | 118 | 0.34 (0.20, 0.59) | |
| HNSCC | 121 | 0.38 (0.22, 0.67) | |
| NSCLC | 615 | 0.42 (0.34, 0.52) | |
| Melanoma | 98 | 0.47 (0.27, 0.82) | |
| Urothelial | 131 | 0.55 (0.32, 0.92) | |
| Hepatocellular | 38 | 0.70 (0.36, 1.36) | |
| Breast | 81 | 0.75 (0.42, 1.33) | |
| Gastroesophageal | 160 | 0.86 (0.54, 1.38) | |
| CRC | 45 | 0.92 (0.32, 2.68) | |

IPS High Better    IPS Low Better

# FIG. 22

Date of Birth
**09/18/1949**

Sex
**Female**

Physician
**Thomas DeFausseOnco**

Institution
**Test Data Institution**

**TEMPUS | IPS**
IMMUNE PROFILE SCORE

Tumor specimen:
Lung
Test Data Management Pathology Lab
Collected 1/2/2021
Received 5/4/2023
Tumor Percentage: 50%

**Notes**

The patient does not currently qualify for
any clinical trials in our database.

# IMMUNE PROFILE SCORE

## Group

**IPS-Ultra High**

## Score

**83**

0    25         70        100
Low     Moderate-   Ultra-High
           High

# INTERPRETATION OF RESULTS

FIG. 23

EP 4 550 337 A1

**1** Indicate responders to ICI <u>outside of</u> FDA-approved labels (e.g., label expansion)

CRC MSS   |   Pan-cancer last line (like TMB)   |   TNBC PDL1 low/negative   |
...

**2** Identify ICI non-responders who may benefit from novel combination therapies (e.g., PD-L1 + TIGIT)

Requires clinical trials with pharma

**3** Refine treatment decisions for patients <u>within</u> FDA-approved ICI labels (e.g., mono vs. combo)

NSCLC   |   Melanoma   |   HNSCC   |   ccRCC   |   Gastric/GEJ   |   ...

Initial validation enables doctors to use Algo with some confidence for these decisions, and more confidence in NSCLC adeno

FIG. 24

# EXAMPLE CLINICAL VALIDATION

In one embodiment, the initial clinical validation aims to use IPS to stratify real world overall survival (rwOS) in a pan-cancer cohort of Tempus patients treated with immune checkpoint inhibitor (ICI)-based regimens, and in relevant clinical subcohorts

## PRIMARY ENDPOINT

| Pan-cancer | Stratify OS in the pan-cancer cohort |
|---|---|
| | *([IPS-High] patients have longer rwOS compared to [IPS-Low])* |

⟩ Supports **generalizability** of IPS as informative prognostic biomarker

## SUBGROUP ANALYSES

| Pan-cancer | Stratify OS within PD-L1 IHC positive subcohort |
|---|---|
| | Stratify OS within PD-L1 IHC negative subcohort |
| | Stratify OS within TMB<10 subcohort |
| | Stratify OS within TMB≥10 (TMB high) subcohort |
| NSCLC | Stratify OS within NSCLC subcohort |
| | Stratify OS within NSCLC adeno PD-L1≥50% subcohort that received ICI monotherapy |

1. Enables **doctors to use IPS** to refine treatment decisions within IHC-directed FDA labels, with some confidence *(further studies required)*
2. Biopharma may request IPS reports for sub-indications supported by additional analyses tailored to the database research query *(e.g., microsatellite stable colorectal cancer (MSS CRC))*

Enables **doctors to use IPS** to refine decisions making such as ICI mono vs. ICI+chemo decision, with more confidence (for example, if patient isn't as likely to respond to IO, may opt to add chemo. If patient is more likely to respond to IO, may opt for ICI mono)

FIG. 25

# EXAMPLE PHARMA USE CASES
The IO Algo can find opportunities for **label expansion**

The IO Dashboard can identify **alternative treatment strategies** among predicted non-responders

**POPULATION OF INTEREST**
*E.g., mCRC MSS*

IPS
Predict responders using IPS

**PREDICTED RESPONDERS**

IPS
Deploy Algo in clinical trial

**LABEL EXPANSION**
Show that **pembro monotherapy** is superior to standard of care in IPS-Low

**PREDICTED NON-RESPONDERS**

Use dashboard to find alternative targets: **TIGIT**

IPS
Deploy Algo in clinical trial

**ALTERNATIVE TREATMENT STRATEGY**
Show that **pembro + anti-TIGIT** is superior to standard of care in IPS-Low

FIG. 26

Tempus patients that received
immunotherapy as 1L or 2L treatment

N=4503

**Excluded**

−Received prior ICI treatment
−Known ECOG PS ≥ 3 assessed at time of treatment
−Tumor purity < 30%
−Samples collected by "bone marrow core biopsy",
"venipuncture", "fine needle aspirate", "fluid aspirate"
−Samples collected from lymph node
−Samples reserved for training

**Included**

−Must have a diagnosis with an ICI approval
−At least 18 years old at stage IV diagnosis
−Stage IV disease at time of sample collection
−Sample collected prior to start of ICI
−Timing of pathologic diagnosis, sample collection
and start of treatment within expected ranges for
standard of care

Eligible Patients
N = 1600

FIG. 27

**DNA**
- TMB

**SINGLE-GENE RNA**
- CD274
- SPP1
- CXCL9
- TNFRSF5
- CD74
- CD276
- IDO1
- PDCD1LG2

**RNA SIGNATURES**
- Immune Exhaustion Signature
- gMDSC Signature
- Meta-Analysis Literature Signature

FIG. 28

EP 4 550 337 A1

FIG. 29A

A

Overall Survival

IPS-H, n = 160
IPS-L, n = 184

Time in months

FIG. 29B

B

Overall Survival

IPS-H, n = 55
IPS-L, n = 108

Time in months

|  | 1L mono | 2L mono | 1L combo | 2L combo |
|---|---|---|---|---|
| **IPS-H** | >24 (>24, >24) | 22.1 (18.3, >24) | >24 (23.0, >24) | 20.75 (16.95, >24) |
| **IPS-L** | 13.1 (10.8, 15.6) | 10.2 (8.56, 13.1) | 12.2 (10.8, 13.8) | 9.77 (8.23, 12.6) |

C

FIG. 29C

| Subgroup | No. of Patients | HR (90% CI) | |
|---|---|---|---|
| All Patients | 1519 | 0.45 (0.40, 0.52) | |
| **PD-L1** | | | |
| Positive | 603 | 0.45 (0.37, 0.56) | |
| Negative | 470 | 0.43 (0.33, 0.56) | |
| **TMB** | | | |
| High | 410 | 0.50 (0.38, 0.65) | |
| Low | 1109 | 0.48 (0.41, 0.57) | |
| **MSI** | | | |
| High | 76 | 0.58 (0.29, 1.18) | |
| Stable | 1440 | 0.46 (0.40, 0.53) | |
| **Additional** | | | |
| TMB-Low, ICI Only | 323 | 0.41 (0.30, 0.57) | |
| MSS, ICI Only, 1L | 309 | 0.33 (0.24, 0.45) | |
| **Age** | | | |
| >=65 | 708 | 0.45 (0.37, 0.55) | |
| <65 | 811 | 0.46 (0.38, 0.56) | |
| **Sex** | | | |
| Male | 907 | 0.45 (0.37, 0.53) | |
| Female | 612 | 0.47 (0.38, 0.59) | |
| **Regimen** | | | |
| ICI Only | 507 | 0.42 (0.33, 0.53) | |
| ICI + Other | 1012 | 0.48 (0.41, 0.57) | |
| **Brain Metastases** | | | |
| Not documented | 1269 | 0.44 (0.37, 0.51) | |
| Documented | 250 | 0.53 (0.39, 0.73) | |
| **Liver Metastases** | | | |
| Not documented | 1173 | 0.49 (0.42, 0.57) | |
| Documented | 346 | 0.41 (0.30, 0.56) | |
| **Cancer Type** | | | |
| RCC | 118 | 0.34 (0.20, 0.59) | |
| HNSCC | 121 | 0.38 (0.22, 0.67) | |
| NSCLC | 615 | 0.42 (0.34, 0.52) | |
| Melanoma | 98 | 0.47 (0.27, 0.82) | |
| Urothelial | 131 | 0.55 (0.32, 0.92) | |
| Hepatocellular | 38 | 0.70 (0.36, 1.36) | |
| Breast | 81 | 0.75 (0.42, 1.33) | |
| Gastroesophageal | 160 | 0.86 (0.54, 1.38) | |
| CRC | 45 | 0.92 (0.32, 2.68) | |

0   0.5   1.5

IPS High Better   IPS Low Better

FIG. 30

**A**

| Analysis | No. of Patients | HR (90% CI) |
|---|---|---|
| **TMB analysis without IPS** | 1519 | |
| TMB | | 0.60 (0.52, 0.70) |
| **TMB analysis with IPS** | 1519 | |
| TMB | | 0.75 (0.64, 0.87) |
| IPS | | 0.49 (0.42, 0.56) |
| **PDL1 analysis without IPS** | 1073 | |
| PDL1 | | 0.86 (0.75, 1.00) |
| **PDL1 analysis with IPS** | 1073 | |
| PDL1 | | 0.94 (0.81, 1.09) |
| IPS | | 0.45 (0.38, 0.53) |
| **MSI analysis without IPS** | 1516 | |
| MSI | | 0.42 (0.30, 0.60) |
| **MSI analysis with IPS** | 1516 | |
| MSI | | 0.48 (0.34, 0.69) |
| IPS | | 0.47 (0.41, 0.53) |

FIG. 31A

**B**

IPS–H; TMB–H, n = 89
IPS–H; TMB–L, n = 71
IPS–L; TMB–H, n = 40
IPS–L; TMB–L, n = 144

Overall Survival

Time in months

FIG. 31B

FIG. 31C

FIG. 31D

FIG. 31E

EP 4 550 337 A1

**F**

| Comparisons | HR |
|---|---|
| B. IPS-H/TMB-L to IPS-L/TMB-L | 0.49 (0.41, 0.58) |
| C. IPS-H/MSS to IPS-L/MSS | 0.41 (0.32, 0.53) |
| D. IPS-H/PDL1- to IPS-L/PDL1- | 0.43 (0.33, 0.56) |
| E. IPS-H/PDL1- to IPS-L/PDL1- (within NSCLC) | 0.43 (0.30, 0.63) |

FIG. 31F

FIG. 32

FIG. 33

100

Communication Network 102

Computing Device 104

Hardware processor(s) 106
Communication system(s) 112
Display(s) 108
Memory 114
Input(s) 110

Server 116

Hardware processor(s) 118
Communication system(s) 124
Display(s) 120
Memory 126
Input(s) 122

FIG. 34

| | Europäisches Patentamt European Patent Office Office européen des brevets | **EUROPEAN SEARCH REPORT** | **Application Number** EP 24 20 9902 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/154284 A1 (LAU DENISE [US] ET AL) 19 May 2022 (2022-05-19) | 1-12 | INV. G16B40/20 |
| Y | * abstract * * paragraphs [0010], [0066], [0112], [0144], [0190], [0334], [0371], [0372]; claims 1-5,20 * | 13-15 | |
| Y | KRATZ JOHANNES R. ET AL: "Genetic and immunologic features of recurrent stage I lung adenocarcinoma", SCIENTIFIC REPORTS, vol. 11, no. 1, 8 December 2021 (2021-12-08), XP093253862, US ISSN: 2045-2322, DOI: 10.1038/s41598-021-02946-0 Retrieved from the Internet: URL:https://www.nature.com/articles/s41598-021-02946-0> | 13-15 | |
| A | * abstract * * page 14, paragraph 2 * | 1-12 | |

TECHNICAL FIELDS SEARCHED (IPC)

G16B

The present search report has been drawn up for all claims

| Place of search | Date of completion of the search | Examiner |
|---|---|---|
| Munich | 25 February 2025 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 9902

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2025

| Patent document cited in search report | Publication date | Patent family member(s) | | Publication date |
|---|---|---|---|---|
| US 2022154284 A1 | 19-05-2022 | EP | 4247980 A2 | 27-09-2023 |
| | | US | 2022154284 A1 | 19-05-2022 |
| | | WO | 2022109607 A2 | 27-05-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63594835 A
- US 789288 A
- US 20200258601 A

### Non-patent literature cited in the description

- **RIZVI H** ; **SANCHEZ-VEGA F** ; **LA K et al.** Molecular Determinants of Response to Anti-Programmed Cell Death (PD)-1 and Anti-Programmed Death-Ligand 1 (PD-L1) Blockade in Patients With Non-Small-Cell Lung Cancer Profiled With Targeted Next-Generation Sequencing. *J Clin Oncol.*, 2018, vol. 36 (7), 633-641
- **MCGRAIL DJ** ; **PILIÉ PG** ; **RASHID NU et al.** High tumor mutation burden fails to predict immune checkpoint blockade response across all cancer types. *Ann Oncol.*, 2021, vol. 32 (5), 661-672
- **CAMILA BRAGANÇA XAVIER et al.** Association between tumor mutational burden (TMB) and mutational profile and its effect on overall survival: A post hoc analysis of patients with TMB-high and TMB-low metastatic cancer treated with immune checkpoint inhibitors (ICI). *JCO*, 2022, vol. 40, 2632-2632
- **ROONEY, M. S.** ; **SHUKLA, S. A.** ; **WU, C. J.** ; **GETZ, G.** ; **HACOHEN, N.** Molecular and Genetic Properties of Tumors Associated with Local Immune Cytolytic Activity. *Cell*, 2015, vol. 160, 48-61
- **AYERS, M. et al.** IFN-γ-related mRNA profile predicts clinical response to PD-1 blockade. *J Clin Invest*, 2017, vol. 127, 2930-2940
- **AUSLANDER, N. et al.** Robust prediction of response to immune checkpoint blockade therapy in metastatic melanoma. *Nat Med*, 2018, vol. 24, 1545-1549
- **HUANG, A. C. et al.** A single dose of neoadjuvant PD-1 blockade predicts clinical outcomes in resectable melanoma. *Nat Med*, 2019, vol. 25, 454-461
- **ALEXANDROV.** *Nature*, 2013
- **ANDERSSON.** *Nat Comms*, 2021
- **AUSLANDER.** *Nat Med*, 2018
- **AYERS.** *JCI*, 2017
- **BEAUBIER.** *Nat Biotech*, 2019
- **BITON.** *Clin Cancer Res*, 2018
- *Cancer Disc*, 2018
- **CABRITA.** *Nature*, 2020
- **CHOWELL.** *Science*, 2018
- **COPPOLA.** *Am J Pathol*, 2021
- **CRISTESCU.** *Clin Cancer Res*, 2022
- **HUANG.** *Nat Med*, 2019
- **JERBY-ARNON.** *Cell*, 2018
- **LAU.** *Nat Comms*, 2022
- **LITCHFIELD.** *Cell*, 2021
- **MANCZINGER.** *Nat Cancer*, 2021
- **ROH.** *Sci Trans Med*, 2017
- **ROONEY.** *Cell*, 2015
- **SADE-FELDMAN.** *Cell*, 2018
- **TURAN.** *BJC*, 2020
- **THOMPSON.** *J Immunother Cancer*, 2020
- **TOMLINS.** *Commun Med*, 2023
- **ZHANG.** *Nt Med*, 2022
- **HERBST RS** ; **BAAS P** ; **KIM D-W et al.** Pembrolizumab versus docetaxel for previously treated, PD-L1-positive, advanced non-small-cell lung cancer (KEYNOTE-010): a randomised controlled trial. *Lancet*, 2016, vol. 387, 1540-50
- **ZHAO B** ; **ZHAO H** ; **ZHAO J.** Efficacy of PD-1/PD-L1 blockade monotherapy in clinical trials. *Ther Adv Med Oncol.*, 2020, vol. 12, 1758835920937612
- **KIM MS** ; **PRASAD V.** Pembrolizumab for all.. *J Cancer Res Clin Oncol.*, 2023, vol. 149, 1357-60
- **CHEN DS** ; **MELLMAN I.** Oncology meets immunology: the cancer-immunity cycle. *Immunity*, 2013, vol. 39, 1-10
- **LITCHFIELD K** ; **READING JL** ; **PUTTICK C et al.** Meta-analysis of tumor- and T cell-intrinsic mechanisms of sensitization to checkpoint inhibition. *Cell*, 2021, vol. 184, 596-614.e14
- **LIN HM** ; **WU Y** ; **YIN Y et al.** Real-world ALK testing trends in patients with advanced non-small-cell lung cancer in the United States. *Clin Lung Cancer*, 2023, vol. 24, e39-49
- Anti-PD1 response prediction dream challenge. *DREAM Challenges*, 12 September 2024, https://dreamchallenges.org/anti-pd1-response-prediction-dream-challenge
- **BEAUBIER N** ; **BONTRAGER M** ; **HUETHER R et al.** Integrated genomic profiling expands clinical options for patients with cancer. *Nat Biotechnol.*, 2019, vol. 37, 1351-60

- **BEAUBIER N** ; **TELL R** ; **LAU D et al.** Clinical validation of the tempus xT next-generation targeted oncology sequencing assay. *Oncotarget*, 2019, vol. 10, 2384-96
- **WENRIC S** ; **DAVISON JM** ; **GUITTAR J et al.** Real-world data validation of the PurIST pancreatic ductal adenocarcinoma gene expression classifier and its prognostic implications. *medRxiv. 2023;2023.02.23.23286356*
- **MICHUDA J** ; **BRESCHI A** ; **KAPILIVSKY J et al.** Validation of a Transcriptome-Based Assay for Classifying Cancers of Unknown Primary Origin. *Mol Diagn Ther.*, 2023, vol. 27, 499-511
- **LEIBOWITZ BD** ; **DOUGHERTY BV** ; **BELL JSK et al.** Validation of genomic and transcriptomic models of homologous recombination deficiency in a real-world pan-cancer cohort. *BMC Cancer*, 2022, vol. 22, 587
- A multi-modal, pan-cancer atlas of tumor-immune states across primary and metastatic disease using a large, real-world database. **JAIN P** ; **STEIN MM** ; **FIELDS P et al.** Regular and Young Investigator Award Abstracts. BMJ Publishing Group Ltd, 2023
- **ERBE R** ; **STEIN MM** ; **RAND TA et al.** Abstract 2281: A tumor-intrinsic signature involving immunosuppression via MIF-CD74 signaling is associated with overall survival in ICT-treated lung adenocarcinoma. *Cancer Res.*, 2024, vol. 84, 2281-2281
- **BARECHE Y** ; **KELLY D** ; **ABBAS-AGHABABAZADEH F et al.** Leveraging big data of immune checkpoint blockade response identifies novel potential targets. *Ann Oncol.*, 2022, vol. 33, 1304-17
- **TSAI W-Y** ; **JEWELL NP** ; **WANG M-C**. A note on the product-limit estimator under right censoring and left truncation. *Biometrika*, 1987, vol. 74, 883
- **UPADHAYA S** ; **HUBBARD-LUCEY VM** ; **YU JX**. Immuno-oncology drug development forges on despite COVID-19. *Nat Rev Drug Discov.*, 2020, vol. 19, 751-2
- KEYTRUDA (pembrolizumab) [package insert. U.S. Food and Drug Administration
- **AGUILAR EJ** ; **RICCIUTI B** ; **GAINOR JF et al.** Outcomes to first-line pembrolizumab in patients with non-small-cell lung cancer and very high PD-L1 expression. *Ann Oncol.*, 2019, vol. 30, 1653-9
- **HOSSEIN BORGHAEI**. ECOG-ACRIN Cancer Research Group. Testing the Timing of Pembrolizumab Alone or With Chemotherapy as First Line Treatment and Maintenance in Non-small Cell Lung Cancer. *ClinicalTrials.gov.*, 24 September 2024, https://clinicaltrials.gov/study/NCT03793179
- **SAHIN IH** ; **CIOMBOR KK** ; **DIAZ LA et al.** Immunotherapy for microsatellite stable colorectal cancers: Challenges and novel therapeutic avenues. *Am Soc Clin Oncol Educ Book.*, 2022, vol. 42, 1-12
- **MARABELLE A** ; **LE DT** ; **ASCIERTO PA et al.** Efficacy of pembrolizumab in patients with noncolorectal high microsatellite instability/mismatch repair-deficient cancer: Results from the phase II KEYNOTE-158 study. *J Clin Oncol.*, 2020, vol. 38, 1-10
- **REARDON DA** ; **BRANDES AA** ; **OMURO A et al.** Effect of nivolumab vs bevacizumab in patients with recurrent glioblastoma: The CheckMate 143 phase 3 randomized clinical trial. *JAMA Oncol.*, 2020, vol. 6, 1003-10
- **MARABELLE A** ; **FAKIH M** ; **LOPEZ J et al.** Association of tumour mutational burden with outcomes in patients with advanced solid tumours treated with pembrolizumab: prospective biomarker analysis of the multicohort, open-label, phase 2 KEYNOTE-158 study. *Lancet Oncol.*, 2020, vol. 21, 1353-65
- **FRIDMAN WH** ; **ZITVOGEL L** ; **SAUTÈS-FRIDMAN C et al.** The immune contexture in cancer prognosis and treatment. *Nat Rev Clin Oncol.*, 2017, vol. 14, 717-34